# EUROPEAN PATENT APPLICATION

(11) **EP 1 479 681 A1**
(43) Date of publication of application: **24.11.2004**
(21) Application number: 03734891.9
(22) Date of filing: 30.01.2003
(51) Int. Cl.: C07D 471/04, A61K 31/437, A61K 31/4375, A61K 31/496, A61K 31/5377, A61K 31/541, A61P 31/10, A61P 31/00

(54) **IMIDAZO(1,2-a)PYRIDINE DERIVATIVE**

(30) Priority: 31.01.2002 JP 2002022767
(71) Applicant: DAIICHI PHARMACEUTICAL CO., LTD., Chuo-ku, Tokyo 103-8234 (JP)
(72) Inventor: Takamura, Makoto, Tokyo 134-8630 (JP); Takahashi, Hisashi, Tokyo 134-8630 (JP); Kawakami, Katsuhiro, Tokyo 134-8630 (JP); Takeshita, Hiroshi, Tokyo 134-8630 (JP); Kimura, Youichi, Tokyo 134-8630 (JP); Watanabe, Jun, Tokyo 134-8630 (JP); Sugimoto, Yuichi, Tokyo 134-8630 (JP); Kitamura, Akihiro, Tokyo 134-8630 (JP); Nakajima, Ryohei, Tokyo 134-8630 (JP); Kanai, Kazuo, Tokyo 134-8630 (JP); Fujisawa, Tetsunori, Takaoka-shi, Toyama 933-8511 (JP)
(74) Representative: Reitstötter - Kinzebach & Partner (GbR)
(86) International application number: PCT/JP2003/000912
(87) International publication number: WO 2003/064422

(57) **Abstract**

A compound reprsented by the following formula (I), its salts or nsolvates thereof capable of specifically or selectively expressig an antifungal activity in a broad spectrum based on the novel mechanism thereof of 1,6-β-glucan synthesis inhibition, and an antifungal agent containing any of them.

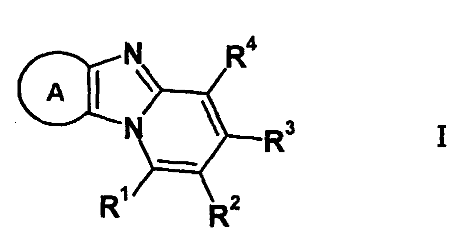

## Description

### TECHNICAL FIELD

The present invention relates to an imidazo[1,2-a]pyridine derivative, its salts or solvates thereof that exhibit an antifungal activity against pathogenic fungi, and to an antifungal agent containing any of them.

### BACKCRUSHED ART

Fungi are known to infect humans, animals and plants to cause various diseases. For example, they cause superficial mycosis in various human tissues such as epidermic corneal layers of skins, keratinous tissues such as nails and hairs, and mucosal epitherlia in oral cavities, and cause subcutaneous mycosis even in deep skin tissues existing in the depth from the body surfaces, and cause deep-seated mycosis even in deep tissues in esophagi, internal organs and brains. Typical pathogenic fungi known to infect humans to cause deep-seatedmycosis are those of the genera *Candida, Cryptococcus* and *Aspergillus;* and typical pathogenic fungi to cause superficial mycosis will be those of the genus *Candida* that infect skins, oral cavities and vaginas, and those of the genus *Trychophyton* that infect the skins of hands and feet. Apart from these, there will be many other various fungi to infect animals and plants.

With the rapid progress in studies and developments relating to antibiotics and medicines for chemical therapy and with wide popularization thereof since 1950s, a lot of medicines for curing bacterial infectious diseases have been developed. Similarly, much effort has been paid to development of antifungal medicines. However, as compared with the development of antibacterial agents for chemical therapy, there are not so many compounds that are at present put in clinical use for antifungal therapy. On the other hand, compromised hosts with immunity depression are on the increase owing to frequent use of antibacterial medicines (antibiotics, chemical therapy agents) in actual clinical sites or caused by malignant tumors, leukemia, organ or bone marrow transplantation, and AIDS (acquired immunodeficiency syndrome), and, as a result, cases with deep-seated mycosis are increasing in these days, and are now therefore problematic in the art.

Typical antifungal agents that are now used in the actual clinical sites are polyenemacrolides, fluoropyrimidines and azoles. They are essentially used for external applications for therapy of superficial mycosis, including, for example, various azole-type medicines, and polyenemacrolide-type nystatin, griseofulvin, terbinafine hydrochloride, butenafine hydrochloride and amorolfine chloride. On the other hand, for therapy of deep-seated mycosis that is significantly on the increase these days, azole-type fluconazole and itraconazole are much used because of their safety as compared with any other medicines, but these are problematic in that their antifungal spectrum is narrow. Amphotericin B, a type of polyenemacrolide medicines, has a broad antifungal spectrum and is highly effective, but it is problematic in point of its toxicity (side effect). Flucytosine, a type of fluoropyrimidine medicines is not toxic, but its use frequently results in emergence of resistance. Accordingly, only a few of medicines that are at present used for therapy of deep-seated mycosis could be on a satisfactory level formedical therapy in point of the antifungal spectrum, the effectiveness and the safety thereof. In addition, fluconazole that is at present the most popular medicine for deep-seated mycosis is poorly effective against some pathogenic fungi such as *Candida glabrata*, *Candida tropicalis, Candida krusei*, and there are emerging some fungi resistant thereto. In the clinical sites, therefore, novel antifungal medicines that overcome these problems are much desired.

On the other hand, a trial of scientifically evaluating the usefulness of substances has been established for development of recent antifungal therapies and novel antifungal agents. The method is with the progress of the studies of mechanisms of antifungal medicines, and it is desired to developmore effective and safer medicines. From the poi nt of the overcomi ng the problem with drug-resistant fungi, it is much desired to develop antifungal agents having a novel mechanisms.

Further, from the point of safety, fungi are eukaryotic cells like human cells differing from bacterial (prokaryotic cells) , and therefore, it is necessary to develop compounds that attack and injure specifically (selectively) fungal cells alone.

Under these circumstances, a medicine capable of inhibiting the synthesis of essential cell wall constitutive components of fungi, namely cell wall polysaccharide synthesis system, or that is, an antifungal agent which targets molecules of cell wall polysaccharide synthetase specifically existing in fungi is expected from the viewpoint of the novelty of the mechanism thereof and from the selective toxicity thereof. For polysaccharides that constitute the cell wall of fungi, known are β-glucan, chitin, chitosan and mannan, of which β-glucan is an essential constitutive component of the cell wall of fungi, and this is grouped into 1,3-β-glucan and 1,6-β-glucan.

For 1,3-β-glucan synthetase inhibitors, heretofore reported are papulacandins [Journal of Antibiotics, Vol. 36, p. 1539 (1983)]; echinocandins [Journal of Medicinal Chemistry, Vol. 38, p. 3271 (1995)]; pneumocandins [Journal ofAntibiotics, Vol. 45, p. 1875 (1992)]; aculeacins [Journal of Biochemistry, Vol. 105, p. 606 (1989)].

However, any 1,6-β-glucan synthetase inhibitor has heretofore not been reported at all, and, in addition, an antifungal agent having a functional mechanism of inhibiting 1,6-β-glucan synthesis has not been known at all.

An obj ect of the present invention is to provide a compound having a wide antifungal spectrum based on its novel mechanism of 1, 6-β-glucan synthesis inhibition and capable of specifically and selectively expressing such a broad antifungal effect, and to provide an antifungal agent containing such a compound, its salt or solvate thereof.

### DISCLOSURE OF THE INVENTION

The present inventors have searched for compounds for the purpose of obtaining those having an antifungal activity of inhibiting 1,6-β-glucan synthetase, and have found out a compound having an effect of inhibiting 1,6-β-glucan synthesis through biopolymer synthesis inhibition experiments based on a [¹⁴C]-glucose intake index. In addition, the present inventors have further investigated as to whether any other compounds structurally similar to the compound have also an antifungal activi ty to pathogenic fungi. As a resul t, the present inventors have found that imidazo[1,2-a]pyridine derivatives of formula (I), its salts and solvates thereof have a broad and potent antifungal effect with a functional mechanism of 1,6-β-glucan synthesis inhibition, and have completed the present invention.

That is, the invention provides the followings:

A compound represented by the following formula (I) , its salt or solvates thereof: 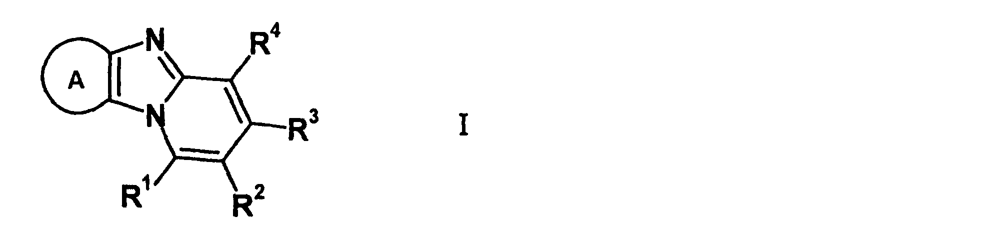 [wherein the moiety A means a benzene ring or a heteroaryl ring (the heteroaryl ring is a 5-membered or 6-membered ring and contains from 1 to 3 hetero atoms of one or more types selected from the group consisting of a nitrogen atom, an oxygen atom and a sulfur atom), and the benzene ring and heteroaryl ring each may have one or more substi tuents of one or more types sel ected from the group consisting of an amino group, a hydroxyl group, a mercapto group, a nitro group, a cyano group, a formyl group, a carboxyl group, a sulfo group, a halogen atom, a heteroaryl group (the heteroaryl group is a 5-membered or 6-membered ring containing from 1 to 3 hetero atoms of one or more types selected from the group consisting of a nitrogen atom, an oxygen atom and a sulfur atom), a group of the following formula: 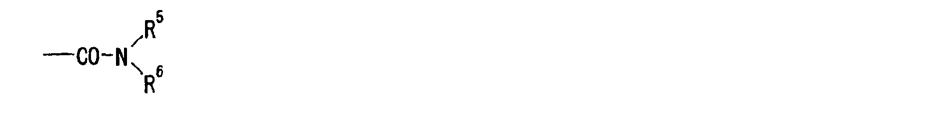 (wherein R⁵ and R⁶ each independently represents a hydrogen atom, an alkyl group having from 1 to 6 carbon atoms or an aryl group having from 6 to 10 carbon atoms),
an alkyl group having from 1 to 6 carbon atoms, an alkenyl group having from 2 to 6 carbon atoms, an alkynyl group having from 2 to 6 carbon atoms, an alkoxy group having from 1 to 6 carbon atoms, a hydroxyalkyl group having from 1 to 6 carbon atoms, an alkylamino group having from 1 to 6 carbon atoms, an alkylthio group having from 1 to 6 carbon atoms, an acyl group having from 2 to 5 carbon atoms, an alkoxycarbonyl group having from 2 to 5 carbon atoms, a cycloalkyl group having from 3 to 6 carbon atoms, an aryl group having from 6 to 10 carbon atoms and an aralkyl group having from 7 to 16 carbon atoms, and the substituents containing a carbon chain of them may bond to each other to form a cyclic structure and fuse to the benzene ring or the heteroaryl ring;
R¹ represents a hydrogen atom, a halogen atom, an amino group, a hydroxyl group, a mercapto group, a ni tro group, a cyano group, a formyl group, a carboxyl group, a group of the following formula: 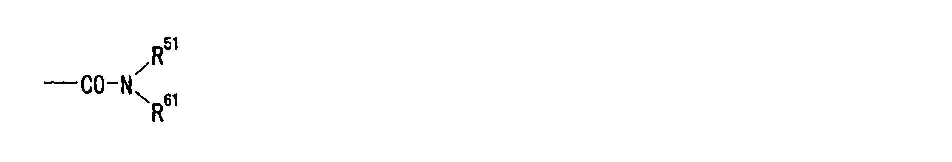 (wherein R⁵¹ and R⁶¹ each independently represents a hydrogen atom, an alkyl group having from 1 to 6 carbon atoms or an aryl group having from 6 to 10 carbon atoms),
an alkyl group having from 1 to 10 carbon atoms, an alkenyl group having from 2 to 10 carbon atoms, an alkynyl group having from 2 to 10 carbon atoms, an alkylamino group having from 1 to 10 carbon atoms, an alkoxy group having from 1 to 10 carbon atoms, an alkylthio group having from 1 to 10 carbon atoms, an acyl group having from 2 to 6 carbon atoms, an alkoxycarbonyl group having from 2 to 7 carbon atoms, a cycloalkyl group having from 3 to 10 carbon atoms, a cycloalkylamino group having from 3 to 10 carbon atoms, a cycloalkyloxy group having from 3 to 10 carbon atoms, a cycloalkylthio group having from 3 to 10 carbon atoms, a cycloalkenyl group having from 4 to 10 carbon atoms, a cycloalkenylamino group having from 4 to 10 carbon atoms, a cycloalkenyloxy group having from 4 to 10 carbon atoms, a cycloalkenylthio group having from 4 to 10 carbon atoms, an aryl group having from 6 to 10 carbon atoms, an arylamino group having from 6 to 10 carbon atoms, an aryloxy group having from 6 to 10 carbon atoms, an arylthio group having from 6 to 10 carbon atoms, a monocyclic-, bicyclic- or spirocyclic-heterocyclic group having from 3 to 10 carbon atoms (containing from 1 to 4 hetero atoms of one or more types selected from the group consisting of a nitrogen atom, an oxygen atom and a sulfur atom), a heteroarylamino group having from 5 to 10 carbon atoms, a heteroaryloxy group having from 5 to 10 carbon atoms, a heteroarylthio group having from 5 to 10 carbon atoms, in which the amino group, the hydroxyl group and the mercapto group may be protected with a protective group;
when R¹ is an amino group, an alkyl group, an alkenyl group, an alkynyl group, an alkylamino group, an alkoxy group, an alkylthio group, an acyl group, an alkoxycarbonyl group, a cycloalkyl group, a cycloalkylamino group, a cycloalkyloxy group, a cycloalkylthio group, a cycloalkenyl group, a cycloalkenylamino group, a cycloalkenyloxy group, a cycloalkenylthio group, an aryl group, an arylamino group, an aryloxy group, an arylthio group, a heteroarylamino group, a heteroaryloxy group or a heteroarylthio group, they may have one or more substituents of one or more types selected from the group consisting of an amino group, an aminoalkyl group having from 1 to 6 carbon atoms (the amino group and the amino group moiety of the aminoalkyl group having from 1 to 6 carbon atoms may be protected with a protective group, and may have one or two alkyl groups each having from 1 to 6 carbon atoms, and when they have two alkyl groups, the groups may be the same or different), a hydroxyl group, a mercapto group, a halogen atom, an alkoxy group having from 1 to 6 carbon atoms, an alkylthio group having from 1 to 6 carbon atoms, an acyl group having from 2 to 7 carbon atoms, an alkoxycarbonyl group having from 2 to 7 carbon atoms, an aryl group having from 6 to 10 carbon atoms, a monocyclic- or bicyclic-heterocyclic group having from 3 to 10 carbon atoms (containing from 1 to 4 hetero atoms of one or more types selected from the group consisting of a nitrogen atom, an oxygen atom and a sulfur atom), an alkylcarbonylamino group having from 2 to 7 carbon atoms and a cycloalkyl group having from 3 to 10 carbon atoms;
when R¹ is a heterocyclic group, it may have one or more substituents of one or more types selected from the group consisting of a halogen atom, an amino group, a hydroxyl group, an oxo group, a group of the following formula: 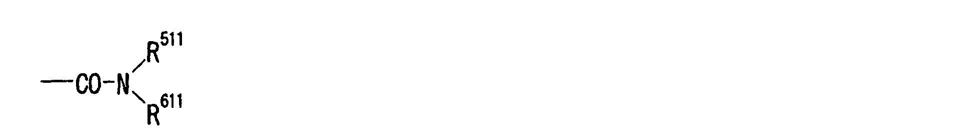 (wherein R⁵¹¹ and R⁶¹¹ each independently represents a hydrogen atom, an alkyl group having from 1 to 6 carbon atoms or an aryl group having from 6 to 10 carbon atoms),
an alkyl group having from 1 to 6 carbon atoms, an alkylamino group having from 1 to 6 carbon atoms, a cycloalkylamino group having from 3 to 6 carbon atoms, an aryl group having from 6 to 10 carbon atoms, a monocyclic- orbicyclic-heterocyclic group having from 3 to 10 carbon atoms (containing from 1 to 4 hetero atoms of one or more types selected from the group consisting of a nitrogen atom, an oxygen atom and a sulfur atom), an alkoxy group having from 1 to 6 carbon atoms, an alkylthio group having from 1 to 6 carbon atoms, a halogenoalkyl group having from 1 to 6 carbon atoms and an aminoalkyl group having from 1 to 6 carbon atoms; and the alkyl group and the alkyl moiety of the alkylamino group, the cycloalkylamino group, the alkoxy group, the alkylthio group, the halogenoalkyl group and the aminoalkyl group may have one or more substituents of one or more types selected from the group consisting of a halogen atom, a hydroxyl group, a carboxyl group, an alkyl group having from 1 to 6 carbon atoms, an alkoxy group having from 1 to 6 carbon atoms, an alkoxycarbonyl group having from 2 to 7 carbon atoms, an alkoxycarbonylamino group having from 2 to 7 carbon atoms, an aryl group having from 6 to 10 carbon atoms, and a monocyclic- or bicyclic-heterocyclic group having from 3 to 10 carbon atoms (containing from 1 to 4 hetero atoms of one or more types selected from the group consisting of a nitrogen atom, an oxygen atom and a sulfur atom); the amino group and the amino group moiety of the aminoalkyl group and the alkylamino group may be protected withaprotective group, andmayhave, as the substituent thereof, one or two alkyl groups each having from 1 to 6 carbon atoms (optionally having one or more substituents of one or more types selected from the group consisting of a hydroxyl group, a halogen atom, an alkoxy group having from 1 to 6 carbon atoms and an alkyl thio group) , and an amino acid, a dipeptide or a polypeptide comprising from 3 to 5 amino acids may be bonded thereto; R² represents a hydrogen atom, a halogen atom, an amino group, a hydroxyl group, a nitro group, a cyano group, a carboxyl group, a group of the following formula: 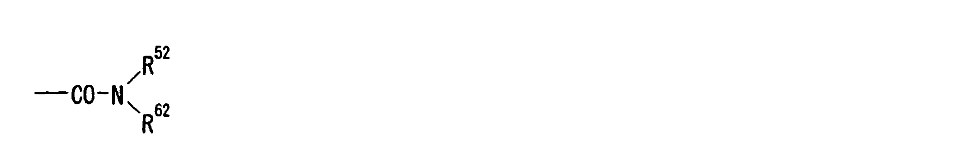 (wherein R⁵² and R⁶² each independently represents a hydrogen atom, an alkyl group having from 1 to 6 carbon atoms or an aryl group having from 6 to 10 carbon atoms),
an alkyl group having from 1 to 20 carbon atoms, an alkenyl group having from 2 to 20 carbon atoms, an alkynyl group having from 2 to 20 carbon atoms, an alkylamino group having from 1 to 20 carbon atoms, an alkoxy group having from 1 to 20 carbon atoms, an acyl group having from 2 to 18 carbon atoms, an alkoxycarbonyl group having from 2 to 18 carbon atoms, a cycloalkyl group having from 3 to 10 carbon atoms, a cycloalkenyl group having from 5 to 10 carbon atoms, a cycloalkylamino group having from 3 to 10 carbon atoms, a cycloalkylalkyl group having from 4 to 16 carbon atoms, a cycloalkyloxy group having from 3 to 10 carbon atoms, an aryl group having from 6 to 10 carbon atoms, an arylamino group having from 6 to 10 carbon atoms, an aralkyl group having from 7 to 16 carbon atoms, an aryloxy group having from 6 to 10 carbon atoms, a monocyclic- or bicyclic-heterocyclic group having from 5 to 10 carbon atoms (containing from 1 to 4 hetero atoms of one or more types selected from the group consisting of a nitrogen atom, an axygen and a sulfur atom), a heteroarylamino group having from 5 to 10 carbon atoms, a heteroarylalkyl group having from 6 to 16 carbon atoms or a heteroaryloxy group having from 5 to 10 carbon atoms, and the amino group and the hydroxyl group may be protected with a protective group;
when R² is an alkyl group, an alkenyl group, an alkynyl group, an alkylamino group, an alkoxy group, an acyl group, an alkoxycarbonyl group, a cycloalkyl group, a cycloalkylamino group or a cycloalkyloxy group, these groups may have one or more substituents of one or more types selected from the group consisting of a halogen atom, an amino group, an imino group, a nitro group, a hydroxyl group, a mercapto group, a carboxyl group, a cyano group, a sulfo group, a dialkylphosphoryl group, a group of the following formula: 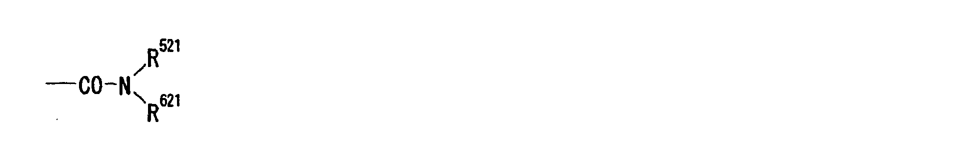 (wherein R⁵²¹ and R⁶²¹ each independently represents a hydrogen atom, an alkyl group having from 1 to 6 carbon atoms or an aryl group having from 6 to 10 carbon atoms),
an alkoxy group having from 1 to 10 carbon atoms, an alkylthio group having from 1 to 10 carbon atoms, an acyl group having from 2 to 8 carbon atoms, an alkoxycarbonyl group having from 2 to 8 carbon atoms, a cycloalkyl group having from 3 to 10 carbon atoms, an aryl group having from 6 to 10 carbon atoms and an arylthio group having from 6 to 10 carbon atoms; the amino group may have one or two substituents selected from the group consisting of a formyl group, an alkyl group having from 1 to 10 carbon atoms, an aminoalkyl group having from 1 to 8 carbon atoms, a hydroxyalkyl group having from 1 to 8 carbon atoms, a mercaptoalkyl group having from 1 to 8 carbon atoms, an acyl group having from 2 to 8 carbon atoms, an alkoxycarbonyl group having from 2 to 8 carbon atoms, a cycloalkyl group having from 3 to 10 carbon atoms, an aryl group having from 6 to 10 carbon atoms, an aralkyl group having from 7 to 16 carbon atoms, a heteroaryl group (the heteroaryl group is a 5-membered or 6-membered ring and contains from 1 to 4 hetero atoms of one or more types selected from the group consisting of a nitrogen atom, an oxygen atom and a sulfur atom), an alkylsulfonyl group having from 1 to 10 carbon atoms and an arylsulfonyl group having from 6 to 10 carbon atoms; when the ami no group has two substituents, theymaybond to each other to form a cyclic structure; the hydroxyl group and the mercapto group may have a substituent selected from the group consisting of an alkyl group having from 1 to 10 carbon atoms, an aminoalkyl group having from 1 to 8 carbon atoms, a hydroxyalkyl group having from 1 to 8 carbon atoms, a mercaptoalkyl group having from 1 to 8 carbon atoms, an acyl group having from 2 to 8 carbon atoms, a cycloalkyl group having from 3 to 10 carbon atoms, an aryl group having from 6 to 10 carbon atoms, an aralkyl group having from 7 to 16 carbon atoms, and a heteroaryl group (the heteroaryl group is a 5-membered or 6-membered ring and contains from 1 to 4 hetero atoms of one or more types selected from the group consisting of a nitrogen atom, an oxygen atom and a sulfur atom) ; the cycloalkyl group may have one or more substituents of one or more types selected from the group consisting of a halogen atom, an amino group, an imino group, a nitro group, a hydroxyl group, a mercapto group, a carboxyl group, a cyano group, a sulfo group, a group of the following formula: 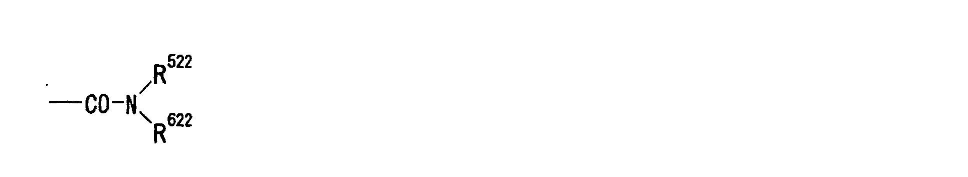 (wherein R⁵²² and R⁶²² each independently represents a hydrogen atom, an alkyl group having from 1 to 6 carbon atoms or an aryl group having from 6 to 10 carbon atoms),
an alkoxy group having from 1 to 10 carbon atoms, an alkylthio group having from 1 to 10 carbon atoms, an acyl group having from 2 to 8 carbon atoms and an alkoxycarbonyl group having from 1 to 8 carbon atoms; the amino group may have one or two substituents selected from the group consisting of a formyl group, an alkyl group having from 1 to 10 carbon atoms, an aminoalkyl group having from 1 to 8 carbon atoms, a hydroxyalkyl group having from 1 to 8 carbon atoms, a mercaptoalkyl group having from 1 to 8 carbon atoms, an acyl group having from 2 to 8 carbon atoms, an alkoxycarbonyl group having from 2 to 8 carbon atoms, a cycloalkyl group having from 3 to 10 carbon atoms, an aryl group having from 6 to 10 carbon atoms, an aralkyl group having from 7 to 16 carbon atoms, a heteroaryl group (the heteroaryl group is a 5-membered or 6-membered ring and contains from 1 to 4 hetero atoms of one or more types selected from the group consisting of a nitrogen atom, an oxygen atom and a sulfur atom), an alkylsulfonyl group having from 1 to 10 carbon atoms and an arylsulfonyl group having from 6 to 10 carbon atoms; when the amino group has two substituents, they may bond to each other to form a cyclic structure;
when R² is an aryl group, an arylamino group, an aralkyl group, an aryloxy group, a heteroaryl group, a heteroarylamino group, a heteroarylalkyl group or a heteroaryloxy group, they may have one or more substituents of one or more types selected from the group consisting of a halogen atom, an amino group, an imino group, a nitro group, a hydroxyl group, a mercapto group, a carboxyl group, a cyano group, a sulfo group, a group of the following formula: 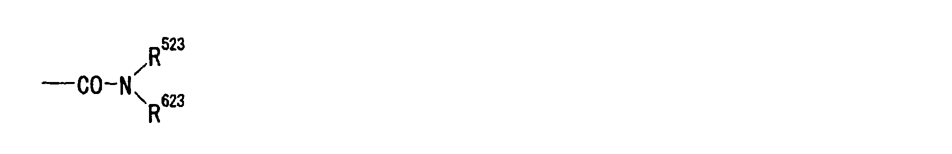 (wherein R⁵²³ and R⁶²³ each independently represents a hydrogen atom, an alkyl group having from 1 to 6 carbon atoms or an aryl group having from 6 to 10 carbon atoms),
an alkoxy group having from 1 to 10 carbon atoms, an alkylthio group having from 1 to 10 carbon atoms, an acyl group having from 2 to 8 carbon atoms, an alkoxycarbonyl group having from 2 to 8 carbon atoms, an aralkyloxy group having from 7 to 16 carbon atoms, an aralkyloxycarbonyl group having from 8 to 17 carbon atoms, an aryl group, and a monocyclic- or bicyclic-heterocyclic group having from 5 to 10 carbon atoms (containing from 1 to 4 hetero atoms of one or more types selected from the group consisting of a nitrogen atom, an oxygen atom and a sulfuratom) ; the amino group may have one or two substituents selected from the group consisting of a formyl group, an alkyl group having from 1 to 10 carbon atoms, an aminoalkyl group having from 1 to 8 carbon atoms, a hydroxyalkyl group having from 1 to 8 carbon atoms, a mercaptoalkyl group having from 1 to 8 carbon atoms, an acyl group having from 2 to 8 carbon atoms, an alkoxycarbonyl group having from 2 to 8 carbon atoms, a cycloalkyl group having from 3 to 10 carbon atoms, an aryl group having from 6 to 10 carbon atoms, an aralkyl group having from 7 to 16 carbon atoms, a heteroaryl group (the heteroaryl group is a 5-membered or 6-membered ring and contains from 1 to 4 hetero atoms of one or more types selected from the group consisting of a nitrogen atom, an oxygen atom and a sulfur atom), an alkylsulfonyl group having from 1 to 10 carbon atoms and an arylsulfonyl group having from 6 to 10 carbon atoms; and when the amino group has two substi tuents, they may bond to each other to form a cyclic structure;
when R² is a heterocyclic group, it may have one or two substituents selected from the group consisting of a halogen atom, an amino group, a hydroxyl group, mercapto group, a carboxyl group, a group of the following formula: 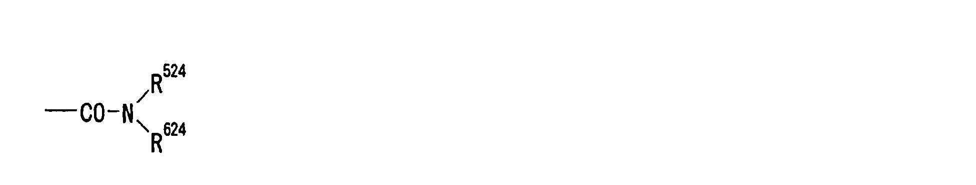 (wherein R⁵²⁴ and R⁶²⁴ each independently represents a hydrogen atom, an alkyl group having from 1 to 6 carbon atoms or an aryl group having from 6 to 10 carbon atoms),
an alkyl group having from 1 to 10 carbon atoms, an alkenyl group having from 2 to 10 carbon atoms, an alkynyl group having from 2 to 10 carbon atoms, an alkoxy group having from 1 to 10 carbon atoms, an alkylthio group having from 1 to 10 carbon atoms, a halogenoalkyl group having from 1 to 10 carbon atoms, an acyl group having from 2 to 10 carbon atoms, an alkoxycarbonyl group having from 2 to 10 carbon atoms and an aryl group having from 6 to 10 carbon atoms; the amino group may have one or two substituents selected from the group consisting of a formyl group, an alkyl group having from 1 to 10 carbon atoms, an aminoalkyl group having from 1 to 8 carbon atoms, a hydroxyalkyl group having from 1 to 8 carbon atoms, a mercaptoalkyl group having from 1 to 8 carbon atoms, an acyl group having from 2 to 8 carbon atoms, an alkoxycarbonyl group having from 2 to 8 carbon atoms, a cycloalkyl group having from 3 to 10 carbon atoms, an aryl group having from 6 to 10 carbon atoms, an aralkyl group having from 7 to 16 carbon atoms, a monocyclic- or bicyclic-heterocyclic group having from 5 to 10 carbon atoms (containing from 1 to 4 hetero atoms of one or more types selected from the group consisting of a nitrogen atom, an oxygen atom and a sulfur atom), a heteroaryl group (the heteroaryl group is a 5-membered or 6-membered ring and contains from 1 to 4 hetero atoms of one or more types selected from the group consisting of a nitrogen atom, an oxygen atom and a sulfur atom), an alkylsulfonyl group having from 1 to 10 carbon atoms and an arylsulfonyl group having from 6 to 10 carbon atoms; and when the amino group has two substituents, they may bond to each other to form a cyclic structure;
R¹ and R² may cooperate to form a 5-membered or 6-membered heterocyclic group (containing from 1 to 3 hetero atoms of one or more types selected from the group consisting of a nitrogen atom, an oxygen atom and a sulfur atom); R³ represents a hydrogen atom, a halogen atom, an amino group, a hydroxyl group, a mercapto group, a nitro group, a cyano group, a formyl group, a carboxyl group, a group of the following formula: 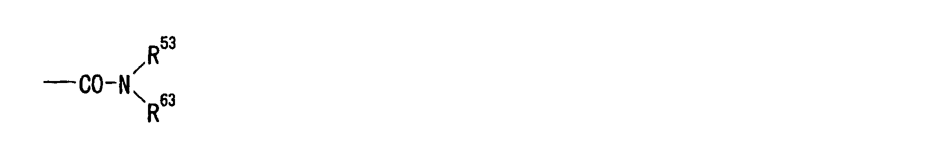 (wherein R⁵³ and R⁶³ each independently represents a hydrogen atom, an alkyl group having from 1 to 6 carbon atoms or an aryl group having from 6 to 10 carbon atoms),
an alkyl group having from 1 to 6 carbon atoms, an alkenyl group having from 2 to 6 carbon atoms, an alkynyl group having from 2 to 6 carbon atoms, an alkoxy group having from 1 to 6 carbon atoms, an alkylthio group having from 1 to 6 carbon atoms, an acyl group having from 2 to 5 carbon atoms, an alkoxycarbonyl group having from 2 to 5 carbon atoms, a cycloalkyl group having from 3 to 7 carbon atoms, a cycloalkenyl group having from 4 to 7 carbon atoms, an aryl group having from 6 to 10 carbon atoms, an aralkyl group having from 7 to 16 carbon atoms and a heteroaryl group (the heteroaryl group is a 5-membered or 6-membered ring and contains from 1 to 4 hetero atoms of one or more types selected from the group consisting of a nitrogen atom, an oxygen atom and a sulfur atom); and the amino group, the hydroxyl group and the mercapto group may be protected with a protective group; when R³ is an alkyl group, an alkenyl group, an alkynyl group, an alkoxy group, an alkylthio group, an acyl group, an alkoxycarbonyl group, a cycloalkyl group, a cycloalkenyl group, an aryl group, an aralkyl group or a heteroaryl group, they may have one or more substituents of one or more types selected from the group consisting of an amino group, a hydroxyl group, a mercapto group, a halogen atom, an alkoxy group having from 1 to 6 carbon atoms, an alkylthio group having from 1 to 6 carbon atoms, an acyl group having from 2 to 5 carbon atoms and an alkoxycarbonyl group having from 2 to 5 carbon atoms; the amino group may have one or two substituents selected from the group consisting of a formyl group, an alkyl group having from 1 to 6 carbon atoms, a cycloalkyl group having from 3 to 6 carbon atoms, an aryl group having from 6 to 10 carbon atoms, a heteroaryl group (the heteroaryl group is a 5-membered or 6-membered ring and contains from 1 to 3 hetero atoms of one or more types selected from the group consisting of a nitrogen atom, an oxygen atom and a sulfur atom) , an acyl group having from 2 to 5 carbon atoms and an alkoxycarbonyl group having from 2 to 5 carbon atoms; and when the amino group has two substituents, they may bond to each other to form a cyclic structure; R⁴ represents a hydrogen atom, a halogen atom, an amino group, a hydroxyl group, a nitro group, a cyano group, a carboxyl group, a sulfo group, a carbamoyl group, a groupof the following formula: 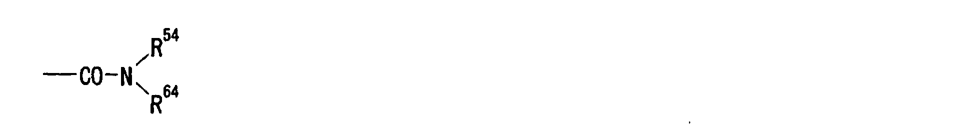 (wherein R⁵⁴ and R⁶⁴ each independently represents a hydrogen atom, an alkyl group having from 1 to 6 carbon atoms or an aryl group having from 6 to 10 carbon atoms),
an alkyl group having from 1 to 6 carbon atoms, an alkenyl group having from 2 to 6 carbon atoms, an alkynyl group having from 2 to 6 carbon atoms, an alkoxy group having from 1 to 6 carbon atoms, an acyl group having from 2 to 5 carbon atoms, an alkoxycarbonyl group having from 2 to 5 carbon atoms, an alkylcarbonyloxy group having from 1 to 6 carbon atoms, an alkyloxysulfonyl group having from 1 to 6 carbon atoms, a cycloalkyl group having from 3 to 6 carbon toms, an aryl group having from 6 to 10 carbon atoms or a heteroaryl group (the heteroaryl group is a 5-membered or 6-membered ring and contains from 1 to 3 hetero atoms of one or more types selected from the group consisting of a nitrogen atom, an oxygen atom and a sulfur atom) ; and the amino group and the hydroxyl group may be protected with a protective group;
when R⁴ is an alkyl group, an alkenyl group, an alkynyl group, an alkoxy group, an acyl group, an alkoxycarbonyl group, a cycloalkyl group, an aryl group or a heterocyclic group, they may have one or more substituents of one or more types selected from the group consisting of an amino group, a hydroxyl group, a mercapto group, a halogen atom, an alkoxy group having from 1 to 6 carbon atoms, an alkylthio group having from 1 to 6 carbon atoms, an acyl group having from 2 to 5 carbon atoms and an alkoxycarbonyl group having from 2 to 5 carbon atoms; the amino group may have one or two substituents selected from the group consisting of a formyl group, an alkyl group having from 1 to 6 carbon atoms, a cycloalkyl group having from 1 to 6 carbon atoms, an aryl group having from 6 to 10 carbon atoms, a heteroaryl group (the heteroaryl group is a 5-membered or 6-membered ring and contains from 1 to 3 hetero atoms of one or more types selected from the group consisting of a nitrogen atom, an oxygen atom and a sulfur atom), an acyl group having from 2 to 5 carbon atoms, an alkoxycarbonyl group having from 2 to 5 carbon atoms, an alkylsulfonyl group having from 1 to 6 carbon atoms and an arylsulfonyl group having from 6 to 10 carbon atoms; and when the amino group has two substituents, they may bond to each other to form a cyclic structure].

The above-mentioned compound, its salt or solvates thereof, wherein A is a benzene ring having one or more substituents of one or more types selected from the group consisting of a carboxyl group, a halogen atom, an alkyl group having from 1 to 6 carbon atoms, an alkoxy group having from 1 to 6 carbon atoms, a hydroxyalkyl group having from 1 to 6 carbon atoms and an alkoxycarbonyl group having from 2 to 5 carbon atoms.

The above-mentioned compound, its salt or solvates thereof, wherein A is a pyridine ring having one or more substituents of one or more types selected from the group consisting of a carboxyl group, a halogen atom, an alkyl group having from 1 to 6 carbon atoms, an alkoxy group having from 1 to 6 carbon atoms, a hydroxyalkyl group having from 1 to 6 carbon atoms and an alkoxycarbonyl group having from 2 to 5 carbon atoms.

The above-mentioned compound, its salt or solvates thereof, wherein R¹ is a halogen atom, a substituted or unsubstituted amino group, a hydroxyl group, a substituted or unsubstituted alkylamino group having from 1 to 10 carbon atoms, a substituted or unsubstituted alkylthio group having from 1 to 10 carbon atoms, a substituted or unsubstituted cycloalkyl group having from 3 to 10 carbon atoms, a substituted or unsubstituted cycloalkenyl group having from 4 to 10 carbon atoms, or a substituted or unsubstituted monocyclic-, bicyclic- or spirocyclic-heterocyclic group having from 3 to 10 carbon atoms (containing from 1 to 4 hetero atoms of one or more types selected from the group consisting of a nitrogen atom, an oxygen atom and a sulfur atom).

The above-mentioned compound, its salt or solvates thereof, wherein R² is a hydrogen atom, a halogen atom, a group of the following formula: 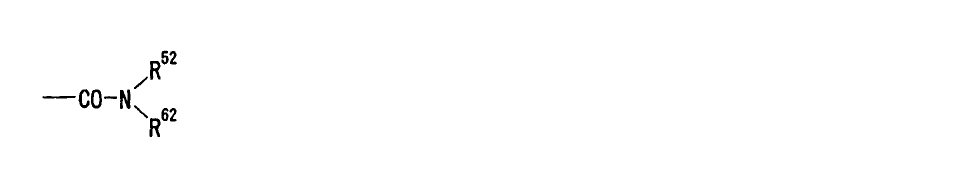 (wherein R⁵² and R⁶² each independently represents a hydrogen atom, an alkyl group having from 1 to 6 carbon atoms or an aryl group having from 6 to 10 carbon atoms),
a substituted or unsubstituted alkyl group having from 1 to 20 carbon atoms, a substituted or unsubstituted alkenyl group having from 2 to 20 carbon atoms, a substituted or unsubstituted cycloalkyl group having from 3 to 10 carbon atoms, a substituted or unsubstituted cycloalkenyl group having from 5 to 10 carbon atoms, a substituted or unsubstituted aryl group having from 6 to 10 carbon atoms, a substituted or unsubstituted aralkyl group having from 7 to 16 carbon atoms, or a substituted or unsubstituted monocyclic- or bicyclic-heterocyclic group having from 5 to 10 carbon atoms (containing from 1 to 4 hetero atoms of one or more types selected from the group consisting of a nitrogen atom, an oxygen atom and a sulfur atom).

The above-mentioned compound, its salt or solvates thereof, wherein R³ is a hydrogen atom, a halogen atom, a substituted or unsubstituted alkyl group having from 1 to 6 carbon atoms or a substituted or unsubstituted cycloalkyl group having from 3 to 7 carbon atoms.

The above-mentioned compound, its salt or solvates thereof, wherein R⁴ is a cyano group, a carboxyl group, a carbamoyl group, a substituted or unsubstituted alkyl group having from 1 to 6 carbon atoms or a substituted or unsubstituted alkoxycarbonyl group having from 2 to 5 carbon atoms.

A pharmaceutical composition containing the above-mentioned compound, its salt or solvates thereof.

An agent for treating an infectous disease, containing the above-mentioned compound, its salt or solvates thereof.

An antifungal agent containing the above-mentioned compound, its salt or solvates thereof.

### (Mode for Carrying out the Invention)

The definitions of the terms used in this description are mentioned below.

The "alkyl group" and the alkyl moiety in the alkyl moiety-containing substituent (e.g., alkoxygroup) maybeeither straight or branched chain form. Specifically, the alkyl group includes a methyl group, an ethyl group, a normal-propyl group, a normal-butyl group, a normal pentyl group, a normal-hexyl group, a normal-heptanyl group, a normal-octanyl group, a normal-nonanyl group, a normal-undecanyl group, a normal-dodecanyl group, a normal-tridecanyl group, a normal-tetradecanyl group, a normal-pentadecanyl group, a normal-hexadecanyl group, a normal-heptadecanyl group, a normal-octadecanyl group, an isopropyl group, an isobutyl group, a secondary-butyl group, a tertiary-butyl group, an isopentyl group, a neopentyl group, a tertiary-pentyl group, an isohexyl group, a 1,1-dimethylpropyl group, an n-heptyl group and an n-octyl group.

The "cycloalkyl group" means a monocyclic- or bicyclic-cycloalkyl group, including, for example, a cyclopropyl group, a cyclobutyl group, a cyclopentyl group, a cyclohexyl group and a bicyclo[3.2.1]oct-2-yl group.

The "alkenyl group" may be either straight or branched chain form, and has one or more carbon-carbon double bonds. Specifically, it includes a vinyl group, a propenyl group, a buten-1-yl group, an isobutenyl group, a penten-1-yl group, a 2-methylbuten-1-yl group, a 3-methylbuten-1-yl group, a hexen-1-yl group, a hepten-1-yl group and an octen-1-yl group.

The "cycloalkenyl group" means a monocyclic- or bicyclic-cycloalkenyl group, including, for example, a 2-cyclopenten-1-yl group, a 2,4-cyclopentadien-1-yl group and a 5-norbornen-2-yl group.

The "alkynyl group" may be either straight or branched chain form, and has one or more carbon-carbon triple bonds. Specifically, it includes an ethynyl group and a propynyl group.

The "halogen atom" means a fluorine atom, a chlorine atom, a bromine atom or an iodine atom.

The "aryl group" means a monovalent group derived from an aromatic ring of an aromatic hydrocarbon by removing one hydrogen atom from the ring. The aromatic ring to constitute the aryl group may be a monocyclic ring or a fused ring. For example, it includes a phenyl group, a naphthyl group, an anthryl group, and an azulenyl group.

The "aralkyl group" means a group formed by substituting the hydrogen atom (s) of an alkyl group for one or more aryl groups such as those mentioned above. For example, i t includes a benzyl group, a benzhydryl group and a trityl group.

The "heterocyclic group" means a group derived from a saturated, partially-saturated or unsaturated heterocyclic compound, and may be monocyclic, bicyclic or spirocyclic. The heterocyclic compound to give the heterocyclic group includes, for example, aziridine, azetidine, pyrrole, furan, thiophene, pyrrolidine, tetrahydrofuran, tetrahydrothiophene, imidazole, pyrazole, imidazolidine, pyrazolidine, oxazole, isoxazole, thiazole, isothiazole, pyridine, dihydropyridine, tetrahydropyridine, piperidine, pyridazine, pyrimidine, triazine, pyrazine, piperazine, pyrrolidone, dioxane, pyran, morpholine, benzofuran, indolidine, benzothiophene, indole, naphthyridine, quinoxaline, quinazoline and chroman. In addition, the following are further mentioned. 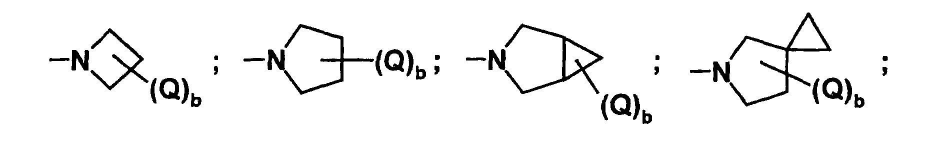 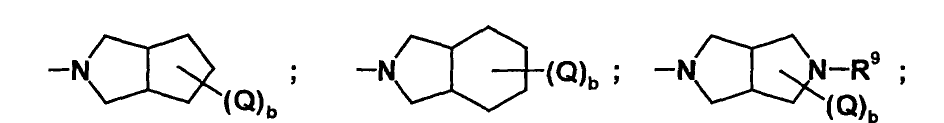 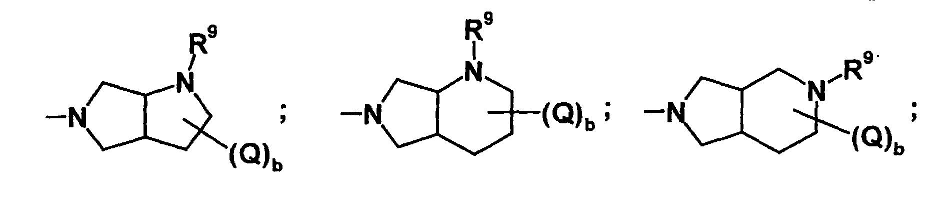 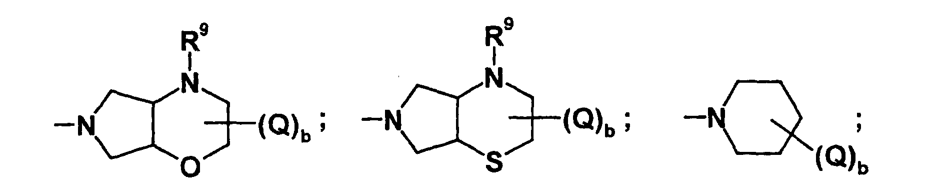 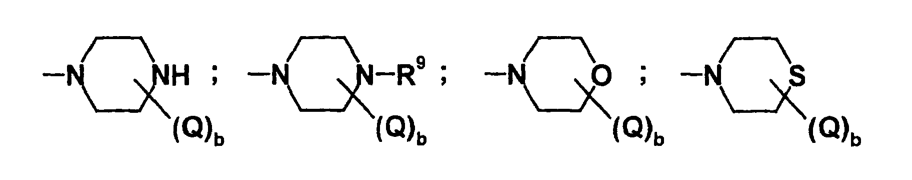 (In these formulae, R⁹ represents an alkyl group having from 1 to 6 carbon atoms, a halogenoalkyl group having from 1 to 6 carbon atoms or a cycloalkyl group having from 3 to 6 carbon atoms; the substituent Q is represented by the following formula:

-N(R¹⁰)(R¹¹),

or the following formula:

-C(R¹²)(R¹³)N(R¹⁰)(R¹¹);

b indicates an integer of 0, 1 or 2, R¹⁰ and R¹¹ each independently represents a hydrogen atom, an alkyl group having from 1 to 6 carbon atoms, a halogenoalkyl group having from 1 to 6 carbon atoms, an amino group, a dipeptide, or a polypeptide comprising from 3 to 5 amino acids; R¹² and R¹³ each independently represents a hydrogen atom, an alkyl group having from 1 to 6 carbon atoms, a halogenoalkyl group having from 1 to 6 carbon atoms, a hydroxyalkyl group having from 1 to 6 carbon atoms, an aminoalkyl group having from 1 to 6 carbon atoms, an alkoxyalkyl group having from 2 to 12 carbon atoms, a cycloalkyl group having from 3 to 6 carbon atoms, a substituted or unsubstituted phenyl group, or a substituted or unsubstituted heteroaryl group.)

R⁹ is preferably a hydrogen atom or an alkyl group. The alkyl group is preferably a methyl group, an ethyl group, a normal-propyl group or an isopropyl group.

R¹⁰ and R¹¹ each is preferably a hydrogen atom or an alkyl group. The alkyl group is preferably a methyl group, an ethyl group, a normal-propyl group or an isopropyl group.

R¹² and R¹³ each is preferably a hydrogen atom, an alkyl group, a halogenoalkyl group, an alkoxyalkyl group, a cycloalkyl group, or a phenyl group. Of those, more preferred are a hydrogen atom, a methyl group, an ethyl group, a fluoromethyl group, a trifluoromethyl group, a 2-fluoromethyl group, a methoxymethyl group, a cyclopropyl group, a cyclobutyl group and a phenyl group.

R¹² and R¹³ may cooperate to form a spirocyclic structure having from 3 to 6 carbon atoms. The spiro-ring may contain a nitrogen atom as a ring constituent atom. Preferred examples of the spirocyclic structure include spirocyclopropyl, spirocyclobutyl and spirocyclopentyl.

The "heteroaryl group" especially means a heteroaromatic group of the above-mentioned heterocyclic groups. For example, it includes a pyrrolyl group, a furyl group, a thienyl group, an imidazolyl group, a pyrazolyl group, an oxazolyl group, an isoxazolyl group, a thiazolyl group, an isothiazolyl group, a pyridyl group, a pyridazinyl group, a pyrimidinyl group, a triazinyl group, a pyrazinyl group, a benzofuryl group, an indolyl group, a naphthyridinyl group, a quinoxalinyl group and a quinazolinyl group.

This description says that the amino group, the hydroxyl group, the mercapto group and others "may be protected with a protective group", in which the "protective group" is not specifically limited and may be any one generally used in this technical field. For example, it includes alkoxycarbonyl groups such as tertiary-butoxycarbonyl group, 2,2,2-trichloroethoxycarbonyl group; aralkyloxycarbonyl groups such as benzyloxycarbonyl group, para-methoxybenzyloxycarbonyl group, para-nitrobenzyloxycarbonyl group; acyl groups such as acetyl group, methoxyacetyl group, trifluoroacetyl group, chloroacetyl group, pivaloyl group, formyl group, benzoyl group; alkyl groups and aralkyl groups such as tertiary-butyl group, benzyl group, para-nitrobenzyl group, para-methoxybenzyl group, triphenylmethyl group; ethers such as methoxymethyl group, tertiary-butoxymethyl group, tetrahydropyranyl group, 2,2,2-trichloroethoxymethyl group; (alkyl and/or aralkyl) -substituted silyl groups such as trimethylsilyl group, isopropyldimethylsilyl group, tertiary-butyldimethylsilyl group, tribenzylsilyl group, tertiary-butyl diphenylsilyl group. The amino group may be protected as phthalimide.

The partial structure and the substituents of the compounds of formula (I) of the invention are described.

In the compounds of the following formula (I): 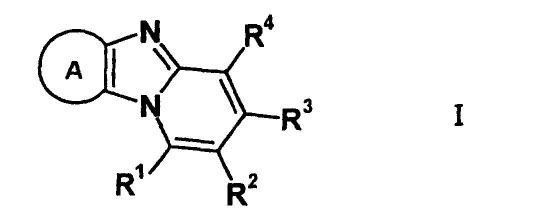 the partial structure A means a benzene ring or a heteroaryl ring (which is a 5-membered or 6-membered ring and contains from 1 to 3 hetero atoms of one or more types selected from the group consisting of a nitrogen atom, an oxygen atom and a sulfur atom) , and these benzene ring and heteroaryl ring each may have one or more substituents of one or more types selected from the group consisting of an amino group, a hydroxyl group, a mercapto group, a nitro group, a cyano group, a formyl group, a carboxyl group, a sulfo group, a halogen atom, a heteroaryl group (the heteroaryl is a 5-membered or 6-membered ring and contains from 1 to 3 hetero atoms of one or more types selected from the group consisting of a nitrogen atom, an oxygen atom and a sulfur atom), a group of the following formula: 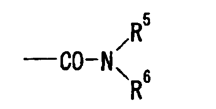 (wherein R⁵ and R⁶ each independently represents a hydrogen atom, an alkyl group having from 1 to 6 carbon atoms, or an aryl group having from 6 to 10 carbon atoms),
an alkyl group having from 1 to 6 carbon atoms, an alkenyl group having from 2 to 6 carbon atoms, an alkynyl group having from 2 to 6 carbon atoms, an alkoxy group having from 1 to 6 carbon atoms, a hydroxyalkyl group having from 1 to 6 carbon atoms, an alkylamino group having from 1 to 6 carbon atoms, an alkylthio group having from 1 to 6 carbon atoms, an acyl group having from 2 to 5 carbon atoms, an alkoxycarbonyl group having from 2 to 5 carbon atoms, a cycloalkyl group having from 3 to 6 carbon atoms, an aryl group having from 6 to 10 carbon atoms and an aralkyl group having from 7 to 16 carbon atoms, and the substituents containing a carbon chain of them may bond to each other to form a cyclic structure and fuse to the benzene ring or the heteroaryl ring.

A is preferably a benzene ring or a pyridine ring, and the substituent which the benzene ring or the pyridine ring may have is preferably one or more groups selected from a carboxyl group, a halogen atom, an alkyl group having from 1 to 6 carbon atoms, an alkoxy group having from 1 to 6 carbon atoms, a hydroxyalkyl group having from 1 to 6 carbon atoms and an alkoxycarbonyl group having from 2 to 5 carbon atoms. Of those, the halogen atom is preferably a fluorine atom or a chlorine atom; the alkyl group is preferably a methyl group; and the alkoxycarbonyl group is preferably an ethoxycarbonyl group.

R¹ represents a hydrogen atom, a halogen atom, an amino group, a hydroxyl group, a mercapto group, a nitro group, a cyano group, a formyl group, a carboxyl group, a group of the following formula: 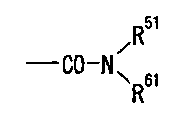 (wherein R⁵¹ and R⁶¹ each independently represents a hydrogen atom, an alkyl group having from 1 to 6 carbon atoms or an aryl group having from 6 to 10 carbon atoms),
an alkyl group having from 1 to 10 carbon atoms, an alkenyl group having from 2 to 10 carbon atoms, an alkynyl group having from 2 to 10 carbon atoms, an alkylamino group having from 1 to 10 carbon atoms, an alkoxy group having from 1 to 10 carbon atoms, an alkylthio group having from 1 to 10 carbon atoms, an acyl group having from 2 to 6 carbon atoms, an alkoxycarbonyl group having from 2 to 7 carbon atoms, a cycloalkyl group having from 3 to 10 carbon atoms, a cycloalkylamino group having from 3 to 10 carbon atoms, a cyaloalkyloxy group having from 3 to 10 carbon atoms, a cycloalkyl thio group having from 3 to 10 carbon atoms, a cycloalkenyl group having from 4 to 10 carbon atoms, a cycloalkenylamino group having from 4 to 10 carbon atoms, a cycloalkenyloxy group having from 4 to 10 carbon atoms, a cycloalkenylthio group having from 4 to 10 carbon atoms, an aryl group having from 6 to 10 carbon atoms, an arylamino group having from 6 to 10 carbon atoms, an aryloxy group having from 6 to 10 carbon atoms, an arylthio group having from 6 to 10 carbon atoms, a monocyclic-, bicyclic- or spirocyclic-heterocyclic group having from 3 to 10 carbon atoms (containing from 1 to 4 hetero atoms of one or more types selected from the group of a nitrogen atom, an oxygen atom and a sulfur atom), a heteroarylamino group having from 5 to 10 carbon atoms, a heteroaryloxy group having from 5 to 10 carbon atoms, a heteroarylthio group having from 5 to 10 carbon atoms, in which the amino group, the hydroxyl group and the mercapto group may be protected with a protective group.

Preferably, R¹ is a halogen atom, an amino group, a hydroxyl group, an alkylamino group having from 1 to 10 carbon atoms, an alkylthio group having from 1 to 10 carbon atoms, a cycloalkyl group having from 3 to 10 carbon atoms, a cycloalkenyl group having from 4 to 10 carbon atoms, or a monocyclic-, bicyclic- or spirocyclic-heterocyclic group having from 3 to 10 carbon atoms (containing from 1 to 4 hetero atoms of one or more types selected from the group consisting of a nitrogen atom, an oxygen atom and a sulfur atom).

R¹ may have one or more substituents in the manner mentioned below.

When R¹ is an amino group, an alkyl group, an alkenyl group, an alkynyl group, an alkylamino group, an alkoxy group, an alkylthio group, an acyl group, an alkoxycarbonyl group, a cycloalkyl group, a cycloalkylamino group, a cycloalkyloxy group, a cycloalkylthio group, a cycloalkenyl group, a cycloalkenylamino group, a cycloalkenyloxy group, a cycloalkenylthio group, an aryl group, an arylamino group, an aryloxy group, an arylthio group, a heteroarylamino group, a heteroaryloxy group or a heteroarylthio group, they may have one or more substituents of one or more types selected from the group consisting of an amino group, an aminoalkyl group having from 1 to 6 carbon atoms (the amino group and the amino group moiety of the aminoalkyl group may be protected with a protective group, and may have one or two alkyl groups each having from 1 to 6 carbon atoms, and when they have two alkyl groups, then the groups may be the same or different), a hydroxyl group, a mercapto group, a halogen atom, an alkoxy group having from 1 to 6 carbon atoms, an alkylthio group having from 1 to 6 carbon atoms, an acyl group having from 2 to 7 carbon atoms, an alkoxycarbonyl group having from 2 to 7 carbon atoms, an aryl group having from 6 to 10 carbon atoms, a monocyclic- or bicyclic-heterocyclic group having from 3 to 10 carbon atoms (containing from 1 to 4 hetero atoms of one or more types selected from the group consisting of a nitrogen atom, an oxygen atom and a sulfur atom), an alkylcarbonylamino group having from 2 to 7 carbon atoms and a cycloalkyl group having from 3 to 10 carbon atoms.

Among these, when R¹ is an amino group, an alkylamino group, an alkylthio group, a cycloalkyl group, a cycloalkylamino group, a cycloalkenyl group or an aryl group, the substituents which they may have are preferably one or more selected from an amino group, an aminoalkyl group having from 1 to 6 carbon atoms (the amino group and the amino group moiety in the alkylamino group having from 1 to 6 carbon atoms may be protected with a protective group, and may have one or two alkyl groups each having from 1 to 6 carbon atoms, and when they have two alkyl groups, the alkyl groups may be the same or different), a hydroxyl group, an aryl group having from 6 to 10 carbon atoms, a monocyclic- or bicyclic-heterocyclic group having from 3 to 10 carbon atoms (containing from 1 to 4 hetero atoms of one or more types selected from the group consisting of a nitrogen atom, an oxygen atom and a sulfur atom), an alkylcarbonylamino group having from 2 to 7 carbon atoms and a cycloalkyl group having from 3 to 10 carbon atoms.

When R¹ is a heterocyclic group, it may have one or more substituents of one or more types selected from the group consisting of a halogen atom, an amino group, a hydroxyl group, an oxo group, a group of the following formula: 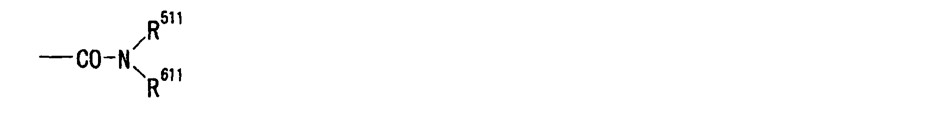 (wherein R⁵¹¹ and R⁶¹¹ each independently represents a hydrogen atom, an alkyl group having from 1 to 6 carbon atoms or an aryl group having from 6 to 10 carbon atoms),
an alkyl group having from 1 to 6 carbon atoms, an alkylamino group having from 1 to 6 carbon atoms, a cycloalkylamino group having from 3 to 6 carbon atoms, an aryl group having from 6 to 10 carbon atoms, a monocyclic- or bicyclic-heterocyclic group having from 3 to 10 carbon atoms (containing from 1 to 4 hetero atoms of one or more types selected from the group of a nitrogen atom, an oxygen atom and a sulfur atom) , an alkoxy group having from 1 to 6 carbon atoms, an alkylthio group having from 1 to 6 carbon atoms, a halogenoalkyl group having from 1 to 6 carbon atoms and an aminoalkyl group having from 1 to 6 carbon atoms; and the alkyl group and the alkyl moiety of the alkylamino group, the cycloalkylamino group, the alkoxy group, the alkylthio group, the halogenoalkyl group and the aminoalkyl group may have one or more substituents of one or more types selected from the group consisting of a halogen atom, a hydroxyl group, a carboxyl group, an alkyl group having from 1 to 6 carbon atoms, an alkoxy group having from 1 to 6 carbon atoms, an alkoxycarbonyl group having from 2 to 7 carbon atoms, an alkoxycarbonylamino group having from 2 to 7 carbon atoms, an aryl group having from 6 to 10 carbon atoms, and a monocyclic- or bicyclic-heterocyclic group having from 3 to 10 carbon atoms (containing from 1 to 4 hetero atoms of one or more types selected from the group of a nitrogen atom, an oxygen atom and a sulfur atom); the amino group and the amino group moiety of the aminoalkyl group and the alkylamino group may be protected with a protective group, and may have, as the substituent thereof, one or two alkyl groups each having from 1 to 6 carbon atoms (optionally having one or more substituents of one or more types selected from the group consisting of a hydroxyl group, a halogen atom, an alkoxy group having from 1 to 6 carbon atoms and an alkylthio group), and an amino acid, a dipeptide or a polypeptide comprising from 3 to 5 amino acids may be bonded thereto.

Among these, preferred examples of the substituent which the heterocyclic group of R¹ may have include an amino group, a hydroxyl group, an alkyl group having from 1 to 6 carbon atoms, an alkylamino group having from 1 to 6 carbon atoms, a cycloalkyl group having from 3 to 6 carbon atoms, a monocyclic- or bicyclic-heterocyclic group having from 3 to 10 carbon atoms (containing from 1 to 4 hetero atoms of one or more types selected from the group consisting of a nitrogen atom, an oxygen atom and a sulfur atom), and an aminoalkyl group having from 1 to 6 carbon atoms; and the alkyl group and the alkyl moiety of the alkylamino group and the aminoalkyl group may have one or more substituents of one or more types selected from the group consisting of a halogen atom, a hydroxyl group, a carboxyl group, an alkyl group having from 1 to 6 carbon atoms, an alkoxy group having from 1 to 6 carbon atoms, an alkoxycarbonyl group having from 2 to 7 carbon atoms, an alkoxycarbonylamino group having from 2 to 7 carbon atoms, an aryl group having from 6 to 10 carbon atoms, and a monocyclic- or bicyclic-heterocyclic group having from 3 to 10 carbon atoms (containing from 1 to 4 hetero atoms of one or more types selected from the group consisting of a nitrogen atom, an oxygen atom and a sulfur atom); the amino group and the amino group moiety of the aminoalkyl group and the alkylamino group may have, as the substituent thereof, one or two alkyl groups each having from 1 to 6 carbon atoms (optionally having, as the substituent thereof, one or more hydroxyl groups) .

R² represents a hydrogen atom, a halogen atom, an amino group, a hydroxyl group, a nitro group, a cyano group, a carboxyl group, a group of the following formula: 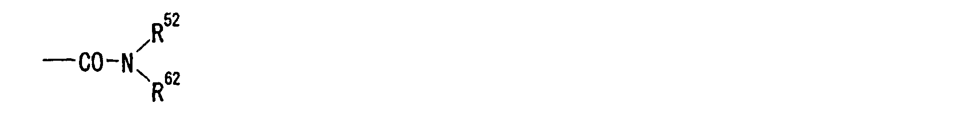 (wherein R⁵² and R⁶² each independently represents a hydrogen atom, an alkyl group having from 1 to 6 carbon atoms or an aryl group having from 6 to 10 carbon atoms),
an alkyl group having from 1 to 20 carbon atoms, an alkenyl group having from 2 to 20 carbon atoms, an alkynyl group having from 2 to 20 carbon atoms, an alkylamino group having from 1 to 20 carbon atoms, an alkoxy group having from 1 to 20 carbon atoms, an acyl group having from 2 to 18 carbon atoms, an alkoxycarbonyl group having from 2 to 18 carbon atoms, a cycloalkyl group having from 3 to 10 carbon atoms, a cycloalkenyl group having from 5 to 10 carbon atoms, a cycloalkylamino group having from 3 to 10 carbon atoms, a cycloalkylalkyl group having from 4 to 16 carbon atoms, a cycloalkyloxy group having from 3 to 10 carbon atoms, an aryl group having from 6 to 10 carbon atoms, an arylamino group having from 6 to 10 carbon atoms, an aralkyl group having from 7 to 16 carbon atoms, an aryloxy group having from 6 to 10 carbon atoms, a monocyclic- or bicyclic-heterocyclic group having from 5 to 10 carbon atoms (containing from 1 to 4 hetero atoms of one or more types selected from the group of a nitrogen atom, an oxygen atom and a sulfur atom), a heteroarylamino group having from 5 to 10 carbon atoms, a heteroarylalkyl group having from 6 to 16 carbon atoms, or a heteroaryloxy group having from 5 to 10 carbon atoms, and the amino group and the hydroxyl group may be protected with a protective group.

R² is preferably a hydrogen atom, a halogen atom, a group of the following formula: 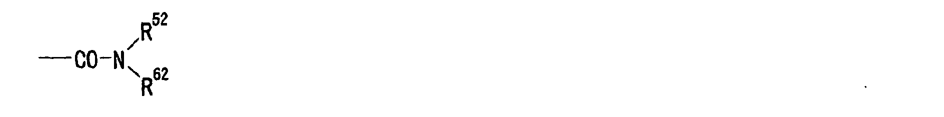 (wherein R⁵² and R⁶² each independently represents a hydrogen atom, an alkyl group having from 1 to 6 carbon atoms or an aryl group having from 6 to 10 carbon atoms),
an alkyl group having from 1 to 20 carbon atoms, an alkenyl group having from 2 to 20 carbon atoms, a cycloalkyl group having from 3 to 10 carbon atoms, a cycloalkenyl group having from 5 to 10 carbon atoms, an aryl group having from 6 to 10 carbon atoms, an aralkyl group having from 7 to 16 carbon atoms, or a monocyclic- or bicyclic-heterocyclic group having from 5 to 10 carbon atoms (containing from 1 to 4 hetero atoms of one or more types selected from the group of a nitrogen atom, an oxygen and a sulfur atom) .

R² may have a substituent in the manner mentioned below.

When R² is an alkyl group, an alkenyl group, an alkynyl group, an alkylamino group, an alkoxy group, an acyl group, an alkoxycarbonyl group, a cycloalkyl group, a cycloalkylamino group or a cycloalkyloxy group, then these groups may have one or more substituents of one or more types selected from the group consisting of a halogen atom, an amino group, an imino group, a nitro group, a hydroxyl group, a mercapto group, a carboxyl group, a cyano group, a sulfo group, a dialkylphosphoryl group, a group of the following formula: 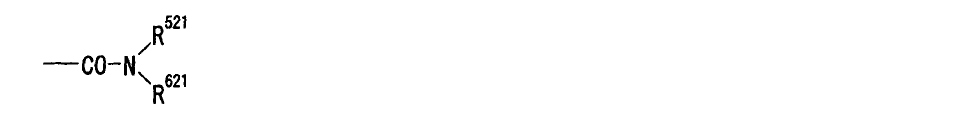 (wherein R⁵²¹ and R⁶²¹ each independently represents a hydrogen atom, an alkyl group having from 1 to 6 carbon atoms or an aryl group having from 6 to 10 carbon atoms),
an alkoxy group having from 1 to 10 carbon atoms, an alkylthio group having from 1 to 10 carbon atoms, an acyl group having from 2 to 8 carbon atoms, an alkoxycarbonyl group having from 2 to 8 carbon atoms, a cycloalkyl group having from 3 to 10 carbon atoms, an aryl group having from 6 to 10 carbon atoms and an aryl thio group having from 6 to 10 carbon atoms; the amino group may have one or two substituents selected from the group consisting of a formyl group, an alkyl group having from 1 to 10 carbon atoms, an aminoalkyl group having from 1 to 8 carbon atoms, a hydroxyalkyl group having from 1 to 8 carbon atoms, a mercaptoalkyl group having from 1 to 8 carbon atoms, an acyl group having from 2 to 8 carbon atoms, an alkoxycarbonyl group having from 2 to 8 carbon atoms, a cycloalkyl group having from 3 to 10 carbon atoms, an aryl group having from 6 to 10 carbon atoms, an aralkyl group having from 7 to 16 carbon atoms, a heteroaryl group (the heteroaryl group is a 5-membered or 6-membered ring and contains from 1 to 4 hetero atoms of one or more types selected from the group consisting of a nitrogen atom, an oxygen atom and a sulfur atom) , an alkylsulfonyl group having from 1 to 10 carbon atoms and an arylsulfonyl group having from 6 to 10 carbon atoms; when the amino group has two substi tuents, they may bond to each other to forma cyclic structure; the hydroxyl group and the mercapto group may have a substituent selected from the group consisting of an alkyl group having from 1 to 10 carbon atoms, an aminoalkyl group having from 1 to 8 carbon atoms, a hydroxyalkyl group having from 1 to 8 carbon atoms, a mercaptoalkyl group having from 1 to 8 carbon atoms, an acyl group having from 2 to 8 carbon atoms, a cycloalkyl group having from 3 to 10 carbon atoms, an aryl group having from 6 to 10 carbon atoms, an aralkyl group having from 7 to 16 carbon atoms, and a heteroaryl group (the heteroaryl group is a 5-membered or 6-membered ring and contains from 1 to 4 hetero atoms of one or more types selected from the group consisting of a nitrogen atom, an oxygen atom and a sulfur atom); the cycloalkyl group may have one or more substituents of one or more types selected from the group consisting of a halogen atom, an amino group, an imino group, a ni tro group, a hydroxyl group, a mercapto group, a carboxyl group, a cyano group, a sulfo group, a group of the following formula: 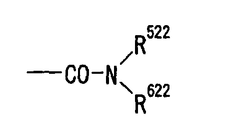 (wherein R⁵²² and R⁶²² each independently represents a hydrogen atom, an alkyl group having from 1 to 6 carbon atoms or an aryl group having from 6 to 10 carbon atoms),
an alkoxy group having from 1 to 10 carbon atoms, an alkylthio group having from 1 to 10 carbon atoms, an acyl group having from 2 to 8 carbon atoms and an alkoxycarbonyl group having from 1 to 8 carbon atoms; the amino group may have one or two substituents selected from the group consisting of a formyl group, an alkyl group having from 1 to 10 carbon atoms, an aminoalkyl group having from 1 to 8 carbon atoms, a hydroxyalkyl group having from 1 to 8 carbon atoms, a mercaptoalkyl group having from 1 to 8 carbon atoms, an acyl group having from 2 to 8 carbon atoms, an alkoxycarbonyl group having from 2 to 8 carbon atoms, a cycloalkyl group having from 3 to 10 carbon atoms, an aryl group having from 6 to 10 carbon atoms, an aralkyl group having from 7 to 16 carbon atoms, a heteroaryl group (the heteroaryl group is a 5-membered or 6-membered ring and contains from 1 to 4 hetero atoms of one or more types selected from the group consisting of a nitrogen atom, an oxygen atom and a sulfur atom), an alkylsulfonyl group having from 1 to 10 carbon atoms, and an arylsulfonyl group having from 6 to 10 carbon atoms; when the amino group has two substituents, they may bond to each other to form a cyclic structure.

Among these, when R² is an alkyl group or an alkenyl group, the substituents which they may have are preferably one or more selected from the group consisting of an amino group, a hydroxyl group, a carboxyl group, a cyano group, an alkoxycarbonyl group having from 2 to 8 carbon atoms, an aryl group having from 6 to 10 carbon atoms and an arylthio group having from 6 to 10 carbon atoms; the amino group may have one or two substituents selected from the group consisting of a formyl group, an acyl group having from 2 to 8 carbon atoms and an alkoxycarbonyl group having from 2 to 8 carbon atoms; when the amino group has two substituents, they may bond to each other to form a cyclic structure; and the hydroxyl group may have an acyl group having from 2 to 8 carbon atoms.

When R² is an aryl group, an arylamino group, an aralkyl group, an aryloxy group, a heteroaryl group, a heteroarylamino group, a heteroarylalkyl group or a heteroaryloxy group, they may have one or more substituents of one or more types selected from the group consisting of a halogen atom, an amino group, an imino group, a nitro group, a hydroxyl group, a mercapto group, a carboxyl group, a cyano group, a sulfo group, a group of the following formula: 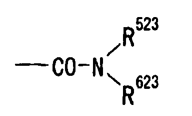 (wherein R⁵²³ and R⁶²³ each independently represents a hydrogen atom, an alkyl group having from 1 to 6 carbon atoms or an aryl group having from 6 to 10 carbon atoms),
an alkoxy group having from 1 to 10 carbon atoms, an alkylthio group having from 1 to 10 carbon atoms, an acyl group having from 2 to 8 carbon atoms, an alkoxycarbonyl group having from 2 to 8 carbon atoms, an aralkyloxy group having from 7 to 16 carbon atoms, an aralkyloxycarbonyl group having from 8 to 17 carbon atoms, an aryl group, and a monocyclic- or bicyclic-heterocyclic group having from 5 to 10 carbon atoms (containing from 1 to 4 hetero atoms of one or more types selected from the group consisting of a nitrogen atom, an oxygen atom and a sulfur atom); the amino group may have one or two substituents selected from the group consisting of a formyl group, an alkyl group having from 1 to 10 carbon atoms, an aminoalkyl group having from 1 to 8 carbon atoms, a hydroxyalkyl group having from 1 to 8 carbon atoms, a mercaptoalkyl group having from 1 to 8 carbon atoms, an acyl group having from 2 to 8 carbon atoms, an alkoxycarbonyl group having from 2 to 8 carbon atoms , a cycloalkyl group having from 3 to 10 carbon atoms, an aryl group having from 6 to 10 carbon atoms, an aralkyl group having from 7 to 16 carbon atoms, a heteroaryl group (the heteroaryl group is a 5-membered or 6-membered ring and contains from 1 to 4 hetero atoms of one or more types selected from the group consisting of a nitrogen atom, an oxygen atom and a sulfur atom), an alkylsulfonyl group having from 1 to 10 carbon atoms and an arylsulfonyl group having from 6 to 10 carbon atoms; and when the amino group has two substituents, they may bond to each other to form a cyclic structure.

Among these, when R² is an aryl group or an aralkyl group, the substituents which they may have are preferably one or more selected from a halogen atom, an amino group, a nitro group, a hydroxyl group, a carboxyl group, a cyano group, an alkoxy group having from 1 to 10 carbon atoms, an alkoxycarbonyl group having from 2 to 8 carbon atoms, an aralkyloxy group having from 7 to 16 carbon atoms and an aralkyloxycarbonyl group having from 8 to 17 carbon atoms; and the amino group may have one or two substituents selected from an acyl group having from 2 to 8 carbon atoms and an alkylsulfonyl group having from 1 to 10 carbon atoms.

When R² is a heterocyclic group, it may have one or two substituents selected from the group consisting of a halogen atom, an amino group, a hydroxyl group, mercapto group, a carboxyl group, a group of the following formula: 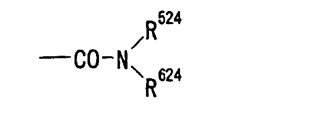 (wherein R⁵²⁴ and R⁶²⁴ each independently represents a hydrogen atom, an alkyl group having from 1 to 6 carbon atoms or an aryl group having from 6 to 10 carbon atoms),
an alkyl group having from 1 to 10 carbon atoms, an alkenyl group having from 2 to 10 carbon atoms, an alkynyl group having from 2 to 10 carbon atoms, an alkoxy group having from 1 to 10 carbon atoms, an alkylthio group having from 1 to 10 carbon atoms, a halogenoalkyl group having from 1 to 10 carbon atoms, an acyl group having from 2 to 10 carbon atoms, an alkoxycarbonyl group having from 2 to 10 carbon atoms and an aryl group having from 6 to 10 carbon atoms; the amino group may have one or two substituents selected from the group consisting of a formyl group, an alkyl group having from 1 to 10 carbon atoms, an aminoalkyl group having from 1 to 8 carbon atoms, a hydroxyalkyl group having from 1 to 8 carbon atoms, a mercaptoalkyl group having from 1 to 8 carbon atoms, an acyl group having from 2 to 8 carbon atoms, an alkoxycarbonyl group having from 2 to 8 carbon atoms, a cycloalkyl group having from 3 to 10 carbon atoms, an aryl group having from 6 to 10 carbon atoms, an aralkyl group having from 7 to 16 carbon atoms, a monocyclic- or bicyclic-heterocyclic group having from 5 to 10 carbon atoms (containing from 1 to 4 hetero atoms of one or more types selected from the group consisting of a nitrogen atom, an oxygen atom and a sulfur atom) , a heteroaryl group (the heteroaryl group is a 5-membered or 6-membered ring and contains from 1 to 4 hetero atoms of one or more types selected from the group consisting of a nitrogen atom, an oxygen atom and a sulfur atom), an alkylsulfonyl group having from 1 to 10 carbon atoms and an arylsulfonyl group having from 6 to 10 carbon atoms; and when the amino group has two substituents, they may bond to each other to form a cyclic structure.

Among these, the substituent which the heterocyclic group of R² may have is preferably an alkyl group having from 1 to 10 carbon atoms or an aryl group having from 6 to 10 carbon atoms.

R¹ and R² may cooperate to form a 5-membered or 6-membered heterocyclic group (containing from 1 to 3 hetero atoms of one or more types selected from the group consisting of a nitrogen atom, an oxygen atom and a sulfur atom) , for example, represented by the following formula: 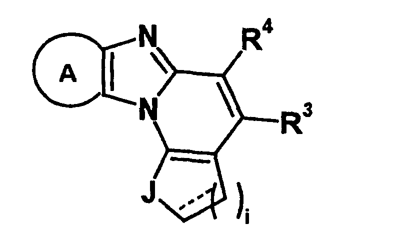 (wherein A, R³ and R⁴ have the same meanings as above; i indicates an integer of 1 or 2; J represents a nitrogen atom, an oxygen atom or a sulfur atom.)

The heterocyclic group to be formed by R¹ and R² may be saturated, partially saturated or unsaturated, and the ring constituent atoms may contain from 1 to 4 hetero atoms selected from a nitrogen atom, an oxygen atom and a sulfur atom in any desired manner.

Specific examples of the heterocyclic structure that may be formed by R¹ and R² are mentioned below. 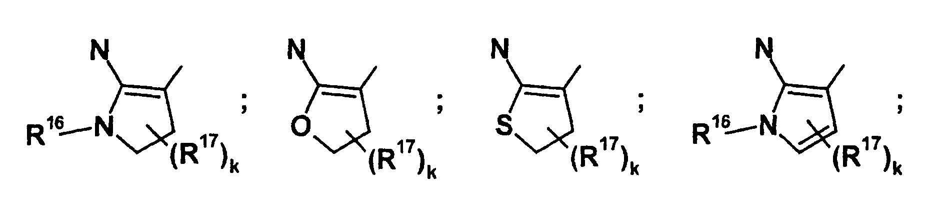 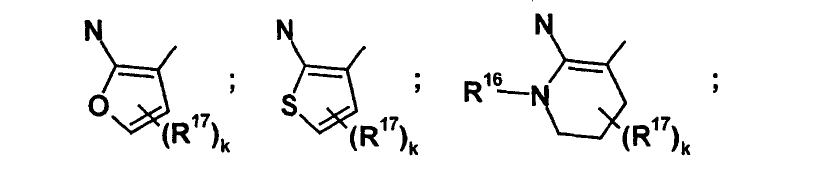 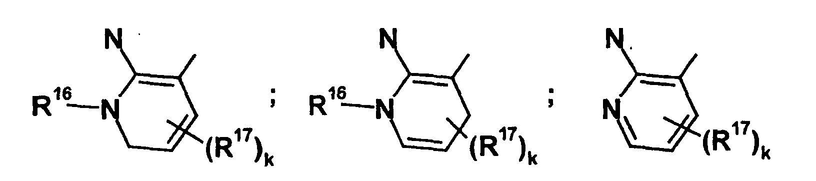 (In these formulae, R¹⁶ represents a hydrogen atom, an alkyl group having from 1 to 6 carbon atoms, an alkenyl group having from 2 to 6 carbon atoms, an alkynyl group having from 2 to 6 carbon atoms, a halogenoalkyl group having from 1 to 6 carbon atoms or a cycloalkyl group having from 3 to 6 carbon atoms; R¹⁷ represents a hydrogen atom, a halogen atom, a substituted or unsubstituted amino group, a hydroxyl group, a thiol group, an alkyl group having from 1 to 6 carbon atoms, an alkenyl group having from 2 to 6 carbon atoms, an alkynyl group having from 2 to 6 carbon atoms, an alkoxy group having from 1 to 6 carbon atoms, an alkylthio group having from 1 to 6 carbon atoms, a halogenoalkyl group having from 1 to 6 carbon atoms, a bicycloalkyl group having from 3 to 6 carbon atoms and optionally having a halogen atom, or a spirocycloalkyl group having from 3 to 6 carbon atoms and optionally having a halogen atom; k indicates an integer of 0, 1 or 2.)

R³ represents a hydrogen atom, a halogen atom, an amino group, a hydroxyl group, a mercapto group, a nitro group, a cyano group, a formyl group, a carboxyl group, a group of the following formula: 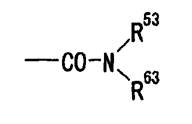 (wherein R⁵³ and R⁶³ each independently represents a hydrogen atom, an alkyl group having from 1 to 6 carbon atoms or an aryl group having from 6 to 10 carbon atoms),
an alkyl group having from 1 to 6 carbon atoms, an alkenyl group having from 2 to 6 carbon atoms, an alkynyl group having from 2 to 6 carbon atoms, an alkoxy group having from 1 to 6 carbon atoms, an alkylthio group having from 1 to 6 carbon atoms, an acyl group having from 2 to 5 carbon atoms, an alkoxycarbonyl group having from 2 to 5 carbon atoms, a cycloalkyl group having from 3 to 7 carbon atoms, a cycloalkenyl group having from 4 to 7 carbon atoms, an aryl group having from 6 to 10 carbon atoms, an aralkyl group having from 7 to 16 carbon atoms and a heteroaryl group (the heteroaryl group is a 5-membered or 6-membered ring and contains from 1 to 4 hetero atoms of one or more types selected from the group consisting of a nitrogen atom, an oxygen atom and a sulfur atom); and the amino group, the hydroxyl group and the mercapto group may be protected with a protective group.

Preferably, R³ is a hydrogen atom, a halogen atom, an alkyl group having from 1 to 6 carbon atoms or a cycloalkyl group having from 3 to 7 carbon atoms.

R³ may have substituents in the manner mentioned below.

When R³ is an alkyl group, an alkenyl group, an alkynyl group, an alkoxy group, an alkylthio group, an acyl group, an alkoxycarbonyl group, a cycloalkyl group, a cycloalkenyl group, an aryl group, an aralkyl group or a heteroaryl group, they may have one or more substituents of one or more types selected from the group consisting of an amino group, a hydroxyl group, a mercapto group, a halogen atom, an alkoxy group having from 1 to 6 carbon atoms, an alkylthio group having from 1 to 6 carbon atoms, an acyl group having from 2 to 5 carbon atoms and an alkoxycarbonyl group having from 2 to 5 carbon atoms; the amino group may have one or two substituents selected from the group consisting of a formyl group, an alkyl group having from 1 to 6 carbon atoms, a cycloalkyl group having from 1 to 6 carbon atoms, an aryl group having from 6 to 10 carbon atoms, a heteroaryl group (the heteroaryl group is a 5-membered or 6-membered ring and contains from 1 to 3 hetero atoms of one or more types selected from the group consisting of a nitrogen atom, an oxygen atom and a sulfur atom) , an acyl group having from 2 to 5 carbon atoms and an alkoxycarbonyl group having from 2 to 5 carbon atoms; and when the amino group has two substituents, they may bond to each other to form a cyclic structure.

Among these, when R³ is an alkyl group or an aryl group, the substituent which they may have is preferably one or more selected from a hydroxyl group, a halogen atom and an alkoxy group having from 1 to 6 carbon atoms.

R⁴ represents a hydrogen atom, a halogen atom, an amino group, a hydroxyl group, a nitro group, a cyano group, a carboxyl group, a sulfo group, a carbamoyl group, a group of the following formula: 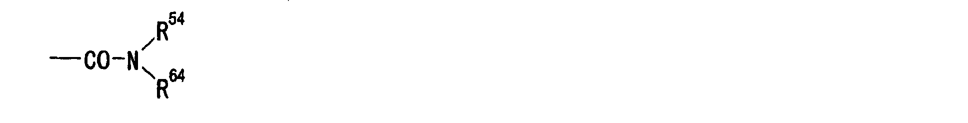 (wherein R⁵⁴ and R⁶⁴ each independently represents a hydrogen atom, an alkyl group having from 1 to 6 carbon atoms or an aryl group having from 6 to 10 carbon atoms),
an alkyl group having from 1 to 6 carbon atoms, an alkenyl group having from 2 to 6 carbon atoms, an alkynyl group having from 2 to 6 carbon atoms, an alkoxy group having from 1 to 6 carbon atoms, an acyl group having from 2 to 5 carbon atoms, an alkoxycarbonyl group having from 2 to 5 carbon atoms, an alkylcarbonyloxy group having from 1 to 6 carbon atoms, an alkyloxysulfonyl group having from 1 to 6 carbon atoms, a cycloalkyl group having from 3 to 6 carbon toms, an aryl group having from 6 to 10 carbon atoms or a heterocyclic groups (the heterocyclic group is a 5-membered or 6-membered ring and contains from 1 to 3 hetero atoms of one or more types selected from the group consisting of a nitrogen atom, an oxygen atom and a sulfur atom); and the amino group and the hydroxyl group may be protected with a protective group.

Preferably, R⁴ is a cyano group, a carboxyl group, a carbamoyl group, an alkyl group having from 1 to 6 carbon atoms, or an alkoxycarbonyl group having from 2 to 5 carbon atoms.

R⁴ may have substituents in the manner mentioned below.

When R⁴ is an alkyl group, an alkenyl group, an alkynyl group, an alkoxy group, an acyl group, an alkoxycarbonyl group, a cycloalkyl group, an aryl group or a heterocyclic group, they may have one or more substituents of one or more types selected from the group consisting of an amino group, a hydroxyl group, a mercapto group, a halogen atom, an alkoxy group having from 1 to 6 carbon atoms, an alkylthio group having from 1 to 6 carbon atoms, an acyl group having from 2 to 5 carbon atoms and an alkoxycarbonyl group having from 2 to 5 carbon atoms; the amino group may have one or two substituents selected from the group consisting of a formyl group, an alkyl group having from 1 to 6 carbon atoms, a cycloalkyl group having from 1 to 6 carbon atoms, an aryl group having from 6 to 10 carbon atoms, a heteroaryl group (the heteroaryl group is a 5-membered or 6-membered ring and contains from 1 to 3 hetero atoms of one or more types selected from the group consisting of a nitrogen atom, an oxygen atom and a sulfur atom), an acyl group having from 2 to 5 carbon atoms, an alkoxycarbonyl group having from 2 to 5 carbon atoms, an alkylsulfonyl group having from 1 to 6 carbon atoms and an arylsulfonyl group having from 6 to 10 carbon atoms; and when the amino group has two substituents, they may bond to each other to form a cyclic structure.

Among these, when R⁴ is an alkyl group, it preferably has a hydroxyl group as a substituent thereof.

Even though not specifically indicated, the aryl group, the heteroaryl group and the heterocyclic group in the description of the partial structure A and the substituents R¹, R², R³ and R⁴ may have one or more substituents selected from the group consisting of a halogen atom, an amino group, a hydroxyl group, a mercapto group, a nitro group, a cyano group, a carboxyl group, a sulfo group, a group of the following formula: 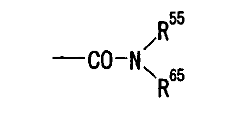 (wherein R⁵⁵ and R⁶⁵ each independently represents a hydrogen atom, an alkyl group having from 1 to 6 carbon atoms or an aryl group having from 6 to 10 carbon atoms),
an alkyl group having from 1 to 10 carbon atoms, an alkenyl group having from 2 to 10 carbon atoms, an alkynyl group having from 2 to 10 carbon atoms, an alkoxy group having from 1 to 10 carbon atoms, an alkylthio group having from 1 to 10 carbon atoms, a halogenoalkyl group having from 1 to 10 carbon atoms, an acyl group having from 2 to 10 carbon atoms, an alkoxycarbonyl group having from 2 to 10 carbon atoms, an aryl group having from 6 to 10 carbon atoms and a heteroaryl group (the heteroaryl group is a 5-membered or 6-membered ring and contains from 1 to 4 hetero atoms of one or more types selected from the group consisting of a nitrogen atom, an oxygen atom and a sulfur atom); the alkyl group, the alkoxy group, the alkylthio group, the acyl group, the alkoxycarbonyl group, the phenyl group and the heteroaryl group may have one or more substituents of one or more types selected from the group consisting of a halogen atom, a hydroxyl group, an alkoxy group having from 1 to 6 carbon atoms and an alkylthio group having from 1 to 6 carbon atoms; the amino group may have one or two substituents selected from the group consisting of a formyl group, an alkyl group having from 1 to 10 carbon atoms, an aminoalkyl group having from 1 to 8 carbon atoms, a hydroxyalkyl group having from 1 to 8 carbon atoms, a mercaptoalkyl group having from 1 to 8 carbon atoms, an acyl group having from 2 to 8 carbon atoms, an alkoxycarbonyl group having from 2 to 8 carbon atoms, a cycloalkyl group having from 3 to 10 carbon atoms, an aryl group having from 6 to 10 carbon atoms, an aralkyl group having from 7 to 16 carbon atoms, a monocyclic- or bicyclic-heterocyclic group having from 5 to 10 carbon atoms (containing from 1 to 4 hetero atoms of one or more types selected from the group consisting of a nitrogen atom, an oxygen atom and a sulfur atom), a heteroaryl group (the heteroaryl group is a 5-membered or 6-membered ring and contains from 1 to 4 hetero atoms of one or more types selected from the group consisting of a nitrogen atom, an oxygen atom and a sulfur atom), an alkylsulfonyl group having from 1 to 10 carbon atoms and an arylsulfonyl group having from 6 to 10 carbon atoms; when the amino group has two substituents, they may bond to each other to form a cyclic structure. Examples of the cyclic structure are mentioned below. 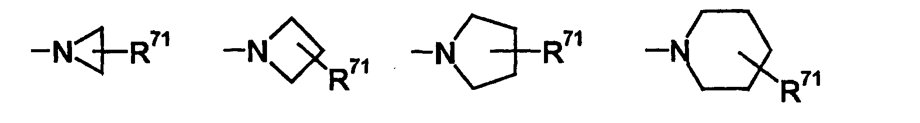 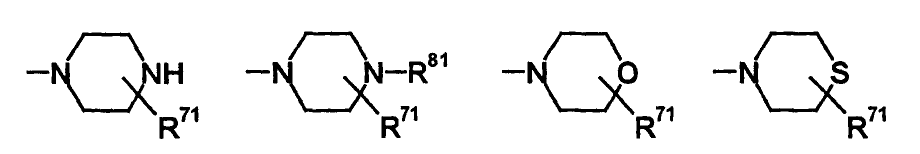 (In these formulae, R⁷¹ represents a hydrogen atom, a halogen atom, a hydroxyl group, a thiol group, an alkyl group having from 1 to 6 carbon atoms, an alkenyl group having from 2 to 6 carbon atoms, an alkynyl group having from 2 to 6 carbon atoms, an alkoxy group having from 1 to 6 carbon atoms, an alkylthio group having from 1 to 6 carbon atoms, a halogenoalkyl group having from 1 to 6 carbon atoms, a bicycloalkyl group having from 3 to 6 carbon atoms and optionally having a halogen atom, or a spirocycloalkyl group having from 3 to 6 carbon atoms and optionally having a halogen atom; R⁸¹ represents an alkyl group having from 1 to 6 carbon atoms or a halogenoalkyl group having from 1 to 6 carbon atoms.)

Even though not specifically indicated, the amino group in the description of the partial structure A and the substituents R¹, R², R³ and R⁴ may have one or two substituents selected from the group consisting of a formyl group, an alkyl group having from 1 to 6 carbon atoms, an acyl group having from 2 to 18 carbon atoms, an alkoxycarbonyl group having from 2 to 18 carbon atoms, an alkylsulfonyl group having from 1 to 18 carbon atoms and an arylsulfonyl group having from 6 to 10 carbon atoms; and when the amino group has two substituents, they may bond to each other to form a cyclic structure. Examples of the cyclic structure are mentioned below. 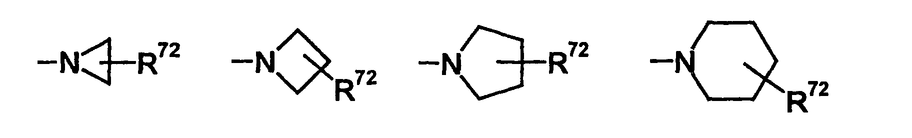 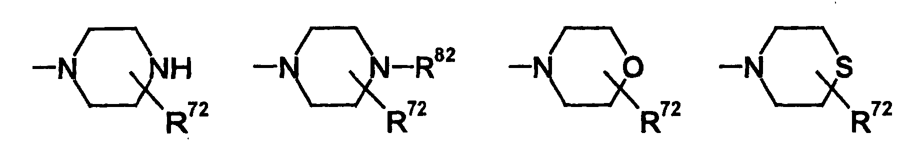 (In these formulae, R⁷² represents a hydrogen atom, a halogen atom, a hydroxyl group, a thiol group, an alkyl group having from 1 to 6 carbon atoms, an alkenyl group having from 2 to 6 carbon atoms, an alkynyl group having from 2 to 6 carbon atoms, an alkoxy group having from 1 to 6 carbon atoms, an alkylthio group having from 1 to 6 carbon atoms, a halogenoalkyl group having from 1 to 6 carbon atoms, a bicycloalkyl group having from 3 to 6 carbon atoms and optionally having a halogen atom, or a spirocycloalkyl group having from 3 to 6 carbon atoms and optionally having a halogen atom; R⁸² represents an alkyl group having from 1 to 6 carbon atoms or a halogenoalkyl group having from 1 to 6 carbon atoms.)

When the compound of formula (I) has a structure in which enantiomers are present, then each enantiomer thereof, a 1/1 racemic mixture of the enantiomers, and other enantiomer mixtures comprising the enantiomers in any desired ratio and having an optical purity of smaller than 100 % are all within the scope of the compound of the invention. When the compound of formula (I) is constituted to have diastereomers, then the single diastereomers and diastereomer mixtures are within the scope of the compound of the invention.

When the compound of formula (I) has a structure in which enantiomers are present, and when such a compound of the invention is administered to human and animals, then it is desirable to administer a compound which comprises a single enantiomer. The term "comprises a single enantiomer" as used herein means not only a case in which it is completely free from the other enantiomer but also a case in which it is in a chemically pure degree. In other word, it is interpretable that the other enantiomer may be present in such a degree that it does not exert influences upon physical constants and biological activities of the compound.

When the compound of formula (I) has a structure in which diastereomers are present, and when such a compound of the invention is administered to human and animals, then it is desirable to administer a compound which comprises a single diastereomer. The term "comprises a single diastereomer" as used herein means not only a case in which it is completely free from the other diastereomer but also a case in which it is in a chemically pure degree. In other word, it is interpretable that the other diastereomer may be present in such a degree that it does not exert influences upon physical constants and biological activities of the compound.

Also, the term "stereochemically pure" as used herein means that, when a compound or the like exists in an isomeric relationship due to the presence of asymmetric carbon atoms, the compound is comprised of only one of them. The term "pure" in thi s case can also be considered inthe same manner as described above.

When the compound of formula (I) is an acid derivative having a phenolic hydroxyl group, a carboxyl group (a carboxylic acid derivative) or a sulfo group (a sulfonic acid derivative) in any desired substituent moiety, then the acid derivative may be a free acid thereof, or may be formed into a salt of the phenolic hydroxyl group, the carboxyl group or the sulfo group.

The salt may be any of inorganic salts or organic salts, including, for example, alkali metal salts such as lithium salts, sodium salts and potassium salts; alkaline earth metal salts such as magnesium salts and calcium salts; ammonium salts; or triethylamine salts, N-methylglutamine salts, tris-(hydroxymethyl)aminomethane salts and the like. These free acid derivatives and their salts may form hydrates.

On the other hand, when the compound of formula (I) is a basic derivative having an amino group or an amine structure in any desired substituent moiety thereof, then the basic derivative maybe a free base thereof, or may form an acid addi tion salt thereof.

Examples of the acid addition salt include inorganic acid salts such as hydrochlorides, sulfates, nitrate, hydrobromides, hydroiodides and phosphates; and organic acid salts such as methanesulfonates, benzenesulfonates, para-toluenesulfonates (sulfonates), acetates, citrates, maleates, fumarates, lactates and tartrates (caboxylates).

These free base derivatives and their salts may form hydrates.

When the compound of formula (I) is a carboxylic acid compound, then the derivative whose the carboxylic acid moiety is an ester is useful as an intermediate for synthesis or as a prodrug. For example, alkyl esters, benzyl esters, alkoxyalkyl esters, phenylalkyl esters and phenyl esters are useful as an intermediate for synthesis.

When the carboxylic acid compound of the invention is used for antifungal purpose, the ester to be used as a prodrug is easily hydrolyzed in living bodies to form its free carboxylic acid. The ester of the type includes, for example, acetoxymethyl esters, pivaloyloxymethyl esters, ethoxycarbonyl esters, choline esters, dimethylaminoethyl esters, 5-indanyl esters and phthalyzinyl esters, as well as oxoalkyl esters such as 5-alkyl-2-oxo-1,3-dioxol-4-ylmethyl esters and 3-acetoxy-2-oxobutyl esters.

When the compound of formula (I) is an amino group-having basic compound, the derivative, in which an amino acid, dipeptide or tripeptide bonds to the amino group, is useful as a prodrug.

The amino acid, dipeptide and tripeptide used for the prodrug are such that the peptide bond to be formed by the carboxyl group therein and the amino group in the compound of formula (I) of the invention is easily cleaved in living bodies to form a free amine. For example, they include amino acids such as glycine, alanine, aspartic acid; dipeptides such as glycine-glycine, glycine-alanine, alanine-alanine; and tripeptides such as glycine-glycine-alanine, glycine-alanine-alanine.

The compound of formula (I) can be produced in various methods. Some preferred and typical examples of the production methods are mentioned below, to which, however, the invention should not be limited. During reaction, the substituents may be optionally protected with a protective group, and the order of converting the substituents (functional groups) is not specifically defined. 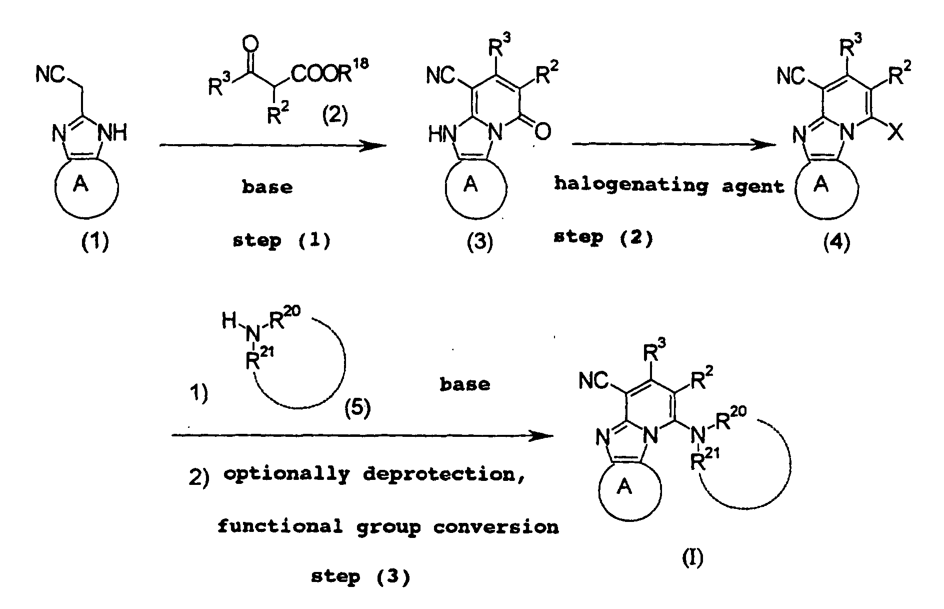 (In the formulae, A, R² and R³ have the same meanings as above; R¹⁸ represents a lower alkyl group such as a methyl group or an ethyl group; X represents a halogen atom; R²⁰ and R²¹ are the same as those mentioned hereinbefore for the substituent that the amino group for R¹ may have.)

Step (1) is the process to prepare compound (3) by condensation and ring-closure in treating compound (1), 2-imidazolylacetonitrile derivative and compound (2), β-ketoester derivative.

The reaction may be carried out using a solvent or without using a solvent. The solvent for use in the reaction may be any solvent that is inert under the reaction condition, and it examples includes chloroform, dichloroethane, dimethylformamide, dimethylacetamide, dimethylsulfoxide, benzene, toluene, chlorobenzene, dichlorobenzene, diethyl ether, tetrahydrofuran, 1,4-dioxane, diphenyl ether, or a mixture thereof. When both or one of the starting materials, compound (1) and compound (2) are liquid, then the reaction of the two is preferably carried out without using a solvent.

Preferably, the reaction is carried out in the presence of an acid receptor such as an inorganic base or an organic base, and it examples include inorganic basic compounds such as acetates, carbonates or hydrogencarbonates with alkali metals, alkaline earth metals or ammonia; or organic basic compounds such as triethylamine, pyridine, 1,8-diazabicycloundecene, N-methylpiperidine, N,N-diisopropylethylamine. Of those, preferably used is ammonium acetate. However, it is desirable that the base to be used is suitably changed depending on the reactivity of the reactants.

The reaction can be carried out generally at a temperature of from room temperature to 200°C. However, when a solvent is used, the temperature preferably is between 25°C and the reflux temperature of the system; and when a solvent is not used, the temperature preferably is between 80°C and 150°C. The reaction is carried out for a period of from 15 minutes to 48 hours and completes generally in about 30 minutes to 6 hours.

Step (2) is the process to prepare compound (4) by treating compound (3) with a halogenating agent. In this step, the carbonyl group moiety of the amido structure of the compound (3) is halogenated and then dehydrated to give the compound (4).

The reaction may be carried out using a solvent or without using a solvent. The solvent for use in the reaction may be any solvent that is inert under the reaction condition, and its examples includes chloroform, dichloroethane, dimethylformamide, dimethylacetamide, dimethylsulfoxide, benzene, toluene, chlorobenzene, dichlorobenzene, diethyl ether, tetrahydrofuran, 1,4-dioxane, diphenyl ether, or a mixture thereof. When the halogenating agent is a solution, then it is desirable that a solvent is not used but an excess halogenating agent is used so that it may serve as a solvent for the reaction.

The halogenating agent may be any one generally used for alcohol halogenation, though not specifically limited thereto. For example, it includes thionyl halides such as thionyl chloride, thionyl bromide and thionyl iodide; sulfuryl halides such as sulfuryl chloride, sulfuryl bromide and sulfuryl iodide; phosphorus trihalides such as phosphorus trichloride, phosphorus tribromide and phosphorus triiodide; phosphorus pentahalides such as phosphorus pentachloride, phosphorus pentabromide and phosphorus pentaiodide; phosphorus oxyhalides such as phosphorus oxychloride, phosphorus oxybromide and phosphorus oxyiodide; dialkylaminosulfite fluorides of the following formula:

(R³⁵)(R³⁶)NSF₃

(wherein R³⁵ and R³⁶ may be the same or different, and each represents an alkyl group having from 1 to 6 carbon atoms, or they may combine to from an alkylene group having from 2 to 6 carbon atoms and optionally including an oxygen atom),
and fluorinating agents such as CF₃CHFCF₂N (C₂H₅)₂, or CF₃CF=CFN(C₂H₅)₂. Preferably, it is a chlorinating agent such as phosphorus oxychloride.

The reaction temperature is generally between room temperature and 200°C. However, when a solvent is used or the halogenating agent used is liquid and serves also as a solvent, then the temperature preferably falls between 25°C and a reflux temperature of the system, more preferably between 50°C and 150°C. The reaction is carried out for a period of from 15 minutes to 48 hours and completes generally in about 30 minutes to 2 hours.

Step (3) is the process to prepare the compound of formula (I) of the invention by treating the compound (4) with amine derivative (5). In this step, the compound (I) of the invention is formed through nucleophilic substitutive reaction of the amine derivative (5) on the aromatic halogen compound (4).

The reaction may be carried out using a solvent or without using a solvent. The solvent for use in the reaction may be any one that is inert under the reaction condition, and its examples includes dimethylsulfoxide, pyridine, acetonitrile, ethanol, chloroform, dimethylformamide, dimethylacetamide, N-methylpyrrolidone, tetrahydrofuran, water, 3-methoxybutanol, or a mixture thereof.

Preferably, the reaction is carried out in the presence of an acid receptor such as an inorganic base or an organic base, for example, alkali metal or alkaline earth metal carbonates or hydrogencarbonates, or organic basic compounds such as triethylamine, pyridine, 1,8-diazabicycloundecene, N-methylpiperidine and N,N-diisopropylethylamine.

The reaction temperature generally is between room temperature and 200°C, preferably between 25 and 150°C. The reaction is carried out for a period of from 30 minutes to 48 hours and completes generally in about 30 minutes to 18 hours.

When the amine derivative (5) to be used in the reaction has an amino group, a hydroxyl group or a thiol group, then it may be protected with a suitable protective group at the substituent thereof. When deprotection is necessary after the reaction, then the protective group may be removed under a suitable condition for it to give the compound of formula (I) of the invention.

As the protective group, it may be any protective group generally used in this technical field, and its example include alkoxycarbonyl groups such as tertiary-butoxycarbonyl group, 2,2,2-trichloroethoxycarbonyl group; aralkyloxycarbonyl groups such as benzyloxycarbonyl group, para-methoxybenzyloxycarbonyl group, para-nitrobenzyloxycarbonyl group; acyl groups such as acetyl group, methoxyacetyl group, trifluoroacetyl group, chloroacetyl group, pivaloyl group, formyl group, benzoyl group; alkyl groups and aralkyl groups such as tertiary-butyl group, benzyl group, paranitrobenzyl group, paramethoxybenzyl group, triphenylmethyl group; ethers such as methoxymethyl group, tertiary-butoxymethyl group, tetrahydropyranyl group, 2,2,2-trichloroethoxymethyl group; and silyl groups such as trimethylsilyl group, isopropyldimethylsilyl group, tertiary-butyldimethylsilyl group, tribenzylsilyl group, tertiary-butyldiphenylsilyl group.

After the reaction is completed, the intended compound in the step (3) may be taken out of the reaction mixture in any ordinary method. For example, after the reaction is completed, a suitable solvent is added to the mixture to precipitate the intended compound, and this is collected through filtration; or water is added to the reaction mixture, and a solvent not miscible with water but capable of dissolving the intended compound is added thereto and the intended compound is extracted, and the extracted organic layer is suitably washed with water, then dried over anhydrous sodium sulfate or anhydrous magnesium sulfate, and thereafter the solvent is evaporated to obtain the intended compound.

In the above-mentioned step (1), when malonate derivative (10) is used in place of the β-ketoester derivative (2), then condensed-cyclized compound (11) may be obtained from 2-imidazolyl acetate derivative (1'), like from the 2-imidazolylacetonitrile derivative (1), according to the method reported in a references (e.g., J. Heterocyclic Chem., 25, 1087 (1988)). The subsequent process of halogenation and nucleophilic substitutive reaction of the resulting aromatic halogenated compound (12) with the amine derivative (5) may be carried out in the same manner as the production method mentioned above to give compound (13) of the invention. 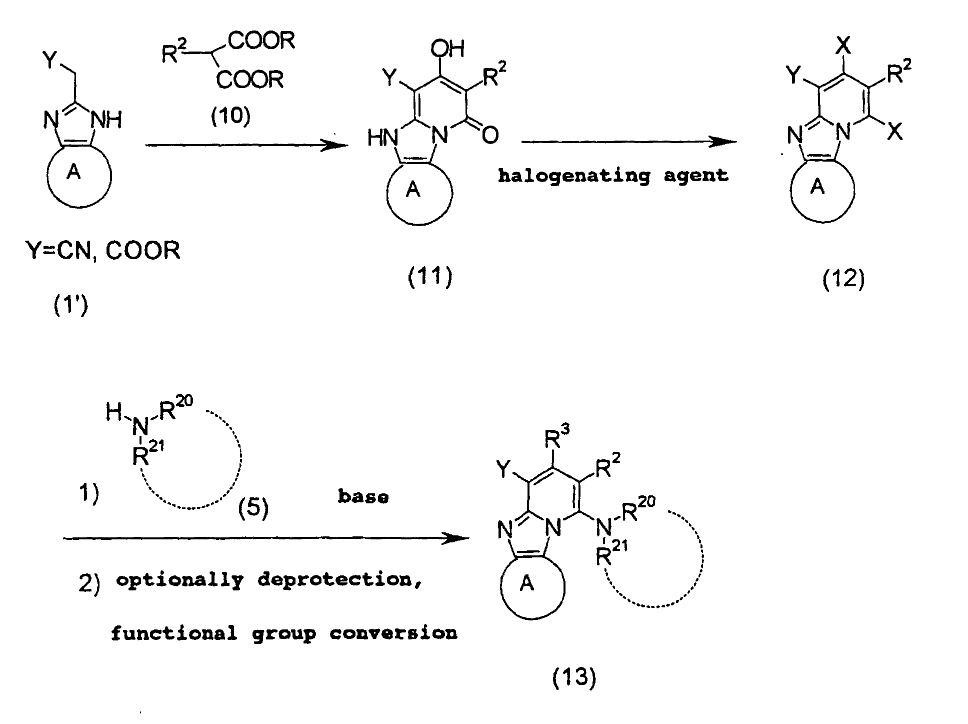 When R¹ in the compound of formula (I) is an aryl group, a heteroaryl group, a cycloalkenyl group, an alkenyl group, a cycloalkyl group, or an alkyl group, then compound (9) may be obtained according to the method reported in references (e.g., J. Heterocyclic Chem., 28, 191 (1991); Chem. Rev., 95, 2457 (1995). Namely, theintented product can be prepared from the compound which is prepared by treating compound (4) with tin compound (7) in the presence of a palladium catalyst such as tetrakis(triphenylphosphine)palladium or dichlorobis(triphenylphosphine)palladium (Stille coupling); or with boron compound (8) (Suzuki coupling).

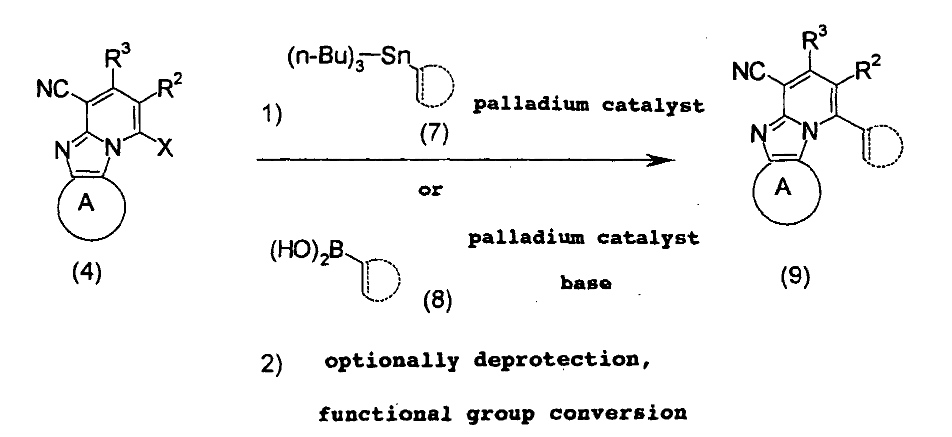

Further, when R¹ is a cycloalkyl group, an alkyl group, an aryl group, a heteroaryl group, a cycloalkenyl group, or an alkenyl group, then 1,3-diketone derivative (14) may be used in place of the β-ketoester derivative (2) in the above-mentioned step (1) also to give condensed-cyclized compound (15), like from the β-ketoester derivative (2). After this step, the substituent having been introduced into the 1-position of the compound may be, if necessary, deprotected or the functional group may be converted to give compound (16) of the invention.

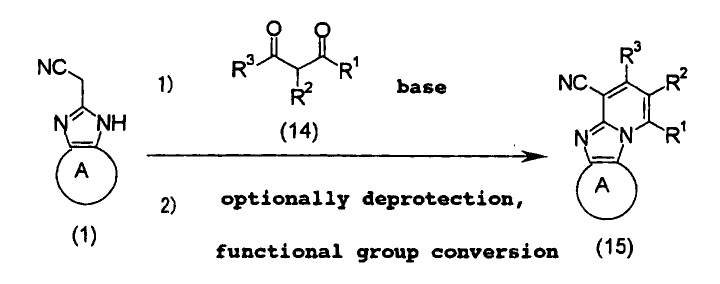

Further, the intended compounds thus obtained in the manner as above may be purified, if necessary, in an ordinary method, for example, through recrystallization, reprecipitation, chromatography or the like.

The compound of the invention specifically (selectively) exhibits an antifungal activity, not exhibiting an antibacterial activity or an anticancer activity, and is active to a broad range of fungi that cause various fungal infectious diseases. Therefore, the compound may be sued for treating, preventing or reducing the diseases caused by such pathogens.

Examples of fungi to which the compound of the invention is effective include those of the genus *Candida* such as *Candida glabrata, Candida krusei, Candida tropicalis;* the genus *Cryptococcus* such as *Cryptococcus neoformans;* the genus *Aspergillus* such as *Aspergillus fumigatus, Aspergillus flavus*; *Pneumocystis carinii;* the genus *Rhisopus;* the genus *Absidia*; the genus *Histoplasma* such as *Histoplasma capsulatum;* the genus *Coccidioides* such as *Coccidioides immitis*; the genus *Blastomyces;* the genus *Paracoccidioides* such as *Paracoccidioides brasiliensis*; the genus *Penicilium*; the genus Pseudallescheria; the genus *Sporothrix;* the genus *Dematiaceous;* the genus *Tricophiton;* the genus *Microsporum;* the genus *Epidermophyton;* the genus *Malassezia;* the genus *Fusarium;* the genus *Trichosporon* such as *Trichosporon cutaneum*; the genus *Hyalohora;* and the genus *Cladosporium.* In addition, further mentioned are *Saccharomyces cerevisiae, Candida albicans; Candida glabrata, Candida krusei, Candida tropicalis, Cryptococcus neoformans, Trichosporon cutaneum*, and *Aspergillus fumigatus*.

The diseases to be caused by these pathogens include internal organ mycosis (deep-seated mycosis) such as candidosis, cryptococosis, aspergillosis, actinomycosis, nocardiosis, mucormycosis, geotrichosis, histoplasmosis, coccidiosis, paracoccidiosis, blastomicosis and penicilliosis, specifically hematomyelia, respiratory system mycosis, digestive system mycosis, urinary tract mycosis, mycotic meningitis; subcutaneous mycosis such as sporotricosis, chromomycosis, mycetoma; and superficial mycosis such as conventional trichophytosis, deep-seated trichophytosis, intractable trichophytosis, nail trichophotosis, tinea versicolor, dermato-candidosis, oral cavity candidosis.

In addition, the compound of the invention is effective also against various fungi that cause fungal infectious diseases in animals.

Based on the antifungal effect against pathogenic fungi thereof, the compound of the invention, its salts and solvates thereof are applicable tomedicines, infectious disease treating agents and antifungal agents as well as medicines for animals and fish, and antifungal preservatives.

The compound of the invention, its salts or solvates thereof may be used for producing medicines, infectious disease treating agents and antifungal agents that contain it. For example, the compound of the invention, its salts or solvates thereof may be used for producing injections that are provided in the form of solutions and for producing liquid preparations. Optionally combined with suitable additives added thereto, the compound of the invention, its salts or solvates thereof may be formulated intomedicines, infectious disease treating agents or antifungal agents in an ordinary method of producing pharmaceutical preparations.

Regarding the form of the antifungal agents that contain any of the compound of the invention, its salt or solvate thereof, for example, there are mentioned oral preparations such as tablets, powders, granules, capsules, solutions, syrups, elixirs, oily or aqueous suspensions.

Injections may contain a stabilizer, a preservative or a dissolution aid, and the solution that contains the auxiliary additives may be put in containers and then freeze-dried into solid preparations, which may be re-formulated into actual preparations before use.

Examples of external applications include solutions, suspensions, emulsions, ointments, gels, creams, lotions, and sprays.

Solid preparations may contain, along with the compound of the invention, its salts or solvates thereof, any pharmaceutically-acceptable additive. For example, the additive includes fillers, vehicles, binders, disintegrators, dissolution promoters, moisturizers, lubricants. These may be suitably selected and mixed with the active ingredient in formulating the preparations.

Liquid preparations include solutions, suspensions and emulsions, to which an additive of a suspending agent and an emulsifier may be added.

For administrating the compound of the invention, its salts or solvates thereof to animals, for example, it may be orally administered thereto directly or after mixed in feed; or after the compound or the like has been formed into a solution thereof, and it may be added to drinking water or feed so as to be orally administered to animals; or the solution may be directly administered to them through injection.

The preparations that contain the compound of the invention, its salts or solvates thereof for administration to animals may be produced according to an ordinary technique known in the art, for example, as powders, granules, soluble powders, syrups, solutions or injections.

Examples of formulation are mentioned below.

| Formulation Example 1 (Capsules): | |
|---|---|
| Compound of Example 1 | 100.0 mg |
| Corn starch | 23.0 mg |
| CMC calcium | 22.5 mg |
| Hydroxymethyl cellulose | 3.0 mg |
| Magnesium stearate | 1.5 mg |
| Total | 150.0 mg |

| Formulation Example 2 (Solutions): | |
|---|---|
| Compound of Example 1 | 1 to 10 g |
| Acetic acid or sodium hydroxide | 0.5 to 2 g |
| Ethyl para-hydroxybenzoate | 0.1 g |
| Pure Water | 88.9 to 98.4 g |
| Total | 100 g |

| Formulation Example 3 (Powdery additive to feed): | |
|---|---|
| Compound of Example 1 | 1 to 10 g |
| Corn starch | 98.5 to 89.5 g |
| Light silicic anhydride | 0.5 g |
| Total | 100 g |

Themethod for administering the medicine of the invention, the dose thereof and the frequency in administering the medicine are not specifically limited, and theymaybe suitably determined depending on various conditions including the type of the pathogenic fungi to be killed by the medicine, the age, the body weight and the condition of the cases to which the medicine is applied. In ordinary oral or parenteral (injection, drip) administration toadults, the dose maybe from 0.1 to 100mg/kg/day, and it may be administered all at once or in multiple times after divided.

### BEST MODE FOR CARRYING OUT THE INVENTION

The invention is described with reference to Examples and Reference Examples,though the invention is not limited thereto.

### [Reference Example 1]

### 2-Ethyl-3-methyl-1-oxo-1H,5H-pyrido[1,2-a]benzimidazole-4-carbonitrile:

A mixture of 10.0 g (63.6 mmol) of (2-benzimidazolyl)acetonitrile, 10.1 g (63.6 mmol) of ethyl 2-ethylacetoacetate and 9.80 g (127 mmol) of ammonium acetate was heated at 140 to 150°C for 25 minutes. After cooling, 200 ml of water was added thereto, then the solid was crushed and decanted, and 100 ml of acetonitrile was added thereto and the solid was washed. The crystal was taken out through filtration to obtain 13.3 g (83 %) of the entitled compound as a pale brown crystal.
MS(EI)m/z:252(M⁺).
¹H-NMR(400MHz, CDCl₃)δ: 1.16(3H, t, J=7.57Hz), 2.50(3H, s), 2.70-2.78(2H, m), 7.36-7.40(1H, m), 7.42-7.52(2H, m), 8.79(1H, d, J=8.30Hz).
IR(ATR) : 2206, 1653, 1628, 1572, 1523, 1460, 1363, 1273, 1201, 1066 cm⁻¹.

### [Reference Example 2]

### 1-Chloro-2-ethyl-3-methylpyrido[1,2-a]benzimidazole-4-carbonitrile:

8.21 g (32.7 mmol) of 2-ethyl-3-methyl-1-oxo-1H,5H-pyrido[1,2-a]benzimidazole-4-carbonitrile was heated under reflux in 40 ml of phosphoryl chloride for 2 hours. After cooling, phosphoryl chloride was evaporated under reduced pressure, and 50 ml of ice-water was added to the residue. The mixture was extracted with chloroform (100 ml × 3). The chloroform layers were combined and dried over anhydrous magnesium sulfate, and the solvent was evaporated under reduced pressure. The resulting residue was washed with diisopropyl ether and taken out through filtration to obtain 7.98 g (90 %) of the entitled compound as a yellow crystal.
MS (EI) m/z : 270 (M+H)⁺.
¹H-NMR(400MHz, CDCl₃)δ: 1.26(3H, t, J=7.57Hz), 2.74(3H, s), 2.92(2H, q, J=7.57Hz), 7.37-7.43(1H, m), 7.56-7.63(1H, m), 8.03(1H, d, J=8.30Hz), 8.58-8.63(1H, m).
IR(ATR): 2225, 1622, 1591, 1469, 1437, 1354, 1304, 1120, 1049 cm⁻¹.

### [Example 1]

### 1-(2-N',N'-Diethylaminoethylamino)-2-ethyl-3-methylpyrido[1,2-a]benzimidazole-4-carbonitrile:

To N,N-dimethylformamide (60 ml) suspension of 3.00 g (10.4 mmol) of 1-chloro-2-ethyl-3-methylpyrido[1,2-a]-benzimidazole-4-carbonitrile was added 2.92 ml (20.8 mmol) of N,N-diethylaminoethylamine. The system was replaced with nitrogen and sealed up, and the mixture was heated at 80°C for 3 hours. After cooling, the solvent was evaporated under reduced pressure, and the residue was dissolved in 100 ml of ethyl acetate. This soution was washed with 50 ml of saturated sodiumbicarbonate solution, and dried over anhydrous magnesium sulfate. The solvent was evaporated under reduced pressure, and the residue was applied to a silica gel column chromatography. This was elutedwith a mixed solvent of dichloromethane/methanol (100/1, v/v) to obtain a crude product of the entitled compound. This was recrystallized from ethanol to obtain 1.62 g (45 %) of the entitled compound as a yellow crystal.
MS(EI)m/z:350(M⁺).
¹H-NMR(400MHz, CDCl₃)δ: 1.13(6H, t, J=7.07Hz), 1.23(3H, t, J=7.56Hz), 2.61-2.72(4H, m), 2.67(3H, s), 2.74-2.85(4H, m), 3.14-3.24(2H, m), 5.40-5.48(1H, m), 7.28-7.34(1H, m), 7.47-7.55(1H, m), 7.96-8.00(1H, m), 8.17(1H, d, J=8.53Hz).
IR(ATR): 2216, 1624, 1593, 1495, 1442, 1369, 1313, 1068 cm⁻¹.

### [Example 2]

### 1-(1-N',N'-Diethylaminoethylamino)-2-ethyl-3-methylpyrido[1,2-a]benzimidazole-4-carbamide:

To an ethanol (750 µl) solution of 70 mg (200 µmol) of 1-(2-N',N'-diethylaminoethylamino)-2-ethyl-3-methylpyrido[1,2-a]benzimidazole-4-carbonitrile was added 84 µl of concentrated sulfuric acid at 0°C, and heated under reflux for 15 hours. After cooling, 10 ml of aqueous 1 N sodium hydroxide solution was added thereto, and the mixture was extracted with ethyl acetate (10 ml × 3). The ethyl acetate layers were combined, and dried over anhydrous magnesium sulfate, and the solvent was evaporated under reduced pressure, and the residue was applied to a silica gel column chromatography. This was eluted with amixedsolventofdichloromethane/methanol (20/1, v/v) toobtain a crude product of the entitled compound. This was washed with diethyl ether and taken out through filtration to obtain 12 mg (16 %) of the entitled compound as a pale yellow crystal.
MS(EI)m/z:368(M⁺).
¹H-NMR(400MHz, CDCl₃)δ: 1.13(6H, t, J=7.08Hz), 1.24(3H, t, J=7.57Hz), 2.58-2.98(11H, m) , 3.05-3.25(2H, m), 5.14 (1H, brs), 5.90(1H, brs), 7.20-7.42(1H, m), 7.97-7.57(1H, m) , 7.84(1H, d, J=8.06Hz), 8.27(1H, d, J=8.30Hz), 10.05(1H, brs).
IR(ATR): 1668, 1595, 1514, 1477, 1450 cm⁻¹.

| | | | |
|---|---|---|---|
| Elemental analysis: C₂₁H₂₉N₅O·0.25H₂O | | | |
| Calcd. | C, 67.80%; | H, 7.99%; | N, 18.83% |
| Found | C, 68.00%; | H, 7.90%; | N, 18.75%. |

### [Reference Example 3]

### 2-n-Butyl-3-methyl-1-oxo-1H,5H-pyrido[1,2-a]benzimidazole-4-carbonitrile:

A mixture of 1.26 g (8.00 mmol) of (2-benzimidazolyl)acetonitrile, 1.49 g (8.00 mmol) of ethyl 2-n-butylacetoacetate and 1.23 g (16.0mmol) of ammonium acetate was heated at 140 to 150°C for 40 minutes. After cooling, 50 ml of water was added thereto, then the solid was crushed and decanted, and 30 ml of acetonitrile was added thereto and the solid was washed. The crystal was taken out through filtration to obtain 2.00 g (90 %) of the entitled compound as a pale brown crystal.
MS(EI)m/z:280(M⁺).
¹H-NMR(400MHz, CDCl₃)δ: 0.96(3H, t, J=7.08Hz), 1.38-1.58(4H, m), 2.49(3H, s), 2.64-2.74(2H, m), 7.32-7.40(1H, m), 7.43-7.52(2H, m), 8.78(1H, d, J=8.04Hz).
IR(ATR): 2208, 1662, 1612, 1549, 1485, 1466 cm⁻¹.

### [Reference Example 4]

### 2-n-Butyl-1-chloro-3-methylpyrido[1,2-a]benzimidazole-4-carbonitrile:

1.50 g (5.37 mmol) of 2-n-butyl-3-methyl-1-oxo-1H,5H-pyrido[1,2-a]benzimidazole-4-carbonitrile was heated under reflux in 7 ml of phosphoryl chloride for 2.5 hours. After cooling, phosphoryl chloride was evaporated under reduced pressure, and 50 ml of ice-water and 50 ml of aqueous 1 N sodium hydroxide solution were added to the residue. The mixture was extracted with chloroform (100 ml × 3). The chloroform layers were combined and dried over anhydrous magnesium sulfate, and the solvent was evaporated under reduced pressure. The resulting residue was washed with diisopropyl ether and taken out through filtration to obtain 1.29 g (80 %) of the entitled compound as a yellow crystal.
MS(EI)m/z:298(M+H)⁺.
¹ H-NMR(400MHz, DMSO-d₆)δ: 0.95(3H, t, J=7.08Hz), 1.38-1.58(4H, m), 2.67(3H, s), 2.76-2.87(2H, m), 7.40-7.49(1H, m), 7.57-7.66(1H, m), 7.88-7.96(1H, m), 8.64-8.72(1H, m).
IR(ATR): 2223, 1624, 1591, 1469, 1354, 1306, 1200 cm⁻¹.

### [Reference Example 5]

### 2-n-Hexyl-3-methyl-1-oxo-1H,5H-pyrido[1,2-a]benzimidazole-4-carbonitrile:

A mixture of 1.26 g (8.00 mmol) of (2-benzimidazolyl)acetonitrile, 1.71 g (8.00 mmol) of ethyl 2-n-hexylacetoacetate and 1. 23 g (16.0 mmol) of ammonium acetate was heated at 140 to 150°C for 40 minutes. After cooling, 50 ml of water was added thereto, then the solid was crushed and decanted, and 30 ml of acetonitrile was added thereto and the mixture was washed. The crystal was taken out through filtration to obtain 1.87 g (76 %) of the entitled compound as a pale brown crystal.
MS(EI)m/z:308(M⁺).
¹H-NMR(400MHz, DMSO-d₆)δ: 0.77-0.98(3H, m), 1.20-1.50(8H, m), 2.35(3H, s), 2.48-2.61 (2H, m), 7.27-7.39(1H, m), 7.42-7.60(2H, m), 8.52-8.62(1H, m).
IR(ATR): 2208, 1653, 1520, 1460 cm⁻¹.

### [Reference Example 6]

### 1-Chloro-2-n-hexyl-3-methylpyrido[1,2-a]benzimidazole-4-carbonitrile:

1.50 g (4.88 mmol) of 2-n-hexyl-3-methyl-1-oxo-1H,5H-pyrido[1,2-a]benzimidazole-4-carbonitrile was heated under reflux in 7 ml of phosphoryl chloride for 2 hours. After cooling, phosphoryl chloride was evaporated under reduced pressure, and 50 ml of ice-water was added to the residue. The mixture was was extracted with chloroform (100ml × 3). The chloroform layers were combined and dried over anhydrous magnesium sulfate, and the solvent was evaporated under reduced pressure. The resulting residue was washed with diisopropyl ether and taken out through filtration to obtain 1.36 g (86 %) of the entitled compound as a yellow crystal.
MS(EI)m/z:326(M+H)⁺.
¹H-NMR(400MHz, DMSO-d₆)δ: 0.82-0.93(3H, m), 1.25-1.59(8H, m), 2.67(3H, s), 2.77-2.86(2H, m), 7.40-7.47(1H, m), 7.57-7.65(1H, m), 7.91(1H, d, J=8.04Hz), 8.67(1H, d, J=8.53Hz).
IR(ATR): 2225, 1624, 1591, 1529, 1469, 1356, 1306, 1194 cm⁻¹.

### [Reference Example 7]

### 3-Methyl-1-oxo-2-phenyl-1H,5H-pyrido[1,2-a]benzimidazole-4-carbonitrile:

A mixture of 1.26 g (8.00 mmol) of (2-benzimidazolyl)acetonitrile, 1.65 g (8.00 mmol) of ethyl 2-phenylacetoacetate and 1.23 g (16. 0 mmol) of ammonium acetate was heated at 140 to 150°C for 40 minutes. After cooling, 50 ml of water was added thereto, then the solid was crushed and decanted, and 30 ml of acetonitrile was added thereto and the solid was washed. The crystal was taken out through filtration to obtain 2.03 g (85 %) of the entitled compound as a pale brown crystal.
MS(EI)m/z:300(M⁺).
¹H-NMR(400MHz, DMSO-d₆)δ: 2.15(3H, s), 6.94-7.01(1H, m), 7.20-7.28(4H, m), 7.31-7.39(2H, m), 7.45(1H, d, J=8.04Hz), 8.43(1H, d, J=8.04Hz).
IR(ATR): 2206, 1645, 1591, 1514, 1466 cm⁻¹.

### [Reference Example 8]

### 1-Chloro-3-methyl-2-phenylpyrido[1,2-a]benzimidazole-4-carbonitrile:

1.50 g (5.01 mmol) of 3-methyl-1-oxo-2-phenyl-1H,5H-pyrido[1,2-a]benzimidazole-4-carbonitrile was heated under reflux in 7 ml of phosphoryl chloride for 2.5 hours. After cooling, phosphoryl chloride was evaporated under reduced pressure, and 50 ml of ice-water and 50 ml of aqueous 1 N sodium hydroxide solution were added to the residue. The mixture was was extractedwith chloroform (100ml × 3). The chloroform layers were combined and dried over anhydrous magnesium sulfate, and the solvent was evaporated under reduced pressure. The resulting residue was washed with diisopropyl ether and taken out through filtration to obtain 1.02 g (64 %) of the entitled compound as a yellow crystal.
MS(EI)m/z:318(M+H)⁺.
¹H-NMR(400MHz, DMSO-d₆)δ: 2.31 (3H, s), 7.39-7.60(6H, m), 7.61-7.68(1H, m), 7.98(1H, d, J=8.55Hz) , 8.65(1H, d, J=8.30Hz).
IR(ATR) : 2225, 1666, 1626, 1593, 1533, 1466, 1379, 1346, 1309, 1219, 1178 cm⁻¹.

### [Reference Example 9]

### 2-Benzyl-3-methyl-1-oxo-1H,5H-pyrido[1,2-a]ben2imidazole-4-carbonitrile:

A mixture of 1.26 g (8.00 mmol) of (2-benzimidazolyl)acetonitrile, 1.76 g (8.00 mmol) of ethyl 2-benzylacetoacetate and 1.23 g (16.0 mmol) of ammonium acetate was heated at 140 to 150°C for 40 minutes. After cooling, 50 ml of water was added thereto, then the solid was crushed and decanted, and 30 ml of acetonitrile was added thereto and the solid was washed. The crystal was taken out through filtration to obtain 2.43 g (97 %) of the entitled compound as a pale brown crystal.
MS(EI)m/z:314(M⁺).
¹H-NMR(400MHz, DMSO-d₆)δ: 2.33(3H, s), 3.96(2H, s), 7.08-7.15(1H, m), 7.18-7.30(5H, m), 7.41-7.47(1H, m), 7.50(1H, d, J=7.80Hz), 8.57(1H, d, J=8.04Hz).
IR(ATR): 2206, 1664, 1614, 1549, 1485, 1466 cm⁻¹.

### [Reference Example 10]

### 2-Benzyl-1-chloro-3-methylpyrido[1,2-a]benzimidazole-4-carbonitrile:

1.50 g (4.79 mmol) of 2-benzyl-3-methyl-1-oxo-1H,5H-pyrido[1,2-a]benzimidazole-4-carbonitrile was heated under reflux in 7 ml of phosphoryl chloride for 3 hours. After cooling, phosphoryl chloride was evaporated under reduced pressure, and 50 ml of ice-water and 50 ml of aqueous 1 N sodium hydroxide solution were added to the residue. The mixture was extracted with chloroform (100ml × 3). The chloroform layers were combined and dried over anhydrous magnesium sulfate, and the solvent was evaporated under reduced pressure. The resulting residue was washed with diisopropyl ether and taken out through filtration to obtain 868 mg (55 %) of the entitled compound (I-10) as a yellow crystal.
MS (EI)m/z:332 (M+H)⁺.
¹H-NMR(400MHz, DMSO-d₆)δ: 2.54(3H, s), 4.33(2H, s), 7.17-7.32(5H, m), 7.43-7.50(1H, m), 7.60-7.68(1H, m), 7.95(1H, d, J=8.30Hz), 8.71(1H, d, J=8.79Hz).
IR(ATR): 2224, 1626, 1593, 1512, 1475, 1446, 1356, 1304, 1227, 1201 cm⁻¹.

### [Reference Example 11]

### Ethyl 2-benzoylbutyrate:

1,2-Dimethoxyethane (30ml) solution of 2.00 g (17.2 mmol) of ethyl butyrate was addeddropwise to 1,2-dimethoxyethane (120 ml) suspension of 1.03 g (25.8 mmol) of sodium hydride under nitrogen atmosphere at 0°C and the resulting mixture was stirred at room temperature for 75 minutes, and then 1,2-dimethoxyethane (30 ml) solution of 5.17 g (34.4 mmol) of ethyl benzoate was added dropwise thereto at 0°C and the mixture was heated under reflux for 4 hours. After cooling, 50 ml of water and 50 ml of saturated ammonium chloride solution were added thereto, and the mixture was extracted with ethyl acetate (100 ml × 3). The ethyl acetate layers were combined and dried over anhydrous magnesium sulfate, and the solvent was evaporated under reduced pressure. The residue was applied to a column chromatography. This was eluted with a mixed solvent of n-hexane/ethyl acetate (30/1, v/v) to obtain 1.83 g (48 %) of the entitled compound as a colorless oil.
MS(EI)m/z:221(M⁺).
¹H-NMR(400MHz, CDCl₃)δ: 0.99(3H, t, J=7.31Hz), 1.17(3H, t, J=7.07Hz), 1.99-2.17(2H, m) , 4.08-4.29(3H, m) , 7.42-7.67(3H, m), 7.96-8.08(2H, m).
IR(ATR): 2974, 1732, 1684, 1597, 1448, 1281, 1255, 1215, 1182, 1157 cm⁻¹.

### [Reference Example 12]

### 2-Ethyl-1-oxo-3-phenyl-1H,5H-pyrido[1,2-a]benzimidazole-4-carbonitrile:

A mixture of 1.26 g (8.00 mmol) of (2-benzimidazolyl)acetonitrile, 1.76 g (8.00 mmol) of ethyl 2-benzoylbutyrate and 1.23 g (16.0 mmol) of ammonium acetate was heated at 140 to 150°C for 40 minutes. After cooling, 50 ml of water was added thereto, then the solid was crushed and decanted, and 30 ml of acetonitrile was added thereto and the solid was washed. The crystal was taken out through filtration to obtain 840 mg (34 %) of the entitled compound as a brown crystal.
MS(EI)m/z:314(M⁺).
¹H-NMR(400MHz, DMSO-d₆)δ: 0.88(3H, t, J=7.56Hz), 2.20-2.27(2H, m), 6.98-7.04(1H, m), 7.21-7.32(3H, m), 7.37-7.50(4H, m), 8.55(1H, d, J=8.04Hz).
IR(ATR): 2216, 1658, 1622, 1601, 1535, 1487, 1456, 1410 cm⁻¹.

### [Reference Example 13]

### 1-Chloro-2-ethyl-3-phenylpyrido[1,2-a]benzimidazole-4-carbonitrile:

678 mg (2.16 mmol) of 2-ethyl-1-oxo-3-phenyl-1H,5H-pyrido[1,2-a]benzimidazole-4-carbonitrile was heated under reflux in 3 ml of phosphoryl chloride for 3 hours. After cooling, phosphoryl chloride was evaporated under reduced pressure, and 30 ml of ice-water was added to the residue. The mixture was extracted with chloroform (50 ml × 3). The chloroform layers were combined and dried over anhydrous magnesium sulfate, and the solvent was evaporated under reduced pressure. The resulting residue was washed with diisopropyl ether and taken out through filtration to obtain 234 mg (33 %) of the entitled compound as a yellow crystal.
MS(EI)m/z:332(M+H)⁺.
¹H-NMR(400MHz, DMSO-d₆)δ: 0.97(3H, t, J=7.32Hz), 2.60(2H, dd, J=15.14, 7.32Hz), 7.47-7.72(7H, m), 7.98(1H, d, J=8.30Hz), 8.78(1H, d, J=8.55Hz).
IR(ATR) : 2229, 1622, 1589, 1522, 1464, 1360, 1309, 1227 cm⁻¹.

### [Example 3]

### 2-n-Butyl-1-(2-N',N'-diethylaminoethylamino)-3-methylpyrido[1,2-a]benzimidazole-4-carbonitrile formic acid salt:

To N, N-dimethylformamide (2 ml) suspension of 30 mg (100 µmol) of 2-n-butyl-1-chloro-3-methylpyrido[1,2-a]-benzimidazole-4-carbonitrile were added 71 µl (500 µmol) of N,N-diethylaminoethylamine and 100 µl (717 µmol) of triethylamine. The system was replaced with nitrogen and sealed up, and the mixure was heated at 80°C for 14 hours. After cooling, the reaction mixture was separated and purified through a preparative HPLC to obtain 32 mg (76 %) of the entitled compound as a yellow crystal.
MS(EI)m/z:378(M⁺).

### [Example 4]

### 2-n-Butyl-1-[(3S)-dimethylaminopyrrolidin-1-yl]-3-methylpyrido[1,2-a]benzimidazole-4-carbonitrile formic acid salt:

According to the production method for 2-n-butyl-1-(2-N',N'-diethylaminoethylamino)-3-methylpyrido[1,2-a]benzimidazole-4-carbonitrile formic acid salt (Example 3) mentioned above, 24 mg (57 %) of the entitled compound was obtained as a yellow crystal from 2-n-butyl-1-chloro-3-methylpyrido[1,2-a]benzimidazole-4-carbonitrile and (3S)-dimethylaminopyrrolidine.
MS(EI)m/z:376(M⁺).

### [Example 5]

### 1-(2-N',N'-Diethylaminoethylamino)-3-methyl-2-phenylpyrido[1,2-a]benzimidazole-4-carbonitrile formic acid salt:

According to the production method for 2-n-butyl-1-(2-N',N'-diethylaminoethylamino)-3-methylpyrido[1,2-a]benzimidazole-4-carbonitrile formic acid salt (Example 3) mentioned above, 25 mg (64 %) of the entitled compound was obtained as a yellow crystal from 1-chloro-3-methyl-2-phenylpyrido[1,2-a]benzimidazole-4-carbonitrile and N,N-diethylaminoethylamine.
MS(EI)m/z:398(M⁺).

### [Example 6]

### 1-[(3S)-Dimethylaminopyrrolidin-1-yl]-3-methyl-2-phenylpyrido[1,2-a]benzimidazole-4-carbonitrile formic acid salt:

According to the production method for 2-n-butyl-1-(2-N',N'-diethylaminoethylamino)-3-methylpyrido[1,2-a]benzimidazole-4-carbonitrile formic acid salt (Example 3) mentioned above, 27 mg (61 %) of the entitled compound was obtained as a yellow crystal from 1-chloro-3-methyl-2-phenylpyrido[1,2-a]benzimidazole-4-carbonitrile and (3S)-dimethylaminopyrrolidine.
MS(EI)m/z:396(M⁺).

### [Example 7]

### 2-Benzyl-1-(2-N',N'-diethylaminoethylamino)-3-methylpyrido[1,2-a]benzimidazole-4-carbonitrile formic acid salt:

According to the production method for 2-n-butyl-1-(2-N',N'-diethylaminoethylamino)-3-methylpyrido[1,2-a]benzimidazole-4-carbonitrile formic acid salt (Example 3) mentioned above, 30 mg (66 %) of the entitled compound was obtained as a yellow crystal from 2-benzyl-1-chloro-3-methylpyrido[1,2-a]benzimidazole-4-carbonitrile and N,N-diethylaminoethylamine.
MS(EI)m/z:412(M⁺).

### [Example 8]

### 2-Benzyl-1-[(3S)-dimethylaminopyrrolidin-1-yl]-3-methylpyrido[1,2-a]benzimidazole-4-carbonitrile formic acid salt:

According to the production method for 2-n-butyl-1-(2-N',N'-diethylaminoethylamino)-3-methylpyrido[1,2-a]benzimidazole-4-carbonitrile formic acid salt (Example 3) mentioned above, 33 mg (73 %) of the entitled compound was obtained as a yellow crystal from 2-benzyl-1-chloro-3-methylpyrido[1,2-a]benzimidazole-4-carbonitrile and (3S)-dimethylaminopyrrolidine.
MS(EI)m/z:410(M⁺).

### [Example 9]

### 1-[(3S)-Dimethylaminopyrrolidin-1-yl]-2-ethyl-3-phenylpyrido[1,2-a]benzimidazole-4-carbonitrile formic acid salt:

According to the production method for 2-n-butyl-1-(2-N',N'-diethylaminoethylamino)-3-methylpyrido[1,2-a]benzimidazole-4-carbonitrile formic acid salt (Example 3) mentioned above, 32 mg (71 %) of the entitled compound was obtained as a yellow crystal from 1-chloro-2-ethyl-3-phenylpyrido[1,2-a]benzimidazole-4-carbonitrile and (3S)-dimethylaminopyrrolidine.
MS(EI)m/z:410 (M⁺).

### [Example 10]

### 2-n-Hexyl-1-(3-dimethylaminoazetidin-1-yl)-3-methylpyrido[1,2-a]benzimidazole-4-carbonitrile:

To N,N-dimethylformamide (2.5 ml) suspension of 163 mg (0.50 mmol) of 1-chloro-2-n-hexyl-3-methylpyrido[1,2-a]benzimidazole-4-carbonitrile were added 95.2 mg (0.55 mmol) of 3-dimethylaminoazetidine dihydrochloride and 349 µl (2.50 mol) of triethylamine, and the resultng mixture was heated at 80°C for 10 hours. After cooling, the solvent was evaporated under reduced pressure, and the residue was applied to a silica gel column chromatography. This was eluted with ethyl acetate to obtain the entitled compound. This compound was recrystallized from isopropyl ether and taken out through filtration to obtain 84 mg (43 %) of the entitled compound as a yellow crystal.
MS(EI)m/z:390(M+H)⁺.
¹H-NMR(400MHz, CDCl₃)δ: 0.94(3H, t, J=7.09Hz), 1.33-1.44(4H, m), 1.47-1.57 (4H, m) , 2.24 (6H, s) , 2.66(3H, s) , 2.83-2.87(2H, m), 3.36(1H, m), 4.14(2H, t, J=6.11Hz), 4.31(2H, t, J=6.85Hz), 7.35(1H, t, J=8.31Hz), 7.51(1H, t, J=8.31Hz), 7.95(1H, d, J=8.31Hz), 8.30(1H, d, J=8.31Hz).
IR(ATR): 2929, 2817, 2221, 1625, 1590, 1467, 1442 cm⁻¹.

| Elemental analysis: C₂₄H₃₁N₅ | | | |
|---|---|---|---|
| Calcd. | C, 74.00%; | H, 8.02%; | N, 17.98% |
| Found | C, 73.72%; | H, 7.97%; | N, 17.88%. |

### [Example 11]

### 2-n-Hexyl-1-[3-(dimethylaminomethyl)azetidin-1-yl]-3-methylpyrido[1,2-a]benzimidazole-4-carbonitrile:

To N,N-dimethylformamide (5.0 ml) suspension of 326 mg (1.00 mmol) of 1-chloro-2-n-hexyl-3-methylpyrido[1,2-a]benzimidazole-4-carbonitrile were added 191 mg (1.02 mmol) of 3-(dimethylaminomethyl)azetidine dihydrochloride and 0.56 ml (4.00 mmol) of triethylamine, and the resulting mixture was heated at 80°C for 15 hours. After cooling, the solvent was evaporated under reduced pressure, and the residue was applied to a silica gel column chromatography. This was eluted with a mixed solvent of chloroform/methanol (10/1, v/v) to obtain 101 mg (25 %) of the entitled compound as a pale brown crystal.
MS(EI)m/z:404(M+H)⁺.
¹H-NMR(400MHz, CDCl₃)δ: 0.94(3H, t, J=7.08Hz), 1.31-1.41(4H, m), 1.46-1.65(4H, m), 2.30(6H, s), 2.64(3H, s), 2.70(2H, d, J=7.32Hz), 2.78-2.82 (2H, m), 3.18 (1H, m), 4.02 (2H, t, J=6.83Hz), 4.40(2H, t, J=7.56Hz), 7.34(1H, t, J=8.30Hz), 7.50(1H, t, J=8.30Hz), 7.94(1H, d, J=8.30Hz), 8.24(1H, d, J=8.30Hz).
IR(ATR): 2929, 2856, 2815, 2763, 2223, 1623, 1590, 1473, 1442 cm⁻¹.

| Elemental analysis: C₂₅H₃₃N₅·0.5H₂O | | | |
|---|---|---|---|
| Calcd. | C, 72.78%; | H, 8.31%; | N, 16.97% |
| Found | C, 72.80%; | H, 8.18%; | N, 16.64%. |

### [Example 12]

### 2-n-Hexyl-1-(4-methylhomopiperazin-1-yl)-3-methylpyrido[1,2-a]benzimidazole-4-carbonitrile:

To N,N-dimethylformamide (5.0 ml) suspension of 335 mg (1.03 mmol) of 1-chloro-2-n-hexyl-3-methylpyrido[1,2-a]benzimidazole-4-carbonitrile were added 141 mg (1.23 mmol) of 1-methylhomopiperazine and 0.42 ml (3.01 mmol) of triethylamine, and the resulting mixture was heated at 80°C for 15 hours. After cooling, the solvent was evaporated under reduced pressure, and the residue was dissolved in chloroform, washed with water and brine, and dried over anhydrous sodium sulfate. The solvent was evaporated under reduced pressure, and the residue was applied to a silica gel column chromatography. This was elutedwith a mixed solvent of chloroform/methanol (10/1, v/v) to obtain 25 mg (6 %) of the entitled compound as a pale brown crystal.
MS(EI)m/z:409(M+H)⁺.
¹ H-NMR(400MHz, CDCl₃)δ: 0.94(3H, t, J=7.10Hz), 1.35-1.40(4H, m), 1.55-1.59(4H, m), 2.04-2.21(2H, m), 2.52(3H, s), 2.70(3H, s), 2.73-2.85(4H, m), 2.88-2.97(2H, m), 3.38-3.45(2H, m), 3.62(1H, m), 3.70(1H, m), 7.36(1H, t, J=8.33Hz), 7.52(1H, t, J=8.33Hz), 7.98(1H, d, J=8.33Hz), 8.48(1H, d, J=8.33Hz).
IR(ATR): 2925, 2852, 2217, 1625, 1592, 1492, 1446 cm⁻¹.

| Elemental analysis: C₂₅H₃₃N₅·H₂O | | | |
|---|---|---|---|
| Calcd. | C, 71.23%; | H, 8.37%; | N, 16.61% |
| Found | C, 71.03%; | H, 8.68%; | N, 16.55%. |

### [Example 13]

### 2-n-Hexyl-3-methyl-1-[3-dimethylaminomethylpyrroldin-1-yl]pyrido[1,2-a]benzimidazole-4-carbonitrile dihydrochloride:

To N,N-dimethylformamide (4 ml) suspension of 250 mg (0.77 mmol) of 1-chloro-2-n-hexyl-3-methylpyrido[1,2-a]benzimidazole-4-carbonitrile were added 148 mg (1.15 mmol) of 3-dimethylaminomethylpyrrolidine and 213 µl (1.53 mmol) of triethylamine. The system was replaced with nitrogen and then sealed up, and the mixture was heated at 80°C for 24 hours. After cooling, the solvent was evaporated under reduced pressure, and the residue was dissolved in 60 ml of chloroform, washed with 20 ml of water, 20 ml of saturated sodium bicarbonate solution and 20 ml of brine, and dried over anhydrous sodium sulfate. The solvent was evaporated under reduced pressure, and the residue was applied to a silica gel column chromatography. This was eluted with a mixed solvent of chloroform/methanol (97/3, v/v) to obtain a crude product of the entitled compound. This was separated and purified by preparative TLC, and then dissolved in 5 ml of ethanol. 2 ml of ethanol solution of 1 N hydrochloric acid was added thereto, and the mixture was stirred at room temperature for 10 minutes, and then the solvent was evaporated. The residue was recrystallized from isopropanol/ethanol, and then taken out through filtration to obtain 150 mg (39 %) of the entitled compound as a pale green crystal.
MS(EI)m/z:417(M⁺).
¹H-NMR(400MHz, D₂O)δ: 0.78(3H, t, J=7.08Hz), 1.18-1.31 (4H, m), 1.36-1.54 (4H, m), 1.95-2.06(1H, m), 2.36-2.47(1H, m), 2.63(6H, s), 2.86(4H, s), 2.88(3H, s), 2.95-3.13(1H, m), 3.30-3.73(4H, m), 7.46-7.50(1H, m), 7.57(1H, s), 7.58(1H, s), 7.99(1H, d, J=8.55Hz).
IR(ATR): 1732, 1626, 1514, 1473 cm⁻¹.

| Elemental analysis: C₂₆H₃₅N₅·2.0HCl·0.25H₂O | | | |
|---|---|---|---|
| Calcd. | C, 63.09%; | H, 7.64%; | N, 14.15% |
| Found | C, 63.05%; | H, 7.64%; | N, 14.10%. |

### [Example 14]

### 2-n-Hexyl-3-methyl-1-(4-methylpiperazin-1-yl)pyrido[1,2-a]benzimidazole-4-carbonitrile:

To N,N-dimethylformamide (4 ml) suspension of 250 mg (0.77 mmol) of 1-chloro-2-n-hexyl-3-methylpyrido[1,2-a]benzimidazole-4-carbonitrile were added 115 mg (1.15 mmol) of N-methylpiperazine and 213 µl (1.53 mmol) of triethylamine. The system was replaced with nitrogen and then sealed up, and the mixture was heated at 80°C for 20 hours. After cooling, the solvent was evaporated under reduced pressure, and the residue was dissolved in 60 ml of chloroform, washed with 20 ml of water, 20 ml of saturated sodium bicarbonate solution and 20 ml of brine, and dried over anhydrous sodium sulfate. The solvent was evaporated under reduced pressure, and the residue was applied to a silica gel column chromatography. This was eluted with a mixed solvent of chloroform/methanol (97/3, v/v) to obtain a crude product of the entitled compound. This was separated and purified by preparative TLC, then recrystallized from isopropanol, and taken out through filtration to obtain 128 mg (43 %) of the entitled compound as a yellow crystal.
MS(EI)m/z:389(M⁺).
¹H-NMR(400MHz, CDCl₃)δ: 0.93(3H, t, J=7.08Hz), 1.35-1.42 (4H, m), 1.50-1.62(4H, m),2.51(3H, s), 2.67(3H, s), 2.72-2.79 (4H, m), 2.81-2.90(2H, m), 3.30-3.37(2H, m), 3.51-3.60(2H, m), 7.35(1H, dt, J=1.22, 7.32Hz), 7.53(1H, ddd, J=0.98, 1.22, 8.06Hz), 7.98(1H, d, J=8.30Hz), 8.79(1H, d, J=8.55Hz).
IR(ATR): 2224, 1628, 1595, 1493 cm⁻¹.

| Elemental analysis: C₂₄H₃₁N₅ | | | |
|---|---|---|---|
| Calcd. | C, 74.00%; | H, 8.02%; | N, 17.98% |
| Found | C, 73.65%; | H, 7.97%; | N, 17.89%. |

### [Examples 15 to 47]

### 1-R^{a}-2-ethyl-3-methylpyrido[1,2-a]benzimidazole-4-carbonitrile:

To N,N-dimethylformamide (1 ml) suspension of 20 mg (0.0736 mmol) of 1-chloro-2-ethyl-3-methylpyrido[1,2-a]benzimidazole-4-carbonitrile were added 30.8 µl (0.221 mmol) of triethylamine and amine (0.147 mmol). The systemwas replaced with nitrogen and then sealed up, and the mixture was heated at 80°C for 20 hours. After cooling, the reaction mixture was separated and purified by preparative HPLC to obtain the entitled compound of the following formula, as in Tables 1 and 2.

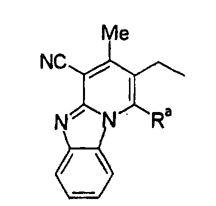

**Table 1**

| Ex. | Amine | Ra | MS EIMS m/s |
|---|---|---|---|
| 15 | morpholine | morpholino | 321 |
| 16 | N,N',N'-trimethylethylenediamine | [2-(N',N'-dimethylamino)ethyl]methylamino | 336 |
| 17 | benzylamine | benzylamino | 341 |
| 18 | 3-piperidine-methanol | (3-hydroxymethyl)piperidin-1-yl | 348 |
| 19 | dl-3-pyrrolidinol | (3-hydroxy)pyrrolidin-1-yl | 320 |
| 20 | 1-methylpiperazine | (4-methyl)piperazin-1-yl | 334 |
| 21 | thiomorpholine | thiomorpholino | 337 |
| 22 | 2-morpholinoethylamine | (2-morpholinoethyl)amino | 363 |
| 23 | pyrrolidine | pyrrolidino | 304 |
| 24 | 1-ethylpropylamine | (1-ethylpropyl)amino | 320 |
| 25 | N,N-dimethylethylenediamine | [2-(N',N'-dimethylamino)ethyl]amino | 322 |
| 26 | N-acetylethylenediamine | [2-(N'-acetylamino)ethyl]amino | 335 |
| 27 | cyclohexanemethylamine | cyclohexanemethylamino | 346 |
| 28 | (S)-(+)-1-(2-pyrrolidinylmethyl)pyrrolidine | (1-pyrrolidinylmethyl)pyrrolidino | 388 |
| 29 | ethyl 4-amino-1-piperidinecarboxylate | (1-methoxycarbonylpiperidin-4-yl)amino | 405 |
| 30 | (R)-(-)-2-amino-1-butanol | 2-(1-hydroxy)butylamino | 324 |
| 31 | (3S)-N,N-dimethylaminopyrrolidine | [3-(N,N-dimethyl)amino]pyrrolidin-1-yl | 350 |
| 32 | (±)-3-(N-acetyl)aminopyrrolidine | [3-(N-acetyl)amino]pyrrolidin-1-yl | 361 |
| 33 | trans-4-aminocyclohexanol | (1-hydroxy)-cyclohexan-4-yl-amino | 348 |
| 34 | 4-hydroxypiperidine | (4-hydroxy)piperidin-1-yl | 336 |

**Table 2**

| Ex. | Amine | Ra | MS EIMS m/s |
|---|---|---|---|
| 35 | t-butyl 2-aminoethylaminecarboxylate | 2-(N'-t-butylcarbonyl)aminoethylamino | 393 |
| 36 | t-butyl 5-aminopentylaminecarboxylate | 5-(N'-t-butylcarbonyl)aminopentylamino | 435 |
| 37 | 3-aminocyclohexylamine | (3-aminocyclohexyl)amino | 348 |
| 38 | 3-amino-2,2-dimethylpropylamine | (3-amino-2,2-dimethyl)propylamino | 336 |
| 39 | (3R)-N'-methylaminopyrrolidine | [(3R)-N'-methylamino]pyrrolidin1-yl | 334 |
| 40 | (3S)-N'-methylaminopyrrolidine | [(3S)-N'-methylamino]pyrrolidin-1-yl | 334 |
| 41 | (3R)-N'-ethylaminopyrrolidine | [(3R)-N'-ethylamino]pyrrolidin-1-yl | 347 |
| 42 | (3R)-N',N'-dimethylaminopyrrolidine | [(3R)-N',N'-dimethylamino]pyrrolidin-1-yl | 347 |
| 43 | (±)-3-aminopiperidine dihydrochloride | (3-aminopiperidin)-1-yl | 334 |
| 44 | (±)-3-aminopiperidine dihydrochloride | (3-piperidinyl)amino | 334 |
| 45 | 4-N',N'-dimethylaminopiperidine | (4-N',N'-dimethylamino)piperidin-1-yl | 362 |
| 46 | (±)-3-N'-methylaminopiperidine dihydrochloride | (3-N'-methylamino)piperidin-1-yl | 320 |
| 47 | 4-pyrrolidinopiperidine | (4-pyrrolidinopiperidin)-1-yl | 388 |

### [Examples 48 and 49]

### 1-R^{b}-2-ethyl-3-methylpyrido[1,2-a]benzimidazole-4-carbonitrile:

A mixture of 1-R^{a}-2-ethyl-3-methylpyrido[1,2-a]benzimidazole-4-carbonitrile and 1 ml of trifluoroacetic acid/methylene chloride (1/1, v/v) was stirred at room temperature for 1 hour. The solvent was evaporated from the reaction mixture, and the resulting residure was dried under reduced pressure to obtain the entitled compound of the following formula, as in Table 3.

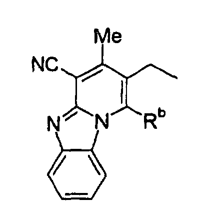

**Table 3**

| Ex. | Ra | Rb | MS EIMS m/s |
|---|---|---|---|
| 48 | 2-(N'-t-butylcarbonyl)aminoethylamino | (2-aminoethyl)amino | 293 |
| 49 | 5-(N'-t-butylcarbonyl)aminopentylamino | (5-aminopentyl)amino | 336 |

### [Reference Example 14]

### Methyl α-(4-fluorobenzyl)acetoacetate:

Totetrahydrofuran (5ml) solution of 623.0 mg (5.00 mmol) of 4-fluorobenzyl bromide were added 301.0 mg (7.1 mmol) of lithium chloride, 755.4 µl (7.00 mmol) of methyl acetoacetate and 1.31 ml (7.50 mmol) of diisopropylethylamine, and then heated under reflux at 80°C for 3.5 hours. After cooling, the solvent was evaporated from the reaction mixture, and 10 ml of chloroform and 3.5 ml of water were added to the residue, and stirred at room temperature for 30 minutes. The organic layer was washed with 5 ml of 1 N hydrochloric acid, and then three times with 3.5 ml of water. The organic layer was separated, and dried over anhydrous magnesium sulfate, and the solvent was evaporated under reduced pressure. The residue was applied to a silica gel flash column chromatography. This was eluted with a mixed solventof n-hexane/ethyl acetate (15/1) to obtain 681.1mg (61 %) of the entitled compound as a colorless oil (this was directly used in the next reaction).
¹H-NMR(400MHz, CDCl₃)δ: 2.18(3H, s), 3.13(2H, d, J=6.84Hz), 3.69(3H, s), 3.75(1H, t, J=7.57Hz), 6.93-6.98(2H, m), 7.11-7.15 (2H, m).

### [Reference Example 15]

### Methyl α-(3-fluorobenzyl)acetoacetate:

According to the production method for methyl α-(4-fluorobenzyl)acetoacetate but using 3-fluorobenzyl bromide in place of 4-fluorobenzyl bromide, the entitled compound was produced.
¹H-NMR(400MHz, CDCl₃)δ: 2.21(3H, s), 3.15-3.17(2H, m), 3.71(3H, s), 3.76-3.80(1H, m), 6.88-6.96(3H, m), 7.22-7.24(1H, m).

### [Reference Example 16]

### Methyl α-(2,4-difluorobenzyl)acetoacetate:

According to the production method for methyl α-(4-fluorobenzyl)acetoacetate but using 2,4-difluorobenzyl bromide inplace of 4-fluorobenzyl bromide, the entitled compound was produced.
¹H-NMR(400MHz, CDCl₃)δ: 2.22(3H, s) , 3.12-3.17(2H, m), 3.70(3H, s), 3.83(1H, t, J=7.58Hz), 6.78-6.80(2H, m), 7.14-7.18(1H, m) .

### [Reference Example 17]

### Methyl α-(4-nitrobenzyl)acetoacetate:

According to the production method for methyl α-(4-fluorobenzyl)acetoacetate but using 4-nitrobenzyl bromi de in place of 4-fluorobenzyl bromide, the entitled compound was produced.
¹H-NMR(400MHz, CDCl₃)δ: 2.24 (3H, s), 3.27(2H, m) , 3.72(3H, s), 3.83(1H, t, J=7.46Hz), 7.37(2H, t, J=8.31Hz), 8.14(2H, t, J=8.80Hz).

### [Reference Example 18]

### Methyl α-(3-nitrobenzyl)acetoacetate:

According to the production method for methyl α-(4-fluorobenzyl)acetoacetate but using 3-nitrobenzyl bromide in place of 4-fluorobenzyl bromide, the entitled compound was produced.
¹H-NMR(400MHz, CDCl₃)δ: 2.25(3H, s), 3.25-3.28(2H, m), 3.72(3H, s), 3.83(1H, t, J=7.46Hz), 7.44-7.48(1H, m), 7.53-7.55(1H, m), 8.06(1H, brs).

### [Reference Example 19]

### Methyl α-(4-benzyloxybenzyl)acetoacetate:

According to the production method for methyl α-(4-fluorobenzyl)acetoacetate but using 4-benzyloxybenzyl chloride in place of 4-fluorobenzyl bromide, the entitled compound was produced.
¹H-NMR(400MHz, CDCl₃)δ: 2.16(3H, s), 3.10(2H, ab, J=7.56,7.56Hz), 3.67(3H, s), 3.73-3.77(1H, m), 5.01(2H, s), 6.86-6.89(2H, m), 7.06-7.09(2H, m), 7.30-7.42(5H, m).

### [Reference Example 20]

### Methyl α-(4-methoxybenzyl)acetoacetate:

According to the production method for methyl α-(4-fluorobenzyl)acetoacetate but using 4-methoxybenzyl chloride in place of 4-fluorobenzyl bromide, the entitled compound was produced.
¹H-NMR(400MHz, CDCl₃)δ: 2.16(3H, s), 3.10 (2H, d, J=7.83Hz), 3.68(3H, s), 3.70-3.76(1H, m), 3.76(3H, s), 6.80(2H, d, J=8.80Hz), 7.08(2H, d, J=8.80Hz).

### [Reference Example 21]

### Methyl α-(4-methoxycarbonylbenzyl)acetoacetate:

According to the production method for methyl α-(4-fluorobenzyl)acetoacetate but using 4-methoxycarbonylbenzyl bromide in place of 4-fluorobenzyl bromide, the entitled compound was produced.
¹H-NMR(400MHz, CDCl₃)δ: 2.20(3H, s), 3.20-3.22(2H, m), 3.71(3H, s), 3.78(1H, t, J=7.57Hz), 3.90(3H, s), 7.25(2H, d, J=8.30Hz), 7.95(2H, d, J=8.30Hz).

### [Reference Example 22]

### 2-(4-Fluorobenzyl)-3-methyl-1-oxo-1H,5H-pyrido[1,2-a]benzimidazole-4-carbonitrile:

A mixture of 477 mg (3.04 mmol) of (2-benzimidazolyl)acetonitrile, 681 mg (3.04 mmol) of methyl α-(4-fluorobenzyl)acetoacetate and 468 mg (6.08 mmol) of ammonium acetate was heated at 140 to 150°C for 30 minutes. After cooling, 10 ml of water was added thereto, then the solid was crushed and decanted, and 5 ml of acetonitrile was added thereto and washed. The crystal was taken out through filtration to obtain 853 mg (85 %) of the entitled compound as a pale red crystal (this was directly used in the next reaction).
EIMSm/z:332(M⁺).

### [Reference Example 23]

### 2-(3-Fluorobenzyl)-3-methyl-1-oxo-1H,5H-pyrido[1,2-a]benzimidazole-4-carbonitrile:

According to the production method for 2-(4-fluorobenzyl)-3-methyl-1-oxo-1H,5H-pyrido[1,2-a]benzimidazole-4-carbonitrile (Reference Example 22) but using methyl α-(3-fluorobenzyl) acetoacetate in place of methyl α-(4-fluorobenzyl)acetoacetate, the entitled compound was produced.
EIMSm/z:332 (M⁺).

### [Reference Example 24]

### 2-(2,4-Difluorobenzyl)-3-methyl-1-oxo-1H,5H-pyrido[1,2-a]benzimidazole-4-carbonitrile:

According to the production method for 2-(4-fluorobenzyl)-3-methyl-1-oxo-1H,5H-pyrido[1,2-a]benzimidazole-4-carboni trile (Reference Example 22) but using methyl α-(2,4-difluorobenzyl)acetoacetate in place of methyl α-(4-fluorobenzyl) acetoacetate, the entitled compound was produced.
EIMSm/z:350(M⁺).

### [Reference Example 25]

### 3-Methyl-2-(4-nitrobenzyl)-1-oxo-1H,5H-pyrido[1,2-a]benzimidazole-9-carbonitrile:

According to the production method for 2-(4-fluorobenzyl)-3-methyl-1-oxo-1H,5H-pyrido[1,2-a]benzimidazole-4-carbonitrile (Reference Example 22) but using methyl α-(4-nitrobenzyl)acetoacetate in place of methyl α-(4-fluorobenzyl)acetoacetate, the entitled compound was produced.
EIMSm/z:359(M⁺).

### [Reference Example 26]

### 3-Methyl-2-(3-nitrobenzyl)-1-oxo-1H,5H-pyrido[1,2-a]benzimidazole-4-carbonitrile:

According to the production method for 2-(4-fluorobenzyl)-3-methyl-1-oxo-1H,5H-pyrido[1,2-a]benzimidazole-4-carbonitrile (Reference Example 22) but using methyl α-(3-nitrobenzyl)acetoacetate in place of methyl α-(4-fluorobenzyl)acetoacetate, the entitled compound was produced.
EIMSm/z:359(M⁺).

### [Reference Example 27]

### 2-(4-Benzyloxybenzyl)-3-methyl-1-oxo-1H,5H-pyrido[1,2-a]benzimidazole-4-carbonitrile:

According to the production method for 2-(4-fluorobenzyl)-3-methyl-1-oxo-1H,5H-pyrido[1,2-a]benzimidazole-4-carbonitrile (Reference Example 22) but using methyl α-(4-benzyloxybenzyl)acetoacetate in place of methyl α-(4-fluorobenzyl)acetoacetate, the entitled compound was produced.
¹H-NMR(400MHz, DMSO-d₆)δ: 2.36(3H, s), 3.91(2H, s), 5.03(2H, s), 6.88(2H, d , J=8.79Hz)), 7.14(2H, d, J=8.79Hz), 7.30-7.42(6H, m), 7.53(2H, d, J=3.42Hz), 8.61(1H, d, J=8.06Hz).

### [Reference Example 28]

### 2-(4-Methoxybenzyl)-3-methyl-1-oxo-1H,5H-pyrido[1,2-a]benzimidazole-4-carbonitrile:

According to the production method for 2-(4-fluorobenzyl)-3-methyl-1-oxo-1H,5H-pyrido[1,2-a]benzimidazole-4-carbonitrile (Reference Example 22) but using methyl α-(4-methoxybenzyl) acetoacetate in place of methyl α-(4-fluorobenzyl)acetoacetate, the entitled compound was produced.
EIMSm/z:372 (M⁺).

### [Reference Example 29]

### 2-(4-Methoxycarbonylbenzyl)-3-methyl-1-oxo-1H,5H-pyrido[1,2-a]benzimidazole-4-carbonitrile:

According to the production method for 2-(4-fluorobenzyl)-3-methyl-1-oxo-1H,5H-pyrido[1,2-a]benzimidazole-4-carbonitrile (Reference Example 22) but using methyl α-(4-methoxycarbonylbenzyl)acetoacetate in place of methyl α-(4-fluorobenzyl)acetoacetate, the entitled compound was produced.
EIMSm/z:372(M⁺).

### [Reference Example 30]

### 1-Chloro-2-(4-fluorobenzyl)-3-methylpyrido[1,2-a]benzimidazole-4-carbonitrile:

853mg (2.57 mmol) of 2-(4-fluorobenzyl)-3-methyl-1-oxo-1H,5H-pyrido[1,2-a]benzimidazole-4-carbonitrile was heated under reflux in 5 ml of phosphoryl chloride for 16 hours. After cooling, phosphoryl chloride was evaporated under reduced pressure, and the residue was dissolved in 30 ml of chloroform. 20 ml of ice water and 60 ml of aqueous 1 N sodium hydroxide solution were added thereto and stirred for 15 minutes. The organic layer was extracted with chloroform (100 ml × 3). The chloroform layers were combined, washed with brine, and dried over anhydrous magnesium sulfate, and the solvent was evaporated under reduced pressure. The resulting residue was washed with a small amount of diisopropyl ether and taken out through filtration to obtain 823 mg (85 %) of the entitled compound as a pale yellow crystal (this was directly used in the next reaction).
EIMSm/z:350(M⁺).

### [Reference Example 31]

### 1-Chloro-2-(3-fluorobenzyl)-3-methylpyrido[1,2 a]benzimidazole-4-carbonitrile:

According to the production method for 1-chloro-2-(4-fluorobenzyl)-3-methylpyrido[1,2-a]benzimidazole-4-carbonitrile (Reference Example 30) but using 2-(3-fluorobenzyl)-3-methyl-1-oxo-1H,5H-pyrido[1,2-a]benzimidazole-4-carbonitrilein place of 2-(4-fluorobenzyl)-3-methyl-1-oxo-1H,5H-pyrido[1,2-a]benzimidazole-4-carbonitrile, the entitled compound was produced.
EIMSm/z:350(M⁺).

### [Reference Example 32]

### 1-Chloro-2-(2,4-difluorobenzyl)-3-methylpyrido[1,2-a]benzimidazole-4-carbonitrile:

According to the production method for 1-chloro-2-(4-fluorobenzyl)-3-methylpyrido[1,2-a]benzimidazole-4-carbonitrile (Reference Example 30) but using 2-(2,4-difluorobenzyl)-3-methyl-1-oxo-1H,5H-pyrido[1,2-a]benzimidazole-4-carbonitrilein place of 2-(4-fluorobenzyl)-3-methyl-1-oxo-1H,5H-pyrido[1,2-a]benzimidazole-4-carbonitrile, the entitled compound was produced.
EIMSm/z:368(M⁺).

### [Reference Example 33]

### 1-Chloro-3-methyl-2-(4-nitrobenzyl)pyrido[1,2-a]benzimidazol-4-yl-carbonitrile:

According to the production method for 1-chloro-2-(4-fluorobenzyl)-3-methylpyrido[1,2-a]benzimidazole-4-carbonitrile (Reference Example 30) but using 3-methyl-2-(4-nitrobenzyl)-1-oxo-1H,5H-pyrido[1,2-a]benzimidazole-4-carbonitrile in place of 2-(4-fluorobenzyl)-3-methyl-1-oxo-1H,5H-pyrido[1,2-a]benzimidazole-4-carbonitrile, the entitled compound was produced.
¹H-NMR(400MHz, CDCl₃)δ: 2.62(3H, s), 4.43(2H, s), 7.30 (2H, d, J=8.79Hz), 7.43-7.46(1H, m), 7.62-7.66(1H, m), 8.07(1H, d, J=8.30Hz), 8.20(2H, d, J=8.55Hz), 8.58(1H, d, J=8.55Hz).

### [Reference Example 34]

### 1-Chloro-3-methyl-2-(3-nitrobenzyl)pyrido[1,2-a]benzimidazol-4-yl-carbonitrile:

According to the production method for 1-chloro-2-(4-fluorobenzyl)-3-methylpyrido[1,2-a]benzimidazole-4-carbonitrile (Reference Example 30) but using 3-methyl-2-(3-nitrobenzyl)-1-oxo-1H,5H-pyrido[1,2-a]benzimidazole-4-carbonitrile in place of 2-(4-fluorobenzyl)-3-methyl-1-oxo-1H,5H-pyrido[1,2-a]benzimidazole-4-carbonitrile, the entitled compound was produced.
¹H-NMR(400MHz, CDCl₃)δ: 2.64(3H, s), 4.42(2H, s), 7.45-7.47(2H, m), 7.50-7.54(1H, m), 7.62-7.64(1H, m), 8.04-8.08(2H, m), 8.14(2H, d, J=8.06Hz), 8.56(1H, d, J=8.55Hz).

### [Reference Example 35]

### 2-(4-Benzyloxybenzyl)-1-chloro-3-methylpyrido[1,2-a]benzimidazole-4-carbonitrile:

According to the production method for 1-chloro-2-(4-fluorobenzyl)-3-methylpyrido[1,2-a]benzimidazole-4-carbonitrile (Reference Example 30) but using 2-(4-benzyloxybenzyl)-3-methyl-1-oxo-1H,5H-pyrido[1,2-a]benzimidazole-4-carbonitrilein place of 2-(4-fluorobenzyl)-3-methyl-1-oxo-1H,5H-pyrido[1,2-a]benzimidazole-4-carbonitrile, the entitled compound was produced.
¹H-NMR(400MHz, CDCl₃)δ: 2.62(3H, s), 4.25(2H, s) , 5.03(2H, s), 6.91(2H, d, J=8.79Hz), 7.01(2H, d, J=8.55Hz), 7.31-7.44(6H, m), 7.60-7.64(1H, m), 8.06(2H, d, J=8.30Hz), 8.61(1H, d, J=8.55Hz).
EIMSm/z:438(M⁺).

### [Reference Example 36]

### 1-Chloro-2-(4-methoxybenzyl)-3-methylpyrido[1,2-a]benzimidazole-4-carbonitrile:

According to the production method for 1-chloro-2-(4-fluorobenzyl)-3-methylpyrido[1,2-a]benzimidazole-4-carbonitrile (Reference Example 30) but using 2-(4-methoxybenzyl)-3-methyl-1-oxo-1H,5H-pyrido[1,2-a]benzimidazole-4-carbonitrile in place of 2-(4-fluorobenzyl)-3-methyl-1-oxo-1H,5H-pyrido[1,2-a]benzimidazole-4-carbonitrile, the entitled compound was produced.
¹H-NMR(400MHz, CDCl₃)δ: 2.63(3H, s), 3.78(3H, s) , 4.26(2H, s), 6.84(2H, d, J=8.55Hz), 7.01(2H, d, J=8.79Hz), 7.43(1H, dd, J=7.57Hz, 8.06Hz), 7.61-7.65(1H, m), 8.07(1H, d, J=8.06Hz), 8.61(1H, d, J=8.55Hz).

### [Reference Example 37]

### 1-Chloro-2-(4-methoxycarbonylbenzyl)-3-methylpyrido[1,2-a]benzimidazol-4-yl-carbonitrile:

According to the production method for 1-chloro-2-(4-fluorobenzyl)-3-methylpyrido[1,2-a]benzimidazole-4-carbonitrile (Reference Example 30) but using 2-(4-methoxycarbonylbenzyl)-3-methyl-1-oxo-1H,5H-pyrido[1,2-a]benzimidazole-4-carbonitrile in place of 2-(4-fluorobenzyl)-3-methyl-1-oxo-1H,5H-pyrido[1,2-a]benzimidazole-4-carbonitrile, the entitled compound was produced.
¹H-NMR(400MHz, CDCl₃)δ: 2.60(3H, s), 3.90(3H, s), 4.37(2H, s), 7.18(2H, d, J=8.06Hz), 7.43(1H, dd, J=7.57Hz, 8.30Hz), 7.61-7.65(1H, m), 7.99(2H, d, J=8.30Hz), 8.06(1H, d, J=8.30Hz) , 8.59(1H, d, J=8.55Hz).

### [Example 50]

### 2-(4-Methoxycarbonylbenzyl)-1-[(3S)-dimethylaminopyrrolidin-1-yl]-3-methylpyrido[1,2-a]benzimidazole-4-carbonitrile:

To N,N-dimethylformamide (10 ml) suspension of 468 mg (1.20 mmol) of 1-chloro-2-(4-methoxycarbonylbenzyl)-3-methylpyrido[1,2-a]benzimidazol-4-yl-carbonitrile were added 228 µl (1.80 mmol) of (3S)-dimethylaminopyrrolidine and 284 µl (2.04 mmol) of triethylamine. The system was replaced with nitrogen and sealed up, and heated at 80°C for 1 hour. After cooling, the solvent was evaporated under reduced pressure, and the residue was dissolved in 50 ml of chloroform, washed successively with 20 ml of water, 20 ml of saturated sodium bicarbonate solution and 20 ml of brine, and dried over anhydrous magnesium sulfate. The solvent was evaporated under reduced pressure, and the residue was applied to a silica gel column chromatography. This was eluted with a mixed solvent of chloroform/methanol (97/3, v/v) to obtain 434 mg (77 %) of a crude product of the entitled compound. This was washed with ethyl acetate and the crystal was taken out through filtration to obtain 112 mg (20 %) of the entitled compound as a yellow crystal.
¹H-NMR(400MHz, DMSO-d₆)δ: 2.11,(6H, s), 2.01-2.20(2H, m), 2.37(3H, s), 2.90-3.32(4H, m), 3.51(1H, brs), 3.83(3H, s), 4.26(2H, brs), 7.36(2H, d, J=8.30Hz), 7.41-7.45(1H, m), 7.55-7.58(1H, m), 7.89(1H, d, J=8.79Hz), 7.89 (2H, d, J=8.30Hz), 8.09-8.23(1H, m).
IR(KBr): 2948, 2771, 2224, 1716, 1591, 1481, 1442, 1294 cm⁻¹.

| Elemental analysis: C₂₈H₂₉N₅O₂ | | | |
|---|---|---|---|
| Calcd. | C, 71.93%; | H, 6.25%; | N, 14.98% |
| Found | C, 71.88%; | H, 6.21%; | N, 15.03%. |

### [Example 51]

### 2-(4-Methoxybenzyl)-1-[(3S)-dimethylaminopyrrolidin-1-yl]-3-methylpyrido[1,2-a]benzimidazole-4-carbonitrile (#51):

According to the production method for 2-(4-methoxycarbonylbenzyl)-1-[(3S)-dimethylaminopyrrolidin-1-yl]-3-methylpyrido[1,2-a]benzimidazole-4-carbonitrile (#50), but using 1-chloro-2-(4-methoxybenzyl)-3-methylpyrido[1,2-a]benzimidazole-4-carbonitrile (I-36) in place of 1-chloro-2-(4-methoxycarbonylbenzyl)-3-methylpyrido[1,2-a]benzimidazole-4-carbonitrile, the entitled compound was produced (BI357102).
¹H-NMR(400MHz, CDCl₃)δ: 2.26,(6H, s), 2.62(2H, m), 2.45(3H, s), 2.90-3.41 (4H, m) , 3.65(1H, brs), 3.78 (3H, s), 4.10(2H, brs) , 6.84(2H, d, J=7.81Hz), 6.97(2H, d, J=7.57Hz), 7.37(1H, brs), 7.54 (1H, dd, J=7.81Hz, 7.08Hz), 8.00(1H, d, J=7.81Hz), 8.14 (1H, brs).
IR(KBr): 2224, 1626, 1591, 1477, 1443, 1244 cm⁻¹.
EIMSm/z:440(M⁺).

| Elemental analysis: C₂₇H₂₉N₅O | | | |
|---|---|---|---|
| Calcd. | C, 73.78%; | H, 6.65%; | N, 15.93% |
| Found | C, 73.43%; | H, 6.67%; | N, 15.75%. |

### [Example 52]

### 2-(4-Benzyloxybenzyl)-1-[(3S)-dimethylaminopyrrolidin-1-yl]-3-methylpyrido[1,2-a]benzimidazole-4-carbonitrile (#52):

According to the production method for 2-(4-methoxycarbonylbenzyl)-1-[(3S)-dimethylaminopyrrolidin-1-yl]-3-methylpyrido[1,2-a]benzimidazole-9-carbonitrile (#50), but using 2-(4-benzyloxybenzyl)-1-chloro-3-methylpyrido[1,2-a]benzimidazole-4-carbonitrile (I-35) in place of 1-chloro-2-(4-methoxycarbonylbenzyl)-3-methylpyrido[1,2-a]benzimidazole-4-carbonitrile, the entitled compound was produced (this was directly used in the next reaction).
¹H-NMR(400MHz, CDCl₃)δ: 2.04-2.30(2H, m), 2.24(6H, brs), 2.46(3H, s), 2.90-3.50(4H, m), 3.64(1H, brs), 4.07-4.10(2H, m), 5.03(2H, m), 6.91(2H, d, J=9.04Hz), 6.97(2H, d, J=8.31Hz) , 7.30-7.43(6H, m), 7.53-7.57(1H, m), 8.02(1H, d, J=8.07Hz), 8.14(1H, brs).

### [Example 53]

### 1-[(3S)-dimethylaminopyrrolidin-1-yl]-3-methyl-2-(4-nitrobenzyl)pyrido[1,2-a]benzimidazole-4-carbonitrile (#53):

According to the production method for 2-(4-methoxycarbonylbenzyl)-1-[(3S)-dimethylaminopyrrolidin-1-yl]-3-methylpyrido[1,2-a]benzimidazole-4-carbonitrile (#50), but using 1-chloro-3-methyl-2-(4-nitrobenzyl)pyrido[1,2-a]benzimidazole-4-carbonitrile (I-33) in place of 1-chloro-2-(4-methoxycarbonylbenzyl)-3-methylpyrido[1,2-a]benzimidazole-4-carbonitrile, the entitled compound was produced (this was directly used in the next reaction).
¹H-NMR(400MHz, CDCl₃)δ: 2.07-2.32,(2H, m), 2.25(6H, s,), 2.44(3H, s), 3.03-3.51(4H, m), 3.67(1H, brs,), 4.30(2H, brs), 7.26-7.29(2H, m) , 7.39-7.42(1H, m) , 7.56-7.60(1H, m) , 8.05(2H, d, J=8.30Hz), 8.19-8.16(1H, m), 8.20(2H, d, J=8.79Hz).

### [Example 54]

### 1-[(3S)-dimethylaminopyrrolidin-1-yl]-3-methyl-2-(3-nitrobenzyl)pyrido[1,2-a]benzimidazole-4-carbonitrile (#54):

According to the production method for 2-(4-methoxycarbonylbenzyl)-1-[(3S)-dimethylaminopyrrolidin-1-yl]-3-methylpyrido[1,2-a]benzimidazole-4-carbonitrile (#50), but using 1-chloro-3-methyl-2-(3-nitrobenzyl)pyrido[1,2-a]benzimidazole-4-carbonitrile (I-34) in place of 1-chloro-2-(4-methoxycarbonylbenzyl)-3-methylpyrido[1,2-a]benzimidazole-4-carbonitrile, the entitled compound was produced (this was directly used in the next reaction).
¹H-NMR(400MHz, CDCl₃)δ: 2.25, (6H, s), 2.32(2H, m) 2.44(3H, s), 3.05-3.49(4H, m) , 3.68(1H, brs) , 4.30(2H, brs), 7.40-7.42 (2H, m), 7.52(1H, dd, J=7.83Hz, 8.06Hz), 7.55-7.59(1H, m), 8.01-8.04(4H, m), 8.13(1H, d, J=7.83Hz).

### [Example 55]

### 1-[(3S)-dimethylaminopyrrolidinyl]-2-(4-fluorobenzyl)-3-methylpyrido[1,2-a]benzimidazole-4-carbonitrile (#55):

According to the production method for 2-(4-methoxycarbonylbenzyl)-1-[(3S)-dimethylaminopyrrolidin-1-yl]-3-methylpyrido[1,2-a]benzimidazole-4-carbonitrile (#50), but using 1-chloro-2-(4-fluorobenzyl)-3-methylpyrido[1,2-a]benzimidazole-4-carbonitrile (I-30) in place of 1-chloro-2-(4-methoxycarbonylbenzyl)-3-methylpyrido[1,2-a]benzimidazole-4-carbonitrile, the entitled compound was produced.
EIMSm/z:428(M⁺).

### [Example 56]

### 1-[(3S)-dimethylaminopyrrolidinyl]-2-(3-fluorobenzyl)-3-methylpyrido[1,2-a]benzimidazole-4-carbonitrile (#56):

According to the production method for 2-(4-methoxycarbonylbenzyl)-1-[(3S)-dimethylaminopyrrolidin-1-yl]-3-methylpyrido[1,2-a]benzimidazole-4-carbonitrile (#50), but using 1-chloro-2-(3-fluorobenzyl)-3-methylpyrido[1,2-a]benzimidazole-4-carbonitrile (I-31) in place of 1-chloro-2-(4-methoxycarbonylbenzyl)-3-methylpyrido[1,2-a]benzimidazole-4-carbonitrile, the entitled compound was produced.
EIMSm/z:428(M⁺).

### [Example 57]

### 2-(2,4-Difluorobenzyl)-1-[(3S)-dimethylaminopyrrolidinyl]-3-methylpyrido[1,2-a]benzimidazole-4-carbonitrile (#57):

According to the production method for 2-(4-methoxycarbonylbenzyl)-1-[(3S)-dimethylaminopyrrolidin-1-yl]-3-methylpyrido[1,2-a]benzimidazole-4-carbonitrile (#50), but using 1-chloro-2-(2,4-difluorobenzyl)-3-methylpyrido[1,2-a]benzimidazole-4-carbonitrile (I-32) in place of 1-chloro-2-(4-methoxycarbonylbenzyl)-3-methylpyrido[1,2-a]benzimidazole-4-carbonitrile, the entitled compound was produced.
EIMSm/z:448(M⁺).

### [Example 58]

### 2-(4-Carboxybenzyl)-1-[(3S)-dimethylaminopyrrolidin-1-yl]-3-methylpyrido[1,2-a]benzimidazole-4-carbonitrile (#58):

To a mixed tetrahydrofuran/water (1/1, v/v) solution (3 ml) of 200 mg (0.428 mmol) of 2-(4-methoxycarbonylbenzyl)-1-[(3S)-dimethylaminopyrrolidin-1-yl]-3-methylpyrido[1,2-a]benzimidazole-4-carbonitrile (#50) was added 55 mg (1.31 mmol) oflithiumhydroxidemonohydrate, andstirredatroomtemperature for 3 hours. 1.40 ml of 1 N hydrochloric acid was added dropwise to the reaction mixture with cooling with ice-water, and this was then stirred for 10 minutes. The solvent was evaporated under reduced pressure, and the residue was recrystallized from water to obtain 104.7 mg (54 %) of the entitled compound as a pale yellow crystal.
EIMSm/z:454(M⁺).
¹H-NMR(400MHz, CDCl₃)δ: 2.38(6H, brs), 2.38-2.54(2H, m), 2.50(3H, s), 3.23-3.52(4H, m), 3.81(1H, brs), 4.21-4.39(2H, m), 7.36(2H, brd, J=6.28Hz), 7.43(1H, m), 7.56(1H, t, J=7.68Hz), 7.88-7.90(3H, m), 8.01, 8.28(each 0.5H, brs).
IR(KBr): 3417, 2958, 2605, 2224, 1707, 1626, 1595, 1487cm⁻¹.

| Elemental analysis: C₂₇H₂₇N₅O₂·2.0-H₂O,HCl. | | | |
|---|---|---|---|
| Calcd. | C, 61.65%; | H, 6.13%; | N, 13.31% |
| Found | C, 61.52%; | H, 6.26%; | N, 13.21%. |

### [Example 59]

### 1-[(3S)-dimethylaminopyrrolidinyl]-2-(4-hydroxybenzyl)-3-methylpyrido[1,2-a]benzimidazole-4-carbonitrile (#59):

36.7 mg of 10 % palladium-carbon catalyst was added to tetrahydrofuran (4 ml) solution of 200 mg (0.388 mmol) of 2-(4-benzyloxybenzyl)-1-[(3S)-dimethylaminopyrrolidinyl]-3-methylpyrido[1,2-a]benzimidazole-4-carbonitrile (#52). The system was degassed to reduced pressure, then replaced with hydrogen, and stirred under atmospheric pressure at room temperature for 20 hours. The reaction mixture was filtered, and the solvent was evaporated from the filtrate. The residue was separated and purified by preparative HPLC, and then the product was further recrystallized from isopropyl ether/ethyl acetate to obtain 94.5 mg (58 %) of the entitled compound as a pale yellow crystal.
EIMSm/z:426(M⁺)
¹H-NMR(400MHz, DMSO-d₆)δ: 2.03-2.22(2H, m), 2.14(6H, s), 2.39 (3H, s), 3.05-3.34(4H, m), 3.52(1H, brs), 4.03(2H, brs) , 6.60 (2H, d, J=8.54Hz), 6. 96 (2H, d, J=8.30Hz), 7.40-7.44(1H, m), 7.53-7.57(1H, m), 7.87(1H, d, J=8.30Hz), 8.23-8.25(1H, m).

| Elemental analysis: C₂₆H₂₇N₅O₂·0.25-H₂O,0.5-HCO₂H | | | |
|---|---|---|---|
| Calcd. | C, 70.26%; | H, 6.34%; | N, 15.46% |
| Found | C, 70.07%; | H, 6.31%; | N, 15.63%. |

### [Example 60]

### 2-(3-Aminobenzyl)-1-[(3S)-dimethylaminopyrrolidinyl]-3-methylpyrido[1,2-a]benzimidazole-4-carbonitrile (#60):

According to the production method for 1-[(3S)-dimethylaminopyrrolidinyl]-2-(4-hydroxybenzyl)-3-methylpyrido[1,2-a]benzimidazole-4-carbonitrile (#59), but using 1-[(3S)-dimethylaminopyrrolidinyl]-3-methyl-2-(3-nitrobenzyl)pyrido[1,2-a]benzimidazole-4-carbonitrile (#54) in place of 2-(4-benzyloxybenzyl)-1-[(3S)-dimethylaminopyrrolidinyl]-3-methylpyrido(1,2-a]benzimidazole-4-carbonitrile, the entitled compound was produced.
¹H-NMR(400MHz, DMSO-d₆)δ: 1.99-2.23(2H, m), 2.15(6H, s), 2.42(3H, s) , 2.89-3.37(4H, m) , 3.52(1H, brs), 4.00(2H, brs) , 6.29(1H, s), 6.35(1H, d, J=7.57Hz), 6.40(1H, d, J=8.06Hz), 6.95(1H, t, J=7.57Hz), 7.41-7.45(1H, m), 7.54-7.58(1H, m), 7.88(1H, d, J=8.06Hz), 8.09,8.24(each0.5H, brs).
IR(KBr): 3437, 3336, 2864, 2775, 2222, 1624, 1593, 1485cm⁻¹.

| Elemental analysis: C₂₆H₂₈N₆·0.5-H₂O | | | |
|---|---|---|---|
| Calcd. | C, 72.03%; | H, 6.74%; | N, 19.38% |
| Found | C, 72.24%; | H, 6.66%; | N, 19.14%. |

### [Example 61]

### 2-(4-Aminobenzyl)-1-[(3S)-dimethylaminopyrrolidinyl]-3-methylpyrido[1,2-a]benzimidazole-4-carbonitrile (#61):

According to the production method for 1-[(3S)-dimethylaminopyrrolidinyl]-2-(4-hydroxybenzyl)-3-methylpyrido[1,2-a]benzimidazole-4-carbonitrile (#59), but using 1-[(3S)-dimethylaminopyrrolidinyl]-3-methyl-2-(4-nitrobenzyl)pyrido[1,2-a]benzimidazole-4-carbonitrile (#53) in place of 2-(4-benzyloxybenzyl)-1-[(3S)-dimethylaminopyrrolidinyl]-3-methylpyrido[1,2-a]benzimidazole-4-carbonitrile, the entitled compound was produced (this was directly used in the next reaction).
EIMSm/z:425(M⁺)

### [Example 62]

### 2-[4-(N-acetylamino)benzyl]-1-[(3S)-dimethylaminopyrrolidinyl]-3-methylpyrido[1,2-a]benzimidazole-4-carbonitrile (#62):

14.5 µl (0.154 mmol) of acetic anhydride was added to 2 ml of pyridine solution of 43.6 mg (0.103 mmol) of 2-(4-aminobenzyl)-1-[(3S)-dimethylaminopyrrolidinyl]-3-methylpyrido[1,2-a]benzimidazole-4-carbonitrile (#61) at 0°C, and then stirred at room temperature for 1 hour. Pyridine was evaporated, and the residue was separated and purified by preparative HPLC. The product was washed with isopropyl ether and 14.8 mg (31 %) of the entitled compound was obtained as a yellow crystal.
EIMSm/z:467(M⁺)
¹H-NMR(400MHz, CD₃OD)δ: 2.08(3H, s), 2.37(3H, brs), 2.54-2.61 (8H, m), 3.41-3.95 (5H, m), 4.20(2H, m), 7.12(2H, d, J=8.30Hz), 7.39-7.43(1H, m), 7.50-7.52(3H, m), 7.77(1H, d, *J*=8.06Hz), 8.07-8.16(1H, m), 8.46(0.5H, brs).
IR(KBr): 3400, 2949, 2773, 2222, 1593, 1512cm⁻¹.

| Elemental analysis: C₂₉H₃₂N₆O₃·0.5-H₂O | | | |
|---|---|---|---|
| Calcd. | C, 66.78%; | H, 6.38%; | N, 16.11% |
| Found | C, 66.92%; | H, 6.44%; | N, 16.45% |

### [Example 63]

### 1-[(3S)-dimethylaminopyrrolidinyl]-3-methyl-2-[4-[N-(methylsulfonyl)amino]benzyl]pyrido[1,2-a]benzimidazole-4-carbonitrile (#63):

According to the production method for 2-[4-(N-acetylamino)benzyl]-1-[(3S)-dimethylaminopyrrolidinyl]-3-methylpyrido[1,2-a]benzimidazole-4-carbonitrile (Example 62), but using methanesulfonyl chloride in place of acetic anhydride, the entitled compound was produced.
EIMSm/z:467(M⁺).
¹H-NMR(400MHz, CD₃OD)δ: 2.56(8H, brs), 2.67(3H, brs), 2.87-2.91(3H, m) , 3.27-3.90(5H, m), 4.17(2H, brs), 7.12-7.19(4H, m), 7.40(1H, brs), 7.50(1H, brs), 7.76(1H, brs), 8.14(1H, m), 8.45(0.5H, brs).
IR(KBr): 3435, 2222, 1626, 1593, 1479, 1153cm⁻¹.

### [Reference Example 38]

### 2-(2-Hydroxyethyl)-3-methyl-1-oxo-1H,5H-pyrido[1,2-a]benzimidazole-4-carbonitrile (I-38):

A mixture of 5.00 g (31.8 mmol) of (2-benzimidazolyl)acetonitrile, 4.08 g (31.8 mmol) of 2-acetylbutyrolactone and 4.90 g (63.6mmol) of ammonium acetate was heated at 140 to 150°C for 80 minutes. After cooling, 100 ml of water was added thereto, then the solid was crushed and decanted, and 50 ml of acetonitrile was added thereto and washed. The crystal was taken out through filtration to obtain 6.28 g (74 %) of the entitled compound (I-38) as a pale brown crystal. MS(EI)m/z:268(M⁺).
¹H-NMR(400MHz, DMSO-d₆)δ: 2.39 (3H, s), 2.73(2H, t, J=6.84Hz), 3.48(2H, t, J=6.84Hz), 4.57(1H, brs), 7.25-7.39(1H, m), 7.42-7.59(2H, m), 8.56(1H, d, J=7.57Hz).
IR(ATR): 3437, 2200, 1655, 1597, 1537, 1469, 1329, 1242, 1219, 1061, 1041 cm⁻¹.

### [Reference Example 39]

### 2-(2-Acetoxyethyl)-3-methyl-1-oxo-1H,5H-pyrido[1,2-a]benzimidazole-4-carbonitrile (I-39):

Amixture of 2.00 g (7.48 mmol) of 2- (2-hydroxyethyl) -3-methyl-1-oxo-1H,5H-pyrido[1,2-a]benzimidazole-4-carbonitrile (I-38), 1.06 ml (11.2 mmol) of acetic anhydride, 9 mg (75 µmol) of 4-dimethylaminopyridine and 7.24 ml (89.6 mmol) of pyridine was stirred at room temperature for 67 hours. 50 ml of ethyl acetate was added thereto, and the solid was taken out through filtration and recrystallized fromethanol to obtain 1.77 g (77 %) of the entitled compound (I-39) as a pale yellow crystal.
MS(EI)m/z:310(M+H)⁺.
¹H-NMR(400MHz, DMSO-d₆)δ: 1.98(3H, s), 2.42(3H, s), 2.90(2H, t, J=7.08Hz), 4.11(2H, t, J=7.08Hz), 7.32-7.40(1H, m), 7.48-7.57(2H, m), 8.58(1H, d, J=8.30Hz).
IR(ATR): 3194, 2204, 1707, 1657, 1614, 1604, 1549, 1259 cm⁻¹.

### [Reference Example 40]

### 2-(2-Acetoxyethyl)-1-chloro-3-methylpyrido[1,2-a]benzimidazole-4-carbonitrile (I-40):

500 mg (1.62 mmol) of 2-(2-acetoxyethyl)-3-methyl-1-oxo-1H,5H-pyrido[1,2-a]benzimidazole-4-carbonitrile (I-39) in 4 ml of phosphoryl chloride was heated under reflux for 90 minutes. After cooling, phosphoryl chloride was evaporated under reduced pressure, and 20 ml of ice-water was added to the residue. This was extracted with chloroform (50 ml x 3). The chloroform layers were combined and dried over anhydrous magnesium sulfate, and the solvent was evaporated under reduced pressure. The resulting residue was washed with diisopropyl ether and taken out through filtration to obtain 438 mg (83 %) of the entitled compound (I-40) as a yellow crystal.
MS(EI)m/z:328(M+H)⁺.
¹H-NMR(400MHz, DMSO-d₆)δ: 2.00 (3H, s), 2.73 (3H, s), 3.21(2H, t, J=7.10Hz), 4.26(2H, t, J=7.10Hz), 7.42-7.49(1H, m), 7.59-7.66(1H, m), 7.93(1H, d, J=8.08Hz), 8.70(1H, d, J=8.57Hz).
IR(ATR): 2224, 1738, 1624, 1589, 1471, 1448, 1354, 1304, 1234, 1200, 1034 cm⁻¹.

### [Example 64]

### 2-(2-Acetoxyethyl)-1-[(3S)-dimethylaminopyrrolidin-2-yl] 3-methylpyrido[1,2-a]benzimidazole-4-carbonitrile (#64):

To N,N-dimethylformamide (10 ml) suspension of 398 mg (1.21 mmol) of 2-(2-acetoxyethyl)-1-chloro-3-methylpyrido[1,2-a]benzimidazole-4-carbonitrile (I-40) were added 231 µl (1.82 mmol) of (3S)-dimethylaminopyrrolidine and 506 µl (3.63 mmol) of triethylamine. The system was replaced with nitrogen and sealed up, and heated at 80°C for 12 hours. After cooling, the solventwas evaporated under reducedpressure, and the residue was dissolved in 50 ml of ethyl acetate. This was washed with 20 ml of saturated sodium bicarbonate solution, and dried over anhydrous magnesium sulfate. The solvent was evaporated under reduced pressure, and the residue was applied to a silica gel column chromatography. This was eluted with amixedsolventofdichloromethane/methanol (30/1, v/v) toobtain a crude product of the entitled compound. This was recrystallizedfromethanol toobtain 203mg (41 %) of theentitled compound (#64) as a yellow crystal.
MS(EI)m/z:406(M⁺).
¹H-NMR(400MHz, CDCl₃)δ: 2.09(3H, s), 2.18-2.51(3H, m), 2.36(6H, s), 2.76(3H, s), 3.08(3H, brs), 3.25-3.81(3H, m), 4.20-4.32(2H, m), 7.38(1H, t, J=7.83Hz), 7.54 (1H, t, J=7.34Hz), 7.91-8.18(1H, m), 8.00(1H, d, J=8.07Hz).
IR(ATR): 2224, 1739, 1626, 1593, 1506, 1481, 1442, 1367, 1300, 1236, 1155, 1034 cm⁻¹.

| Elemental analysis: C₂₃H₂₇N₅O₂ | | | |
|---|---|---|---|
| Calcd. | C, 68.13%; | H, 6.71%; | N, 17.27% |
| Found | C, 67.93%; | H, 6.67%; | N, 17.40%. |

### [Examples 65 and 66]

### 2,3-Dihydro-4-methyl-1H-furano[2,3-b]pyrido[1,2-a]benzimidazole-5-carbonitrile (#65) and 1-[(3S)-dimethylaminopyrrolidin-1-yl]-2-(2-hydroxyethyl)-3-methylpyrido[1,2-a]benzimidazole-4-carbonitrile (#66):

Aqueous (1 ml) solution of 79 mg (573 µmol) of potassium carbonate was added to methanol (4 ml) solution of 155 mg (382 µmol) of 2-(2-acetoxyethyl)-1-[(3S)-dimethylaminopyrrolidin-1-yl]-3-methylpyrido[1,2-a]benzimidazole-4-carbonitrile (#64) at 0°C, and stirred at room temperature for 3 hours. The solvent was evaporated under reduced pressure, and the residue was dissolved in 50 ml of chloroform, washed with 20 ml of water, and dried over anhydrous magnesium sulfate. The solvent was evaporated under reduced pressure, and the residue was applied to a silica gel column chromatography. From the eluate with dichloromethane/methanol (30/1, v/v) and from the eluate with dichloromethane/methanol (10/1, v/v), crude products of the entitled compound (#65) and the entitled compound (#66) were obtained, respectively. Each was washed with diethyl ether and taken out through filtration to obtain 56mg (59 %) of the entitled compound (#65) as a milky white crystal and 14 mg (10 %) of the entitled compound (#66) as a yellow crystal, respectively. #65
MS(EI)m/z:250(M⁺).
¹H-NMR(400MHz, DMSO-d₆)δ: 2.54 (3H, s), 3.53(2H, t, J=8.79Hz), 5.21(2H, t, J=8.79Hz), 7.33-7.39(1H, m), 7.52-7.57(1H, m), 7.82(1H, d, J=8.30Hz), 8.08(1H, d, J=8.30Hz).
IR(ATR): 2212, 1641, 1560, 1498, 1415, 1267, 1227 cm⁻¹.

| Elemental analysis: C₁₅H₁₁N₃O₅·0.2H₂O | | | |
|---|---|---|---|
| Calcd. | C, 71.25%; | H, 4.54%; | N, 16.62% |
| Found | C, 71.48%; | H, 4.28%; | N, 16.89%. |

#66
MS(EI)m/z:364(M⁺).
¹H-NMR(400MHz, CDCl₃)δ: 2.14-2.46(3H, m), 2.37 (6H, s), 2.56(3H, s), 2.91-3.15(3H, m), 3.36-3.80(3H, m), 3.89-4.07(2H, m), 7.21-7.39(1H, m), 7.46-7.55(1H, m), 7.87-8.11(2H, m).
IR(ATR): 3282, 2225, 1628, 1593, 1477, 1444, 1410, 1375, 1306 cm⁻¹.

| Elemental analysis: C₂₁H₂₅N₅O·0.5H₂O | | | |
|---|---|---|---|
| Calcd. | C, 67.72%; | H, 7.04%; | N, 18.80% |
| Found | C, 67.84%; | H, 6.79%; | N, 18.84%. |

### [Example 67]

### 2-(2-Acetoxyethyl)-1-(2-N',N'-diethylaminoethylamino)-3-methylpyrido[1,2-a]benzimidazole-4-carbonitrile (#67):

To N,N-dimethylformamide (10 ml) suspension of 500 mg (1.53 mmol) of 2-(2-acetoxyethyl)-1-chloro-3-methylpyrido[1,2-a]benzimidazole-4-carbonitrile (I-40) were added 321 µl (2.29 mmol) of N,N-diethylaminoethylamine and 640 µl (4.59 mmol) of triethylamine. The system was replaced with nitrogen and sealed up, and heated at 80°C for 12 hours. After cooling, the solvent was evaporated under reduced pressure, and the residue was dissolved in 50ml of ethyl acetate. The solution was washed with 20 ml of aqueous saturated sodium bicarbonate solution, and dried over anhydrous magnesium sulfate. The solvent was evaporated under reduced pressure, and the residue was applied to a silica gel column chromatography. This was eluted with a mixed solvent of dichloromethane/methanol (30/1, v/v) to obtain a crude product of the entitled compound. This was recrystallized from ethanol to obtain 391 mg (63 %) of the entitled compound (#67) as a yellow crystal.
MS(EI)m/z:408(M⁺).
¹H-NMR(400MHz, CDCl₃)δ: 1.20 (6H, t, J=7.08Hz), 2.06(3H, s), 2.66(4H, q, J=7.08Hz), 2.69(3H, s), 2.73(2H, t, J=5.62Hz), 3.15(2H, t, J=7.08Hz), 3.20-3.28(2H, m), 4.24(2H, t, J=7.08Hz), 5.73(1H, t, J=5.86Hz), 7.31-7.38(1H, m), 7.50-7.58(1H, m), 7.98(1H, d, J=8.05Hz), 8.20(1H, d, J=8.55Hz).
IR(ATR): 3327, 2214, 1732, 1626, 1593, 1500, 1444, 1373, 1304, 1234, 1041 cm⁻¹.

### [Example 68]

### 1-(2-N',N'-diethylaminoethylamino)-2-(2-hydroxyethyl)-3-methylpyrido[1,2-a]benzimidazole-4-carbonitrile (#68):

Aqueous (1 ml) solution of 81 mg (589 µmol) of potassium carbonate was added to methanol (4 ml) solution of 160 mg (393 µmol) of 2-(2-acetoxyethyl)-1-(2-N',N'-diethylaminoethylamino)-3-methylpyrido[1,2-a]benzimidazole-4-carbonitrile (#67) at 0°C, and the mixure was stirred at room temperature for 14 hours. The solvent was evaporated under reduced pressure, and the residue was dissolved in 50 ml of chloroform. The solution was washed with 20 ml of water, and dried over anhydrous magnesium sulfate. The solvent was evaporated under reduced pressure, and the residue was recrystallized from ethanol to obtain 102 mg (71 %) of the entitled compound (#68) as a yellow compound.
MS(EI)m/z:366(M⁺).
¹ H-NMR(400MHz, CDCl₃)δ: 1.12(6H, t, J=7.08Hz), 2.50-2.73(6H, m), 2.68(3H, s), 2.92-3.03(4H, m), 4.13(2H, t, J=5.37Hz), 5.79(1H, t, J=6.10Hz), 7.08-7.14(1H, m) , 7.39-7.46(1H, m), 7.58(1H, d, J=8.30Hz), 7.80(1H, d, J=8.06Hz).
IR(ATR) : 3257, 2216, 1626, 1597, 1496, 1466, 1448, 1371, 1309, 1236, 1200, 1053 cm⁻¹.

### [Example 69]

### 2-(2-Dimethylphosphorylethyl)-1-methoxy-3-methylpyrido[1,2-a]benzimidazole-4-carbonitrile (#69):

A mixture of 100 mg (274 µmol) of 1-(2-N',N'-diethylaminoethylamino)-2-(2-hydroxyethyl)-3-methylpyrido[1,2-a]benzimidazole-4-carbonitrile (#68), 15 mg (137 µmol) of sodium carbonate and 1 ml of trimethyl phosphate was heated at 200°C for 5 minutes. After cooling, 5 ml of water was added thereto, the resulting solid was taken out through filtration, washed with a small amount of diethyl ether and taken out through filtration to obtain 55 mg (52 %) of the entitled compound (#69) as a pale dark brown crystal.
MS(EI)m/z:390(M⁺).
¹H-NMR(400MHz, DMSO-d₆)δ: 2.45 (3H, s) , 2.94-3.02(2H, m), 3.62(6H, dd, J=10. 99, 1.47Hz), 4.01-4.12(2H, m), 4.08(3H, s), 7.37-7.43(1H, m), 7.55-7.62(1H, m), 7.76(1H, d, J=8.30Hz), 8.67(1H, d, J=7.81Hz).
IR(ATR): 2202, 1658, 1604, 1593, 1537, 1477, 1273, 1036, 1012 cm⁻¹.

| Elemental analysis: C₁₈H₂₀N₃O₅P | | | |
|---|---|---|---|
| Calcd. | C, 55.53%; | H, 5.18%; | N, 10.79% |
| Found | C, 55.65%; | H, 5.15%; | N, 10.89%. |

### [Example 70]

### 1-[(3S)-dimethylaminopyrrolidin-1-yl]-3-methyl-2-(2-phenylthioethyl)pyrido[1,2-a]benzimidazole-4-carbonitrile (#70):

144 mg (660 µmol) of diphenyl disulfide was added to a mixture of 80 mg (220 µmol) of 1-[(3S)-dimethylaminopyrrolidin-1-yl]-2-(2-hydroxyethyl)-3-methylpyrido[1,2-a]benzimidazole-4-carbonitrile (#66), 164 µl (660 µmol) of tri-n-butyl phosphine and 178 µl (2.20 mmol) of pyridine at room temperature, and the resulting mixture was stirred for 19 hours. This was diluted with 20 ml of chloroform. The solution was washed with 10 ml of aqueous 1 N sodium hydroxide solution, and dried over anhydrous magnesium sulfate. The solvent was evaporated under reduced pressure, and the residue was applied to a silica gel column chromatography. This was eluted with a mixed solvent of dichloromethane/methanol (20/1, v/v) to obtain a crude product of the entitled compound. This was recrystallized from ethanol to obtain 61 mg (61 %) of the entitled compound (#70) as a yellow crystal.
MS(EI)m/z:456(M⁺).
¹H-NMR(400MHz, CDCl₃)δ: 1.37-1.48(1H, m), 2.02-2.19(1H, m), 2.21-2.41(2H, m), 2.31(6H, s), 2.60(3H, s), 2.91-3.76(7H, m), 7.23-7.60(7H, m), 7.91-8.11(1H, m), 7.99(1H, d, J=8.30Hz).
IR(ATR) : 2222, 1626, 1593, 1483, 1441, 1408, 1371, 1304, 1207, 1155, 1061 cm⁻¹.

| Elemental analysis: C₂₇H₂₉N₅S·0.25H₂O | | | | |
|---|---|---|---|---|
| Calcd. | C, 70.48%; | H, 6.46%; | N, 15.22%; | S, 6.97% |
| Found | C, 70.67%; | H, 6.25%; | N, 15.34%; | S, 7.11%. |

### [Example 71]

### 1-[(3S)-dimethylaminopyrrolidin-1-yl]-2-(2-hetanoyloxyethyl)-3-methylpyrido[1,2-a]benzimidazole-4-carbonitrile (#71):

108 µl (413 µmol) of heptanoic anhydride was added to a mixture of 100 mg (275 µmol) of 1-[(3S)-dimethylaminopyrrolidin-1-yl]-2-(2-hydroxyethyl)-3-methylpyrido[1,2-a]benzimidazole-4-carbonitrile (#66) and 534 µl (6.60 mmol) of pyridine at room temperature, and stirred for 18 hours. The solvent was evaporated under reducedpressure, and the residue was dissolved in 20 ml of ethyl acetate. The solution was washed with 10 ml of aqueous saturated sodium bicarbonate solution and dried over anhydrous magnesium sulfate. The solvent was evaporated under reduced pressure, and the residue was applied to a silica gel column chromatography. This was eluted with a mixed solvent of dichloromethane/methanol (30/1, v/v) to obtain a crude product of the entitled compound. This was recrystallized from ethyl acetate/n-hexane to obtain 69 mg (53 %) of the entitled compound (#71) as a yellow crystal.
MS(EI)m/z:476(M⁺).
¹H-NMR(400MHz, CDCl₃)δ: 0.83-0.92(3H, m), 1.28(6H, brs), 1.51-1.68(2H, m), 2.15-2.50(5H, m) , 2.36(6H, s), 2.77(3H, s), 3.07(3H, brs), 3.25-3.80 (3H, m), 4.21-4.33(2H, m) , 7.33-7.41(1H, m), 7.50-7.59(1H, m), 7.97-8.15(1H, m), 8.00(1H, d, J=8.07Hz).
IR(ATR): 2224, 1728, 1624, 1589, 1475, 1446, 1306, 1169 cm⁻¹.

| Elemental analysis: C₂₈H₃₇N₅O₂ | | | |
|---|---|---|---|
| Calcd. | C, 70.71%; | H, 7.84%; | N, 14.72% |
| Found | C, 70.42%; | H, 7.82%; | N, 14.69%. |

### [Reference Example 41]

### 2-Ethoxycarbonylmethyl-3-methyl-1-oxo-1H,5H-pyrido[1,2-a]benzimidazole-4-carbonitrile (I-41):

A mixture of 3.00 g (19.1 mmol) of (2-benzimidazolyl)acetonitrile, 4.13 g (19.1 mmol) of diethyl acetyl succinate and 2.94 g (38.2 mmol) of ammonium acetate was heated at 140 to 150°C for 90 minutes. After cooling, 100 ml of water was added thereto, the solid was crushed and decanted, and 50 ml of acetonitrile was added thereto and washed. The crystal was taken out through filtration to obtain 1.91 g (32 %) of the entitled compound (I-41) as a pale brown crystal.
MS(EI)m/z:310(M⁺).
¹H-NMR(400MHz, DMSO-d₆)δ: 1.19(3H, t, J=7.10Hz), 2.36(3H, s), 3.65(2H, s), 4.07(2H, dd, J=14.21, 7.10Hz), 7.33-7.40(1H, m), 7.49-7.57(2H, m), 8.55(1H, d, J=8.08Hz).
IR(ATR): 2210, 1743, 1653, 1529, 1464, 1367, 1323, 1192, 1155, 1026 cm⁻¹.

### [Reference Example 42]

### 1-Chloro-2-ethoxycarbonylmethyl-3-methylpyrido[1,2-a]-benzimidazole-4-carbonitrile (I-42):

1.90 g (6.14 mmol) of 2-ethoxycarbonylmethyl-3-methyl-1-oxo-1H,5H-pyrido[1,2-a]benzimidazole-4-carbonitrile (I-41) in 7 ml of phosphoryl chloride was heated under reflux for 2.5 hours. After cooling, phosphoryl chloride was evaporated under reduced pressure, and 20 ml of ice-water and 20 ml of aqueous 1 N sodium hydroxide solution were added to the residue. This mixture was extracted with chloroform (50 ml × 3). The chloroform layers were combined and dried over anhydrous magnesium sulfate, and the solvent was evaporated under reduced pressure. The resulting residue was washed with diisopropyl ether and taken out through filtration to obtain 1.91 g (95 %) of the entitled compound (I-42) as a yellow crystal.
MS(EI)m/z:328(M+H)⁺.
¹ H-NMR(400MHz, DMSO-d₆)δ: 1.21 (3H, t, J=7. 07Hz) , 2. 65 (3H, s), 4.04(2H, s), 4.15(2H, dd, J=14.14, 7.07Hz), 7.43-7.50(1H, m), 7.61-7.68(1H, m), 7.95(1H, d, J=8.04Hz), 8.67(1H, d, J=8.78Hz).
IR(ATR) : 2225, 1738, 1626, 1591, 1473, 1329, 1300, 1205, 1176, 1024 cm⁻¹

### [Example 72]

### 1-[(3S)-dimethylaminopyrrolidin-1-yl]-2-ethoxycarbonylmethyl-3-methylpyrido[1,2-a]benzimidazole-4-carbonitrile (#72):

To N,N-dimethylformamide (30 ml) suspension of 900 mg (2.75 mmol) of 1-chloro-2-ethoxycarbonylmethyl-3-methylpyrido[1,2-a]benzimidazole-4-carbonitrile were added 523 µl (4.12 mmol) of (3S)-dimethylaminopyrrolidine and 1.15 ml (8.25 mmol) of triethylamine. The system was replaced with nitrogen and sealed up, and heated at 80°C for 16 hours. After cooling, the solvent was evaporated under reduced pressure, and the residue was dissolved in 50 ml of ethyl acetate. This was washed with 20 ml of saturated sodium bicarbonate solution, and dried over anhydrous magnesium sulfate. The solvent was evaporated under reduced pressure, and the residue was applied to a silica gel column chromatography. This was eluted with a mixed solvent of dichloromethane/methanol (30/1, v/v) to obtain a crude product of the entitled compound. This was recrystallized from ethanol/n-hexane to obtain 859 mg (77 %) of the entitled compound (#72) as a yellow crystal.
MS(EI)m/z:406(M⁺).
¹H-NMR(400MHz, CDCl₃)δ: 1.30(3H, t, J=7.08Hz), 2.15-2.50(3H, m), 2.34 (6H, s), 2.61(3H, s), 2.97-3.93(6H, m), 4.18-4.35 (2H, m), 7.33-7.44(1H, m), 7.52-7.61(1H, m), 7.91-8.17(1H, m), 8.01(1H, d, J=8.55Hz).
IR(ATR): 2220, 1730, 1479, 1442, 1369, 1302, 1211, 1173, 1157, 1022 cm⁻¹.

| Elemental analysis: C₂₃H₂₇N₅O₂ | | | |
|---|---|---|---|
| Calcd. | C, 68.13%; | H, 6.71%; | N, 17.27% |
| Found | C, 67.85%; | H, 6.70%; | N, 17.29%. |

### [Reference Example 43]

### 3-Methyl-1-oxo-1H,5H-pyrido[1,2-a]benzimidazole-4-carbonitrile (I-43):

A mixture of 3.00 g (19.1 mmol) of (2-benzimidazolyl)acetonitrile, 2.43 ml (19.1 mmol) of ethyl acetoacetate and 2.94 g (38.2mmol) of ammoniumacetate was heated at 140 to 150°C for 50 minutes. After cooling, 100 ml of water was added thereto, then the solid was crushed and decanted, and 50 ml of acetonitrile was added thereto and washed. The crystal was taken out through filtration to obtain 3.63 g (85 %) of the entitled compound (I-43) as a pale brown crystal.
MS(EI)m/z:224(M⁺).
¹H-NMR(400MHz, DMSO-d₆)δ: 2.35(3H, s), 5.95(1H, s), 7.31-7.40(1H, m), 7.48-7.59(2H, m), 8.54(1H, d, J=8.06Hz).
IR(ATR): 2206, 1662, 1566, 1522, 1454, 1396, 1369, 1325, 1279, 1252, 1211, 1113, 1078, 1012 cm⁻¹.

### [Reference Example 44]

### 1-Chloro-3-methylpyrido[1,2-a]benzimidazole-4-carbonitrile (I-44):

1.50 g (6.72 mmol) of 3-methyl-1-oxo-1H,5H-pyrido[1,2-a]benzimidazole-4-carbonitrile (I-43) in 5 ml of phosphoryl chloride was heated under reflux for 2 hours. After cooling, phosphoryl chloride was evaporated under reduced pressure, and 20 ml of ice-water and 20 ml of aqueous 1 N sodium hydroxide solution were added to the residue. This mixture was extracted with chloroform (50 ml × 3). The chloroform layers were combined and dried over anhydrous magnesium sulfate, and the solvent was evaporated under reduced pressure. The resulting residue was washed with diisopropyl ether and taken out through filtration to obtain 1.04 g (64 %) of the entitled compound (I-44) as a yellow crystal.
MS (EI)m/z:242 (M+H)⁺.
¹H-NMR(400MHz, DMSO-d₆)δ: 2.62 (3H, s), 7.33(1H, m), 7.45(1H, dd, J=8.55, 7.33Hz), 7.62(1H, dd, J=8.30, 7.32Hz), 7.93(1H, d, J=8.30Hz), 8.63(1H, d, J=8.55Hz).
IR(ATR): 2222, 1626, 1595, 1496, 1444, 1358, 1286, 1171 cm⁻¹.

### [Example 73]

### 1-[(3S)-dimethylaminopyrroldin-1-yl]-3-methylpyrido[1,2-a]benzimidazole-4-carbonitrile (#73):

To N, N-dimethylformamide (8 ml) suspension of 200 mg (828 µmol) of 1-chloro-3-methylpyrido[1,2-a]benzimidazole-4-carbonitrile (I-44) were added 126 µl (993 µmol) of (3S)-dimethylaminopyrrolidine and 346 µl (2.48 mmol) of triethylamine. The system was replaced with nitrogen and sealed up, and heated at 140°C for 20 minutes. After cooling, the solvent was evaporated under reduced pressure, and the residue was dissolved in 30 ml of chloroform. The solution was washed with 15 ml of aqueous saturated sodium bicarbonate solution, and dried over anhydrous magnesium sulfate. The solvent was evaporated under reduced pressure, and the residue was applied to a silica gel column chromatography. This was eluted with a mixed solvent ofdichloromethane/methanol (20/1, v/v) toobtainacrudeproduct of the entitled compound. This was recrystallized from ethyl acetate to obtain 151 mg (57 %) of the entitled compound (#73) as a yellow crystal.
MS(EI)m/z:320(M⁺).
¹H-NMR(400MHz, CDCl₃)δ: 2.01-2.23(1H, m), 2.28-2.40(1H, m), 2.32(6H, s), 2.65(3H, s), 3.00-3.20(1H, m), 3.20-3.39(2H, m), 3.51(2H, brs), 6.25(1H, s), 7.32-7.40(1H, m), 7.50-7.58(1H, m), 7.98(2H, d, J=8.55Hz).
IR(ATR) : 2222, 1628, 1597, 1514, 1475, 1442, 1417, 1348, 1308, 1201, 1153, 1117 cm⁻¹.

| Elemental analysis: C₁₉H₂₁N₅ | | | |
|---|---|---|---|
| Calcd. | C, 71.45%; | H, 6.63%; | N, 21.93% |
| Found | C, 71.40%; | H, 6.91%; | N, 21.86%. |

### [Reference Example 45]

### 3-Methyl-1-oxo-2-(3-phthalimidopropyl)-1H,5H-pyrido[1,2-a]benzimidazole-4-carbonitrile (I-45):

A mixture of 1.98 g (12.6 mmol) of (2-benzimidazolyl)acetonitrile, 4.00 g (12.6 mmol) of ethyl 2-(3-phthalimidopropyl)acetoacetate and 1.94 g (25.2 mmol) of ammonium acetate was heated at 140 to 150°C for 20 minutes. After cooling, 100 ml of water was added thereto, then the solid was crushed and decanted, and 50 ml of acetonitrile was added thereto and washed. The crystal was taken out through filtration to obtain 4.43 g (86 %) of the entitled compound (I-45) as a pale brown crystal.
MS(EI)m/z:411(M⁺).
¹H-NMR(400MHz, DMSO-d₆)δ: 1.74-1.85(2H, m), 2.36(3H, s), 2.58-2.65(2H, m), 3.64(2H, t, J=7.08Hz), 7.29-7.36(1H, m), 7.45-7.53(2H, m), 7.77-7.87(4H, m), 8.52(1H, d, J=8.06Hz).
IR(ATR): 2208, 1697, 1668, 1606, 1543, 1468, 1398, 1038 cm⁻¹.

### [Reference Example 46]

### 1-Chloro-3-methyl-2-(3-phthalimidopropyl)pyrido[1,2-a]benzimidazole-4-carbonitrile (I-46):

1.00 g (2.44 mmol) of 3-methyl-1-oxo-2-(3-phthalimidopropyl)-1H,5H-pyrido[1,2-a]benzimidazole-4-carbonitrile (I-45) in 5 ml of phosphoryl chloride was heated under reflux for 4.5 hours. After cooling, phosphoryl chloride was evaporated under reduced pressure, and 20 ml of ice-water and 20 ml of aqueous 1 N sodium hydroxide solution were added to the residue. This mixture was extracted with chloroform (50 ml × 3). The chloroform layers were combined and dried over anhydrous magnesium sulfate, and the solvent was evaporated under reduced pressure. The resulting residue was washed with diisopropyl ether and taken out through filtration to obtain 962 mg (92 %) of the entitled compound (I-46) as a yellow crystal. MS(EI)m/z:429(M+H)⁺.
¹H-NMR(400MHz, CDCl₃)δ: 1.96-2.07(2H, m), 2.72(3H, s), 2.90-3.00(2H, m), 3.85-3.94 (2H, m), 7.37-7.44(1H, m), 7.57-7.64(1H, m), 7.72-7.79(2H, m), 7.85-7.92(2H, m), 8.00-8.05(1H, m), 8.53-8.60(1H, m).
IR(ATR) : 2224, 1707, 1466, 1435, 1400, 1371, 1306, 1225, 1188, 1028 cm⁻¹.

### [Example 74]

### 1-[(3S)-dimethylaminopyrroldin-1-yl]-3-methyl-2-(3-phthalimidopropyl)pyrido[1,2-a]benzimidazole-4-carbonitrile (#74):

To N,N-dimethylformamide (55 ml) suspension of 2.33 g (5.43 mmol) of 1-chloro-3-methyl-2-(3-phthalimidopropyl)pyrido[1,2-a]benzimidazole-4-carbonitrile (I-46) were added 827 µl (6.52 mmol) of (3S)-dimethylaminopyrrolidine and 2.27 ml (16.3 mmol) of triethylamine. The system was replaced with nitrogen and sealed up, and heated at 80°C for 17 hours. After cooling, the solvent was evaporated under reduced pressure, and the residue was dissolved in 100 ml of chloroform. This solution was washed with 50 ml of saturated sodium bicarbonate solution, and dried over anhydrous magnesium sulfate. The solvent was evaporated under reduced pressure, and the residue was applied to a silica gel column chromatography. This was eluted with a mixed solvent of dichloromethane/methanol (20/1, v/v) to obtain a crudeproduct of the entitled compound. This was washed with diisopropyl ether and taken out through filtration to obtain 2.13 g (77 %) of the entitled compound (#74) as a yellow crystal.
MS(EI)m/z:507 (M⁺).
¹H-NMR(400MHz, CDCl₃)δ: 1.93-2.05(2H, m), 2.08-2.38(2H, m), 2.25(6H, s), 2.64-2.81(2H, m), 2.68(3H, s), 3.15-3.75(5H, m), 3.85-3.96(2H, m), 7.31-7.40(1H, m), 7.49-7.58(1H, m), 7.72-7.80(2H, m), 7.85-7.92(2H, m), 7.95-8.08(1H, m), 7.98(1H, d, J=8.33Hz).
IR(ATR): 2222, 1705, 1500, 1466, 1442, 1400, 1369, 1308, 1221 cm⁻¹.

### [Reference Example 47]

### 2-(3-Aminopropyl)-1-[(3S)-dimethylaminopyrrolidin-1-yl]-3-methylpyrido[1,2-a]benzimidazole-4-carbonitrile trihydrochloride (I-47):

40 ml of aqueous 6 N hydrochloric acid solution was added to acetic acid (40 ml) solution of 2.10 g (4.15 mmol) of 2-[(3S)-dimethylaminopyrroldin-1-yl]-3-methyl-2-(3-phthalimidopropyl)pyrido[1,2-a]benzimidazole-4-carbonitrile (#74) at room temperature, and heated under reflux for 18 hours. After cooling, the solvent was evaporated under reduced pressure, the residue was dissolved in 50 ml of water, the insoluble material was removed through filtration, and the solvent was evaporated from the filtrate. The resulting residue was dissolved in 40 ml of ethanol, and 971 µl (13.9 mmol) of propylene oxide was added thereto at 0°C, and stirred at room temperature for 14 hours. After cooling with ice, 80 ml of ethyl acetate was added thereto, and the precipitate was taken out through filtration to obtain 1.65 g (82 %) of the entitled compound (I-47) as a yellow crystal.
MS(EI)m/z:377(M⁺).
IR(ATR): 3380, 2222, 1628, 1595, 1477, 1444, 1373, 1298, 1157 cm⁻¹.

### [Example 75]

### 2-[3-(N-acetylamino)propyl]-1-[(3S)-dimethylaminopyrrolidin-1-yl]-3-methylpyrido[1,2-a]benzimidazole-4-carbonitrile (#75):

287 µl (2.06 mol) of triethylamine was added to dichloromethane (5 ml) suspension of 200 mg (412 µmol) of 2-(3-aminopropyl)-1-[(3S)-dimethylaminopyrrolidin-1-yl]-3-methylpyrido[1,2-a]benzimidazole-4-carbonitrile trihydrochloride (I-47) and 58 µl (617 µmol) of acetic anhydride at 0°C, and the resulting mixure was stirred at room temperature for 22 hours. 20 ml of water was added thereto, and this mixure was extracted with chloroform (50 ml x 3). The chloroform layers were combined and dried over anhydrous magnesium sulfate, and the solvent was evaporated under reduced pressure. The residue was applied to a silica gel column chromatography. This was eluted with a mixed solvent of dichloromethane/methanol (10/1, v/v) to obtain a crude product of the entitled compound. This was recrystallized from diisopropyl ether/n-hexane to obtain 74 mg (43 %) of the entitled compound (#75) as a pale yellow crystal.
MS(EI)m/z:419(M+H)⁺.
¹H-NMR(400MHz, CDCl₃)δ: 1.77-1.88(2H, m), 2.05(3H, s), 2.21(1H, brs), 2.36(6H, s), 2.40-2.49(1H, m) , 2.62-2.80(2H, m), 2.68(3H, s), 3.10(1H, brs), 3.20-3.80(6H, m), 7.32-7.40(1H, m), 7.48-7.59(1H, m), 7.90-8.18(1H, m), 7.99(1H, d, J=8.06Hz).
IR(ATR): 2224, 1639, 1562, 1504, 1487, 1442, 1371, 1306 cm⁻¹.

| Elemental analysis: C₂₄H₃₀N₆O·1.5H₂O | | | |
|---|---|---|---|
| Calcd. | C, 64.70%; | H, 7.47%; | N, 18.86% |
| Found | C, 64.92%; | H, 7.40%; | N, 19.22%. |

### [Example 76]

### 2-[3-(N-tert-butoxycarbonylamino)propyl]-1-[(3S)-dimethylaminopyrrolidin-1-yl]-3-methylpyrido[1,2-a]benzimidazole-4-carbonitrile (#76):

287 µl (2.06 mol) of triethylamine was added to dichloromethane (4 ml) suspension of 200 mg (412 µmol) of 2-(3-aminopropyl)-1-[(3S)-dimethylaminopyrrolidin-1-yl]-3-methylpyrido[1,2-a]benzimidazole-4-carbonitrile trihydrochloride (I-47) and 142 µl (617 µmol) of di-tert-butyl dicarbonate at 0°C, and the resulting mixure was stirred at room temperature for 16 hours. The solvent was evaporated under reduced pressure, and the residue was dissolved in 50 ml of chloroform. This solution was washed with 25 ml of aqueous saturated sodium bicarbonate solution, and dried over anhydrous magnesium sulfate. The solvent was evaporated under reduced pressure, and the residue was applied to a silica gel column chromatography. This was eluted with a mixed solvent of dichloromethane/methanol (30/1, v/v) to obtain a crude product of the entitled compound. This was recrystallized from ethyl acetate/n-hexane to obtain 119 mg (61 %) of the entitled compound (#76) as a yellow crystal.
MS(EI)m/z:477(M+H)⁺.
¹H-NMR(400MHz, CDCl₃)δ: 1.47(9H, s), 1.79(2H, brs), 2.18-2.30(1H, m), 2.32-2.47(1H, m) , 2.37 (6H, s), 2.65-2.78(2H, m), 2.69(3H, s), 3.02-3.78(7H, m), 7.32-7.40(1H, m), 7.50-7.57(1H, m), 7.98-8.16(1H, m), 8.00(1H, d, J=8.30Hz).
IR(ATR) : 2224, 1682, 1518, 1508, 1491, 1444, 1365, 1311, 1277, 1244, 1165, 1140 cm⁻¹.

| Elemental analysis: C₂₇H₃₆N₆O₂·1.5H₂O | | | |
|---|---|---|---|
| Calcd. | C, 64.39%; | H, 7.81%; | N, 16.69% |
| Found | C, 64.44%; | H, 7.34%; | N, 16.73 |

### [Example 77]

### 2-(3-Aminopropyl)-1-[(3S)-dimethylaminopyrrolidin-1-yl]-3-methylpyrido[1,2-a]benzimidazole-4-carbonitrile trihydrochloride (#77):

1.5 ml of concentrated hydrochloric acid was added to methanol (3 ml) solution of 75 mg (157 µmol) of 2-[3-(N-tert-butoxycarbonylamino)propyl]-1-[(3S)-dimethylaminopyrrolidin-1-yl]-3-methylpyrido[1,2-a]benzimidazole-4-carbonitrile (#76) at 0°C, and the resulting mixture was stirred at room temperature for 2.5 hours. The solvent was evaporated under reduced pressure, and the residue was washed with dii sopropyl ether ad taken out through filtration to obtain 58 mg (76 %) of the entitled compound (#77) as a yellow crystal.
MS (EI)m/z:377 (M⁺).
¹H-NMR(400MHz, D₂O)δ: 1.94(2H, brs), 2.43(1H, brs), 2.68(3H, s), 2.79(3H, brs) , 2.89(3H, s), 3.00(3H, s), 3.12-3.21(2H, m), 3.38-4.08(4H, m), 4.28-4.40(1H, m), 7.49-7.58(1H, m), 7.61-7.73(2H, m), 7.88-8.08(1H, m).
IR(ATR): 3359, 2225, 1631, 1520, 1477, 1412, 1379 cm⁻¹.

### [Example 78]

### 5-Methyl-1,2,3,4-tetrahydropyrido[2,3-b]pyrido[1,2 a]benzimidazole-6-carbonitrile (#78):

29 µl (592 µmol) of hydrazine monohydrate was added to ethanol (4 ml) suspension of 200 mg (395 µmol) of 1-[(3S)-dimethylaminopyrroldin-1-yl]-3-methyl-2-(3-phthalimidopropyl)pyrido[1,2-a]benzimidazole-4-carbonitrile (#74) at room temperature, and the reslting mixture was heated under reflux for 80 minutes. After cooling, the precipitate was taken out through filtration to obtain 82 mg (79 %) of the entitled compound (#78) as a yellow crystal.
MS(EI)m/z:263(M⁺).
¹H-NMR(400MHz, DMSO-d₆)δ: 1.82-1.90(2H, m), 2.45(3H, s), 2.69-2.75(2H, m), 3.51-3.59(2H, m), 7.24-7.30(1H, m), 7.46-7.52(1H, m), 7.73(1H, d, J=8.08Hz), 7.83-7.90(1H, m), 8.42(1H, d, J=8.33Hz).
IR(ATR): 3437, 2202, 1624, 1591, 1550, 1522, 1454, 1408, 1336, 1304, 1265, 1244, 1201 cm⁻¹.

### [Reference Example 48]

### Methyl 2-butyl-3-oxo-valerate (I-48):

To acetone (25 ml) solution of 1. 30 g (10.0mmol) of methyl 3-oxo-valerate were added 1.07 ml (10.0 mmol) of 1-bromobutane and 1.11 g of potassium carbonate, and the resulting mixture was heated at 60°C for 2 hours, and further heated under reflux for 2 hours. After cooling, the reaction mixture was filtered, and the solvent was evaporated from the filtrate. The residue was applied to a silica gel flash column chromatography. This was eluted with a mixed solvent of n-hexane/ethyl acetate (97/3, v/v) to obtain 477 mg (32 %) of the entitled compound as a colorless oil (this was directly used in the next reaction).
¹H-NMR(400MHz, CDCl₃)δ: 0.89(3H, t, J=7.20Hz), 1.07(3H, t, J=7.20Hz), 1.23-1.36(4H, m), 1.81-1.88(2H, m), 2.46-2.63(2H, m), 3.45(1H, t, J=7.45Hz), 3.72(3H, s).

### [Reference Example 49]

### Ethyl 2-butyl-3-oxo-n-caproate (I-49):

According to the production method for methyl 2-butyl-3-oxo-valerate (Reference Example 48) but using ethyl 3-oxo-n-caproate in place of methyl 3-oxo-valerate, the entitled compound was produced.
¹H-NMR(400MHz, CDCl₃)δ: 0.89(3H, t, J=7.09Hz), 0.91(3H, t, J=7.34Hz), 1.26(3H, t, J=7.09Hz), 1.31-1.36(4H, m), 1.59-1.64(2H, m), 1.80-1.87 (2H, m) , 2.43-2.56(2H, m), 3.41(1H, t, J=7.34Hz), 4.18(2H, q, J=7.09Hz).

### [Reference Example 50]

### Ethyl 2-butyl-3-oxo-n-heptanoate (I-50):

According to the production method for methyl 2-butyl-3-oxo-valerate (Reference Example 48) but using ethyl 3-oxo-n-heptanoate in place of methyl 3-oxo-valerate, the entitled compound was produced.
¹H-NMR(400MHZ, CDCL₃)δ: 0.89(3H, t, J=7.24Hz), 0.90(3H, t, J=7.34Hz), 1.24-1.36(6H, m), 1.26(3H, t, J=7.09Hz), 1.53-1.60(2H, m), 1.80-1.87(2H, m), 2.43-2.57(2H, m), 3.41(1H, t, J=7.34Hz), 4.18(2H, q, J=7.09Hz).

### [Reference Example 51]

### Methyl 2-butyl-4-methoxy-3-oxo-butyrate (I-51):

According to the production method for methyl 2-butyl-3-oxo-valerate (Reference Example 48) but using methyl 4-methoxy-3-oxo-butyrate in place of methyl 3-oxo-valerate, the entitled compound was produced.
¹H-NMR(400MHz, CDCl₃)δ: 0.89(3H, t, J=7.75Hz), 1.24-1.34(4H, m), 1.85-1.87 (2H, m), 3.42 (3H, s), 3.58 (1H, t, J=7.24Hz), 3.72 (3H, s), 4.10(1H, s), 4.11(1H, s).

### [Reference Example 52]

### Ethyl 2-butyl-4-methyl-3-oxo-valerate (I-52):

According to the production method for methyl 2-butyl-3-oxo-valerate (Reference Example 48) but using ethyl 4-methyl-3-oxo-valerate in place of methyl 3-oxo-valerate, the entitled compound was produced.
¹H-NMR(400MHz, CDCl₃)δ: 0.89(3H, t, J=7.20Hz), 1.10(3H, d, J=6.84Hz), 1.12(3H, d, J=6.35Hz), 1.21-1.34(4H, m), 1.26(3H, t, J=7.32Hz), 1.83-1.86(2H, m), 2.79(1H, dq, J=6.84Hz, 6.84Hz), 3.59(1H, t, J=7.20Hz), 4.17(2H, q, J=7.33Hz).

### [Reference Examples 53 to 56]

### 2-Butyl-3-R^{d}-1-oxo-1H,5H-pyrido[1,2-a]benzimidazole-4-carbonitrile (I-53 to I-56):

A mixture of (2-benzimidazolyl)acetonitrile, R^{c}-3-R^{d}-3-oxo-propionate (I-48 to I-51), and ammonium acetate was heated at 140 to 150°C for 30 minutes. After cooling, 10 ml of water was added thereto, then the solid was crushed and decanted, and 5 ml of acetonitrile was added thereto and washed. The crystal was taken out through filtration to obtain the entitled compound of the following formula as a pale red crystal, as in Table 4 (this was directly used in the next reaction).

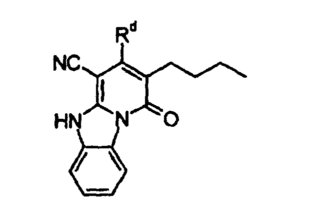

**Table 4**

| Example | Rd | Rc | MS EIMS m/s |
|---|---|---|---|
| 53 | ethyl | methyl | 294 |
| 54 | n-propyl | ethyl | 308 |
| 55 | n-butyl | ethyl | 322 |
| 56 | (2-methoxymethyl) | methyl | 310 |

### [Reference Example 57]

### 2-Butyl-3-isopropyl-1-oxo-1H,5H-pyrido[1,2-a]benzimidazole-4-carbonitrile (I-57):

According to the production method for 2-butyl-3-R-1-oxo-1H,5H-pyrido[1,2-a]benzimidazole-4-carbonitrile (I-53 to I-56) but using 2-(benzimidazolyl)acetonitrile and ethyl 3-isopropyl-3-oxo-propionate in place of R^{c}-3-R^{d}-3-oxo-propionate, a crude product of the entitled compound was obtained. This was applied to a flash column chromatography eluting with a mixed solution of chloroform/ethyl acetate (97/3, v/v) to obtain 229 mg of a mixture of the entitled compound and 3-isopropyl-1-oxo-1H,5H-pyrido[1,2-a]benzimidazole-4-carbonitrile as a brown crystal. EIMSm/z:312(M⁺)

### [Reference Examples 58 to 61]

### 1-Chloro-2-butyl-3-R-1H,5H-pyrido[1,2-a]benzimidazole-4-carbonitrile (I-58 to I-61):

2-Butyl-3-R^{d}-1-oxo-1H,5H-pyrido[1,2-a]benzimidazole-4-carbonitrile (I-53 to I-58) in 3 ml of phosphoryl chloride was heated under reflux for 16 hours. After cooling, phosphoryl chloride was evaporated under reduced pressure, and the residue was dissolved in 15 ml of chloroform. 10 ml of ice water and 30 ml of aqueous 1 N sodium hydroxide were added thereto, and the resulting mixture was stirred for 15 minutes. The organic layer was extracted with chloroform (20 ml × 3). The combined chloroform layer was washed with brine, and dried over anhydrous magnesium sulfate. The solvent was evaporated under reduced pressure to give a residue, which was washed with a small amount of diisopropyl ether, and then taken out through filtration to obtain the entitled compound of the following formula, as in Table 5.

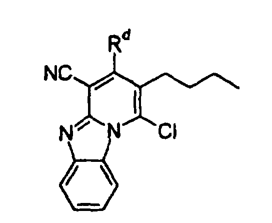

**Table 5**

| Example | Rd | MS EIMS m/s |
|---|---|---|
| 58 | ethyl | 312 |
| 59 | n-propyl | 325 |
| 60 | n-butyl | 340 |
| 61 | (2-methoxymethyl) | 328 |

### [Reference Example 62]

### 1-chloro-2-butyl-3-isopropyl-1H,5H-pyrido[1,2-a]benzimidazole-4-carbonitrile (I-62):

According to the production method for 1-chloro-2-butyl-3-R-1H,5H-pyrido[1,2-a]benzimidazole-4-carbonitrile (I-58 to I-61), but using 2-butyl-3-isopropyl-1-oxo-1H,5H-pyrido[1,2-a]benzimidazole-4-carbonitrile (I-57) in place of 2-butyl-3-R-1-oxo-1H,5H-pyrido[1,2-a]benzimidazole-4-carbonitrile, a crude product of the entitled compound was obtained. This was applied to a flash column chromatography, and eluted with a mixed solution of chloroform/ethyl acetate (95/5, v/v) to obtain 274 mg of a mixture of the entitled compound and 1-chloro-3-isopropyl-1H,5H-pyrido[1,2-a]benzimidazole-4-carbonitrile as a yellow crystal.
EIMSm/z : 325 (M⁺).

### [Examples 79 to 83]

### 2-Butyl-1-[(3S)-dimethylaminopyrroldinyl]-3-R-2-[4-[N-(methylsulfonyl)amino]benzyl]pyrido[1,2-a]benzimidazole-4-carbonitrile (#79 to #83):

To N,N-dimethylformamide (2 ml) suspension of 1-chloro-2-butyl-3-R^{d}-1H,5H-pyrido[1,2-a]benzimidazole-4-carbonitrile (I-58 to I-62, 0.10 mmol) were added 41.8 µl (0.30 mmol) of triethylamine and 24.5 µl (0.20 mmol) of (3S)-dimethylaminopyrrolidine. The system was replaced with nitrogen and sealed up, and heated at 80°C for 20 hours. After cooling, the reaction mixture was separated and purified by preparative HPLC to obtain the entitled compound of the following formula, as in Table 6.

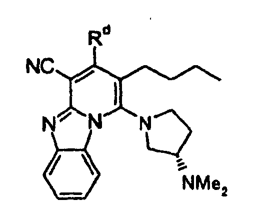

**Table 6**

| Example | Rd | MS EIMS m/s |
|---|---|---|
| 79 | ethyl | 389 |
| 80 | n-propyl | 403 |
| 81 | n-butyl | 417 |
| 82 | (2-methoxymethyl) | 406 |
| 83 | i-propyl | 403 |

### [Reference Example 63]

### Ethyl α-n-decylacetoacetate (I-63):

To acetone (10 ml) solution of 650 mg (5.00 mmol) of ethyl acetoacetate were added 1.25 ml (6.00 mmol) of 1-bromodecane and 700 mg of potassium carbonate, and the resulting mixture was heated at 60°C for 2 hours, and then further heated under reflux for 2 hours. After cooling, the reaction mixture was filtered, and the solvent was evaporated from the filtrate. The residue was dissolved in 30 ml of ethyl acetate, and the solution was washed successively with 10 ml of aqueous 10 % potassium carbonate solution and 10 ml of brine. The organic layer was taken out and dried over anhydrous sodium sulfate. The solvent was evaporated under reduced pressure to give a residue, which was applied to a silica gel column chromatography. This was eluted with a mixed solvent of n-hexane/ethyl acetate (9/1 to 6/1, v/v) to obtain 392 mg (29 %) of the entitled compound as a colorless oil (this was directly used in the next reaction).
¹H-NMR(400MHz, CDCl₃)δ: 0.88(3H, t, J=7.10Hz), 1.20-1.37(19H, m), 1.76-1.91 (2H, m) , 2.22 (3H, s), 3.39(1H, t, J=7.59Hz) 4.19(2H, q, J=7.10Hz).

### [Reference Example 64]

### Ethyl α-(1-cyclohexen-3-yl)acetoacetate (I-64):

According to the production method for (I-63), 1.95 g (15.0 mmol) of ethyl acetoacetate and 2.07 ml (18.0 mmol) of 3-bromocyclohexene in 40 ml of acetone in the presence of potassium carbonate (2.5 g) were heated under reflux for 3.5 hours, and then the mixture was purified to obtain 2.68 g (85 %) of the entitled compound as a colorless oil (this was directly used in the next reaction).
¹H-NMR(400MHz, CDCl₃)δ: 1.27(3H, t, J=7.32Hz), 1.30-1.38(1H, m), 1.53-1.65(1H, m), 1.68-1.85(2H, m), 1.98-2.02(2H, m), 2.24(3H, d, J=3.17Hz), 2.90-3.01(2H, m), 3.36(1H, dd, J=7.57, 9.77Hz), 4.17-4.23(2H, m), 5.42-5.52(1H, m), 5.74-5.82(1H, m).

### [Reference Example 65]

### 2-N-decyl-3-methyl-1-oxo-1H,5H-pyrido[1,2-a]benzimidazole-4-carbonitrile (I-65):

A mixture of 227 mg (1.44 mmol) of (2-benzimidazolyl)acetonitrile, 390 mg (1.44 mmol) of ethyl α-n-decylacetoacetate (I-63) and 223 mg (2.88 mmol) of ammonium acetate was heated at 140 to 150°C for 1.5 hours. After cooling, 10 ml of water was added thereto, and the solid was crushed and decanted. 5 ml of acetonitrile was added thereto and washed, and the crystal was taken out through filtration to obtain 432 mg (82 %) of the entitled compound as a pale brown crystal.
MS(EI)m/z:363(M⁺).
¹H-NMR(400MHz, DMSO-d₆)δ: 0.87(3H, t, J=6.86Hz), 1.19-1.43(14H, m), 1.45-1.57(2H, m), 2.47(3H, s), 2.67(2H, t, J=7.84Hz), 7.28-7.36(1H, m), 7.45(2H, d, J=4.16Hz), 8.76(1H, d, J=8.08Hz).
IR(ATR): 2915, 2848, 2202, 1666, 1552, 1465 cm⁻¹.

### [Reference Example 66]

### 2-(1-Cyclohexen-3-yl)-3-methyl-1-oxo-1H,5H-pyrido[1,2-a]benzimidazole-4-carbonitrile (I-66):

A mixture of 314 mg (2.00 mmol) of (2-benzimidazolyl)acetonitrile, 421 mg (2.00 mmol) of ethyl α-(1-cyclohexen-3-yl)acetoacetate (I-64) and 308 mg (4.00 mmol) of ammonium acetate was heated at 140 to 150°C for 1 hour. After cooling, 10 ml of water was added thereto, and the solid was crushed and decanted. 5 ml of acetonitrile was added thereto and washed, and the crystal was taken out through filtration to obtain 324 mg (53 %) of the entitled compound as a colorless crystal.
MS(EI)m/z:303(M⁺).
¹ H-NMR (400MHz, CDCl₃)δ: 1.60-2.28(6H, m) ,2.55(3H, s), 4.10(1H, brs), 5.67(1H, t, J=10.25Hz), 5.78-5.88(1H, m), 7.33-7.38(1H, m), 7.46(2H, d, J=3.19Hz), 8.78(1H, d, J=8.06Hz).
IR(ATR): 2200, 1653, 1614, 1541, 1465 cm⁻¹.

### [Reference Example 67]

### 2-(1-Cyclohexyl)-3-methyl-1-oxo-1H,5H-pyrido[1,2-a]benzimidazole-4-carbonitrile (I-67):

500 mg of 10 % palladium-carbon (water content 50 %) was added to ethanol (80 ml) solution of 340 mg (1.12 mmol) of 2-(1-cyclohexen-3-yl)-3-methyl-1-oxo-1H,5H-pyrido[1,2-a]benzimidazole-4-carbonitrile (I-66). The mixture was hydrogenated at room temperature under 1 atmosphere for 1 hour. The palladium-carbon catalyst was removed through filtration, and the solvent was evaporated from the filtrate to obtain 340 mg (99 %) of the entitled compound as a colorless crystal.
MS (FAB) m/z : 305 (M⁺).
¹H-NMR(400MHz, CDCl₃)δ: 1.25-1.43(3H, m),1.55(2H, brd, J=12.0Hz), 1.71(1H, brs), 1.78-1.90(2H, m), 2.25-2.42(2H, m), 2.50(3H, s), 2.83(1H, brs), 7.25-7.33(1H, m), 7.38-7.47(2H, m) , 8.74(1H, d, J=8.08Hz).
IR(ATR): 2208, 1655, 1608, 1537, 1466 cm⁻¹.

### [Reference Example 68]

### 2-Isopropyl-3-methyl-1-oxo-1H,5H-pyrido[1,2-a]benzimidazole-4-carbonitrile (I-68):

A mixture of 1.57 g (10.0 mmol) of (2-benzimidazolyl)acetonitrile, 1.72 g (10.0 mmol) of ethyl α-isopropylacetoacetate and 1.54 g (20.0 mmol) of ammonium acetate was heated at 140 to 150°C for 1 hour. After cooling, 20 ml of water was added thereto, and the solid was crushed and decanted. 10 ml of acetonitrile was added thereto and washed, and the crystal was taken out through filtration to obtain 1.76 g (66 %) of the entitled compound as a colorless crystal.
MS(EI)m/z:265(M⁺).
¹H-NMR(400MHz, CDCl₃)δ: 1.41(6H, d, J=6.84Hz), 2.49(3H, s), 3.23-3.32(1H, m), 7.25-7.36(1H, m), 7.38-7.52(2H, m), 8.75(1H, dd, J=0.73, 8.06Hz).
IR(ATR): 2200, 1653, 1614, 1541, 1465 cm⁻¹.

### [Reference Example 69]

### 1-Chloro-2-n-decyl-3-methylpyrido[1,2-a]benzimidazole-4-carbonitrile (I-69):

According to the production method for (I-2), 402 mg (1.11 mmol) of 2-n-decyl-3-methyl-1-oxo-1H,5H-pyrido[1,2-a]benzimidazole-4-carbonitrile (I-65) in 7 ml of phosphoryl chloride was heated under reflux for 1 hour to obtain 369 mg (87 %) of the entitled compound as a yellow crystal.
MS(EI)m/z:381(M⁺).
¹H-NMR(400MHz, CDCl₃)δ: 0.89(3H, t, J=6.84Hz), 1.24-1.42(12H, m), 1.43-1.52(2H, m), 1.54-1.65(2H, m), 2.73(3H, s), 2.82-2.90 (2H, m), 7.38-7.42(1H, m), 7.57-7.61(1H, m) , 8.03(1H, d, J=8.30Hz),8.60(1H, d, J=8.55Hz).
IR(ATR): 2918, 2852, 2223, 1624, 1591, 1469 cm⁻¹.

### [Reference Example 70]

### 1-Chloro-2-(1-cyclohexen-3-yl)-3-methylpyrido[1,2-a]benzimidazole-4-carbonitrile (I-70):

According to the production method for (I-2) , 299 mg (0.99 mmol) of 2-(1-cyclohexen-3-yl)-3-methyl-1-oxo-1H,5H-pyrido[1,2-a]benzimidazole-4-carbonitrile (I-66) in 5 ml of phosphoryl chloride was heated under reflux for 2 hours to obtain 276 mg (87 %) of the entitled compound as a yellow crystal. MS(EI)m/z:321(M⁺).
¹H-NMR(400MHz, CDCl₃)δ: 1.75-1.90(2H, m),1.96-2.08(2H, m), 2.18-2.24(2H, m), 2.82(3H, brs), 4.40(1H, brs) , 7.39(1H, t, J=8.57Hz), 7.59(1H, t, J=8.33Hz), 8.02(1H, d, J=7.10Hz), 8.60(1H, d, J=8.33Hz).
IR(ATR): 2222, 1622, 1589 cm⁻¹.

### [Reference Example 71]

### 1-Chloro-2-cyclohexyl-3-methylpyrido[1,2-a]benzimidazole-4-carbonitrile (I-71):

According to the production method for (I-2), 340 mg (1.11 mmol) of 2-cyclohexyl-3-methyl-1-oxo-1H,5H-pyrido[1,2-a]benzimidazole-4-carbonitrile (I-67) in 10 ml of phosphoryl chloride was heated under reflux for 2 hours to obtain 340 mg (94 %) of the entitled compound as a yellow crystal. MS(EI)m/z:323(M⁺).
¹H-NMR(400MHz, CDCl₃)δ: 1.15-1.55(3H, m), 1.68-1.89(2H, m), 1.90-2.02(2H, m), 2.28-2.42(1H, m), 2.75-3.18(6H, m), 7.41-7.46(1H, m), 7.62(1H, t, J=7.32Hz), 8.07(1H, d, J=8.30Hz), 8.65(1H, d, J=8.55Hz).
IR(ATR): 2927, 1624, 1444 cm⁻¹.

### [Reference Example 72]

### 1-Chloro-2-isopropyl-3-methylpyrido[1,2-a]-benzimidazole-4-carbonitrile (I-72):

According to the production method for (I-2), 1.71 g (6.45 mmol) of 2-isopropyl-3-methyl-1-oxo-1H,5H-pyrido[1,2-a]benzimidazole-4-carbonitrile (I-68) in 20 ml of phosphoryl chloride was heated under reflux for 2 hours to obtain 1.57 g (86 %) of the entitled compound as a yellow crystal. MS(EI)m/z:283(M⁺).
¹H-NMR(400MHz, CDCl₃)δ: 1.50(6H, d, J=7.35Hz), 2.81(3H, s), 3.50-3.80(1H, m), 7.38-7.45(1H, m) , 7.58-7.64(1H, m) , 8.04(1H, dd, J=0.74, 8.34Hz), 8.62(1H, dd, J=0.74, 8.57Hz).
IR(ATR): 2224, 1624, 1589, 1444 cm⁻¹.

### [Example 84]

### 2-n-Decyl-1-[(3S)-dimethylaminopyrrolidin-1-yl]-3-methylpyrido[1,2-a]benzimidazole-4-carbonitrile (#84):

To N,N-dimethylformamide (6ml) suspension of 346 mg (0.91 mmol) of 1-chloro-2-n-decyl-3-methylpyrido[1,2-a]-benzimidazole-4-carbonitrile (I-69) were added 173 µl (1.37 mmol) of (3S)-dimethylaminopyrrolidine and 253 µl (1.82 mmol) of triethylamine. The system was replaced with nitrogen and sealed up, and heated at 80°C for 20 hours. After cooling, the solvent was evaporated under reduced pressure, and the residue was dissolved in 50 ml of chloroform. This solution was washed successively with 20 ml of water, 20 ml of saturated sodium bicarbonate solution and 20 ml of brine, and dried over anhydrous magnesium sulfate. The solvent was evaporated under reduced pressure, and the residue was applied to a silica gel column chromatography. This was eluted with a mixed solvent of chloroform/methanol (99/1 to 95/5, v/v) to obtain a crude product of the entitled compound. This was separated and purified by preparative TLC, and then recrystallized from n-hexane/2-propanol to obtain 130 mg (31 %) of the entitled compound as a yellow crystal.
MS(EI)m/z:459(M⁺).
¹H-NMR(400MHz, CDCl₃)δ: 0.89(3H, t, J=6.89Hz), 1.20-1.85(18H, m), 2.10-3.80(7H, m), 2.35(6H, s), 2.69(3H, s), 2.44(3H, s), 2.46(3H, s), 2.60-2.73(2H, m), 7.30-7.40(1H, m), 7.52(1H, t, J=8.08Hz), 7.97(1.5H, brd, J=8.08Hz), 8.10(0.5H, brs).
IR(ATR): 2920, 2222, 1626, 1593, 1443 cm⁻¹.

| Elemental analysis::C₂₉H₄₁N₅ | | | |
|---|---|---|---|
| Calcd. | C, 75.77%; | H, 8.99%; | N, 15.24% |
| Found | C, 75.40%; | H, 9.03%; | N, 14.99%. |

### [Example 85]

### 2-(1-Cyclohexen-3-yl)-1-[(3S)-dimethylaminopyrrolidin-1-yl]-3-methylpyrido[1,2-a]benzimidazole-4-carbonitrile (#85):

To N,N-dimethylformamide (6 ml) suspension of 264 mg (0.82 mmol) of 1-chloro-2-(1-cyclohexen-3-yl)-3-methylpyrido[1,2-a]benzimidazole-4-carbonitrile (I-70) were added 156 µl (1.23 mmol) of (3S)-dimethylaminopyrrolidine and 228 µl (1.64 mmol) of triethylamine. The system was replaced with nitrogen and sealed up, and heated at 80°C for 6.5 hours. After cooling, the solvent was evaporated under reduced pressure, and the residue was dissolved in 50 ml of chloroform. This solution was washed successively with 20 ml of water, 20 ml of saturated sodium bicarbonate solution and 20 ml of brine, and dried over anhydrous magnesium sulfate. The solvent was evaporated under reduced pressure, and the residue was applied to a silica gel column chromatography. This was eluted with a mixed solvent of chloroform/methanol (97/3, v/v) to obtain a crude product of the entitled compound. This was recrystallized from methanol/ethanol to obtain 60 mg (18 %) of the entitled compound as a pale yellow crystal.
MS(EI)m/z:399(M⁺).
¹H-NMR(400MHz, CDCl₃)δ: 1.68-2.12(6H, m), 2.15-2.30(3H, m), 2.35 (6H, s), 2.35-2.47(1H, m), 2.77 (3H, s), 2.98-3.21(1H, m), 3.23-4.08 (3H, m) , 5.58-5.70 (1H, m) , 5.92 (1H, brs), 7.30-7.38 (1H, m), 7.52(1H, t, J=8.08Hz), 7.98(1H, d, J=8.08Hz), 8.05(1H, dd, J=8.08, 45.1Hz).
IR(ATR): 2222, 1626, 1593, 1483 cm⁻¹.

| Elemental analysis: C₂₅H₂₉N₅ | | | |
|---|---|---|---|
| Calcd. | C, 75.15%; | H, 7.32%; | N, 17.53% |
| Found | C, 74.87%; | H, 7.27%; | N, 17.39%. |

### [Example 86]

### 2-Cyclohexyl-1-[(3S)-dimethylaminopyrrolidin-1-yl]-3-methylpyrido[1,2-a]benzimidazole-4-carbonitrile (#86):

To N,N-dimethylformamide (8ml) suspension of 320 mg (0.99 mmol) of 1-chloro-2-cyclohexyl-3-methylpyrido[1,2-a]benzimidazole-4-carbonitrile (I-71) were added 188 µl (1.48 mmol) of (3S)-dimethylaminopyrrolidine and 412 µl (2 .96 mmol) of triethylamine. The system was replaced with nitrogen and sealed up, and heated at 80°C for 3 hours. After cooling, the solvent was evaporated under reduced pressure, and the residue was dissolved in 60 ml of chloroform. This solution was washed with 20 ml of water, 20 ml of saturated sodiumbicarbonate solution and 20 ml of brine, and dried over anhydrous magnesium sulfate. The solvent was evaporated under reduced pressure, and the residue was applied to a silica gel column chromatography. This was eluted with a mixed solvent of chloroform/methanol (97/3, v/v) to obtain a crude product of the entitled compound. This was recrystallized from n-hexane/isopropanol to obtain 110 mg (28 %) of the entitled compound as a pale yellow crystal.
MS (EI)m/z:401 (M⁺).
¹H-NMR(400MHz, CDCl₃)δ: 1.28-1.60(3H, m), 1.70-2.13(7H, m), 2.18-2.30(1H, m), 2.36(6H, s), 2.36-2.50(1H, m), 2.86(3H, s), 2.94-3.78(6H, m), 7.30-7.40(1H, m), 7.52(1H, t, J=7.35Hz), 7.97(1H, d, J=8.08Hz), 8.06(1H, dd, J=7.84, 59.0Hz).
IR(ATR): 2224, 1626, 1587, 1473, 1441 cm⁻¹.

| Elemental analysis: C₂₅H₃₁N₅·0.5H₂O | | | |
|---|---|---|---|
| Calcd. | C, 73.14%; | H, 7.86%; | N, 17.12% |
| Found | C, 73.46%; | H, 7.68%; | N, 17.12%. |

### [Example 87]

### 2-Isopropyl-1-[(3S)-dimethylaminopyrrolidin-1-yl]-3-methylpyrido[1,2-a]benzimidazole-4-carbonitrile (#87):

To N,N-dimethylformamide (8ml) suspensionof 309mg (1.09 mmol) of 1-chloro-2-isopropyl-3-methylpyrido[1,2-a]benzimidazole-4-carbonitrile (I-72) were added 207 µl (1.63 mmol) of (3S)-dimethylaminopyrrolidine and 454 µl (3.27 mmol) of triethylamine. The system was replaced with nitrogen and sealed up, and heated at 80°C for 6 hours. After cooling, the solvent was evaporated under reduced pressure, and the residue was dissolved in 60 ml of chloroform. This solution was washed with 20 ml of water, 20ml of saturated sodium bicarbonate solution and 20 ml of brine, and dried over anhydrous magnesium sulfate. The solvent was evaporated under reduced pressure, and the residue was applied to a silica gel column chromatography. This was eluted with a mixed solvent of chloroform/methanol (97/3, v/v) to obtain a crude product of the entitled compound. This was recrystallized from n-hexane/chloroform/diethyl ether to obtain 214 mg (54 %) of the entitled compound as a pale yellow crystal.
MS(EI)m/z:361(M⁺).
¹H-NMR(400MHz, CDCl₃)δ: 1.47(6H, d, J=7.35Hz), 2.18-2.50(2H, m), 2.36(6H, s), 2.82 (3H, s), 2.98-3.80 (6H, m), 7.30-7.92(1H, m), 7.52(1H, t, J=7.59Hz), 7.98(1.5H, d, J=7.84Hz), 8.08-8.15(0.5H, m).
IR(ATR): 2224, 1626, 1591, 1485, 1442 cm⁻¹.

| Elemental analysis: C₂₂H₂₇N₅·0.5H₂O | | | |
|---|---|---|---|
| Cacld. | C, 72.20%; | H, 7.57%; | N, 19.14% |
| found | C, 72.49%; | H, 7.71%; | N, 19.30%. |

### [Reference Example 73]

### Ethyl 3-cyclopropyl-3-oxopropionate (I-73):

To ethyl acetate (100 ml) suspension of potassium ethyl malonate (17.0 g, 0.10 mol) were added triethylamine (34.7 ml, 0.25 mol) and magnesium chloride (14.3 g, 0.15 mol) with cooling with ice, and then the resulting mixture was stirred at 40°C for 20 hours. To tetrahydrofuran (50 ml) solution of cyclopropanecarboxylicacid (4.30 g, 50.0 mmol) were added oxalyl chloride (4.36 ml, 50.0 mmol) and a catalytic amount of N,N-dimethylformamide with cooling with ice, and the resulting mixture was stirred as such for 1 hour and then at room temperature for 1 hour. The above-mentioned malonic acid solution was added to this acid chloride solution with cooling with ice, and the resulting mixture was stirred at room temperature for 20 hours. The reaction mixture was poured into 300 ml of aqueous 10 % citric acid solution, and the mixture was extracted with ethyl acetate (300 ml × 3). The organic layer was successively washed with 500 ml of aqueous saturated sodium bicarbonate solution and 300 ml of brine, and dried over sodium sulfate. The solvent was evaporated, and7.26g (93%) of the entitled compound was obtained as a colorless oil (this was directly used in the next reaction). ¹H-NMR(400MHz, CDCl₃)δ: 0.94-0.99(2H, m), 1.10-1.15(2H, m), 1.28(3H, t, J=7.08Hz), 2.01-2.06(1H, m), 3.57 (2H, s), 4.21(2H, q, J=7.08Hz).

### [Reference Example 74]

### Ethyl 2-cyclopropanecarbonylhexanoate (I-74):

To acetone (50 ml) solution of 3.50 g (22.4 mmol) of ethyl 3-cyclopropyl-3-oxopropionate were added 2.89 ml (26.9 mmol) of 1-bromobutane and 3 g of potassium carbonate, and the resulting mixture was heated under reflux for 7 hours. After cooling, the reaction mixture was filtered, and the solvent was evaporated from the filtrate. The residue was applied to a silica gel column chromatography. This was eluted with a mixed solvent of n-hexane/ethyl acetate (97/3, v/v) to obtain 2.56 g (54 %) of the entitled compound as a colorless oil (this was directly used in the next reaction).
¹H-NMR(400MHz, CDCl₃)δ: 0.88-0.94(5H, m),1.06-1.09(2H, m), 1.23-1.36(7H, m), 1.85-1.92(2H, m) , 2.04-2.10(1H, m) , 3.53(1H, d, J=7.32Hz), 4.20(2H, q, J=7.08Hz).

### [Reference Example 75]

### 2-n-Butyl-3-cyclopropyl-1-oxo-1H,5H-pyrido[1,2-a]benzimidazole-4-carbonitrile (I-75):

A mixture of 475 mg (3.02 mmol) of (2-benzimidazolyl)acetonitrile, 642 mg (3.02 mmol) of ethyl 2-cyclopropanecarbonylhexanoate (I-74) and 466 mg (6.04 mmol) of ammonium acetate was heated at 140 to 150°C for 3.5 hours. After cooling, 30 ml of water was added thereto, and the solution was washed with 10 ml of water. The organic layer was dried over sodium sulfate, and the solvent was evaporated. The residue was applied to a silica gel column chromatography and eluted with a mixed solvent of chloroform/methanol (97/3, v/v) to obtain a crude product of the entitled compound. This was washed with diethyl ether and taken out through filtration to obtain 62 mg (7 %) of the entitled compound as a colorless crystal.
MS(EI)m/z:305(M⁺).
¹H-NMR(400MHz, CDCl₃)δ: 0.92-1.02(5H, m), 1.18-1.24(2H, m), 1.41-1.50(2H, m), 1.54-1.61 (2H, m), 1.99-2.05(1H, m), 2.83-2.90(2H, m), 7.31-7.37(1H, m) , 7.44-7.47 (2H, m), 8.77(1H, d, J=8.30Hz), 10.91(1H, brs).
IR(ATR): 2218, 1662, 1610, 1541, 1466 cm⁻¹.

### [Reference Example 76]

### 2-n-Butyl-1-chloro-3-cyclopropylpyrido[1,2-a]benzimidazole-4-carbonitrile (I-76):

According to the production method for (I-2), 610 mg (2.00 mmol) of 2-n-butyl-3-cyclopropyl-1-oxo-1H,5H-pyrido[1,2-a]benzimidazole-4-carbonitrile (I-75) in 20 ml of phosphoryl chloride was heated under reflux for 30 minutes to obtain 459 mg (71 %) of the entitled compound as a yellow crystal.
MS(EI)m/z:323(M⁺).
¹H-NMR(400MHz, CDCl₃)δ: 1.02(3H, t, J=7.32Hz), 1.09-1.13(2H, m),1.32-1.38(2H, m), 1.48-1.65(4H, m), 2.08-2.15(1H, m), 3.08-3.12(2H, m), 7.38-7.42(1H, m), 7.56-7.61(1H, m), 8.04(1H, d, J=8.30Hz), 8.61(1H, d, J=8.55Hz).
IR(ATR): 2231, 1618, 1587, 1462 cm⁻¹.

### [Example 88]

### 2-n-Butyl-3-cyclopropyl-1-[(3S)-dimethylaminopyrrolidin-1-yl]pyrido[1,2-a]benzimidazole-4-carbonitrile (#88):

To N,N-dimethylformamide (7 ml) suspension of 324 mg (1.00 mmol) of 2-n-butyl-1-chloro-3-cyclopropylpyrido[1,2-a]benzimidazole-4-carbonitrile (I-76) were added 190 µl (1.50 mmol) of (3S)-dimethylaminopyrrolidine and 278 µl (2.00 mmol) of triethylamine. The system was replaced with nitrogen and sealed up, and heated at 80°C for 10 hours. After cooling, the solvent was evaporated under reduced pressure, and the residue was dissolved in 60 ml of chloroform. This solution was washed with 20 ml of water, 20ml of saturated sodium bicarbonate solution and 20 ml of brine, and dried over anhydrous sodium sulfate. The solvent was evaporated under reduced pressure, and the residue was applied to a silica gel column chromatography. This was eluted with a mixed solvent of chloroform/methanol (97/3, v/v) to obtain a crude product of the entitled compound. This was separated and purified by preparative TLC, and then recrystallized from n-hexane/diethyl ether/dichloromethane to obtain 250 mg (62 %) of the entitled compound as a yellow crystal.
MS(EI)m/z:401(M⁺).
¹H-NMR(400MHz, CDCl₃)δ: 1.00-1.12(5H, m), 1.31(2H, brd, J=8.55Hz), 1.50-1.70(4H, m),2.09-2.48(3H, m), 2.36(6H, s), 2.82-3.80(7H, s), 7.34(1H, brt, J=7.32Hz), 7.52(1H, t, J=7.08Hz), 7.99(1.5H, d, J=8.30Hz), 8.10(0.5H, brs).
IR(ATR): 2224, 1622, 1589, 1479, 1441 cm⁻¹.

| Elemental analysis: C₂₅H₃₁N₅ | | | |
|---|---|---|---|
| Calcd. | C, 74.78%; | H, 7.78%; | N, 17.44% |
| Found | C, 74.62%; | H, 8.05%; | N, 17.43%. |

### [Examples 89 and 90]

### 2-Benzyloxycarbonylmethyl-1-[(3S)-dimethylaminopyrrolidin-1-yl]-3-methylpyrido[1,2-a]benzimidazole-4-carbonitrile (#89) and 2-cyanomethyl-1-[(3S)-dimethylaminopyrrolidin-1-yl]-3-methylpyrido[1,2-a]benzimidazole-4-carbonitrile (#90):

Tetrahydrofuran (20 ml) solution of 2.00 g (10.4 mmol) of benzyl acetoacetate was added dropwise to tetrahydrofuran (60 ml) suspension of 499 mg (12.5 mmol) of sodium hydride under a nitrogen atmosphere at 0°C, and the resulting mixture was stirred at room temperature for 40 minutes, and then tetrahydrofuran (20 ml) solution of 3. 58 g (15.6mmol) of benzyl 2-bromoacetate was added dropwise thereto at 0°C, and the resulting mixture was heated under reflux for 14 hours. After cooling, 50 ml of water and 50 ml of saturated ammonium chloride solution were added thereto, and the resulting mixture was extracted with ethyl acetate (100 ml × 3). The ethyl acetate layers were combined and dried over anhydrous magnesium sulfate. And the solvent was evaporated under reduced pressure. The residue was applied to a column chromatography. This was eluted with a mixed solvent of n-hexane/ethyl acetate (10/1, v/v) to obtain 2.95 g of a colorless oil.

A mixture of 2.95 g of the above colorless oil, 1.23 g (7.83 mmol) of (2-benzimidazolyl) acetonitrile and 1.21 g (15.7 mmol) of ammonium acetate was heated at 140 to 150°C for 3 hours. After cooling, the solvent was evaporated. 50 ml of water was added to the residue, and the solid was crushed and decanted. 50ml of acetani trile was added thereto and washed, and the crystal was taken out through filtration to obtain 481 mg of a dark brown crystal.

481 mg of the above dark brown crystal in 3 ml of phosphoryl chloride was heated under reflux for 2 hours. After cooling, phosphoryl chloride was evaporated under reduced pressure. 30 ml of ice-water and 30 ml of aqueous 1 N sodium hydroxide solution were added to the residue, and this mixture was then extracted with chloroform (50 ml × 3). The chloroform layers were combined and dried over anhydrous magnesium sulfate. The solvent was evaporated under reduced pressure. The resulting residue was washed with diisopropyl ether and taken out through filtration to obtain 336 mg of a yellow crystal.

To N,N-dimethylformamide (10 ml) suspension of 336 mg of the above yellow crystal were added 164 µl (1.29 mmol) of (3S)-dimethylaminopyrrolidine and 360 µl (2.58 mmol) of triethylamine. The system was replaced with nitrogen and sealed up, and heated at 80°C for 16 hours. After cooling, the solvent was evaporated under reduced pressure, and the residue was dissolved in 50 ml of ethyl acetate. This solution was washed with 20 ml of saturated sodium bicarbonate solution, and dried over anhydrous magnesium sulfate. The solvent was evaporated under reduced pressure, and the residue was applied to a silica gel column chromatography. From the eluate with dichloromethane/methanol (20/1, v/v), crude products of the entitled compound (#89) and the entitled compound (#90) were obtained. These were recrystallized from ethanol to obtain 80 mg (1.6 %) of the entitled compound (#89) as a yellow crystal, and 88 mg (2.4 %) of the entitled compound (#90) as a yellow crystal.
#89
MS(EI)m/z:468(M⁺).
¹ H-NMR(400MHz, CDCl₃)δ: 2.08-2.39(3H, m), 2.27(6H, s), 2.54(3H, s), 2.85-3.06(1H, m), 3.12-3.95(5H, m), 5.21(2H, brs), 7.29-7.35(6H, m) , 7.50-7.60(1H, m), 7.89-8.10(1H, m), 8.00(1H, d, J=8.06Hz).
IR(ATR): 2224, 1732, 1487, 1444, 1375, 1306, 1213, 1151 cm⁻¹.

| Elemental analysis: C₂₈H₂₉N₅O₂ | | | |
|---|---|---|---|
| Calcd. | C, 71.93%; | H, 6.25%; | N, 14.98% |
| Found | C, 72.18%; | H, 6.23%; | N, 14.91%. |

#90
MS(EI)m/z:359(M⁺).
¹H-NMR(400MHz, CDCl₃)δ: 2.28-2.90(3H, m), 2.38(6H, s), 2.79(3H, s), 3.02-3.10(1H, m), 3.29-4.05(5H, m), 7.38-7.44(1H, m), 7.52-7.60(1H, m), 7.90-8.03(1H, m), 8.01(1H, d, J=8.35Hz).
IR(ATR): 2225, 1630, 1597, 1508, 1442, 1412, 1375, 1306 cm⁻¹.

| Elemental analysis: C₂₁H₂₂N₆·0.25H₂O | | | |
|---|---|---|---|
| Calcd. | C, 69.49%; | H, 6.25%; | N, 23.15% |
| found | C, 69.55%; | H, 6.28%; | N, 22.76%. |

### [Example 91]

### [4-Cyano-1-[(3S)-dimethylaminopyrrolidin-1-yl]-3-methylpyrido[1,2-a]benzimidazol-2-yl]acetic acid (#91):

6 mg of 10 % palladium-carbon (water content 50 %) was added to methanol (5 ml) suspension of 61 mg (130 µmol) of 2-benzyloxycarbonylmethyl-1-[(3S)-dimethylaminopyrrolidin-1-yl]-3-methylpyrido[1,2-a]benzimidazole-4-carbonitrile (#89). Hydrogenation of the compound was carried out at room temperature under 1 atmosphere of hydrogen for 17 hours. 5 ml of methanol was added thereto to dissolve the precipitate, and then the palladium-carbon catalyst was removed through filtration. The solvent was evaporated from the filtrate to obtain a crude product of the entitled compound (#91). This was recrystallized from ethanol to obtain 23 mg (47 %) of the entitled compound (#91) as a yellow crystal.
MS(FABI)m/z:378(M⁺).
¹H-NMR(400MHz, DMSO-d₆)δ: 2.04-2.18(1H, m), 2.24 (6H, s), 2.27-2.37(1H, m), 2.57(3H, s) , 2.97-3.90(7H, m), 7.36-7.45(1H, m), 7.50-7.58(1H, m), 7.85(1H, d, J=8.31Hz), 8.10(1H, brs).
IR(ATR): 2218, 1701, 1628, 1595, 1491, 1441, 1369, 1304, 1186 cm⁻¹.

| Elemental analysis: C₂₁H₂₃N₅O₂·0.25H₂O | | | |
|---|---|---|---|
| calcd. | C, 65.27%; | H, 6.26%; | N, 18.12% |
| Found | C, 65.22%; | H, 6.32%; | N, 18.10%. |

### [Reference Example 77]

### Ethyl 3-methoxy-2-phenylacrylate (I-77):

3.88 g (11.3 mmol) of methoxymethyltriphenylphosphonium chloride was added to tetrahydrofuran (80 ml) suspension of 415 mg (10.4 mmol) of sodium hydride under a nitrogen atmosphere at 0°C, and the resulting mixture was stirred at room temperature for 1.5 hours. Then, tetrahydrofuran (20 ml) solution of 1. 50 ml (9.44 mmol) of ethyl phenylglyoxylate was added dropwise thereto at 0°C, and the resulting mixture was stirred at room temperature for 16 hours. 50 ml of water was added thereto, and the mixture was extracted with ethyl acetate (100 ml × 3). The ethyl acetate layers were combined, and dried over anhydrous magnesium sulfate, and the solvent was evaporated under reduced pressure. The residue was applied to a silica gel column chromatography, and eluted with amixed solvent of n-hexane/ethyl acetate (10/1, v/v) to obtain 1.4 8 g (76%) of the entitled compound (I-77) as a colorless oil.
MS(EI)m/z:207(M⁺).
¹H-NMR(400MHz, CDCl₃)δ: 1.276(1.35H, t, J=7.08Hz), 1.279(1.65H, t, J=7.08Hz), 3.85(1.35H, s) , 3.90(1.65H, s), 4.19-4.30(2H, m), 6.64(0.55H, s), 7.22-7.40(5H, m), 7.55(0.45H, s).
IR(ATR): 1697, 1626, 1250, 1188, 1119, 1053, 1028 cm⁻¹.

### [Reference Example 78]

### 1-Oxo-2-phenyl-1H,5H-pyrido[1,2-a]benzimidazole-4 carbonitrile (I-78):

A mixture of 303 mg (1.92 mmol) of (2-benzimidazolyl)acetonitrile, 397 mg (1.92 mmol) of ethyl 3-methoxy-2-phenylacrylate (I-77) and 296 mg (3.84 mmol) of ammonium acetate was heated at 140 to 150°C for 3.5 hours. After cooling, 10 ml of water was added thereto, and the solid was crushed and decanted. 10 ml of acetonitrile was added thereto and washed, and the crystal was taken out through filtration to obtain 105 mg (19 %) of the entitled compound (I-78) as a dark brown crystal.
MS(EI)m/z:286(M⁺).
¹H-NMR(400MHz, DMSO-d₆)δ: 7.28(1H, t, J=7.35Hz), 7.34-7.45(3H, m), 7.51-7.61(2H, m), 7.75(2H, d, J=7.10Hz), 8.07(1H, s), 8.69(1H, d, J=8.33Hz).
IR(ATR): 2204, 1662, 1614, 1558, 1487, 1464, 1446, 1271, 1230, 1126 cm⁻¹.

### [Reference Example 79]

### 1-Chloro-2-phenylpyrido[1,2-a]benzimidazole-4-carbonitrile (I-79):

97 mg (340 µmol) of 1-oxo-2-phenyl-1H,5H-pyrido[1,2-a]benzimidazole-4-carbonitrile (I-78) in 2 ml of phosphoryl chloride was heated under reflux for 3 hours. After cooling, phosphoryl chloride was evaporated under reduced pressure, 15 ml of ice-water and 15 ml of aqueous 1 N sodium hydroxide solution were added to the residue, and this was extracted with chloroform (50 ml × 3). The chloroform layers were combined and dried over anhydrous magnesium sulfate, and the solvent was evaporated under reduced pressure. The resulting residue was washed with diisopropyl ether and taken out through filtration to obtain 76 mg (74 %) of the entitled compound (I-79) as a yellow crystal.
MS(EI)m/z:304(M+H)⁺.
¹H-NMR(400MHz, CDCl₃)δ: 7.42-7.60(6H, m), 7.63-7.70(1H, m), 7.90(1H, s) , 8.11 (1H, dd, J=8.30, 0.73Hz), 8.69(1H, dd, J=8.79, 0.73Hz).
IR(ATR): 2227, 1552, 1462, 1444, 1363, 1346, 1309, 1234, 1176, 1149, 1115 cm⁻¹.

### [Example 92]

### 1-[(3S)-Dimethylaminopyrrolidin-1-yl]-2-phenylpyrido[1,2-a]benzimidazole-4-carbonitrile (#92):

To N,N-dimethylformamide (3 ml) solution of 70 mg (230 µmol) of 1-chloro-2-phenylpyrido[1,2-a]benzimidazole-4-carbonitrile (I-79) were added 35 µl (277 µmol) of (3S)-dimethylaminopyrrolidine and 96 µl (690 µmol) of triethylamine. The system was replaced with nitrogen and sealed up, and heated at 80°C for 16 hours. After cooling, the solvent was evaporated under reduced pressure, and the residue was dissolved in 30 ml of chloroform. This solution was washed with 15 ml of saturated sodium bicarbonate solution, and dried over anhydrous magnesium sulfate. The solvent was evaporated under reduced pressure, and the residue was applied to a silica gel column chromatography. This was eluted with a mixed solvent of dichloromethane/methanol (30/1, v/v) to obtain a crude product of the entitled compound. This was washed with diisopropyl ether and taken out through filtration to obtain 64 mg (73 %) of the entitled compound (#92) as a yellow crystal.
MS(EI)m/z:382(M⁺).
¹H-NMR(400MHz, CDCl₃)δ: 1.87(1H, brs), 2.10-2.22(1H, m), 2.18(6H, s), 2.70(1H, brs), 2.92-3.45(4H, m), 7.31-7.36(2H, m), 7.37-7.43(1H, m) , 7.45-7.56(3H, m), 7.57-7.62(1H, m), 7.76(1H, d, J=0.73Hz), 8.00-8.11(2H, m).
IR(ATR): 2229, 1500, 1471, 1431, 1392, 1365, 1338, 1296, 1159, 1155, 1065 cm⁻¹.

| Elemental analysis: C₂₄H₂₃N₅ | | | |
|---|---|---|---|
| Calcd. | C, 75.56%; | H, 6.08%; | N, 18.36% |
| found | C, 75.33%; | H, 6.10%; | N, 18.35%. |

### [Reference Example 80]

### Ethyl 2-ethyl-1-oxo-1H,5H-pyrido[1,2-a]benzimidazole-4-carboxylate (I-80):

2.00 g (9.79 mmol) of ethyl (2-benzimidazolyl)acetate was added to a mixture of 1.83ml (19.6mmol) of phosphoryl chloride and 2.28 ml (29.4 mmol) of N,N-dimethylformamide at 0°C, and the resulting mixture was heated at 95°C for 50 minutes. After cooling, 50 ml of ice-water was added thereto, and potassium carbonate was added to this mixture until it became alkaline, and then this was extracted with chloroform (100 ml x 3). The chloroform layers were combined and dried over anhydrous magnesium sulfate, and the solvent was evaporated under reduced pressure. The residue was washed with ethyl acetate and taken out through filtration to obtain a dark brown crystal.

Butyric anhydride (10 ml) suspension of the dark brown crystal synthesized above was heated at 170°C for 70 minutes. After cooling, the solvent was evaporated under reducedpressure, and the residue was washed with diisopropyl ether and taken out through fil tration to obtain 713 mg (26%) of the entitled compound (I-80) as an orange crystal.
MS(EI)m/z:285(M⁺).
¹H-NMR(40OMHz, DMSO-d₆)δ: 1.16(3H, t, J=7.33Hz), 1.34(3H, t, J=7.08Hz), 2.45-2.60(2H, m), 4.34(2H, q, J=7.08Hz), 7.31-7.39(1H, m), 7.48-7.53(1H, m), 7.68(1H, d, J=7.81Hz), 7.85(1H, s), 8.67(1H, d, J=7.81Hz).
IR(ATR): 3203, 1685, 1637, 1606, 1552, 1462, 1369, 1323, 1240, 1182, 1134, 1107, 1090 cm⁻¹.

### [Reference Example 81]

### Ethyl 1-chloro-2-ethylpyrido[1,2-a]benzimidazole-4-carboxylate (I-81):

400 mg (1.41 mmol) of ethyl 2-ethyl-1-oxo-1H,5H-pyrido[1,2-a]benzimidazole-4-carboxylate (I-80) in 3 ml of phosphoryl chloride was heated under reflux for 3 hours. After cooling, phosphoryl chloride was evaporated under reduced pressure, 15 ml of ice-water and 15 ml of aqueous 1 N sodium hydroxide solution were added to the residue, and this was extracted with chloroform (50 ml × 3). The chloroform layers were combined and dried over anhydrous magnesium sulfate, and the solvent was evaporated under reduced pressure. The resulting residue was washed with a small amount of methanol and diethyl ether and taken out through filtration to obtain 220 mg (52 %) of the entitled compound (I-81) as a dark brown crystal.
MS(EI)m/z:303(M+H)⁺.
¹H-NMR(400MHz, DMSO-d₆)δ: 1.27 (3H, t, J=7.57Hz), 1.42(3H, t, J=7.08Hz), 2.97(2H, q, J=7.57Hz), 4.47-4.58(2H, m), 7.61-7.70(1H, m), 7.80-7.88(1H, m), 8.08(1H, d, J=8.06Hz), 8.64(1H, brs), 8.97(1H, d, J=8.55Hz).
IR(ATR): 1720, 1502, 1473, 1458, 1315, 1219, 1159 cm⁻¹.

### [Example 93]

### Methyl 1-[(3S)-dimethylaminopyrrolidin-1-yl]-2-ethylpyrido[1,2-a]benzimidazole-4-carboxylate (#93):

To N,N-dimethylformamide (3 ml) solution of 98 mg (324 µmol) of ethyl 1-chloro-2-ethylpyrido[1,2-a]benzimidazole-4-carboxylate (I-81) were added 49 µl (388 µmol) of (3S)-dimethylaminopyrrolidine and 135 µl (972 µmol) of triethylamine. The system was replaced with nitrogen and sealed up, and heated at 80°C for 22 hours. After cooling, the solvent was evaporated under reduced pressure, and the residue was dissolved in 30 ml of chloroform. This solution was washed with 15 ml of saturated sodium bicarbonate solution, and dried over anhydrous magnesium sulfate. The solvent was evaporated under reduced pressure, and the residue was applied to a preparative TLC. This was eluted with a mixed solvent of dichloromethane/methanol (20/1, v/v) to obtain a mixture of the entitled compound (#93) and ethyl 1-[(3S)-dimethylaminopyrroldin-1-yl]-2-ethylpyrido[1,2-a]benzimidazole-4-carboxylate.

Methanol (2 ml) solution of the mixture synthesized above was stirred at room temperature for 4 days. The solvent was evaporated under reduced pressure to obtain 22 mg (18 %) of the entitled compound (#93) as a yellow crystal.
MS(EI)m/z:367(M⁺).
¹H-NMR(400MHz, CDCl₃)δ: 1.32-1.40(3H, m), 2.10-2.48(2H, m), 2.36(6H, s), 2.72(2H, brs), 3.08(1H, brs), 3.21-3.78(4H, m), 4.08(3H, s), 7.34(1H, t, J=7.57Hz), 7.47-7.55(1H, m), 8.04(1H, d, J=8.30Hz), 8.10-8.29(1H, m), 8.18(1H, s).

### [Reference Example 82]

### 3-Tri-n-butylstannyl-2-cyclopenten-1-one (I-82):

15.6 ml (23.8 mmol) of n-butyllithium (1.53 M hexane solution) was added to tetrahydrofuran (40 ml) solution of 13.2 ml (26.2 mmol) of hexabutylditin under nitrogen atmosphere at -78°C, and the resulting mixture was stirred for 70 minutes. At the temperature, tetrahydrofuran (10 ml) solution of 3.00 g (23.8 mmol) of 3-ethoxy-2-cyclopenten-1-one was added dropwise thereto, and the resulting mixture was stirred for 1.5 hours. At -78°C, 10 ml of aqueous saturated ammonium chloride solution was added thereto, and this was warmed up to room temperature and poured into 30 ml of diethyl ether. The combined organic layer was dried over anhydrous magnesium sulfate, and the solvent was evaporated under reduced pressure. The residue was applied to a silica gel column chromatography. With n-hexane, a less-polar side product was eluted out. Further by eluting with a mixed solvent of n-hexane/ethyl acetate (30/1, v/v), 6.07 g (69 %) of the entitled compound (I-82) was obtaned as a colorless oil.
¹H-NMR(400MHz, CDCl₃)δ: 0.90(9H, t, J=7.32Hz), 1.01-1.09(6H, m), 1.28-1.39(6H, m), 1.48-1.56(6H, m), 2.28-2.33(2H, m) , 2.84 (2H, dt, J=7.08, 2.20Hz), 6.36-6.39(1H, m).
IR(ATR): 2954, 2922, 2852, 1705, 1670, 1552, 1458, 1377, 1238, 1221, 1174 cm⁻¹.

### [Reference Example 83]

### 3-Tri-n-butylstannyl-2-cyclopenten-1-ol (I-83):

Diethyl ether (25 ml) solution of 2.00 g (5.39 mmol) of 3-tri-n-butylstannyl-2-cyclopenten-1-one (I-82) was added to diethyl ether (25 ml) solution of 307 mg (8.08 mmol) of lithium aluminium hydride under nitrogen atmosphere at -40°C, and the resulting mixture was stirred for 20 minutes, and then further stirred at -20°C for 2.5 hours. 307 µl of water, 307 µl of aqueous 1 N sodium hydroxide solution, 614 µl of water and anhydrous magnesium sulfate were added thereto, and the resulting mixture was stirred for 30 minutes, and the insoluble material was filtrated through a pad of Celite. The solvent was evaporated from the filtrate under reduced pressure. The resulting residue was applied to a silica gel column chromatography and eluted with a mixed solvent of n-hexane/ethyl acetate (10/1, v/v) to obtain 1. 90 g (95%) of the entitled compound (I-83) as a colorless oil.
¹H-NMR(400MHz, CDCl₃)δ: 0.81-1.00(15H, m), 1.23-1.38(6H, m), 1.45-1.55(6H, m), 1.60-1.70(1H, m), 2.16-2.27(1H, m), 2.33-2.43(1H, m), 2.55-2.66(1H, m), 9.81-4.90(1H, m), 5.89-6.00(1H, m).
IR(ATR): 3306, 2954, 2924, 2846, 1456, 1377, 1070, 1043 cm⁻¹.

### [Reference Example 84]

### N-(3-Tri-n-butylstannyl-2-cyclopenten-1-yl)phthalimide (I-84):

2.23 ml (5.12 mmol) of diethyl azodicarboxylate (40 % toluene solution) was added to tetrahydrofuran (50 ml) solution of 1.82 g (4.88 mmol) of 3-tri-n-butylstannyl-2-cyclopenten-1-ol (I-83), 718 mg (4.88 mmol) of phthalimide and 1.34 g (5.12 mmol) of triphenylphosphine at 0°C, and the resulting mixture was stirred at room temperature for 43 hours. The solvent was evaporated under reduced pressure, and the residue was applied to a silica gel column chromatography. This was eluted with a mixed solvent of n-hexane/ethyl acetate (20/1, v/v) to obtain 1.30 g (53 %) of the entitled compound (I-84) as a colorless oil.
¹H-NMR(400MHz, CDCl₃)δ: 0.80-1.02(15H, m), 1.21-1.39(6H, m), 1.41-1.63(6H, m), 1.98-2.15(1H, m), 2.20-2.33(1H, m), 2.50-2.70(1H, m), 2.86-2.98(1H, m), 5.34-5.42(1H, m), 5.60-5.72(1H, m), 7.63-7.71(2H, m), 7.74-7.85(2H, m).
IR(ATR) : 2954, 2924, 2852, 1770, 1709, 1458, 1389, 1354, 1329, 1105, 1072 cm⁻¹.

### [Reference Example 85]

### 1-(N-tert-Butoxycarbonylamino)-3-tri-n-butylstannyl-2-cyclopentene (I-85):

155 µl (3.20 mmol) of hydrazine monohydrate was added to ethanol (20 ml) solution of 1.07 g (2.13 mmol) of N-(3-tri-n-butylstannyl-2-cyclopenten-1-yl)phthalimide (I-84) at room temperature, and the resulting mixture was stirred for 30 hours. The insoluble material was removed through filtration, and the solvent was evaporated from the filtrate under reduced pressure. The residue was dissolved in 50 ml of dichloromethane, and the solution was washed with 25 ml of aqueous 1 N sodium hydroxide solution, and the aqueous layer was extracted with dichloromethane (50 ml × 3). The dichloromethane layers were combined and dried over anhydrous magnesium sulfate, and the solvent was evaporated under reduced pressure. The residue was applied to a silica gel column chromatography. This was eluted with a mixed solvent of dichloromethane/methanol (20/1, v/v) to obtain a colorless oil.

290 µl (1.26 mmol) of di-tert-butyl carbonate was added to dichloromethane (10 ml) solution of the colorless oil synthesized above and 240 µl (1.73 mol) of triethylamine at 0°C, and the resulting mixture was stirred at room temperature for 22 hours. The solvent was evaporated under reduced pressure, and the residue was dissolved in 50 ml of ethyl acetate. The solution was washed successively with 25 ml of aqueous saturated sodium bicarbonate solution, 25 ml of water and 25 ml of brine, and dried over anhydrous magnesium sulfate. The solvent was evaporated under reduced pressure, and the residue was applied to a silica gel column chromatography, and eluted with a mixed solvent of n-hexane/ethyl acetate (25/1, v/v) to obtain 444 mg (44 %) of the entitled compound (I-85) as a colorless oil.
¹H-NMR(400MHz, CDCl₃)δ: 0.79-1.01(15H, m), 1.22-1.37(6H, m), 1.40-1.62(7H, m), 1.45(9H, s), 2.21-2.41(2H, m) , 2.47-2.58(1H, m), 4.57(1H, brs), 4.72(1H, brs), 5.71-5.82(1H, m).
IR(ATR) : 2956, 2925, 2852, 1705, 1491, 1456, 1365, 1244, 1171, 1045 cm⁻¹.

### [Example 94]

### 1-[3-(N-tert-Butoxycarbonylamino)-1-cyclopenten-1-yl]-2-ethyl-3-methylpyrido[1,2-a]benzimidazole-4-carbonitrile (#94):

1,9-Dioxane (7 ml) solution of 300 mg (635 µmol) of 1-(N-tert-butoxycarbonylamino)-3-tri-n-butylstannyl-2-cyclopentene (I-85), 171 mg (635 µmol) of 1-chloro-2-ethyl-3-methylpyrido[1,2-a]benzimidazole-4-carbonitrile (I-2), 22 mg (32 µmol) of dichlorobis(triphenylphosphine)palladium and 5 crystals of 2,6-di-tert-butyl-4-methylphenol was heated under reflux for 43 hours. After cooling, the solvent was evaporated under reduced pressure, and the residue was suspended in 10 ml of cyclohexane and vigorously stirred for 30 minutes. The precipitate was taken out through filtration. After washing with cyclohexane, the filtrate was applied to a silica gel column chromatography and eluted with a mixed solvent of dichloromethane/methanol (50/1, v/v) to obtain a crude product of the entitled compound. This was washed with diethyl ether and taken out through filtration to obtain 181 mg (68 %) of the entitled compound (#94) as a yellow crystal.
MS(EI)m/z:417(M⁺).
¹H-NMR(400MHz, CDCl₃)δ: 1.14-1.26(3H, m), 1.50(9H, s), 1.98-2.10(1H, m), 2.49-3.02(8H, m), 4.75-4.96(1H, m) , 5.15(1H, brs), 6.19-6.22(1H, m), 7.26-7.37(1H, m), 7.49-7.58(1H, m), 7.59(0.6H, d, J=8.55Hz), 7.85(0.4H, d, J=8.30Hz), 7.99-8.07(1H, m).
IR(ATR): 3388, 2222, 1712, 1496, 1448, 1365, 1306, 1232, 1163, 1051 cm⁻¹.

### [Example 95]

### 1-(3-Amino-1-cyclopenten-1-yl)-2-ethyl-3 methylpyrido[1,2-a]benzimidazole-4-carbonitrile (#95):

4 ml of aqueous 5 N hydrochloric acid solution was added to tetrahydrofuran (4 ml) solution of 180 mg (432 µmol) of 1-[3-(N-tert-butoxycarbonylamino)-1-cyclopenten-1-yl]-2-ethyl-3-methylpyrido[1,2-a]benzimidazole-4-carbonitrile (#94) at 0°C, and the resulting mixture was stirred at room temperature for 22 hours. 25 ml of aqueous 1 N sodium hydroxide solution was added thereto, and the mixture was extracted with ethyl acetate (30 ml × 3). The combined ethyl acetate layer was dried over anhydrous magnesium sulfate, the solvent was evaporated under reduced pressure, and the residue was applied to a silica gel column chromatography. This was eluted with amixedsolventofdichloromethane/methanol (10/1, v/v) toobtain a crude product of the entitled compound. This was washed with diisopropyl ether and taken out through filtration to obtain 91 mg (67 %) of the entitled compound (#95) as a yellow crystal.
MS(EI)m/z:317(M⁺).
¹H-NMR(400MHz, CDCl₃)δ: 1.13-1.28(3H, m), 1.88-2.01(1H, m), 2.49-3.07(8H, m), 4.43-4.52(1H, m), 6.10-6.13(0.4H, m), 6.16-6.20(0.6H, m), 7.20-7.37(1H, m), 7.48-7.56(1H, m), 7.63(0.6H, d, J=8.30Hz), 7.97-8.08(1.4H, m).
IR(ATR): 2222, 1624, 1593, 1496, 1446, 1363, 1304, 1228, 1047 cm⁻¹.

| Elemental analysis: C₂₀H₂₀N₄·0.5H₂O | | | |
|---|---|---|---|
| Calcd. | C, 73.82%; | H, 6.50%; | N, 17.22% |
| Found | C, 74.22%; | H, 6.43%; | N, 16.85%. |

### [Example 96]

### 1-[(3S)-Aminopyrrolidin-1-yl]-2-ethyl-3-methylpyrido[1,2-a]benzimidazole-4-carbonitrile (#96):

To N,N-dimethylformamide (15 ml) suspension of 500 mg (1.85 mmol) of 1-chloro-2-ethyl-3-methylpyrido[1,2-a]benzimidazole-4-carbonitrile (#I-2) were added 239 mg (2.78 mmol) of (3S)-aminopyrrolidine and 774 µl (5.55 mmol) of triethylamine. The system was replaced with nitrogen and sealed up, and heated at 80°C for 17 hours. After cooling, the solvent was evaporated under reduced pressure, the residue was dissolved in 30 ml of chloroform, and the solution was washed with 20 ml of saturated sodium bicarbonate solution. The aqueous layer was extracted with chloroform (30 ml × 3), and the combined chloroform layer was dried over anhydrous magnesium sulfate. The solvent was evaporated under reduced pressure, and the residue was applied to a silica gel column chromatography. This was eluted with a mixed solvent of di chloromethane/methanol (10/1, v/v) to obtain a crude product of the entitled compound. This was recrystallized from ethanol to obtain 316 mg (54 %) of the entitled compound (#96) as a yellow crystal.
MS(EI)m/z:320(M⁺).
¹H-NMR(400MHz, CDCl₃)δ: 1.30(3H, t, J=7.57Hz), 1.94-2.08(1H, m), 2.36-2.52(1H, m), 2.60-2.96(2H, m), 2.71(3H, s), 3.00-3.88(4H, m), 4.00-4.11(1H, m), 7.29-7.39(1H, m), 7.48-7.58(1H, m), 7.90-8.05(1H, m), 7.99(1H, d, J=8.30Hz).
IR(ATR): 2220, 1628, 1593, 1498, 1479, 1442, 1408, 1306 cm⁻¹.

| Elemental analysis: C₁₉H₂₁N₅ | | | |
|---|---|---|---|
| Calcd. | C, 71.45%; | H, 6.63%; | N, 21.93% |
| Found | C, 71.11%; | H, 6.66%; | N, 21.72%. |

### [Reference Example 86]

### Ethyl 2-cyanomethyl-1H-benzimidazole-5-carboxylate (I-86):

To N,N-dimethylformamide (10 ml) solution of 2,85 g (15.8 mmol) of ethyl 3,4-diaminobenzoate were added 1.48 g (17.4 mmol) of cyanoacetic acid, 3.64 g (19.0 mmol) of 1-ethyl-3-(3-diethylaminopropyl)carbodiimide hydrochloride and 2.14 g (15.8 mmol) of 1-hydroxybenzotriazole, and the resulting mixture was stirred at room temperature for 15 hours. The reaction mixture was diluted with 100 ml of chloroform, and the solution was washed successively with 30 ml of aqueous saturated sodium bicarbonate solution and 30 ml of brine. The organic layer was taken out, and dried over anhydrous sodium sulfate, and the solvent was evaporated under reduced pressure. The residue was dissolved in 20 ml of acetic acid, and the solution was heated under reflux for 3 hours. After cooling, acetic acid was evaporated under reduced pressure, and the residue was applied to a silica gel column chromatography. This was eluted with a mixed solvent of chloroform/methanol (95/5, v/v) to obtain an acetic acid salt of the entitled compound. This was dissolved in 100 ml of chloroform, and the solution was washed successively with 50 ml of aqueous saturated sodium bicarbonate solution and 30 ml of brine. The organic layer was dried over anhydrous sodium sulfate, and the solvent was evaporated under reduced pressure to obtain 2.43 g (67 %) of the entitled compound as a pale brown crystal.
MS(EI)m/z:230(M+H)⁺.
¹H-NMR(400MHz, CDCl₃)δ: 1.41(3H, t, J=7.08Hz), 4.19(2H, s), 4.41(2H, q, J=7.08Hz), 7.30-7.85(1H, m), 8.01(1H, dd, J=1.47, 8.55Hz), 8.15-8.55(1H, m).
IR(ATR): 1681, 1623, 1537, 1423, 1369, 1300cm⁻¹.

### [Reference Example 87]

### Mixture of ethyl 2-n-butyl-4-cyano-3-methyl-1-oxo-1H,5H-pyrido[1,2-a]benzimidazole-7-carboxylate and ethyl 2-n-butyl-4-cyano-3-methyl-1-oxo-1H,5H-pyrido[1,2-a]benzimidazole-8-carboxylate (I-87):

A mixture of 1.98 g (8.64 mmol) of ethyl 2-cyanomethyl-1H-benzimidazole-5-carboxylate (I-86), 1.23 g (6.62 mmol) of ethyl α-n-butylacetoacetate and 1.02 g (13.2 mmol) of ammonium acetate was heated at 140 to 150°C for 30 minutes. After cooling, 20 ml of water was added thereto, and the solid was crushed and decanted. 10 ml of acetonitrile was added thereto and washed, and the crystal was taken out through filtration to obtain 2.10 g (69 %) of the entitled compound as a colorless crystal (neither divided nor purified, this was used in the next reaction).
MS(ESI)m/z:352(M⁺).

### [Reference Example 88]

### Mixture of ethyl 2-n-butyl-1-chloro-4-cyano-3-methylpyrido[1,2-a]benzimidazole-7-carboxylate and ethyl 2-n-butyl-4-cyano-1-chloro-3-methylpyrido[1,2-a]benzimidazole-8-carboxylate (I-88):

2.08 g (5.94 mmol) of the mixture of ethyl 2-n-butyl-4-cyano-3-methyl-1-oxo-1H,5H-pyrido[1,2-a]benzimidazole-7-carboxylate and ethyl 2-n-butyl-4-cyano-3-methyl-1-oxo-1H,5H-pyrido[1,2-a]benzimidazole-8-carboxylate synthesized above (I-87) in 10 ml of phosphoryl chloride was heated under reflux for 40 minutes. After cooling, phosphoryl chloride was evaporated under reduced pressure, 20 ml of ice-water was added to the residue, and this mixture was extracted with chloroform (50 ml × 3). The combined chloroform layer was washed with brine and dried over anhydrous magnesium sulfate, and the solvent was evaporated under reduced pressure. The resulting residue was washed with a small amount of ethanol and diethyl ether, and taken out through filtration to obtain 1.86 g (85%) of the entitled compound as a paleyellow crystal (neither divided nor purified, this was used in the next reaction).
MS(ESI)m/z:370(M⁺).

### [Examples 97 and 98]

### Ethyl 2-n-butyl-4-cyano-1-(2-N',N'-diethylaminoethylamino)-3-methylpyrido[1,2-a]benzimidazole-8-carboxylate (#97) and ethyl 2-n-butyl-4-cyano-1-(2-N',N'-diethylaminoethylamino)-3-methylpyrido[1,2-a]benzimidazole-7-carboxylate hydrochloride (#98):

To N,N-dimethylformamide (15 ml) suspension of 740 mg (2.00 mmol) of the mixture of the above synthesized ethyl 2-n-butyl-4-cyano-1-chloro-3-methylpyrido[1,2-a]benzimidazo le-7-carboxylate and ethyl 2-n-butyl-4-cyano-1-chloro-3-methylpyrido[1,2-a]benzimidazole-8-carboxylate (I-88) were added 422 µl (3.00 mmol) of N,N-diethylaminoethylamine and 835 µl (6.00 mmol) of triethylamine. The system was replaced with nitrogen and sealed up, and heated at 80°C for 15 hours. After cooling, the solvent was evaporated under reduced pressure, and the residue was dissolved in 200 ml of ethyl acetate. The solution was washed successively with 50 ml of water, 50 ml of aqueous saturated sodium bicarbonate solution and 50 ml of brine, and dried over anhydrous sodium sulfate, and the solvent was evaporated under reduced pressure. Ethanol and a small amount of diethyl ether were added to the residue, and the crystal thus formed was taken out through filtration, and this was recrystallizedfromethanol/diethyl ether to obtain 160 mg (18 %) of the entitled compound (8-ester, #97) as a pale yellow crystal. The filtrate was concentrated under reduced pressure, and the residue was dissolved in ethanol. 1.55 ml of 1 N hydrochloric acid was added thereto. The mixture was stirred at room temperature for 20 minutes, and the crystal thus formed was taken out through filtration. The filtrate was concentrated under reduced pressure, and the residue was recrystallized from methanol/ethanol to obtain 232 mg (24 %) of the entitled compound (7-ester hydrochloride, #98) as a yellow crystal. 8-ethyl ester
(#97)
MS(EI)m/z:449(M⁺).
¹H-NMR(400MHz, CDCl₃)δ: 1.00-1.08(3H, m), 1.11-1.19(6H, m), 1.44-1.68(7H, m), 2.67-2.90(11H, m), 3.11-3.20(2H, m), 4.43(2H, q, J=7.10Hz), 7.93(1H, d, J=8.82Hz), 8.21(1H, d, J=8.82Hz), 8.88(1H, s).
IR(ATR): 2968, 1709, 1597, 1491 cm⁻¹.

| Elemental analysis: C₂₆H₃₅N₅O₂ | | | |
|---|---|---|---|
| Cacld. | C, 69.46%; | H, 7.85%; | N, 15.58% |
| Found | C, 69.44%; | H, 7.88%; | N, 15.67%. |

7-ethyl ester hydrochloride (#98)
MS(EI)m/z:449(M⁺).
¹H-NMR(400MHz, DMSO-d₆)δ: 0.96(3H, t, J=7.08Hz), 1.20(6H, t, J=7.32Hz), 1.39(3H, t, J=6.48Hz), 1.42-1.56(4H, m), 2.64(3H, s), 2.74-2.85(2H, m), 3.05-3.16(4H, m), 3.30-3.58(4H, m), 4.38(2H, q, J=7.08Hz), 6.47(1H, d, J=6.59Hz), 7.96(1H, d, J=8.79Hz),8.29(1H, d, J=8.79Hz), 10.48(1H, brs).
IR(ATR): 1712, 1631, 1601, 1498, 1465 cm⁻¹.

| Elemental analysis: C₂₆H₃₅N₅O₂·HCl·0.25H₂O | | | |
|---|---|---|---|
| Calcd. | C, 63.66%; | H, 7.50%; | N, 14.28% |
| Found | C, 63.70%; | H, 7.53%; | N, 14.34%. |

### [Example 99]

### 2-n-Butyl-4-cyano-1-(2-N',N'-diethylaminoethylamino)-3-methylpyrido[1,2-a]benzimidazole-7-carboxylic acid (#99):

10 ml of aqueous 1 N sodium hydroxide solution was added to ethanol (5 ml) suspension of 179 mg (0.36 mmol) of ethyl 2-n-butyl-4-cyano-1-(2-N',N'-diethylaminoethylamino)-3-methylpyrido[1,2-a]benzimidazole-7-carboxylate (#98), and the resulting mixture was stirred at room temperature for 3 hours. With concentrated hydrochloric acid and 1 N hydrochloric acid added thereto, the reaction mixture was neutralized, and then extracted with chloroform (50 ml × 3). The organic layer was dried over anhydrous sodium sulfate, and the solvent was evaporated under reduced pressure. The residue was recrystallized from ethanol/diethyl ether to obtain 100 mg (65%) of the entitled compound as a pale yellow crystal.
MS(EI)m/z:421 (M⁺).
¹H-NMR(400MHz, DMSO-d₆)δ: 0.89(6H, t, J=6.86Hz), 0.96(3H, t, J=6.61Hz), 1.40-1.51(4H, m) , 2.45(4H, q, J=6.86Hz), 2.50 (3H, s), 2.67(2H, t, J=5.63Hz), 2.72-2.80(2H, m), 3.23-3.28(2H, m), 5.95-6.01(1H, m), 7.91(1H, d, J=8.57Hz),8.28(1H, d, J=8.82Hz), 8.32(1H, d, J=1.22Hz).
IR(ATR): 2960, 2217, 1627, 1592 cm⁻¹.

| Elemental analysis: C₂₄H₃₁N₅O₂·0.5H₂O | | | |
|---|---|---|---|
| Calcd. | C, 66.95%; | H, 7.49%; | N, 16.27% |
| Found | C, 66.64%; | H, 7.28%; | N, 16.18%. |

### [Example 100]

### Ethyl 2-n-butyl-4-cyano-1-[(3S)-dimethylaminopyrrolidin-1-yl]-3-methylpyrido[1,2-a]benzimidazole-7-carboxylate (#100):

To N,N-dimethylformamide (20 ml) suspension of 945 mg (2.56 mmol) of the above synthesized mixture of ethyl 2-n-butyl-4-cyano-1-chloro-3-methylpyrido[1,2-a]benzimidazole-7-carboxylate and ethyl 2-n-butyl-4-cyano-1-chloro-3-methylpyrido[1,2-a]benzimidazole-8-carboxylate (I-88) were added 486 µl (3.83 mmol) of (3S)-dimethylaminopyrrolidine and 1.07 ml (7.68 mmol) of triethylamine. The system was replaced with nitrogen and sealed up, and heated at 80°C for 20 hours. After cooling, the solvent was evaporated under reducedpressure and the residue was dissolved in 200 ml of ethyl acetate. The solution was successively washed with 50 ml of water, 50 ml of aqueous saturated sodium bicarbonate solution and 50 ml of brine, and dried over anhydrous sodium sulfate, and the solvent was evaporated under reduced pressure. The residue was applied to a silica gel column chromatography, and eluted with a mixed solvent of chloroform/methanol (93/7, v/v) to obtain a mixture of the entitled compound. A small amount of ethanol and diethyl ether was added thereto, and the crystal thus formed was taken out through filtration to obtain 110 mg (10 %) of the entitled compound (7-ester, #100) as a pale yellow crystal.
7-ethyl eser (#100)
MS(EI)m/z:447(M⁺).
¹H-NMR(400MHz, CDCl₃)δ: 1.00-1.09(3H, m), 1.44(3H, t, J=7.10Hz), 1.56-1.67(4H, m), 1.70(2H, brs), 2.10-2.29(1H, m) , 2.35(6H, s), 2.36-2.48(1H, m), 2.69(3H, s), 2.98-3.80(5H, m), 4.43(2H, q, J=7.10Hz), 7.98-8.26(2H, m), 8.67(1H, s).
IR(ATR): 2954, 1714, 1294, 1214 cm⁻¹.

| Elemental analysis: C₂₆H₃₃N₅O₂ | | | |
|---|---|---|---|
| Calcd. | C, 69.77%; | H, 7.43%; | N, 15.65% |
| Found | C, 69.47%; | H, 7.36%; | N, 15.64%. |

### [Reference Example 89]

### 5,6-Dichloro-1H-benzimidazole-2-acetonitrile (I-89):

According to the production method for (I-86), 1.02 g (30%) of the entitled compound was produced as a pale red amorphous substance from 2.65 g (15.0 mmol) of 4,5-dichloro-1,2-phenylenediamine, 1.40 g (16.5 mmol) of cyanoacetic acid, 3.45 g (18.0 mmol) of 1-ethyl-3-(3-dimethylaminopropyl)carbodiimide hydrochloride and 2.03 g (15.0 mmol) of 1-hydroxybenzotriazole. (Not crystallized, this was directly used in the next reaction.)
¹H-NMR(400MHz, CDCl₃)δ: 4.08(2H, s), 7.05-7.80(2H, m).

### [Reference Example 90]

### 5,6-Dimethyl-1H-benzimidazole-2-acetonitrile (I-90):

According to the production method for (I-86), 1.20 g (25 %) of the entitled compound was produced as a pale orange crystal from 3.52 g (25.9 mmol) of 4,5-dimethyl-1,2-phenylenediamine, 2.42 g (28.5 mmol) of cyanoacetic acid, 5.95 g (31.0 mmol) of 1-ethyl-3-(3-dimethylaminopropyl)carbodiimide hydrochloride and 3.49 g (25.9 mmol) of 1-hydroxybenzotriazole.
MS(EI)m/z:185(M⁺).
¹H-NMR(400MHz, CDCl₃)δ: 2.29(3H, s), 2.30(3H, s), 4.31(2H, s), 7.24(1H, s), 7.35(1H, s), 12.31(1H, brs).
IR(ATR): 2271, 1537, 1452, 1306cm⁻¹.

### [Reference Example 91]

### 2-n-Butyl-7,8-dichloro-3-methyl-1-oxo-1H,5H-pyrido-[1,2-a]benzimidazole-4-carbonitrile (I-91):

According to the production method for (I-87), 966 mg (61 %) of the entitled compound was produced as a pale reddish brown crystal from 1.02 g (4.52 mmol) of 5,6-dichloro-1H-benzimidazole-2-acetonitrile (I-89), 1.26 g (6.78 mmol) of ethyl α-n-butylacetoacetate and 697 mg (9.04 mmol) of ammonium acetate.
MS(EI)m/z:348(M+H)⁺.
¹H-NMR(400MHz, CDCl₃)δ: 0.94-1.00(3H, m), 1.38-1.57(4H, m), 2.45-2.51(3H, m), 2.65-2.72(2H, m), 7.53(1H, d, J=6.61Hz), 8.86(1H, d, J=6.37Hz).
IR(ATR): 2216, 1662, 1616, 1547, 1464cm⁻¹.

### [Reference Example 92]

### 2-n-Butyl-3,7,8-trimethyl-1-oxo-1H,5H-pyrido[1,2-a]benzimidazole-4-carbonitrile (I-92):

According to the production method for (I-87), 1.01 g (76%) of the entitled compound was produced as a colorless crystal from 800 mg (4.32 mmol) of 5,6-dimethyl-1H-benzimidazole-2-acetonitrile (I-90), 828 mg (4.45 mmol) of ethyl α-n-butylacetoacetate and 666 mg (8.64 mmol) of ammonium acetate.
MS (EI) m/z:307 (M⁺).
¹H-NMR(400MHz, DMSO-d₆)δ: 0.92 (3H, t, J=6.59Hz) , 1.30-1.50(4H, m), 2.33-2.40(9H, m), 2.45-2.60 (2H, m), 7.25(1H, s), 8.37(1H, s), 13.13(1H, s).
IR(ATR): 2206, 1658, 1610, 1540, 1475 cm⁻¹.

### [Reference Example 93]

### 2-n-Butyl-1,7,8-trichloro-3-methylpyrido[1,2-a]benzimidazole-4-carbonitrile (I-93):

According to the production method for (I-88), 914 mg (2.62 mmol) of 2-n-butyl-7,8-dichloro-3-methyl-1-oxo-1H,5H-pyrido[1,2-a]benzimidazole-4-carbonitrile (I-91) in 8 ml of phosphoryl chloride was heated under reflux for 3 hours and then processed to obtain 946 mg (98 %) of the entitled compound as a pale yellow crystal.
MS(EI)m/z:366(M⁺).
¹H-NMR(400MHz, CDCl₃)δ: 1.02(3H, t, J=6.84Hz), 1.48-1.58(4H, m) , 2.76(3H, s), 2.88-2.92(2H, m), 8.06(1H, s), 8.67(1H, s).
IR(ATR): 2227, 1626, 1589, 1471, 1437cm⁻¹.

### [Reference Example 94]

### 2-n-Butyl-1-chloro-3,7,8-trimethylpyrido[1,2-a]benzimidazole-4-carbonitrile (I-94):

According to the production method for (I-88), 960 mg (3.12 mmol) of 2-n-butyl-3,7,8-trimethyl-1-oxo-1H,5H-pyrido[1,2-a]benzimidazole-4-carbonitrile (I-92) in 10 ml of phosphoryl chloride was heated under reflux for 30 minutes and then processed to obtain 1.00 g (quantitative) of the entitled compound as a pale yellow crystal.
MS(EI)m/z:325(M⁺).
¹H-NMR(400MHz, CDCl₃)δ: 1.04(3H, t, J=7.01Hz), 1.50-1.71(4H, m), 2.37, 2.48, 2.89(each 3H, s), 2.95-3.08(2H, m), 7.78, 8.48(each 1H, s).
IR(ATR): 1623, 1504, 1473, 1292 cm⁻¹.

### [Example 101]

### 2-n-Butyl-7,8-dichloro-1-[(3S)-dimethylaminopyrrolidin-1-yl]-3-methylpyrido[1,2-a]benzimidazole-4-carbonitrile (#101):

To N,N-dimethylformamide (5 ml) suspension of 300 mg (0.82 mmol) of 2-n-butyl-1,7,8-trichloro-3-methylpyrido[1,2-a]benzimidazole-4-carbonitrile (I-93) were added 156 µl (1.23 mmol) of (3S) -dimethylaminopyrrolidine and 341 µl (2.45 mmol) of triethylamine. The system was replaced with nitrogen and sealed up, and heated at 80°C for 2.5 hours. After cooling, the solvent was evaporated under reduced pressure and the residue was dissolved in 50 ml of ethyl acetate. The solution was successively washed with 10ml of water, 20ml of aqueous saturated sodium bicarbonate solution and 20 ml of brine, and dried over anhydrous sodium sulfate, and the solvent was evaporated under reduced pressure. The residue was applied to a silica gel column chromatography, and eluted with a mixed solvent of chloroform/methanol (97/3, v/v) to obtain 350 mg (96 %) of a crude product of the entitled compound as a yellow crystal. This was recrystallized from ethanol/chloroform to obtain 135 mg (38 %) of the entitled compound as a pale yellow crystal.
MS(EI)m/z:444(M⁺).
¹H-NMR(400MHz, CDCl₃)δ: 1.04(3H, t, J=6.59Hz), 1.50-1.72(6H, m), 2.38(6H, s), 2.60-2.68(2H, m), 2.68(3H, s), 3.00-3.12(1H, m), 3.20-3.35(1H, m), 3.45-3.76(3H, m), 8.00(1H, s), 8.09, 8.69(each 0.5H, brs).
IR(ATR): 2224, 1628, 1589, 1481, 1435 cm⁻¹.

| Elemental analysis: C₂₃H₂₇Cl₂N₅·0.5H₂O | | | |
|---|---|---|---|
| Calcd. | C, 60.93%; | H, 6.22%; | N, 15.45% |
| Found | C, 61.01%; | H, 6.17%; | N, 15.41%. |

### [Example 102]

### 2-n-Butyl-1-[(3S)-dimethylaminopyrrolidin-1-yl]-3,7,8-trimethylpyrido[1,2-a]benzimidazole-4-carbonitrile (#102):

To N,N-dimethylformamide (6ml) suspension of 399mg (1.22 mmol) of 2-n-butyl-1-chloro-3,7,8-trimethylpyrido[1,2-a]benzimidazole-4-carbonitrile (I-94) were added 233 µl (1.83 mmol) of (3S)-dimethylaminopyrrolidine and 339 µl (2.44 mmol) of triethylamine. The system was replaced with nitrogen and sealed up, and heated at 80°C for 15 hours. After cooling, the solvent was evaporated under reduced pressure and the residue was dissolved in 80 ml of chloroform. The solution was successively washed with 20ml of water, 30ml of aqueous saturated sodium bicarbonate solution and 30 ml of brine, and dried over anhydrous sodium sulfate, and the solvent was evaporated under reduced pressure. The residue was applied to a silica gel column chromatography, and eluted with a mixed solvent of chloroform/methanol (97/3, v/v) to obtain a crude product of the entitled compound. This was crystallized from 2-propanol/diethyl ether and then taken out through filtration to obtain 120 mg (24 %) of the entitled compound as a yellow crystal.
FABMSm/z:403(M⁺).
¹H-NMR(400MHz, CDCl₃)δ: 1.03(3H, brs), 1.48-1.70(4H, m), 1.76(2H, brs), 2.36(6H, s), 2.44(3H, s), 2.46(3H, s), 2.60-2.73(2H, m), 2.66(3H, s), 2.98-3.82(5H, m), 7.73(1H, s), 7.75, 7.95(each 0.5H, brs).
IR(ATR): 2222, 1597, 1481, 1454 cm⁻¹.

| Elemental analysis: C₂₅H₃₃N₅ | | | |
|---|---|---|---|
| Calcd. | C, 74.40%; | H, 8.24%; | N, 17.35% |
| Found | C, 74.07%; | H, 8.23%; | N, 17.44% |

### [Reference Example 95]

### Ethyl [(3S)-1-benzylpyrrolidin-3-yl]methylaminoacetate (I-95):

To 1,2-dichloroethane (52 ml) solution of 1.18 ml (5.20 mmol) of ethyl glyoxylate (polymer form, 40 to 50 % toluene solution) were added 1.00 ml (5.20 mmol) of (3S)-1-benzyl-3-methylaminopyrrolidine and 1.65 g (7.80 mmol) of sodium triacetoxyborohydride at room temperature, and 5.20 ml of acetic acid was added thereto at 0°C, and then this mixture was stirred at room temperature for 27.5 hours. Aqueous 1 N sodium hydroxide solution was added thereto, and this was extracted with chloroform. The organic layers were combined and dried over anhydrous magnesium sulfate. The solvent was evaporated under reduced pressure, and the resulting residue was applied to a silica gel column chromatography. From the eluate with dichloromethane/methanol (10/1, v/v) , 1.06 g (74 %) of the entitled compound was obtained as a yellow oil.
MS(ESI)m/z:277 (M+1)⁺.
¹H-NMR(CDCl₃)δ: 1.26(3H, t, J=7.1Hz), 1.72-1.82(1H, m), 1.97-2.08(1H, m), 2.34 (3H, s), 2.44-2.81 (4H, m), 3.20-3.33 (3H, m), 3.56-3.70(2H, m) , 4.17(2H, q, J=7.1Hz), 7.29-7.37(5H, m). IR(ATR): 1732, 1452, 1250, 1182, 1126, 1028 cm

### [Reference Example 96]

### Ethyl [(3S)-pyrrolidin-3-yl]methylaminoacetate dihydrochloride (I-96):

To ethanol (23 ml) solution of 800 mg (2.89 mmol) of ethyl [(3S)-1-benzylpyrrolidin-3-yl]methylaminoacetate (I-95) were added 80 mg of 10 % palladium-carbon catalyst and 5.79 ml (5.79 mmol) of 1 mol/liter hydrochloric acid (in ethanol), and the resulting mixture was stirred under hydrogen atmosphere for 23 hours. The catalyst was removed through filtration, and the solvent was evaporated under reduced pressure. The resulting residue was azeotropically boiled with benzene to obtain 738 mg (99 %) of the entitled compound as an amorphous substance. MS(ESI)m/z:186(M⁺).
¹H-NMR(D₂O) δ: 1.12-1.19(3H, m) , 2.11-2.24(1H, m), 2.45-2.57(1H, m), 2.93(3H, s), 3.25-3.36(1H, m), 3.44-3.57(2H, m), 3.76(1H, dd, J=13.2, 8.5Hz), 4.07-4.30(5H, m).
IR(ATR): 1739, 1444, 1404, 1377, 1284, 1225, 1020 cm⁻¹.

### [Example 103]

### Ethyl [(3S)-1-[4-cyano-3-methyl-2-phenylpyrido[1,2-a]benzimidazol-1-yl]pyrrolidin-3-yl]methylaminoacetate (#103):

To N,N-dimethylformamide (15 ml) suspension of 714 mg (2.75 mmol) of ethyl [(3S)-pyrrolidin-3-yl]methylaminoacetate dihydrochloride (I-96) were added 2.16 ml (15.5 mmol) of triethylamine and 492 mg (1.55 mmol) of 1-chloro-3-methyl-2-phenylpyrido[1,2-a]benzimidazole-4-carbonitrile (I-8). The system was replaced with nitrogen and sealed up, and heated at 80°C for 4.5 hours. After cooling, the solvent was evaporated under reduced pressure and the residue was dissolved in chloroform. The solution was washed with aqueous saturated sodium bicarbonate solution and dried over anhydrous magnesium sulfate. The solvent was evaporated under reduced pressure, and the resulting residue was applied to a silica gel column chromatography. From the eluate with dichloromethane/methanol (50/1, v/v), a crude product of the entitled compound was obtained, and this was recrystallized from ethanol to obtain 519 mg (72 %) of the entitled compound as a yellow crystal.
MS(ESI)m/z:468(M+1)⁺.
¹H-NMR(CDCl₃)δ: 1.21-1.30(3H, m), 1.90-2.02(1H, m), 2.23-2.37(1H, m), 2.28(3H, s), 2.31(3H, s),2.63-3.67(5H, m), 3.17(2H, brs), 4.11-4.20(2H, m), 7.20-7.38(3H, m), 7.48-7.60(4H, m), 7.87-8.20(1H, m), 8.01(1H, d, J=8.3Hz).
IR(ATR) : 2222, 1749, 1630, 1591, 1473, 1442, 1427, 1298, 1192, 1128 cm⁻¹.

| Elemental analysis: C₂₈H₂₉N₅O₂ | | | |
|---|---|---|---|
| Calcd. | C, 71.93%; | H, 6.25%; | N, 14.98% |
| Found | C, 71.70%; | H, 6.22%; | N, 14.94%. |

### [Example 104]

### [(3S)-1-[4-Cyano-3-methyl-2-phenylpyrido[1,2-a]benzimidazol-1-yl]pyrrolidin-3-yl]methylaminoacetic acid (#104):

257 µl (257 µmol) of aqueous 1 N sodium hydroxide solution was added to tetrahydrofuran/ethanol (4 ml) (1/1, v/v) mixed solution of 100mg (214µmol) of ethyl [(3S)-1-[4-cyano-3-methyl-2-phenylpyrido[1,2-a]benzimidazol-1-yl]pyrrolidin-3-yl]methylaminoacetate (#103) at 0°C, and the resulting mixture was stirred at room temperature for 20 hours. Water (2 ml) and aqueous 1 N hydrochloric acid (1 ml) were added thereto, and then aqueous 1 N sodium hydroxide solution (700 µl) was added thereto, and the mixture was extracted with a mixed solvent of chloroform/methanol (20/1, v/v). This was dried over anhydrous magnesium sulfate, and the solvent was evaporated under reduced pressure. The resulting residue was washed with diethyl ether and taken out through filtration to obtain 34 mg (36 %) of the entitled compound as a yellow crystal.
MS(ESI)m/z:440(M+1)⁺.
¹H-NMR(CD₃OD)δ: 1.76-2.37(2H, m), 2.32(3H, s), 2.67(3H, s), 2.82-3.78(7H, m), 7.36-7.52(3H, m), 7.57-7.68(4H, m), 7.84-8.20(2H, m).
IR(ATR): 2222, 1624, 1589, 1469, 1441, 1375, 1298 cm⁻¹.

### [Reference Example 97]

### (3S)-1-Benzyl-3-[[2-(tert-butoxycarbonylamino)ethyl]-methylamino]pyrrolidine (I-97):

According to the production method for (I-95), 1,2-dichloroethane (63 ml) solution of 1.00 ml (6.28 mmol) of tert-butyl N-(2-oxoethyl)carbamate, 1.21 ml (5.20 mmol) of (3S)-1-benzyl-3-methylaminopyrrolidine, 2.00 g (9.42 mmol) of sodium triacetoxyborohydride and 6.30 ml of acetic acid was stirred at room temperature for 24 hours and processed to obtain 1.36 g (65 %) of the entitled compound as a yellow oil.
MS(EI)m/z:334(M+1)⁺.
¹H-NMR(CDCl₃)δ: 1.44(9H, s), 1.72-1.84(1H, m), 1.98-2.08(1H, m), 2.26(3H, s), 2.47-2.72(5H, m), 2.76-2.84 (1H, m) , 3.18-3.30(3H, m), 3.61-3.71(2H, m), 5.25(1H, brs), 7.22-7.37(5H, m).
IR(ATR): 1705, 1495, 1454, 1363, 1248, 1167 cm⁻¹.

### [Reference Example 98]

### (3S)-3-[[2-(tert-Butoxycarbonylamino)ethyl]methylamino]-pyrrolidine dihydrochloride (I-98):

According to the production method for (I-96), 40 mg of 10 % palladium-carbon catalyst and 2.40 ml (2.40 mmol) of 1 mol/liter hydrochloric acid (in ethanol) were added to ethanol (10 ml) solution of 400 mg (1.20 mmol) of (3S)-1-benzyl-3-[[2-(tert-butoxyearbonylamino)ethyl]-methylamino]pyrrolidine (I-97), and stirred under hydrogen atmosphere for 67 hours, and then processed to obtain 345 mg (91 %) of the entitled compound as a brown oil.
MS(ESI)m/z:249(M+1)⁺.
¹H-NMR(D₂O)δ: 1.32 (9H, s), 2.07-2.11(1H, m), 2.40-2.60(1H, m), 2.87(3H, s), 3.19-3.58(7H, m), 3.73-3.82(1H, m), 4.11-4.21(1H, m).
IR(ATR): 1691, 1516, 1367, 1277, 1250, 1167 cm⁻¹.

### [Reference Example 99]

### 1-[(3S)-3-[[2-(tert-Butoxycarbonylamino)ethyl]methylamino]pyrrolidin-1-yl]-3-methyl-2-phenylpyrido[1,2-a]benzimidazole-4-carbonitrile (I-99):

According to the production method for (#103), N,N-dimethylformamide (9 ml) solution of 336 mg (1.06 mmol) of (3S)-3-[[2-(tert-butoxycarbonylamino)ethyl]methylamino]-pyrrolidine dihydrochloride (I-98), 617 µl (4.43 mmol) of triethylamine and 281 mg (885 µmol) of 1-chloro-3-methyl-2-phenylpyrido[1,2-a]benzimidazole-4-carbonitrile (I-8) was heated at 80°C for 6 hours and then processed. This was washed with diisopropyl ether and a precipitate was collected by filtration to obtain 191 mg (41 %) of the entitled compound as a yellow crystal.
MS(EI)m/z:525(M+1)⁺.
¹H-NMR(CDCl₃)δ: 1.44(9H, s), 1.80-2.14(1H, m), 2.09(3H, s), 2.20-2.48(3H, m), 2.30(3H, s), 2.66-3.60(7H, m), 4.81(1H, brs), 7.19-7.40(3H, m), 7.42-7.60(4H, m), 7.81-8.20(1H, m), 8.02(1H, d, J=7.8Hz).
IR(ATR): 2222, 1709, 1626, 1589, 1469, 1442, 1365, 1298, 1248, 1167 cm⁻¹.

### [Example 105]

### 1-[(3S)-3-[(2-Acetylaminoethyl)methylamino]pyrrolidin-1-yl]-3-methyl-2-phenylpyrido[1,2-a]benzimidazole-4-carbonitrile (#105):

4 ml of concentrated hydrochloric acid was added to methanol (8 ml) solution of 188 mg (358 µmol) of 1-[(3S)-3-[[2-(tert-butoxycarbonylamino)ethyl]methylamino]pyrrolidin-1-yl]-3-methyl-2-phenylpyrido[1,2-a]benzimidazole-4-carbonitrile (I-99) at 0°C, and then stirred at room temperature for 27.5 hours. The solvent was evaporated under reduced pressure, and the resulting residue was azeotropically boiled with benzene.

51 µl (537 µmol) of acetic anhydride was added to dichloromethane (4 ml) solution of the residue, and 249 µl (1.79 mmol) of triethylamine was added thereto at 0°C, and stirred at room temperature for 5 days. Water was added thereto, extracted with chloroform, and dried over anhydrous magnesium sulfate. The solvent was evaporated under reduced pressure, and the resulting residue was applied to a silica gel column chromatography. From the eluate with dichloromethane/methanol (30/1, v/v), a crude product of the entitled compound was obtained, and this was recrystallized from ethanol/n-hexane to obtain 85 mg (51 %) of the entitled compound as a yellow crystal.
MS(ESI)m/z:467(M+1)⁺.
¹H-NMR(CDCl₃)δ: 1.80-2.18(4H, m), 2.11(3H, s), 2.26-2.44(3H, m), 2.31(3H, s) , 2.68-3.65(7H, m), 5.78(1H, brs), 7.21-7.40(3H, m), 7.48-7.60(4H, m), 7.85-8.21(1H, m), 8.03(1H, d, J=8.1Hz). IR(ATR): 2220, 1643, 1628, 1473, 1442, 1298, 1132 cm⁻¹.

| Elemental analysis: C₂₈H₃₀N₆O·0.5H₂O | | | |
|---|---|---|---|
| Calcd. | C, 70.71%; | H, 6.57%; | N, 17.67% |
| Found | C, 70.78%; | H, 6.52%; | N, 17.65%. |

### [Reference Example 100]

### (3S)-1-Benzyl-3-[[2-(tert-butyldimethylsiloxy)ethyl]methylamino]pyrrolidine (I-100):

According to the production method for (I-95), 1,2-dichloroethane (57 ml) solution of 1.00 ml (5.74 mmol) of (tert-butyldimethylsiloxy)acetaldehyde, 1.10 ml (5.74 mmol) of (3S)-1-benzyl-3-methylaminopyrrolidine, 541 mg (2.55 mmol) of sodium triacetoxyborohydride and 5.70 ml of acetic acid was stirred at room temperature for 26 hours and processed to obtain 482 mg (24 %) of the entitled compound as a brown oil.
MS(ESI)m/z:349(M+1)⁺.
¹H-NMR(CDCl₃)δ: 0.05(6H, s), 0.88(9H, s), 1.78-1.89(1H, m), 1.99-2.10(1H, m) , 2.35 (3H, s), 2.50-2.88(6H, m), 3.28-3.37(1H, m), 3.61-3.70(2H, m), 3.75(2H, t, J=6.3 Hz) , 7.23-7.36(5H, m). IR(ATR): 1462, 1379, 1362, 1252, 1103, 1057cm⁻¹.

### [Reference Example 101]

### (3S)-3-[(2-Hydroxyethyl)methylamino]pyrrolidine dihydrochloride (I-101):

According to the production method for (I-96), 50 mg of 10 % palladium-carbon catalyst and 2.76 ml (2.76 mmol) of 1 mol/liter hydrochloric acid (in ethanol) were added to ethanol (11 ml) solution of 482 mg (1.38 mmol) of (3S)-1-benzyl-3-[[2-(tert-butyldimethylsiloxy)ethyl]methylamino]pyrrolidine (I-100), and stirred under hydrogen atmosphere for 23 hours, and then processed to obtain 218 mg (73%) of the entitled compound as a brown oil.
MS(ESI)m/z:235(M+2)⁺.
¹H-NMR(D₂O)δ: 2.11-2.23(1H, m), 2.49-2.60(1H, m), 2.87(3H, d, J=1.7Hz), 3.20-3.37(3H, m), 3.43-3.59 (2H, m) , 3.75-3.85(3H, m), 4.14-4.27(1H, m).

### [Example 106]

### 1-[(3S)-3-[(2-Hydroxyethyl)methylamino]pyrrolidin-1-yl]-3-methyl-2-phenylpyrido[1,2-a]benzimidazole-4-carbonitrile (#106):

According to the production method for (#103), N,N-dimethylformamide (10 ml) solution of 221 mg (972 µmol) of (3S)-3-[(2-hydroxyethyl)methylamino]pyrrolidine dihydrochloride (I-101), 677 µl (4.86 mmol) of triethylamine and 309 mg (972 µmol) of 1-chloro-3-methyl-2-phenylpyrido[1,2-a]benzimidazole-4-carbonitrile (I-8) was heated at 80°C for 13 hours and then processed. This was recrystallized from ethanol to obtain 196 mg (47 %) of the entitled compound as a yellow crystal.
MS(ESI)m/z:426(M+1)⁺.
¹H-NMR(CDCl₃)δ: 1.85-2.17(1H, m), 2.12(3H, s), 2.23-2.53(3H, m), 2.31(3H, s), 2.70-3.64(7H, m), 7.20-7.39(3H, m), 7.48-7.60(4H, m), 7.87-8.12(1H, m), 8.02(1H, d, J=8.3 Hz).
IR(ATR): 2222, 1628, 1591, 1442, 1427, 1371, 1298, 1130, 1090, 1045 cm⁻¹.

| Elemental analysis: C₂₆H₂₇N₅O·0.25H₂O | | | |
|---|---|---|---|
| Calcd. | C, 72.62%; | H, 6.45%; | N, 16.33% |
| Found | C, 72.44%; | H, 6.45%; | N, 16.33% |

### [Reference Example 102]

### 1-[(3S)-Aminopyrrolidin-1-yl]-3-methyl-2-phenylpyrido[1,2-a]benzimidazole-4-carbonitrile (I-102):

395 µl (2.83 mmol) of triethylamine and 99 µl (1.13 mmol) of (3S)-aminopyrrolidine were added to N,N-dimethylformaldehyde (10 ml) suspension of 300 mg (944 µmol) of 1-chloro-3-methyl-2-phenylpyrido[1,2-a]benzimidazole-4-carbonitrile (I-8). The system was replaced with nitrogen and sealed up, and heated at 80°C for 16 hours. After cooling, the solvent was evaporated under reduced pressure, the residue was dissolved in chloroform, washed with aqueous saturated sodium bicarbonate solution, and dried over anhydrous magnesium sulfate. The solvent was evaporated under reduced pressure, and the resulting residue was applied to a silica gel column chromatography. From the eluate with dichloromethane/methanol (20/1, v/v), a crude product of the entitled compound was obtained, and this was washed with diisopropyl ether and was collected by filtration to obtain 146 mg (42 %) of the entitled compound as a yellow crystal.
MS(ESI)m/z:368(M+1)⁺.
¹H-NMR(CDCl₃)δ: 1.80-2.35(2H, m), 2.31(3H, s), 2.72-3.80(5H, m), 7.20-7.30(2H, m), 7.33(1H, t, J=7.3Hz), 7.47-7.59(4H, m), 7.97-8.05(1H, m), 8.01(1H, d, J=8.3Hz).
IR(ATR) : 2222, 1624, 1589, 1466, 1441, 1408, 1373, 1300 cm⁻¹.

### [Example 107]

### 1-[(3S)-3-Benzylaminopyrrolidin-1-yl]-3-methyl-2-phenylpyrido[1,2-a]benzimidazole-4-carbonitrile (#107):

To 1,2-dichloroethane (4 ml) solution of 130 mg (354 µmol) of 1-[(3S)-aminopyrrolidin-1-yl]-3-methyl-2-phenylpyrido[1,2-a]benzimidazole-4-carbonitrile (I-102) were added 43 µl (425 µmol) of benzaldehyde and 113 mg (531 µmol) of sodium triacetoxyborohydride at room temperature, and 400 µl of acetic acid was added thereto at 0°C, and then stirred at room temperature for 24 hours. Aqueous 1 N sodium hydroxide solution was added thereto, and extracted with chloroform. The combined organic layers were dried over anhydrous magnesium sulfate. The solvent was evaporated under reduced pressure, and the resulting residue was applied to a silica gel column chromatography. From the eluate with dichloromethane/methanol (40/1, v/v), a crude product of the entitled compound was obtained, and this was recrystallized from ethanol to obtain 99 mg (61 %) of the entitled compound as a yellow crystal.
MS(ESI)m/z:458(M+1)⁺.
¹H-NMR(CDCl₃)δ: 1.72-1.90(1H, m), 2.31(3H, s), 2.67-2.92(1H, m), 3.07-3.80(7H, m), 7.20-7.39(8H, m), 7.43-7.58(4H, m), 7.99-8.09(1H, m), 8.01(1H, d, J=8.3Hz).
IR(ATR): 2222, 1624, 1587, 1493, 1469, 1441, 1300 cm⁻¹.

| Elemental analysis: C₃₀H₂₇N₅ | | | |
|---|---|---|---|
| Calcd. | C, 78.75%; | H, 5.95%; | N, 15.31% |
| Found | C, 78.42%; | H, 5.90%; | N, 15.13%. |

### [Reference Example 103]

### 1-[3-(tert-Butoxycarbonylamino)-1-cyclopenten-1-yl]-3-methyl-2-phenylpyrido[1,2-a]benzimidazole-4-carbonitrile (I-103):

1,4-Dioxane (45 ml) solution of 1.35 g (4.25 mmol) of 1-chloro-3-methyl-2-phenylpyrido[1,2-a]benzimidazole-4-carbonitrile (I-8), 2.01 g (4.25 mmol) of 1-(tert-butoxycarbonylamino)-3-tri-n-butylstannyl-2-cyclopentene (I-85), 149 mg (213 µmol) of dichlorobis(triphenylphosphine)palladium and 10 crystals of 2,6-di-tert-butyl-4-methylphenol was heated under reflux for 18.5 hours. After cooling, the solvent was evaporated under reduced pressure, the residue was suspended in cyclohexane and vigorously stirred for 1 hour. The precipitate was collected by filtration, washed with cyclohexane, and then applied to a silica gel column chromatography. From the eluate with dichloromethane/methanol (50/1, v/v), a crude product of the entitled compound was obtained. This was washed with diethyl ether and taken out through filtration to obtain 1.52 g (77 %) of the entitled compound as a yellow crystal.
MS(ESI)m/z:465(M+1)⁺.
¹H-NMR(CDCl₃)δ: 1.39-1.49(9H, m), 1.65-1.85(1H, m), 2.13-2.49(5H, m), 2.71-2.90(1H, m), 3.37-3.45(1H, m), 4.67-4.87(1H, m), 5.70-5.82(1H, m), 7.04-7.59(7.71H, m), 7.80-7.87(0.29H, m), 8.04(1H, d, J=8.3Hz).
IR(ATR): 2222, 1707, 1485, 1446, 1365, 1302, 1236, 1165 cm⁻¹.

### [Reference Example 104]

### 1-[3-(Amino-1-cyclopenten-1-yl)-3-methyl-2-phenylpyrido[1,2-a]benzimidazole-4-carbonitrile (I-104):

35 ml of 5 N hydrochloric acid was added to tetrahydrofuran (35 ml) solution of 1.61 g (3.47 mmol) of 1-[3-(tert-butoxycarbonylamino)-1-cyclopenten-1-yl]-3-methyl-2-phenylpyrido[1,2-a]benzimidazole-4-carbonitrile (I-103) at 0°C, and stirred at room temperature for 14 hours. Aqueous 1 N sodium hydroxide solution was added thereto, extracted with chloroform, and dried over anhydrous magnesium sulfate. The solvent was evaporated under reduced pressure, and the resulting residue was washed with diisopropyl ether and a precipitate was collected by filtration to obtain 1.15 g (91 %) of the entitled compound as a yellow crystal.
MS(ESI)m/z: 365(M+1)⁺.
¹H-NMR(CDCl₃)δ: 1.67-1.77 (1H, m), 2.13-2.52(5H, m), 2.71-2.91(1H, m), 3.77-3.84(0.29H, m), 4.03-4.10(0.71H, m), 5.72-5.76(0.29H, m), 5.79-5.84(0.71H, m), 7.07-7.65 (7.71H, m), 7.96(0.29H, d, J=8.1Hz), 8.04(1H, d, J=8.3Hz).
IR(ATR): 2222, 1620, 1587, 1483, 1444, 1300, 1236 cm⁻¹.

### [Example 108]

### 1-(3-Diethylamino-1-cyclopenten-1-yl)-3-methyl-2 phenylpyrido[1,2-a]carbonitrile-4-carbonitrile (#108):

To methanol (22 ml) suspension of 800 mg (2.20 mmol) of 1-[3-(amino-1-cyclopenten-1-yl)-3-methyl-2-phenylpyrido[1,2-a]benzimidazole-4-carbonitrile (I-104) were added 1.78 ml (22.0 mmol) of 37 % formaldehyde solution, 415 mg (6.60 mmol) of sodium cyanoborohydride and methanol (2 ml) at room temperature, and 2 ml of acetic acid was added thereto at 0°C and stirred at room temperature for 1 hour. Aqueous 1 N sodium hydroxide solution was added thereto, and extracted with chloroform, and the combined organic layers were dried over anhydrous magnesium sulfate. The solvent was evaporated under reduced pressure, and the resulting residue was applied to a silica gel column chromatography. From the eluate with dichloromethane/methanol (30/1, v/v), a crude product of the entitled compound was obtained. This was washed with diisopropyl ether and was collected by filtration to obtain 639 mg (74 %) of the entitled compound as a yellow crystal.
MS(ESI)m/z:393(M+1)⁺.
¹H-NMR(CDCl₃)δ: 1.78-2.40(12H, m), 2.78-2.92(1H, m), 3.29-3.38(0.28H, m), 4.11-4.19(0.72H, m), 5.83-5.88(0.28H, m), 5.91-5.96 (0. 72H, m) , 7.08-7.63 (7.72H, m), 7.94-7.99 (0.28H, m), 8.03-8.08(1H, m).
IR(ATR) : 2224, 1620, 1589, 1481, 1444, 1302, 1234, 1072 cm⁻¹.

| Elemental analysis: C₂₆H₂₄N₄·0.25H₂O | | | |
|---|---|---|---|
| Calcd. | C, 78.66%; | H, 6.22%; | N, 14.11% |
| Found | C, 78.91%; | H, 6.11%; | N, 14.19%. |

### [Reference Example 105]

### (2S)-2-Benzyloxycarbonylamino-4-(4-cyano-2-ethyl-3-methylpyrido[1,2-a]benzimidazol-1-ylamino)butanoic acid (I-105):

To N,N-dimethylformamide (120 ml) suspension of 3.30 g (12.2 mmol) of 1-chloro-2-ethyl-3-methylpyrido[1,2-a]benzimidazole-4-carbonitrile (I-2) were added 3.40 ml (24.4 mmol) of triethylamine and 3.70 g (14.7 mmol) of (2S)-2-benzyloxycarbonylamino-4-aminobutanoic acid. The system was replaced with nitrogen and sealed up, and heated at 80°C for 22 hours. After cooling, the solvent was evaporated under reduced pressure, and water was added thereto, and extracted with chloroform. The combined chloroform layers were dried over anhydrous magnesium sulfate. The solvent was evaporated under reduced pressure, and the resulting residue was applied to a silica gel column chromatography. From the eluate with dichloromethane/methanol (5/1, v/v), a crudeproduct of the entitled compound was obtained, and this was washed with diethyl ether and was collected by filtration to obtain 1.91 g (32 %) of the entitled compound as a yellow crystal.
MS(ESI)m/z:486(M+1)⁺.
¹H-NMR(DMSO-d₆)δ: 1.03-1.19(3H, m), 1.87-1.99(1H, m), 2.05-2.19(1H, m), 2.60(3H, s), 2.72-2.87(2H, m), 3.12-3.41(2H, m), 3.70-3.80(1H, m), 4.85-5.05(3H, m), 7.20-7.42(6H, m), 7.96-7.52(1H, m), 7.78(1H, d, J=8.3 Hz), 8.10-8.20(1H, m).
IR(ATR): 2218, 1701, 1626, 1593, 1529, 1495, 1444, 1311, 1275, 1248, 1228, 1051 cm⁻¹.

### [Reference Example 106]

### 1-[(3S)-3-Benzyloxycarbonylamino-2-oxo-pyrrolidin-1-yl]-2-ethyl-3-methylpyrido[1,2-a]benzimidazole-4-carbonitrile (I-106):

1.55 ml (11.1 mmol) of triethylamine was added to dichloromethane (150 ml) solution of 1.80 g (4.08 mmol) of benzotriazol-1-yloxytris(dimethylamino)phosphonium under nitrogen atmosphere, and dichloromethane/N,N-dimethylformamide (60 ml) (2/1, v/v) mixed solution of (2S)-2-benzyloxycarbonylamino-4-(4-cyano-2-ethyl-3-methylpyrido[1,2-a]benzimidazol-1-ylamino)butanoic acid (I-105) was added dropwise thereto at room temperature over a period of 25 minutes, and stirred for 24 hours. The solvent was evaporated under reduced pressure, and the residue was dissolved in chloroform, washed with aqueous saturated sodium bicarbonate solution, and dried over anhydrous magnesium sulfate. The solvent was evaporated under reduced pressure, and the resulting residue was applied to a silica gel column chromatography. From the eluate with dichloromethane/methanol (50/1, v/v) , a crude product of the entitled compound was obtained, and this was washed with diisopropyl ether and was collected by filtration to obtain 767 mg (44 %) of the entitled compound as a yellow crystal.
MS(ESI)m/z:468(M+1)⁺.
¹H-NMR(CDCl₃)δ: 1.24 (3H, t, J=7.3Hz), 2.51-2.82(6H, m), 2.99-3.10(1H, m), 3.60-3.76(1H, m), 3.91-4.00(0.29H, m), 4.05-4.15(0.71H, m), 4.52-4.67(1H, m), 5.10-5.25(2H, m), 5.60(1H, brs), 7.29-7.48(6.71H, m), 7.50-7.59(1H, m), 7.78-7.88(0.29H, m), 7.97-8.07(1H, m).
IR(ATR): 2225, 1712, 1498, 1446, 1415, 1308, 1279, 1246, 1223, 1182, 1045 cm⁻¹.

### [Example 109]

### 1-[[(3S)-3-Amino-2-oxo-pyrrolidin-1-yl]-2-ethyl-3-methylpyrido[1,2-a]benzimidazole-4-carbonitrile (#109):

Methanol (15 ml) solution of 690 mg (1.48 mmol) of 1-[(3S)-3-benzyloxycarbonylamino-2-oxo-pyrrolidin-1-yl]-2-ethyl-3-methylpyrido[1,2-a]benzimidazole-4-carbonitrile (I-106) and 70 mg of 10 % palladium-carbon catalyst was stirred under hydrogen atmosphere for 25.5 hours. The catalyst was removed through filtration, the solvent was evaporated under reduced pressure, and the resulting residue was applied to a silica gel column chromatography. From the eluate with dichloromethane/methanol (50/1, v/v), a crude product of the entitled compound was obtained, and this was recrystallized from ethanol to obtain 227 mg (46 %) of the entitled compound as a yellow crystal.
MS(ESI)m/z:334(M+1)⁺.
¹H-NMR(CDCl₃)δ: 1.21-1.31(3H, m), 2.20-2.43(1H, m), 2.55-2.78(5H, m), 2.80-2.93(1H, m), 3.60-3.70(1H, m), 3.95-4.14(2H, m), 7.32-7.92(1H, m), 7.49(0.5H, d, J=8.6 Hz), 7.52-7.59(1H, m), 7.86(0.5H, d, J=8.6 Hz), 8.02(1H, dd, J=8.3, 0.5Hz).
IR(ATR): 2227, 1709, 1631, 1502, 1448, 1419, 1308, 1281, 1227 cm⁻¹.

| Elemental analysis: C₁₉H₁₉N₅O | | | |
|---|---|---|---|
| Calcd. | C, 68.45%; | H, 5.74%; | N, 21.01% |
| Found | C, 68.15%; | H, 5.72%; | N, 20.73% |

### [Example 110]

### 1-[2-(Diethylamino)ethylthio]-2-ethyl-3-methylpyrido[1,2-a]benzimidazole-4-carbonitrile (#110):

To benzene (5 ml) suspension of 150 mg (556 µmol) of 1-chloro-2-ethyl-3-methylpyrido[1,2-a]benzimidazole-4-carbonitrile (I-2) were added 249 µl (1.67 mmol) of 1,8-diazabicyclo[5.4.0]-7-undecene and 113 mg (667 µmol) of 2-diethylaminoethanethiol hydrochloride. The system was replaced with nitrogen and sealed up, and stirred at room temperature for 2.5 hours. The solvent was evaporated under reduced pressure, and aqueous saturated sodium bicarbonate solution was added thereto, and extracted with chloroform. The combined chloroform layers were dried over anhydrous magnesium sulfate. The solvent was evaporated under reduced pressure, and the resulting residue was applied to a silica gel column chromatography. From the eluate with dichloromethane/methanol (5/1, v/v), a crude product of the entitled compound was obtained, and this was recrystallized from ethyl acetate to obtain 111 mg (55 %) of the entitled compound as a yellow crystal.
MS(ESI)m/z:367(M+1)⁺.
¹H-NMR(CDCl₃)δ: 0.96(6H, t, J=7.1Hz), 1.25(3H, t, J=7.3Hz), 2.48(4H, q, J=7.1Hz), 2.70-2.79(2H, m), 2.74(3H, s), 2.95-3.02(2H, m), 3.14(2H, q, J=7.3Hz), 7.36-7.42(1H, m), 7.53-7.60(1H, m), 8.03(1H, dd, J=8.1, 0.5Hz), 8.98(1H, d, J=8.8_{H}z).
IR(ATR) : 2224, 1618, 1577, 1464, 1446, 1429, 1373, 1331, 1292, 1194, 1063 cm⁻¹.

| Elemental analysis: C₂₁H₂₆N₄S | | | | |
|---|---|---|---|---|
| Calcd. | C, 67.98%; | H, 7.20%; | N, 15.10%; | S, 8.64% |
| Found | C, 68.40%; | H, 6.87%; | N, 15.21%; | S, 8.48%. |

### [Reference Example 107]

### 3-Methyl-1-oxo-1H,5H-pyrido[1,2-a]benzimidazole-4-carbonitrile (I-107)]

A mixture of 3.00 g (19.1 mmol) of (2-benzimidazolyl)acetonitrile, 2.43 ml (19.1 mmol) of ethyl acetoacetate and 2. 94 g (38.2 mmol) of ammonium acetate was heated at 140 to 150°C for 50 minutes. After cooling, water and acetonitrile were added thereto, and the solid was crushed and was collected by filtration. The resulting crystal was washed with water and acetonitrile to obtain 3.63 g (85%) of the entitled compound as a pale dark brown crystal.
MS(ESI)m/z:224 (M+1)⁺.
¹H-NMR(DMSO-d₆)δ: 2.35(3H, s), 5.95(1H, s), 7.31-7.40(1H, m), 7.48-7.59(2H, m), 8.54(1H, d, J = 8.1 Hz).
IR(ATR) : 2206, 1662, 1566, 1522, 1454, 1396, 1369, 1325, 1279, 1252, 1211, 1113, 1078, 1012 cm⁻¹.

### [Reference Example 108]

### 1-Chloro-3-methylpyrido[1,2-a]benzimidazole-4-carbonitrile (I-108):

1.50 g (6.72 mmol) of 3-methyl-1-oxo-1H,5H-pyrido[1,2-a]benzimidazole-4-carbonitrile (I-107) was heated under reflux in 5 ml of phosphoryl chloride for 2 hours. After cooling, phosphoryl chloride was evaporated under reduced pressure, and ice-water and aqueous 1 N sodium hydroxide solution were added to the residue, and this was extracted with chloroform. The combined chloroform layers were dried over anhydrous magnesium sulfate, and the solvent was evaporated under reduced pressure. The resulting residue was washed with diisopropyl ether and a precipitate was collected by filtration to obtain 1.04 g (64 %) of the entitled compound as a yellow crystal.
MS(ESI)m/z:242(M+1)⁺.
¹H-NMR(DMSO-d₆)δ: 2.62(3H, s), 7.33(1H, s), 7.45(1H, dd, J=8.5, 7.3Hz), 7.62(1H, dd, J=8.3, 7.3Hz), 7.93(1H, d, J=8.3Hz), 8.63(1H, d, J=8.5Hz).
IR(ATR): 2222, 1626, 1595, 1496, 1444, 1358, 1286, 1171 cm⁻¹

### [Example 111]

### 1-[(3S)-Dimethylaminopyrrolidin-1-yl]-3-methylpyrido[1,2-a]benzimidazole-4-carbonitrile (#111):

To N,N-dimethylformaldehyde (8 ml) suspension of 200 mg (828 µmol) of 1-chloro-3-methylpyrido[1,2-a]benzimidazole-4-cabonitrile (T-11) were added 346 µl (2.48 mmol) of triethylamine and 126 µl (993 µmol) of (3S)-dimethylaminopyrrolidine. The system was replaced with nitrogen and sealed up, and heated at 140°C for 20 minutes. After cooling, the solvent was evaporated under reduced pressure, and the residue was dissolved in chloroform, washed with aqueous saturated sodium bicarbonate solution, and dried over anhydrous magnesium sulfate. The solvent was evaporated under reduced pressure, and the resulting residue was applied to a silica gel column chromatography. From the eluate with dichloromethane/methanol (20/1, v/v) , a crude product of the entitled compound was obtained, and this was recrystallized from ethyl acetate to obtain 69 mg (26 %) of the entitled compound as a yellow crystal.
MS(ESI)m/z:320(M+1)⁺.
¹H-NMR(CDCl₃)δ: 2.01-2.13(1H, m), 2.28-2.40(1H, m), 2.32(6H, s), 2.65(3H, s), 3.00-3.10(1H, m) , 3.20-3.39(2H, m), 3.51 (2H, brs), 6.25(1H, s), 7.32-7.40(1H, m), 7.50-7.58(1H, m), 7.98 (2H, d, J=8.5Hz).
IR(ATR): 2222, 1628, 1597, 1514, 1475, 1442, 1417, 1348, 1308, 1201, 1153, 1117 cm⁻¹.

| Elemental analysis: C₁₉H₂₁N₅ | | | |
|---|---|---|---|
| Calcd. | C, 71.45%; | H, 6.63%; | N, 21.93% |
| Found | C, 71.40%; | H, 6.91%; | N, 21.86%. |

### [Reference Example 109]

### 2-(2-Hydroxybutyl)-3-methyl-1-oxo-1H,5H-pyrido[1,2-a]benzimidazole-4-carbonitrile (I-109):

To 1,4-dioxane (100 ml) suspension of 691 mg (17.3 mmol) of sodium hydride was added 1,4-dioxane (30 ml) solution of 2.00 ml (15.7 mmol) of ethyl acetoacetate under nitrogen atmosphere at room temperature, and stirred for 5 minutes, and then 1,4-dioxane (30 ml) solution of 2.71 ml (31.4 mmol) of 1,2-butylene oxide was added thereto at room temperature, and heated under reflux for 20 hours. After cooling, the solvent was evaporated under reduced pressure, and the residue was dissolved in ethyl acetate, and washed with aqueous saturated sodium bicarbonate solution and 1 N hydrochloric acid, and the aqueous layer was extracted with ethyl acetate. The combined ethyl acetate layers were dried over anhydrous magnesium sulfate, and the solvent was evaporated under reduced pressure. The resulting residue was applied to a silica gel column chromatography. From the eluate with n-hexane/ethyl acetate (4/1, v/v), a colorless oil was obtained.

A mixture of the oil, 312 mg (1.98 mmol) of (2-benzimidazolyl)acetonitrile and 305 mg (3.96 mmol) of ammonium acetate was heated at 140 to 150°C for 20 minutes. After cooling, water and acetonitrile were added thereto, and the solid was crushed and was collected by filtration. The resulting crystal was washed with water and acetonitrile to obtain 459 mg (10 %) of the entitled compound as a dark brown crystal. MS(ESI)m/z:295(M⁺).
¹H-NMR(DMSO-d₆)δ: 0.89(3H, t, J=7.3Hz), 1.30-1.50(2H, m), 2.41(3H, s), 2.55(1H, dd, J=13.4, 12.1Hz), 2.73(1H, dd, J=13.4, 5.1Hz), 3.58 (1H, brs), 4.38(1H, brs), 7.30-7.39(1H, m), 7.45-7.55(2H, m), 8.53-8.63(1H, d, J=8.1Hz).
IR(ATR) : 3363, 2204, 1643, 1589, 1537, 1468, 1288, 1252 cm⁻¹.

### [Reference Example 110]

### 2-(2-Acetoxybutyl)-3-methyl-1-oxo-1H,5H-pyrido[1,2-a]benzimidazole-4-carbonitrile (I-110):

To acetic anhydride (214 µl, 2.27 mmol) suspension of 447 mg (1.51 mmol) of 2-(2-hydroxybutyl)-3-methyl-1-oxo-1H,5H-pyrido[1,2-a]benzimidazole-4-carbonitrile (I-109) were added 1.47 ml (18.1 mmol) of pyridine and 2 mg (15 µmol) of dimethylaminopyridine at 0°C, and stirred at room temperature for 4.5 hours. Ethyl acetate was added thereto, then washed wi th water and brine, and dried over anhydrous magnesium sulfate. The solvent was evaporated under reduced pressure. The resul ting residue was washed with acetonitrile and a precipitate was collectedby filtration to obtain 417 mg (82 %) of the entitled compound as a pale dark brown crystal.
MS(ESI)m/z:338(M+1)⁺.
¹H-NMR(DMSO-d₆)δ: 0.80-0.95(3H, m), 1.50-1.70(2H, m), 1.90(3H, s), 2.43(3H, s), 2.70-2.79(1H, m), 2.86-2.98(1H, m), 4.90-5.00(1H, m), 7.30-7.41(1H, m), 7.48-7.59(2H, m), 8.58(1H, d, J=8.1Hz).
IR(ATR): 2206, 1730, 1660, 1603, 1550, 1466, 1240 cm⁻¹.

### [Reference Example 111]

### 2-(2-Acetoxybutyl)-1-chloro-3-methylpyrido[1,2-a]benzimidazole-4-carbonitrile (I-111):

According to the production method for (I-108), 415 mg (1.23 mmol) of 2-(2-acetoxybutyl)-3-methyl-1-oxo-1H,5H-pyrido[1,2-a]benzimidazole-4-carbonitrile (I-110) was heated under reflux in 3 ml of phosphoryl chloride for 2.5 hours and processed to obtain 417 mg (95 %) of the entitled compound as a yellow crystal.
MS(ESI)m/z:356(M+1)⁺.
¹H-NMR(DMSO-d₆)δ: 0.90-0.97(3H, m), 1.65-1.80(2H, m), 1.86(3H, s), 2.73(3H, s), 3.12-3.19(2H, m), 5.01-5.10(1H, m), 7.42-7.48(1H, m), 7.60-7.67(1H, m), 7.93(1H, d, J=8.3Hz), 8.70(1H, d, J=8.5Hz).
IR(ATR): 2227, 1728, 1630, 1473, 1354, 1292, 1238 cm⁻¹.

### [Reference Example 112]

### 2-(2-Acetoxybutyl)-1-[(3S)-dimethylaminopyrrolidin-1-yl]-3-methylpyrido[1,2-a]benzimidazole-4-carbonitrile (I-112):

To N,N-dimethylformaldehyde (11 ml) suspension of 413 mg (1.16 mmol) of 2-(2-acetoxybutyl)-1-chloro-3-methylpyrido[1,2-a]benzimidazole-4-carbonitrile (I-111) were added 485 µl (3.48 mmol) of triethylamine and 177 µl (1.39 mmol) of (3S)-dimethylaminopyrrolidine. The system was replaced with nitrogen and sealed up, and heated at 80°C for 21.5 hours. After cooling, the solvent was evaporated under reduced pressure, and the residue was dissolved in chloroform, washed with aqueous saturated sodium bicarbonate solution, and dried over anhydrous magnesium sulfate. The solvent was evaporated under reduced pressure, and the resulting residue was applied to a silica gel column chromatography. From the eluate with dichloromethane/methanol (40/1, v/v), a crude product of the entitled compound was obtained, and this was washed with diisopropyl ether and was collected by filtration to obtain 230 mg (46 %) of the entitled compound as a yellow crystal.
MS (ESI)m/z:434 (M+1)⁺.
¹H-NMR(CDCl₃)δ: 0.98-1.08(3H, m), 1.70-1.80(2H, m), 1.86(3H, s), 2.12-2.28(1H, m), 2.36(6H, s), 2.39-2.49(1H, m), 2.76(1.5H, s), 2.77(1.5H, s), 2.86-3.13 (3H, m), 3.35-3.80(4H, m), 5.10-5.28(1H, m) , 7.32-7.40(1H, m), 7.50-7.58 (1H, m), 7.90-8.09(1H, m), 8.01(1H, d, J=8.3Hz).
IR(ATR) : 2222, 1734, 1626, 1589, 1473, 1441, 1406, 1375, 1300, 1240 cm⁻¹.

### [Example 112]

### 1-[(3S)-Dimethylaminopyrrolidin-1-yl]-2-(2-hydroxybutyl)-3-methylpyrido[1,2-a]benzimidazole-4-carbonitrile (#112):

2.5 ml of concentrated hydrochloric acid was added to methanol (2.5 ml) solution of 104 mg (240 µmol) of 2-(2-acetoxybutyl)-1-[(3S)-dimethylaminopyrrolidin-1-yl]-3-methylpyrido[1,2-a]benzimidazole-4-carbonitrile (I-112) at 0°C, and then s ti rred at room temperature for 3 hours. The solvent was evaporated under reduced pressure, and the residue was dissolved in chloroform, and washed with aqueous saturated sodium bicarbonate solution, and the aqueous layer was extracted with chloroform. The combined chloroform layers were dried over anhydrous magnesium sulfate. The solvent was evaporated under reduced pressure, and the resulting residue was applied to a silica gel column chromatography. From the eluate with dichloromethane/methanol (20/1, v/v), a crude product of the entitled compound was obtained, and this was washed with diisopropyl ether and was collected by filtration to obtain 60 mg (64 %) of the entitled compound as a yellow crystal.
MS(ESI)m/z:392(M+1)⁺.
¹H-NMR(CDCl₃)δ: 1.11-1.29(3H, m), 1.71-1.85(2H, m), 2.11-2.49(11H, m), 2.67-3.21(4H, m), 3.31-4.48(4H, m), 7.22-7.32(1H, m), 7.41-7.51(1H, m), 7.78-8.09(2H, m).
IR(ATR) : 3265, 2224, 1626, 1591, 1475, 1444, 1408, 1298, 1155, 1057 cm⁻¹.

| Elemental analysis: C₂₃H₂₉N₅O·0.25H₂O | | | |
|---|---|---|---|
| Calcd. | C, 69.76%; | H, 7.51%; | N, 17.68% |
| Found | C, 70.11%; | H, 7.47%; | N, 17.56%. |

### [Reference Example 113]

### α-Acetyl-γ-phenyl-γ-butyrolactone (I-113):

To 1,4-dioxane (100 ml) suspension of 739 mg (18.5 mmol) of sodium hydride was added 1,4-dioxane (25 ml) solution of 2.00 ml (15.4 mmol) of ethyl acetoacetate under nitrogen atmosphere at 0°C, and stirred at room temperature for 35 minutes. Then, 1,4-dioxane (25 ml) solution of 3.50 ml (30.7 mmol) of styrene oxide was added thereto at 0°C, and heated under reflux for 14 hours. After cooling, aqueous saturated ammonium chloride solution was added thereto, the solvent was evaporated under reduced pressure, and the remaining aqueous layer was extracted with ethyl acetate. The combined ethyl acetate layers were dried over anhydrous magnesium sulfate, the solvent was evaporated under reduced pressure, and the resulting residue was applied to a silica gel column chromatography. From the eluate with n-hexane/ethyl acetate (5/1, v/v) , 732 mg (23 %) of the entitled compound was obtained as a yellow oil.
MS(EI)m/z:204(M⁺).
¹H-NMR(CDCl₃)δ: 2.24 (0.5H, ddd, J=13.2, 9.0, 7.8Hz) , 2.48(1.5H, s), 2.51(1.5H, s), 2.66(0.5H, ddd, J=13.4, 9.0, 6.6Hz), 2.78(0.5H, ddd, J=13.4, 11.0, 10.0Hz), 3.12-3.19(0.5H, m) , 3.84-3.93(1H, m), 5.44(0.5H, dd, J=10.0, 6.6Hz), 5.57(0.5H, dd, J=15.2, 7.6Hz), 7.30-7.45(5H, m).
IR(ATR): 1766, 1716, 1360, 1329, 1223, 1149 cm⁻¹.

### [Reference Example 114]

### 2-(2-Hydroxy-2-phenylethyl)-3-methyl-1-oxo-1H,5H-pyrido[1,2-a]benzimidazole-4-carbonitrile (I-114):

According to the production method for (I-107), a mixture of 725 mg (3.55 mmol) of α-acetyl-γ-phenyl-γ-butyrolactone (I-113), 558 mg (3.55 mmol) of (2-benzimidazolyl) acetonitrile and 547 mg (7.10 mmol) of ammonium acetate was heated at 140 to 150°C for 1 hour and processed to obtain 951 mg (78 %) of the entitled compound as a pale brown crystal.
MS(ESI)m/z:344(M+1)⁺.
¹H-NMR(DMSO-d₆)δ: 2.15(3H, s) , 2.77-2.84 (1H, m), 2.88-2.95(1H, m), 4.76-4.83(1H, m), 5.19-5.25(1H, m), 7.18-7.24(1H, m), 7.27-7.41(5H, m), 7.51(2H, d, J=3.7Hz), 8.63(1H, d, J=8.1Hz), 13.4(1H, brs).
IR(ATR): 3286, 2210, 1645, 1572, 1533, 1269, 1034 cm⁻¹.

### [Reference Example 115]

### 2-(2-Acetoxy-2-phenylethyl)-3-methyl-1-oxo-1H,5H-pyrido[1,2-a]benzimidazole-4-carbonitrile (I-115):

To acetic anhydride (391 µl, 4.15 mmol) suspension of 949 mg (2.76 mmol) of 2-(2-hydroxy-2-phenylethyl)-3-methyl-1-oxo-1H,5H-pyrido[1,2-a]benzimidazole-4-carbonitrile (I-114) were added 2.68 ml (33.1 mmol) of pyridine and 3 mg (28 µmol) of dimethylaminopyridine at 0°C, and 1 ml of pyridine was added thereto at room temperature and then stirred for 24 hours. Ethyl acetate was added thereto and suspended, and then this was filtered and washed with ethyl acetate to obtain 313 mg (29 %) of the entitled compound as a milky white crystal.
MS(ESI)m/z:386(M+1)⁺.
¹H-NMR(DMSO-d₆)δ: 1.97(3H, s) , 2.23(3H, s), 3.02(1H, dd, J=13.9, 5.4 Hz), 3.12(1H, dd, J=13.9, 8.5Hz), 5.85-5.93(1H, m), 7.26-7.41 (6H, m), 7.53(2H, d, J=3.9Hz), 8.62(1H, d, J=8.1Hz), 13.5(1H, brs).
IR(ATR): 2212, 1732, 1664, 1614, 1549, 1466, 1225 cm⁻¹.

### [Examples 113 and 114]

### 1-[(3S)-Dimethylaminopyrroldin-1-yl]-2-(2-hydroxy-2-phenylethyl)-3-methylpyrido[1,2-a]benzimidazole-4-carbonitrile (#113), and

### 1-[(3S)-Dimethylaminopyrroldin-1-yl]-3-methyl-2-styrylpyrido[1,2-a]benzimidazole-4-carbonitrile (#114):

309 mg (802 µmol) of 2-(2-acetoxy-2-phenylethyl)-3-methyl-1-oxo-1H,5H-pyrido[1,2-a]benzimidazole-4-carbonitrile (I-115) was heated under reflux in 8ml of phosphoryl chloride for 5 hours. After cooling, phosphoryl chloride was evaporated under reduced pressure, and ice-water and aqueous 1 N sodium hydroxide solution were added to the residue, and this was extracted with chloroform. The combined chloroform layers were dried over anhydrous magnesium sulfate, and the solvent was evaporated under reduced pressure. The resulting residue was washed with diisopropyl ether and a precipitate was collected by filtration to obtain a yellow solid.

To N,N-dimethylformaldehyde (7 ml) suspension of the yellow solid were added 192 µl (1.38 mmol) of triethylamine and 131 µl (1.03 mol) of (3S)-dimethylaminopyrrolidine. The system was replaced with nitrogen and sealed up, and heated at 80°C for 6 hours. After cooling, the solvent was evaporated under reduced pressure, and the residue was dissolved in chloroform, washed with aqueous saturated sodium bicarbonate solution, and dried over anhydrous magnesium sulfate. The solvent was evaporated under reduced pressure, and the resulting residue was applied to a silica gel column chromatography. From the eluate with dichloromethane/methanol (30/1, v/v) , ayellowsolid was obtained.

5 ml of concentrated hydrochloric acid was added to methanol (5 ml) solution of the yellow solid at 0°C, and stirred at room temperature for 1.5 hours. The solvent was evaporated under reduced pressure, and the resulting residue was dissolved in chloroform, and washed with aqueous saturated sodium bicarbonate solution, and the aqueous layer was extracted with chloroform. The combined chloroform layers were dried over anhydrous magnesium sulfate, the solvent was evaporated under reduced pressure, and the resulting residue was applied to a silica gel column chromatography. From the eluate with dichloromethane/methanol (20/1, v/v), crude products of the two entitled compounds were obtained. These were washed with diisopropyl ether and taken out through filtration to obtain 26 mg (8 %) of 1-[(3S)-dimethylaminopyrroldin-1-yl]-2-(2-hydroxy-2-phenylethyl)-3-methylpyrido[1,2-a]benzimidazole-4-carbonitrile (#113), and 48 mg (15 %) of 1-[(3S)-dimethylaminopyrroldin-1-yl]-3-methyl-2-styrylpyrido[1,2-a]benzimidazole-4-carbonitrile (#114), both as a yellow oil.

### 1-[(3S)-Dimethylaminopyrroldin-1-yl]-2 -(2-hydroxy-2-phenylethyl)-3-methylpyrido[1,2-a]benzimidazole-4-carbonitrile (#113):

MS(ESI)m/z:440(M+1)⁺.
¹H-NMR(CDCl₃)δ: 2.05-2.58(11H, m), 2.90-4.00 (7H, m), 5.00-5.32(1H, m), 7.22-7.62(7H, m), 7.80-8.11(2H, m).
IR(ATR): 3301, 2225, 1626, 1591, 1477, 1444, 1406, 1300, 1057, 1041 cm⁻¹.

| Elemental analysis: C₂₇H₂₉N₅O | | | |
|---|---|---|---|
| Calcd. | C, 73.78%; | H, 6.65%; | N, 15.93% |
| found | C, 73.45%; | H, 6.66%; | N, 15.64%. |

### 1-[(3S)-Dimethylaminopyrroldin-1-yl]-3-methyl-2-styrylpyrido[1,2-a]benzimidazole-4-carbonitrile (#114):

MS(ESI)m/z:422(M+1)⁺.
¹H-NMR(CDCl₃)δ: 2.00-2.11(1H, m), 2.19-2.31(1H, m), 2.23(6H, s), 2.68(3H, s), 2.82-3.54(3H, m), 3.67-3.76(2H, m), 6.66(1H, d, J = 16.4Hz), 6.97(1H, d, J=16.4Hz), 7.33-7.40(2H, m), 7.41-7.48(2H, m), 7.51-7.59(3H, m), 7.99-8.09(1H, m), 8.01(1H, d, J=8.1Hz).
IR(ATR): 2222, 1624, 1587, 1496, 1475, 1442, 1373, 1300 cm⁻¹.

| Elemental analysis: C₂₇H₂₇N₅·0.25H₂O | | | |
|---|---|---|---|
| Calcd. | C, 76.12%; | H, 6.51%; | N, 16.44% |
| Found | C, 76.28%; | H, 6.39%; | N, 16.17%. |

### [Reference Example 116]

### 2-(2-Benzenesulfinylethyl)-1-[(3S)-dimethylaminopyrrolidin-1-yl]-3-methylpyrido[1,2-a]benzimidazole-4-carbonitrile (I-116):

218 mg (1.02 mmol) of sodium metaperiodate was added to methanol/benzene/water (5.13 ml) (32/1/8, v/v) mixed solution of 232 mg (509 µmol) of 1-[(3S)-dimethylaminopyrrolidin-1-yl]-3-methyl-2-(2-phenylthioethyl)pyrido[1,2-a]benzimidazole-4-carboni trile (#70) at room temperature, and stirred for 18 hours. The reaction mixture was extracted with chloroform, and dried over anhydrous magnesium sulfate, and the solvent was evaporated under reduced pressure. The resulting residue was applied to a silica gel column chromatography. From the eluate with dichloromethane/methanol (30/1, v/v), 49 mg (20 %) of the entitled compound was obtained as a yellow crystal.
MS(ESI)m/z:472(M+1)⁺.
¹H-NMR(CDCl₃)δ: 2.09-2.21(1H, m), 2.25-2.40(1H, m), 2.33 and 2.35 (6H, s), 2.53 and 2.54 (3H, s), 2.67-3.75(9H, m), 7.32-7.40(1H, m), 7.50-7.65(4H, m), 7.68-7.73(2H, m), 7.93-8.15(1H, m), 7.98(1H, d, J=8.3 Hz).
IR(ATR): 2222, 1626, 1593, 1506, 1481, 1442, 1373, 1306, 1041 cm⁻¹.

### [Example 115]

### 1-[(3S)-Dimethylaminopyrroldin-1-yl]-3-methyl-2-vinylpyrido[1,2-a]benzimidazole-4-carbonitrile (#115):

Bromobenzene (3 ml) solution of 46 mg (97.5 µmol) of 2-(2-benzenesulfinylethyl)-1-[(3S)-dimethylaminopyrrolidin-1-yl]-3-methylpyrido[1,2-a]benzimidazole-4-carbonitrile (I-116) was heated under reflux for 15 hours. The solvent was evaporated under reduced pressure, and the resulting residue was applied to a silica gel column chromatography. From the eluate with dichloromethane/methanol (30/1, v/v), a crude product of the entitled compound was obtained. This was washed with diisopropyl ether and taken out through filtration to obtain 9 mg (27 %) of the entitled compound as a yellow crystal.
MS(ESI)m/z:346(M+1)⁺,
¹H-NMR(CDCl₃)δ: 2.01-2.15(1H, m), 2.28-2.39(1H, m), 2.33(6H, s), 2.61(3H, s), 2.91-3.02(1H, m), 3.30-3.68 (4H, m), 5.38(1H, dd, J=17.8, 1.5 Hz), 5.75(1H, dd, J=11.0, 1.5Hz), 6.66(1H, dd, J=17.8, 11.0Hz), 7.33-7.40(1H, m), 7.51-7.59(1H, m), 7.99(1H, d, J=8.1Hz), 8.04(1H, brd, J=8.3Hz).
IR(ATR) : 2220, 1626, 1591, 1475, 1442, 1408, 1373, 1346, 1296, 1209, 1155 cm⁻¹.

| Elemental analysis: C₂₁H₂₃N₅·0.25H₂O | | | |
|---|---|---|---|
| Calcd. | C, 72.08%; | H, 6.77%; | N, 20.01% |
| Found | C, 72.35%; | H, 6.65%; | N, 19.78%. |

### [Reference Example 117]

### Ethyl (3-fluorophenyl)acetate (I-117):

1.41 ml (15.6 mmol) of thionyl chloride was added to ethanol (65 ml) solution of 2.00 g (13.0 mmol) of (3-fluorophenyl)acetic acid under nitrogen atmosphere at 0°C, and stirred at room temperature for 2 hours. Water was added thereto, and the solvent was evaporated under reduced pressure. Ethyl acetate was added to the remaining aqueous layer. This was washed with aqueous saturated sodium bicarbonate solution and brine, and dried over anhydrous magnesium sulfate, and the solvent was evaporated under reduced pressure to obtain 2.35 g (99 %) of the entitled compound as a colorless oil.
MS(EI)m/z:182 (M⁺).
¹H-NMR(CDCl₃)δ: 1.26(3H, t, J=7.1Hz), 3.61(2H, s), 4.16(2H, q, J=7.1Hz), 6.94-7.10(3H, m), 7.24-7.33(1H, m).
IR(ATR): 1732, 1591, 1489, 1450, 1255, 1142, 1030 cm⁻¹

### [Reference Example 118]

### Ethyl 2-(3-fluorophenyl)acetoacetate (I-118):

To tetrahydrofuran (100 ml) suspension of 619 mg (15.5 mmol) of sodium hydride was added tetrahydrofuran (30 ml) solution of 2.35g (12.9mmol) of ethyl 2-(3-fluorophenyl)acetate (I-117) under nitrogen atmosphere at 0°C, and stirred at room temperature for 2 hours. 1.89 ml (19.3 mmol) of ethyl acetate was added thereto at 0°C, and this was heated under reflux for 14 hours. After cooling, aqueous saturated ammonium chloride solution was added thereto, and extracted with ethyl acetate. The combined ethyl acetate layers were dried over anhydrous magnesium sulfate, the solvent was evaporated under reduced pressure, and the resulting residue was applied to a silica gel column chromatography. From the eluate with n-hexane/ethyl acetate (10/1, v/v), 1.38 g (48 %) of the entitled compound was obtained as a yellow oil.
MS (FAB) m/z : 225 (M+1)⁺.
¹H-NMR(CDCl₃)δ: 1.13-1.31(3H, m), 1.86(1.29H, s), 2.21(1.71H, s), 4.10-4.29(2H, m), 4.68(0.57H, s), 6.79-7.14(3H, m), 7.23-7.99(1H, m), 13.09-13.13(0.43H, m).
IR(ATR): 1720, 1641, 1612, 1589, 1333, 1275, 1230, 1176, 1140 cm⁻¹.

### [Reference Example 119]

### 2-(3-Fluorophenyl)-3-methyl-1-oxo-1H,5H-pyrido[1,2-a]benzimidazole-4-carbonitrile (I-119):

According to the production method for (I-107), a mixture of 700 mg (3.12 mmol) of ethyl 2-(3-fluorophenyl)acetoacetate (I-118), 491 mg (3.12 mmol) of (2-benzimidazolyl)acetonitrile and 481 mg (6.24 mmol) of ammonium acetate was heated at 140 to 150°C for 1 hour and processed to obtain 498 mg (50 %) of the entitled compound as a pale brown crystal.
MS(ESI)m/z:318(M+1)⁺.
¹H-NMR(CDCl₃)δ: 2.36(3H, s), 7.00-7.12(3H, m), 7.35-7.41(2H, m), 7.45-7.52(2H, m), 8.74(1H, d, J=8.1Hz), 10.42(1H, brs).
IR(ATR): 2200, 1668, 1608, 1583, 1545, 1442, 1414, 1298, 1219 cm⁻¹

### [Reference Example 120]

### 1-Chloro-2-(3-fluorophenyl)-3-methylpyrido[1,2-a]benzimidazole-4-carbonitrile (I-120):

According to the production method for (I-108), 497 mg (1.57 mmol) of 2-(3-fluorophenyl)-3-methyl-1-oxo-1H,5H-pyrido[1,2-a]benzimidazole-4-carbonitrile (I-119) was heated under reflux in 8 ml of phosphoryl chloride and processed to obtain 433 mg (82 %) of the entitled compound as a yellow crystal.
MS(EI)m/z:336(M+1)⁺.
¹H-NMR(CDCl₃)δ: 2.43(3H, s), 7.00-7.05(1H, m), 7.08(1H, d, J=7.8Hz), 7.20-7.25(1H, m), 7.40-7.47(1H, m), 7.51-7.58(1H, m), 7.61-7.67 (1H, m), 8.08(1H, d, J=8.3 Hz), 8.56(1H, d, J=8.5 Hz).
IR(ATR): 2229, 1583, 1462, 1444, 1311, 1234, 1190

### [Example 116]

### 1-[(3S)-Dimethylaminopyrrolidin-1-yl]-2-(3-fluorophenyl)-3-methylpyrido[1,2-a]benzimidazole-4-carbonitrile (#116):

According to the production method for (I-112), N,N-dimethylformaldehyde (6 ml) solution of 200 mg (596 µmol) of 1-chloro-2-(3-fluorophenyl)-3-methylpyrido[1,2-a]benzimidazole-4-carbonitrile (I-120), 166 µl (1.19 mmol) of triethylamine and 91 µl (715 µmol) of (3S) -dimethylaminopyrrolidine was heated at 80°C for 13.5 hours and processed, and then a crude product was recrystallized from ethanol to obtain 161 mg (65 %) of the entitled compound as a yellow crystal.
MS (ESI)m/z:414 (M+1)⁺.
¹H-NMR(CDCl₃)δ: 1.80-2.50(2H,m), 2.15(6H, d, J=3.7Hz) , 2.31(3H, s), 2.65-3.82(5H, m), 6.96-7.12(2H, m), 7.18-7.28(1H, m), 7.35(1H, dd, J=8.3, 7.1 Hz), 7.48-7.60(2H, m), 7.82-8.22(1H, m), 8.02(1H, d, J=8.3Hz).
IR(ATR): 2222, 1581, 1498, 1466, 1442, 1298, 1263, 1196 cm⁻¹.

| Elemental analysis: C₂₅H₂₄FN₅ | | | | |
|---|---|---|---|---|
| Calcd. | C, 72.62%; | H, 5.85%; | N, 16.94%; | F, 4.59% |
| Found | C, 72.54%; | H, 5.76%; | N, 16.74%; | F, 4.65%. |

### [Reference Example 121]

### Ethyl 2-(4-benzyloxyphenyl)acetate (I-121):

To tetrahydrofuran (75 ml) suspension of 639 mg (16.0 mmol) of sodium hydride were added tetrahydrofuran (30 ml) solution of 2.00 g (11.1 mmol) of ethyl (4-hydroxyphenyl) acetate and tetrahydrofuran (30 ml) solution of 491 mg (1.33 mmol) of tetrabutylammonium iodide and 2.37 ml (20.0 mmol) of benzyl bromide under nitrogen atmosphere at 0°C, and stirred at room temperature for 18 hours. Water and aqueous saturated ammonium chloride solution were added thereto, and extracted with ethyl acetate. The combined ethyl acetate layers were dried over anhydrous magnesium sulfate, and the solvent was evaporated under reduced pressure. The resulting residue was applied to a silica gel column chromatography. From the eluate with n-hexane/ethyl acetate (5/1, v/v), 2.67 g (89 %) of the entitled compound was obtained as a colorless oil.
MS(ESI)m/z:271(M+1)⁺.
¹H-NMR(CDCl₃)δ: 1.20-1.29(3H, m), 3.55(2H, s), 4.14(2H, q, J=7.1Hz), 5.05(2H, s), 6.89-6.98(2H, m), 7.15-7.27(3H, m), 7.30-7.46 (4H, m).
IR(ATR): 1730, 1510, 1454, 1298, 1236, 1221, 1176, 1149, 1026 cm⁻¹.

### [Reference Example 122]

### Ethyl 2-(4-benzyloxyphenyl)acetoacetate (I-122):

According to the production method for (I-118), tetrahydrofuran (75 ml) suspension of 353 mg (8.83 mmol) of sodium hydride, 1.99 g (7.36 mmol) of ethyl 2-(4-benzyloxyphenyl)acetate (I-121) and 1.08 ml (11.0 mmol) of ethyl acetate was heated under reflux for 14 hours andprocessed to obtain 1.25 g (54 %) of the entitled compound as colorless oil.
MS(ESI)m/z:313(M+1)⁺.
¹H-NMR(CDCl₃)δ: 1.16-1.32(3H, m), 1.85(1.5H, s), 2.17(1.5H, s), 4.10-4.29(2H, m) , 4.62(0.5H, s) , 5.01-5.12(2H, m) , 6.90-7.02(2H, m), 7.05-7.12(1H, m), 7.20-7.50(6H, m), 13.08-13.13(0.5H, m). IR(ATR): 1716, 1608, 1508, 1240, 1223, 1176, 1142, 1024 cm⁻¹.

### [Reference Example 123]

### 2-(4-Benzyloxyphenyl)-3-methyl-1-oxo-1H,5H-pyrido[1,2-a]benzimidazole-4-carbonitrile (I-123):

According to the production method for (I-107), a mixture of 1.03g (3.30mmol) of ethyl 2-(4-benzyloxyphenyl)acetoacetate (I-122), 518 mg (3.30 mmol) of (2-benzimidazolyl)acetonitrile and 509 mg (6.60 mmol) of ammonium acetate was heated at 140 to 150°C for 1 hour and processed to obtain 997 mg (75 %) of the entitled compound as a pale brown crystal.
MS(ESI)m/z:406(M+1)⁺.
¹H-NMR(DMSO-d₆)δ: 2.22(3H, s) , 5.14 (2H, s), 7.05(2H, d, J=8.3Hz), 7.19(2H, d, J=8.3Hz), 7.30-7.66(8H, m) , 8.54(1H, d, J=8.1Hz), 13.56(1H, s).
IR(ATR): 2204, 1668, 1614, 1545, 1466, 1460, 1238, 1182, 1109 cm⁻¹.

### [Reference Example 124]

### 2-(4-Benzyloxyphenyl)-1-chloro-3-methylpyrido[1,2-a]benzimidazole-4-carbonitrile (I-124):

According to the production method for (I-108), 992 mg (2.45 mmol) of 2-(4-benzyloxyphenyl)-3-methyl-1-oxo-1H,5H-pyrido[1,2-a]benzimidazole-4-carbonitrile (I-123) was heated under reflux in 5 ml of phosphoryl chloride for 2 hours and processed to obtain 966 mg (93 %) of the entitled compound as a yellow crystal.
MS(ESI)m/z:424 (M+1)⁺.
¹H-NMR(CDCl₃)δ: 2.43(3H, s), 5.15(2H, s), 7.12-7.25(4H, m), 7.36-7.55(6H, m), 7.58-7.68(1H, m), 8.06(1H, d, J=8.3Hz), 8.56(1H, dd, J=8.6, 0.7Hz).
IR(ATR): 2224, 1593, 1510, 1446, 1306, 1240, 1174 cm⁻¹.

### [Reference Example 125]

### 2-(4-Benzyloxyphenyl)-1-[(3S)-dimethylaminopyrroldin-1-yl]-3-methylpyrido[1,2-a]benzimidazole-4-carbonitrile (I-125):

According to the production method for (I-112), N,N-dimethylformaldehyde (7 ml) solution of 300 mg (708 µmol) of 2-(4-benzyloxyphenyl)-1-chloro-3-methylpyrido[1,2-a]benzimidazole-4-carbonitrile (I-124), 197 µl (1.42 mmol) of triethylamine and 108 µl (849 µmol) of (3S) -dimethylaminopyrrolidine was heated at 80°C for 11 hours and processed, and then this was washed with diisopropyl ether and a precipitate was collected by filtration to obtain 271 mg (76 %) of the entitled compound as a yellow crystal.
MS(ESI)m/z:502(M+1)⁺.
¹H-NMR(CDCl₃)δ: 1.80-2.20(2H, m), 2.14(6H, s), 2.32(3H, s), 2.70-3.60(5H, m) , 5.16(2H, s), 7.10-7.20(4H, m), 7.31-7.59(7H, m), 7.88-8.18(1H, m), 8.01(1H, d, J=8.3Hz).
IR(ATR): 2224, 1589, 1493, 1468, 1377, 1300, 1232, 1176 cm⁻¹.

### [Example 117]

### 1-[(3S)-Dimethylaminopyrrolidin-1-yl]-2-(4-hydroxyphenyl)-3-methylpyrido[1,2-a]benzimidazole-4-carbonitrile (#117):

Tetrahydrofuran/methanol (11 ml) (6/6, v/v) mixed solution of 200 mg (399 µmol) of 2-(9-benzyloxyphenyl)-1-[(3S)-dimethylaminopyrroldin-1-yl]-3-methylpyrido[1,2-a]benzimidazole-4-carbonitrile (I-125) and 40 mg of 10 % palladium-carbon catalyst was stirred under hydrogen atmosphere for 70 hours. The catalyst was removed through filtration, the solvent was evaporated under reduced pressure, and the resulting residue was applied to a silica gel column chromatography. From the eluate with dichloromethane/methanol (10/1, v/v), a crude product of the entitled compound was obtained. This was recrystallized from ethanol to obtain 61 mg (37 %) of the entitled compound as a yellow crystal.
MS(ESI)m/z:412(M+1)⁺.
¹H-NMR(DMSO-d₆)δ: 1.80-2.30 (2H, m), 2.03(6H, s), 2.23(3H, s), 2.70-3.50(5H, m), 6.91(2H, d, J=8.8Hz), 7.08-7.20(2H, m), 7.38(1H, dd, J=7.6, 7.6Hz), 7.50-7.58(1H, m), 7.85(1H, d, J=8.3 Hz), 7.93-8.20(1H, m), 9.70(1H, s).
IR(ATR): 2222, 1591, 1496, 1473, 1442, 1377, 1275, 1236, 1165 cm⁻¹.

| Elemental analysis: C₂₅H₂₅N₅O·0.25H₂O | | | |
|---|---|---|---|
| Calcd. | C, 72.18%; | H, 6.18%; | N, 16.83% |
| Found | C, 72.30%; | H, 6.08%; | N, 16.98%. |

### [Reference Example 126]

### Ethyl 2-(2-methylthiazol-4-yl)acetate (I-126):

Ethanol (400 ml) solution of 5.43 ml (39.9 mmol) of ethyl 4-chloroacetoacetate and 3.00 g (39.9 mmol) of thioacetamide was heated under reflux for 5 hours. Aqueous saturated sodium bicarbonate solution was added thereto, and extracted with ethyl acetate. The combined ethyl acetate layers were dried over anhydrous magnesium sulfate. The solvent was evaporated under reduced pressure, and the resulting residue was applied to a silica gel column chromatography. From the eluate with n-hexane/ethyl acetate (5/1, v/v), 5.08 g (69 %) of the entitled compound was obtained as a yellow oil.
MS (ESI) m/z:186(M+1)⁺.
¹H-NMR(CDCl₃)δ: 1.28(3H, t, J=7.1Hz), 2.70 (3H, s), 3.78(2H, s), 4.19(2H, q, J=7.1Hz), 7.00(1H, d, J=0.7Hz).
IR(ATR): 1732, 1367, 1252, 1178, 1151, 1030 cm⁻¹.

### [Reference Example 127]

### Ethyl 2-(2-methylthiazol-4-yl)acetoacetate (I-127):

According to the production method for (I-118), tetrahydrofuran (80 ml) suspension of 356 mg (8.91 mmol) of sodium hydride, 1.50 g (8.10 mmol) of ethyl 2-(2-methylthiazol-4-yl)acetate (I-126) and 949 µl (9.72 mmol) of ethyl acetate was heated under reflux for 3 hours and processed to obtain 429 mg (23 %) of the entitled compound as a yellow oil.
MS(ESI)m/z:227(M⁺).
¹H-NMR(CDCl₃)δ: 1.21(0.81H, dt, J=6.4, 0.7Hz), 1.29(1.38H, dt, J=6.4, 0.7Hz), 1.37(0.81H, dt, J=6.4, 0.7Hz), 1.93(0.81H, d, J=0.7Hz), 2.27(1.38H, s), 2.39(0.81H, s), 2.69-2.75(3H, m), 4.15-4.34(2H, m), 5.02(0.46H, s), 6.91(0.27H, s), 7.25-7.27(0.46H, m), 7.96(0.27H, s) , 13.25(0.27H, d, J=0.7Hz), 14.60(0.27H, s).
IR(ATR): 1716, 1608, 1369, 1340, 1242, 1196, 1147, 1065 cm⁻¹.

### [Reference Example 128]

### 3-Methyl-2-(2-methylthiazol-4-yl)-1-oxo-1H,5H-pyrido[1,2-a]benzimidazole-4-carbonitrile (I-128):

According to the production method for (I-107), a mixture of 429 mg (1.89 mmol) of ethyl 2-(2-methylthiazol-4-yl)acetoacetate (I-127), 346 mg (2.20 mmol) of (2-benzimidazolyl) acetonitrile and 339 mg (4.40 mmol) of ammonium acetate was heated at 140 to 150°C for 50 minutes and processed to obtain 456 mg (75 %) of the entitled compound as a dark brown crystal.
MS(ESI)m/z:321(M+1)⁺.
¹H-NMR(DMSO-d₆)δ: 2.34(3H, s), 2.69(3H, s), 7.30-7.38(1H, m), 7.45-7.60(3H, m), 8.55(1H, d, J=8.1Hz).
IR(ATR): 2212, 1653, 1604, 1537, 1483, 1464, 1406, 1371, 1279, 1240, 1171, 1136 cm⁻¹.

### [Reference Example 129]

### 1-Chloro-3-methyl-2-(2-methylthiazol-4-yl)pyrido[1,2-a]-benzimidazole-4-carbonitrile (I-129):

According to the production method for (I-108), 807 mg (2.52 mmol) of 3-methyl-2-(2-methylthiazol-4-yl)-1-oxo-1H,5H-pyrido[1,2-a]benzimidazole-4-carbonitrile (I-128) was heated under reflux in 3 ml of phosphoryl chloride for 2 hours and processed to obtain 276 mg (32 %) of the entitled compound as a yellow crystal.
MS (ESI)m/z:339 (M+1)⁺.
¹H-NMR(CDCl₃)δ: 2.49(3H, s), 2.83(3H, s), 7.29(1H, s), 7.40-7.50(1H, m), 7.59-7.70(1H, m), 8.07(1H, d, J=8.3Hz), 8.56(1H, d, J=8.5 Hz).
IR(ATR): 2227, 1620, 1589, 1487, 1454, 1267, 1200 cm⁻¹.

### [Example 118]

### 1-[(3S)-Dimethylaminopyrrolidin-1-yl]-3-methyl-2-(2-methylthiazol-4-yl)pyrido[1,2-a]benzimidazole-4-carbonitrile (#118):

According to the production method for (I-112), N,N-dimethylformaldehyde (6 ml) solution of 210 mg (620 µmol) of 1-chloro-3-methyl-2-(2-methylthiazol-4-yl)pyrido[1,2-a]benzimidazole-4-carbonitrile (I-129), 259 µl (1.86 mmol) of triethylamine and 94 µl (744 µmol) of (3S)-dimethylaminopyrrolidine was heated at 80°C for 7 hours and processed, and then this was recrystallized from ethanol to obtain 149 mg (58 %) of the entitled compound as a yellow crystal.
MS(ESI)m/z:417(M+1)⁺.
¹H-NMR(CDCl₃)δ: 1.70-1.90(1H, m), 2.06-2.30(1H, m), 2.19(6H, s), 2.38(3H, s), 2.83(3H, s), 3.12-3.62(5H, m), 7.15(1H, s), 7.32-7.43(1H, m), 7.50-7.62(1H, m), 7.93-8.15(1H, m) , 8.02(1H, d, J=8.1Hz).
IR(ATR): 2220, 1618, 1587, 1487, 1452, 1439, 1406, 1371, 1340, 1302, 1176, 1147 cm⁻¹.

| Elemental analysis: C₂₃H₂₄N₆S | | | | |
|---|---|---|---|---|
| Calcd. | C, 66.32%; | H, 5.81%; | N, 20.18%; | S, 7.70% |
| Found | C, 66.04%; | H, 5.74%; | N, 19.97%; | S, 7.56%. |

### [Reference Example 130]

### Ethyl 2-(2-ethylthiazol-4-yl)acetate (I-130):

According to the production method for (I-126), ethanol (65 ml) solution of 10.8 g (65.6 mmol) of ethyl 4-chloroacetoacetate and 5.85 g (65.6 mmol) of thiopropionamide was heated under reflux for 5.5 hours and processed to obtain 11.1 g (85 %) of the entitled compound as a pale yellow oil. MS (ESI)m/z:200 (M+1)⁺.
¹H-NMR(CDCl₃)δ: 1.28(3H, dt, J=7.1, 0.5Hz), 1.38(3H, dt, J=7.6, 0.5Hz), 3. 02 (2H, q, J=7.6Hz), 3.79(2H, s), 4.19(2H, q, J=7 .1Hz), 7.03(1H, d, J=0.5 Hz).
IR(ATR): 1734, 1367, 1252, 1151, 1030 cm⁻¹.

### [Reference Example 131]

### Ethyl 2-(2-ethylthiazol-4-yl)acetoacetate (I-131):

According to the production method for (I-118), tetrahydrofuran (80 ml) suspension of 1.92 g (48.1 mmol) of sodium hydride, 8.00 g (40.1 mmol) of ethyl 2-(2-ethylthiazol-4-yl)acetate (I-130) and 5.88 ml (60.2 mmol) of ethyl acetate was heated under reflux for 2 hours and processed to obtain 3.53 g (37 %) of the entitled compound as a pale yellow oil.
MS(ESI)m/z:242(M+1)⁺.
¹H-NMR(CDCl₃)δ: 1.17-1.44(6H, m), 1.94(0.81H, d, J=0.7Hz), 2.26 (1.29H, s), 2.39(0.90H, s), 2.99-3.10(2H, m), 4.15-4.34 (2H, m), 5.03(0.43H, s), 6.92(0.27H, s), 7.28 (0.43H, d, J=0.5Hz), 7.47(0.30H, s), 13.24(0.30H, d, J=0.7Hz), 14.69(0.27H, s).
IR(ATR): 1718, 1697, 1606, 1338, 1242, 1147 cm⁻¹.

### [Reference Example 132]

### 2-(2-Ethylthiazol-4-yl)-3-methyl-1-oxo-1H,5H-pyrido[1,2-a]benzimidazole-4-carbonitrile (I-132):

According to the production method for (I-107), a mixture of 2.00 g (8.29 mmol) of ethyl 2-(2-ethylthiazol-4-yl)acetoacetate (I-131), 1.30 g (8.29 mmol) of (2-benzimidazolyl)acetonitrile and 1.28 g (16.6 mmol) of ammonium acetate was heated at 140 to 150°C for 1.5 hours and processed to obtain 1.99 g (72 %) of the entitled compound as a dark brown crystal.
MS(ESI)m/z:335(M+1)⁺.
¹H-NMR(CDCl₃)δ: 1.34 (3H, t, J=7.6Hz), 2.42 (3H, s), 2.95 (2H, q, J=7.6Hz), 7.12-7.22(1H, m), 7.31-7.48(3H, m), 8.52(1H, d, J=8.3Hz).
IR(ATR): 2204, 1664, 1614, 1550, 1466, 1242, 1192, 1124 cm⁻¹.

### [Reference Example 133]

### 1-Chloro-2-(2-ethylthiazol-4-yl)-3-methylpyrido[1,2-a]benzimidazole-4-carbonitrile (I-133):

According to the production method for (I-108), 1.00 g (2.99 mmol) of 2-(2-ethylthiazol-4-yl)-3-methyl-1-oxo-1H,5H-pyrido[1,2-a]benzimidazole-4-carbonitrile (I-132) was heated under reflux in 30 ml of phosphoryl chloride for 19 hours and processed to obtain 867 mg (82 %) of the entitled compound as a yellow crystal.
MS(ESI)m/z:353(M+1)⁺.
¹H-NMR(CDCl₃)δ: 1.48(3H, dt, J=7.6, 0. 7Hz) , 2.49(3H, d, J=1.8Hz), 3.10-3.19(2H, m), 7.31(1H, s) , 7.39-7.47(1H, m) , 7.59-7.67(1H, m), 8.06(1H, d, J=8.3Hz), 8.52-8.60(1H, m).
IR(ATR) : 2229, 1620, 1593, 1477, 1448, 1421, 1302, 1263, 1201, 1142 cm⁻¹.

### [Example 119]

### 1-[(3S)-Dimethylaminopyrrolidin-1-yl]-2-(2-ethylthiazol-4-yl)-3-methylpyrido[1,2-a]benzimidazole-4-carbonitrile (#119):

According to the production method for (I-112), N,N-dimethylformaldehyde (9 ml) solution of 300 mg (850 µmol) of 1-chloro-2-(2-ethylthiazol-4-yl)-3-methylpyrido[1,2-a]benzimidazole-4-carbonitrile (I-133), 237 µl (1.70 mmol) of triethylamine and 129 µl (1.02 mmol) of (3S)-dimethylaminopyrrolidine was heated at 80°C for 14.5 hours and processed, and then this was recrystallized from ethanol to obtain 236 mg (65 %) of the entitled compound as a yellow crystal.
MS(ESI)m/z:431 (M+1)⁺.
¹H-NMR(CDCl₃)δ: 1.47(3H, t, J=7.6Hz), 1.62-1.95(1H, m), 2.05-2.24(1H, m), 2.18(6H, s), 2.37 (3H, s), 2.60-3.77(5H, m), 3.14(2H, q, J=7.6Hz), 7.18(1H, s), 7.34(1H, dd, J=8.1, 7.3Hz), 7.51-7.59(1H, m), 7.92-8.10(1H, m), 8.00(1H, d, J=8.1Hz).
IR(ATR): 2218, 1620, 1591, 1489, 1460, 1439, 1302, 1146 cm⁻¹.

| Elemental analysis: C₂₄H₂₆N₆S | | | | |
|---|---|---|---|---|
| Calcd. | C, 66.95%; | H, 6.09%; | N, 19.52%; | S, 7.45% |
| Found | C, 66.74%; | H, 6.02%; | N, 19.42%; | S, 7.55%. |

### [Reference Example 134]

### Ethyl 2-(2-phenylthiazol-4-yl)acetate (I-134):

According to the production method for (I-126), ethanol (50 ml) solution of 6.80 ml (50.0 mmol) of ethyl 4-chloroacetoacetate and 6.86 g (50.0 mmol) of thiobenzamide was heated under reflux for 5.5 hours and processed to obtain 10.6 g (86 %) of the entitled compound as a yellow oil.
MS(ESI)m/z:248(M+1)⁺.
¹H-NMR(CDCl₃)δ: 1.29(3H, t, J=7.1Hz), 3.89(2H, d, J=0.7Hz), 4.22 (2H, q, J=7.1Hz), 7.19 (1H, d, J=0.7Hz), 7.39-7.47(3H, m), 7.90-7.98(2H, m).
IR(ATR): 1732, 1246, 1153, 1030 cm⁻¹.

### [Reference Example 135]

### Ethyl 2-(2-phenylthiazol-4-yl)acetoacetate (I-135):

According to the production method for (I-118), tetrahydrofuran (65 ml) suspension of 1.55 g (38.8 mmol) of sodium hydride, 8.00 g (32.3 mmol) of ethyl 2-(2-phenylthiazol-4-yl)acetate (I-134) and 4.74 ml (48.5 mmol) of ethyl acetate was heated under reflux for 3 hours and processed to obtain 5.47 g (59 %) of the entitled compound as a yellow oil.
MS(ESI)m/z:290(M+1)⁺.
¹H-NMR(CDCl₃)δ: 1.23(1.20H, t, J=7.1Hz), 1.31 (0.78H, t, J=7.1Hz), 1.40(1.02H, t, J=7.1Hz), 2.02(1.20H, d, J=0.7Hz), 2.32(0.78H, s), 2.45(1.02H, s) , 4.20-4.39(2H, m), 5.14(0.26H, s), 7.09(0.40H, s), 7.41-7.53(3.26H, m), 7.68(0.34H, s), 7.87-8.02(2H, m), 13.33(0.40H, d, J=0.7Hz), 14.76(0.34H, s).
IR(ATR): 1720, 1697, 1639, 1606, 1338, 1238, 1178, 1136, 1061, 1043 cm⁻¹.

### [Reference Example 136]

### 3-Methyl-1-oxo-2-(2-phenylthiazol-4-yl)-1H,5H-pyrido[1,2-a]benzimidazole-4-carbonitrile (I-136):

According to the production method for (I-107), a mixture of 2.00 g (6.91 mmol) of ethyl 2-(2-phenylthiazol-4-yl)acetoacetate (I-135), 1.09 g (6.91 mmol) of (2-benzimidazolyl)acetonitrile and 1.07 g (13.8 mmol) of ammonium acetate was heated at 140 to 150°C for 50 minutes and processed to obtain 1.97 g (75 %) of the entitled compound as a pale brown crystal.
MS(ESI)m/z:383(M+1)⁺.
¹H-NMR(DMSO-d₆)δ: 2.45(3H, s), 7.36-7.43(1H, m) , 7.45-7.60(5H, m), 7.81(1H, d, J=1.3Hz), 7.95-8.00(2H, m), 8.59(1H, d, J=8.1Hz).
IR(ATR): 2206, 1676, 1614, 1552, 1466, 1240, 1196, 1144 cm⁻¹.

### [Reference Example 137]

### 1-Chloro-3-methyl-2-(2-phenylthiazol-4-yl)pyrido[1,2-a]benzimidazole-4-carbonitrile (I-137):

According to the production method for (I-108), 1.00 g (2.61 mmol) of 3-methyl-1-oxo-2-(2-phenylthiazol-4-yl)-1H,5H-pyrido[1,2-a]benzimidazole-4-carbonitrile (I-136) was heated under reflux in 26 ml of phosphoryl chloride for 18 hours and processed to obtain 898 mg (86 %) of the entitled compound as a yellow crystal.
MS(ESI)m/z:401(M+1)⁺.
¹H-NMR(CDCl₃)δ: 2.56(3H, s), 7.41-7.53(5H, m), 7.61-7.68(1H, m), 7.98-8.05(2H, m), 8.08(1H, d, J=8.3 Hz), 8.58(1H, d, J=8.6Hz).
IR(ATR): 2231, 1624, 1589, 1469, 1446, 1433, 1302, 1198 cm⁻¹.

### [Example 120]

### 1-[(3S)-Dimethylaminopyrrolidin-1-yl]-3-methyl-2-(2-phenylthiazol-4-yl)pyrido[1,2-a]benzimidazole-4-carbonitrile (#120):

According to the production method for (I-112), N,N-dimethylformaldehyde (8 ml) solution of 300 mg (748 µmol) of 1-chloro-3-methyl-2-(2-phenylthiazol-4-yl)pyrido[1,2-a]benzimidazole-4-carbonitrile (I-137), 209 µl (1.50 mmol) of triethylamine and 114 µl (898 µmol) of (3S)-dimethylaminopyrrolidine was heated at 80°C for 5 hours and processed, and then this was recrystallized from ethanol to obtain 226 mg (63 %) of the entitled compound as a yellow crystal.
MS(ESI)m/z:479(M+1)⁺.
¹H-NMR(CDCl₃)δ: 1.75-2.22 (2H, m), 2.12(6H, s), 2.45(3H, s), 2.63-3.78(5H, m), 7.30-7.41(2H, m), 7.44-7.61(4H, m), 7.89-8.17(4H, m).
IR(ATR): 2225, 1587, 1460, 1442, 1300 cm⁻¹.

| Elemental analysis: C₂₈H₂₆N₆S·0.5H₂O | | | | |
|---|---|---|---|---|
| Calcd. | C, 68.97%, | H, 5.58%, | N, 17.23%; | S, 6.58% |
| Found | C, 68.85%, | H, 5.45%, | N, 17.29%, | S, 6.69%. |

### [Reference Example 138]

### 3-Oxocyclopent-1-enecarbonitrile (I-138):

10.1 g (80.06 mmol) of 3-ethoxy-2-cyclopenten-1-one was dissolved in dichloromethane (100 ml), and 2.56 g (8.06 mmol) of zinc iodide and 22.3 ml (160.11 mmol) of trimethylsilylcyanide were added thereto at 0°C. At the temperature, this was stirred for 15 minutes, and then warmed up to room temperature, andfurther stirred for 29 hours. 1 M hydrochloric acid (50 ml) was added thereto at 0°C, and stirred at room temperature for 1 hour. The solution was extracted with chloroform. The organic layer was washed with aqueous saturated sodium hydrogencarbonate solution and then brine, and dried over anhydrous sodium sulfate. The solvent was evaporated, and the resulting residue was applied to a silica gel column chromatography. From the eluate with hexane/ethyl acetate =2/1, 5.363g (63%) of the entitled compound was obtained as a reddish brown oil.
¹H-NMR(CDCl₃)δ: 2.52-2.57 (2H, m) , 2.92(1H, dt, J=2.2, 7.1Hz), 6.76(1H, t, J=2.2Hz).

### [Reference Example 139]

### 3-Hydroxycyclopent-1-enecarbonitrile (I-139):

6.71 g (62.65 mmol) of 3-oxocyclopent-1-enecarbonitrile (I-138) was dissolved in methanol (268 ml), and 25.67 g (68. 91 mmol) of cerium chloride 7-hydrate was added thereto at room temperature. At the temperature, this was stirred for 5 minutes, and then cooled to 0°C. 2.37 g (62.65 mmol) of sodium borohydride was gradually added to the solution, and stirred at the temperature for 2 hours. At 0°C, aqueous saturated ammonium chloride solution was added to the reaction mixture. Methanol was evaporated under reduced pressure. The resulting insoluble material was separated through filtration, and this was extracted with ethyl acetate. The organic layer was washed with brine, and dried over anhydrous sodium sulfate. The solvent was evaporated, and the resulting residue was applied to a silica gel column chromatography. From the eluate with hexane/ethyl acetate=1/1, 6.65 g (97%) of the entitled compound was obtained as a colorless oil.
MS(EI)m/z:109(M⁺).
HRMS(EI)m/z:109.0522(Calcd for C₆H₇NO 109.0527).
¹H-NMR(CDCl₃)δ: 1.77-1.85(1H, m), 2.31-2.44(1H, m), 2.50-2.59(1H, m), 2.70-2.79(1H, m), 3.08(1H, d, J=6.1Hz), 4.92-5.00(1H, m), 6.63(1H, q, J=2.2Hz).
IR(ATR): 3400, 2224, 1327, 1140, 1039, 966, 879 cm⁻¹.

### [Reference Example 140]

### 3-(tert-Butyldiphenylsilanyloxy)-cyclpent-1-enecarbonitrile (I-140):

900 mg (8.5 mmol) of 3-hydroxycyclopent-1-enecarbonitrile (I-139) was dissolved in N,N-dimethylformamide (40 ml) , and 730 mg (10.72 mmol) of imidazole and 2.57 ml (9.90 mmol) of tert-butylchlorodiphenylsilane were added thereto under nitrogen atmosphere at room temperature. This was stirred for 17 hours, and then aqueous saturated ammonium chloride solution was added to the reaction mixture. This was extracted with ethyl acetate. The organic layer was washed with brine, and dried over anhydrous sodium sulfate. The solvent was evaporated, and the resulting residue was applied to a silica gel column chromatography. From the eluate with hexane/ethyl acetate= 98/2, 2.597 g (91%) of the entitled compound was obtained as a colorless oil.
MS(EI)m/z:290(M⁺).
HRMS (EI)m/z:290.1012 (Calcd for C₁₈H₁₆NOSi 290.1001).
¹H-NMR(CDCl₃)δ: 1.05(9H, s), 1.87-1.96(1H, m), 2.12-2.22(1H, m), 2.34-2.43(1H, m), 2.62-2.71(1H, m), 4.90-4.95(1H, m), 6.37(1H, q, J=2.2Hz), 7.38-7.48(6H, m), 7.62-7.68(4H, m).
IR(ATR): 2224, 1427, 1111, 822, 741, 702 cm⁻¹.

### [Reference Example 141]

### 3-(tert-Butyldiphenylsilyloxy)cyclopent-1-enecarbaldehyde (I-141):

2.02 g (5.81 mmol) of 3-(tert-butyldiphenylsilanyloxy)-cyclpent-1-enecarbonitrile (I-140) was dissolved in anhydrous tetrahydrofuran, and 9.4 ml (8.72 mmol) of aluminium diisobutylhydride (0.93 M hexane solution) was gradually added thereto under nitrogen atmosphere at -78°C. At the temperature, this was stirred for 30 minutes, and then for 6 hours at room temperature. Aqueous saturated ammonium chloride solution (2 ml) was added to the reaction mixture at 0°C, and stirred at room temperature for 1 hour. This suspension was diluted with tetrahydrofuran, and the insoluble material was separated through filtration. The filtrate was concentrated under reduced pressure. The resulting residue was separated with chloroform and aqueous saturated ammonium chloride solution, and the organic layer was washed with brine and dried over anhydrous sodium sulfate. The solvent was evaporated, and the resulting residue was applied to a silica gel column chromatography. From the eluate with hexane/ethyl acetate = 95/5, 1.566 g (77 %) of the entitled compound was obtained as a colorless gel.
¹H-NMR(CDCl₃)δ: 1.07(9H, s), 1.88-1.98(1H, m), 2.18-2.30(2H, m), 2.59-2.68(1H, m), 5.00-5.06(1H, m), 6.53-6.57(1H, m), 7.38-7.48(6H, m), 7.67-7.71(4H, m), 9.75(1H, s).
IR(ATR): 1685, 1427, 1111, 1063, 824, 741, 702 cm⁻¹.

### [Reference Example 142]

### 3-(tert-Butyldiphenylsilyloxy)cyclopent-1-enecarboxylic acid methoxymethylamide (I-142):

273 mg (0.78 mmol) of 3-(tert-butyldiphenylsilyloxy)-cyclopent-1-enecarbaldehyde (I-141) was dissolved in tert-butanol (8 ml) and water (3 ml), and 1.56 ml (3.12 mmol) of 2-methyl-2-butene and 182 mg (1.17 mmol) of sodium dihydrogenphosphate were added thereto at room temperature. After stirring for 5 minutes, 268mg (2.34 mmol) of sodiumchlorite was added thereto. At the temperature, this was stirred for 2.5 hours, and aqueous 1 M hydrochloric acid was added thereto so as to make it have a pH of 4. This solution was extracted with ethyl acetate, and the organic layer was washed with brine, and dried over anhydrous sodium sulfate. The solvent was evaporated to obtain 3- (tert-butyldiphenylsilyloxy) cyclopent-1-enecarboxylic acid as a colorless oil. This compound was dissolved in dichloromethane (8 ml) and 435 µl (3.12 mmol) of triethylamine, 99 mg (1.01 mmol) of N,O-dimethylhydroxylamine hydrochloride, 137 mg (1.01 mmol) of 1-hydroxybenzotriazole and 99 mg (1.01 mmol) of 1-ethyl-3-(3-dimethylaminopropyl)-carbodiimide hydrochloride were added thereto in nitrogen atmosphere at room temperature. At the temperature, this was stirred for 12 hours, and aqueous saturated ammonium chloride was added thereto. This solution was extracted with dichloromethane, and the organic layer was washed with brine, and dried over anhydrous sodium sulfate. The solvent was evaporated, and the resulting residue was applied to a silica gel column chromatography. From the eluate with hexane/ethyl acetate = 4/1, 224 mg (70 %) of the entitled compound was obtained as a colorless gel.
MS(FAB)m/z:410(M+1)⁺.
HRMS (FAB)m/z:410.2447 (Calcd for C₂₄H₃₂NO₃Si 410.2152).
¹H-NMR(CDCl₃)δ: 1.06-1.91(1H, m), 2.08-2.19(1H, m), 2.37-2.50(1H, m), 2.70-2.80(1H, m), 4.92-4.99(1H, m), 6.19-6.22(1H, m), 7.33-7.45(6H, m), 7.64-7.70(4H, m).
IR(ATR): 1645, 1610, 1427, 1105, 1072, 1041, 894, 740, 700 cm⁻¹.

### [Reference Example 143]

### 1-[3-(tert-Butyldiphenylsilyloxy)cyclopent-1-enyl]-2-phenylethanone (I-143):

1.55 g (3.78 mmol) of 3-(tert-butyldiphenylsilyloxy)-cyclopent-1-enecarboxylic acid methoxymethylamide (I-142) was dissolved in anhydrous tetrahydrofuran (15 ml), and 2.08 ml (4.16 mmol) of magnesium benzyl chloride was gradually added thereto under nitrogen atmosphere at 0°C. At the temperature, this was stirred for 6 hours, and aqueous saturated ammonium chloride was added thereto at 0°C. Under reduced pressure, the organic layer was evaporated, and the residue was extracted with ethyl acetate. The organic layer was washed with brine, and dried over anhydrous sodium sulfate. The solvent was evaporated, and the resulting residue was applied to a silica gel column chromatography. From the eluate with hexane/ethyl acetate = 96/4, 778 mg (47 %) of the entitled compound was obtained as a colorless gel.
MS(EI)m/z:383(M-57)⁺.
HRMS(EI)m/z:383.1474(Calcd for C₂₅H₂₃O₂Si 383.1468).
¹H-NMR(CDCl₃)δ: 1.06(9H, s), 1.80-1.91(1H, m), 2.10-2.20(1H, m), 2.20-2.32(1H, m), 2.58-2.60(1H, m), 3.86(2H, s), 4.97-5.20(1H, m), 6.41-6.47(1H, m), 7.12-7.14(2H, m), 7.20-7.30(8H, m), 7.35-7.50(6H, m), 7.66-7.72(4H, m).
IR(ATR): 1668, 1427, 1105, 1068, 824, 741, 700 cm⁻¹.

### [Reference Example 144]

### 1-[3-(tert-Butyldiphenylsilyloxy)cyclopentyl]-2-phenylethanone (I-144):

4.455 g (10.11 mmol) of 1-[3-(tert-butyldiphenylsilyloxy)cyclopent-1-enyl]-2-phenylethanone (I-143) was dissolved in ethyl acetate (45 ml), and 668 mg (15 wt.%) of 10 % palladium-carbon was added thereto at room temperature. Under atmospheric pressure of hydrogen at room temperature, this was stirred for 1.5 hours. The catalyst was removed through filtration, and the solvent was evaporated under reduced pressure to obtain the entitled compound as a colorless oil. The residue was used in the next reaction without further purification.
MS(FAB)m/z:443(M+1)⁺.
HRMS(FAB)m/z:443.2401(Calcd for C₂₉H₃₅O₂Si 443.2406).
¹H-NMR(CDCl₃)δ: 1.03(9H, s), 1.55-1.80(3H, m), 1.83-2.00(2H, m), 2.00-2.12(1H, m), 2.79(1H, dq, J=5.4, 5.4Hz), 3.71(2H, s), 4.21(1H, dq, J=5.4, 5.4Hz), 7.15-7.50(11H, m), 7.60-7.70(4H, m).
IR(ATR): 1709, 1427, 1109, 702 cm⁻¹.

### [Reference Example 145]

### 1-(3-Hydroxycyclopentyl)-2-phenylethanone (I-145):

1-[3-(tert-Butyldiphenylsilyloxy)cyclopentyl]-2-phenylethanone (I-144) obtained in the above was dissolved in tetrahydrofuran (50 ml), and 15.2 ml (15.17 mmol) of tetrabutylammonium fluoride (1 M tetrahydrofuran solution) was added thereto at 0°C. At the temperature, this was stirred for 2 hours, and then stirred for 13 hours at room temperature. Aqueous saturated ammonium chloride solution was added to this solution, and the organic solvent was evaporated under reduced pressure. The resulting residue was extracted with ethyl acetate. The organic layer was washed with brine, and dried over anhydrous sodium sulfate. The solvent was evaporated, and the resulting residue was applied to a silica gel column chromatography. From the eluate with hexane/ethyl acetate = 3/2, 1.528 g (yield in the two steps, 74%) of the entitled compound was obtained as a pale yellow oil.
MS(EI)m/z:204(M⁺).
HRMS(FAB)m/z:204.1122(Calcd for C₁₃H₁₆O₂ 204.1150).
¹H-NMR(CDCl₃)δ: 1.58-2.06(6H, m), 2.12(1H, br), 3.15(0.2H, dq, J=2.4, 7.8Hz), 3.26-3.34(0.8H, m), 3.74(1.6H, s), 3.80(0.4H, d, J=7.8Hz), 4.25-4.29(0.2H, m), 4.37-4.41(0.8H, m), 7.18-7.35(5H, m).
IR(ATR): 3415, 1701, 1495, 1454, 1072, 1030, 987 cm⁻¹.

### [Reference Example 146]

### 1-(3-Methoxymethoxycyclopentyl)-2-phenylethanone (I-146):

1.52 g (7.44 mmol) of 1-(3-hydroxycyclopentyl)-2-phenylethanone (I-145) was dissolved in dichloromethane (30 ml) and 6.48 ml (37.21 mmol) of diisopropylethylamine, 182 mg (1.49 mmol) of 4-dimethylaminopyridine and 2.26 ml (29.77 mmol) of chloromethyl methyl ether were added thereto under nitrogen atmosphere at 0°C. After stirring at room temperature for 15 hours, this solution was separated with dichloromethane and 0.5 M aqueous hydrochloric acid solution. The organic layer was washed with brine, and dried over anhydrous sodium sulfate. The solvent was evaporated, and the resulting residue was applied to a silica gel column chromatography. From the eluate with hexane/ethyl acetate=7/1, 1.698 g (92%) of the enti tled compound was obtained as a colorless oil.
MS(EI)m/z:248(M⁺).
HRMS(EI)m/z:248.1427(Calcd for C₁₅H₂₀O₃ 248.1412).
¹H-NMR(CDCl₃)δ: 1.71-2.11(6H, m), 2.94(0.5H, dq, J=8.1, 8.5Hz), 3.19-3.29(0.5H, m), 3.33(1.5H, s), 3.34(1.5H, s), 3.75(2H, d, J=1.7Hz), 4.11(0.5H, dq, J=5.6, 5.6Hz), 4.19-4.23(0.5H, m), 4.59(1H, s), 4.61(1H, d, J=2.7Hz), 7.19-7.34(5H, m).
IR(ATR): 1709, 1146, 1095, 1038, 916, 700 cm⁻¹.

### [Reference Example 147]

### 1-(3-Methoxymethoxycyclopentyl)-2-phenylbutane-1,3-dione (I-147):

448 mg (10.26 mmol) of sodium hydride (55 % mineral oil suspension) was dissolved in dimethoxyethane (5 ml), and a solution prepared by dissolving 15-crown-5 (2 drops) in dimethoxyethane (1 ml) was added thereto under nitrogen atmosphere. Next, this was cooled to 0°C, anda solution prepared by dissolving 1.698 g (6.84 mmol) of 1-(3-methoxymethoxycyclopentyl)-2-phenylethanone (I-146) in dimethoxyethane (28 ml) was added dropwise thereto. At the temperature, this was stirred for 15 minutes, and then 1.34 ml (13.68 mmol) of ethyl acetate was added thereto. At room temperature, this was stirred for 15 minutes, and then heated under reflux for 10 hours. After cooling to room temperature, this was further stirred for 12 hours. The reaction mixture was poured into aqueous saturated ammonium chloride solution, and the organic layer was evaporated under reduced pressure. The residue was extracted with ethyl acetate. The organic layer was washed with brine, and dried over anhydrous sodium sulfate. The solvent was evaporated, and the resulting residue was applied to a silica gel column chromatography. From the eluate with hexane/ethyl acetate=19/1, 516 mg (26%) of the entitled compound was obtained as a colorless oil.
MS(EI)m/z:290(M⁺).
HRMS(EI)m/z:290.1516(Calcd for C₁₇H₂₂O₄ 290.1518).
¹H-NMR(CDCl₃)δ: 1.50-2.02(6H, m), 1.86(1.5H, s), 1.87(1.5H, s), 2.58(0.5H, dq, J=8.6, 8.6Hz), 2.86(0.5H, dq, J=7.8, 8.3Hz), 3.24(1.5H, s), 3.34(1.5H, s), 3.98(0.5H, dq, J=5.9, 5.9), 4.20-4.24(0.5H, m), 4.51(1H, dd, J=6.9, 9.3Hz), 4.61(1H, s), 7.15-7.51(5H, m).
IR(ATR): 1732, 1599, 1147, 1097, 1039, 704 cm⁻¹.

### [Reference Example 148]

### 1-(3-Methoxymethoxycyclopentyl)-3-methyl-2-phenylbenzo[4,5]imidazo[1,2-a]pyridine-4-carbonitrile (I-148):

260 mg (0.895 mmol) of 1-(3-methoxymethoxycyclopentyl)-2-phenylbutane-1,3-dione (I-147), 174 mg (0.94 mmol) of (2-benzylimidazole)acetonitrile and 138 mg (1.79 mmol) of ammonium acetate were heated under nitrogen atmosphere at 140°C for 4 hours. After cooling to room temperature, the residue was fractionated with 10 % methanol-containing chloroform and brine. The organic layer was washed with brine, and dried over anhydrous sodium sulfate. The solvent was evaporated, and the resulting residue was applied to a silica gel column chromatography. From the eluate with chloroform, 230 mg (62 %) of the entitled compound was obtained as a brown solid.
MS(EI)m/z:411(M⁺).
HRMS(EI)m/z:411.1948(Calcd for C₂₆H₂₅O₂N₃ 411.1947).
¹H-NMR(CDCl₃)δ: 1.55-2.30(6H, m), 2.23(1.5H, s), 2.24 (1.5H, s), 3.30(1.5H, s), 3.33(1.5H, s), 3.80-3.90(0.5H, m), 4.04-4.13(0.5H, m), 4.14-4.25 (0.5H, m), 4.52-4.62(0.5H, m), 4.60 (2H, s), 7.17-7.58(7H, m), 8.06(2H, d, J=8.3Hz).
IR(ATR) : 2220, 1481, 1144, 1097, 1038, 916, 762, 735, 710 cm⁻¹.

### [Reference Example 149]

### 1-(3-Hydroxycyclopentyl)-3-methyl-2-phenylbenzo[4,5]imidazo[1,2-a]pyridine-4-carbonitrile (I-149):

1-(3-Methoxymethoxycyclopentyl)-3-methyl-2-phenylbenzo[4,5]imidazo[1,2-a]pyridine-4-carbonitrile (I-148) was dissolved in tetrahydrofuran (2 ml), and 6 N hydrochloric acid (2 ml) was added thereto at room temperature. After stirred at 65°C for 6 hours, this was cooled to room temperature. The reaction mixture was poured into 1 N aqueous sodium hydroxide solution, and extracted with chloroform. The organic layer was washed with brine, and dried over anhydrous sodium sulfate. The solvent was evaporated, and the resulting residue was purified through preparative thin-layer chromatography (eluent: chloroform/methanol = 95/5) to obtain 158 mg (including impurity) of the entitled compound as a yellow solid.
MS(ESI)m/z:368(M+1)⁺.
¹H-NMR(CDCl₃)δ: 1.55-2.15 (6H, m), 2.22(1.5H, s), 2.24(1.5H, s), 2.27-2.40(1H, m), 3.84-4.02(0.5H, m), 4.30-4.44(1H, m), 4.50-4.61(0.5H, m), 7.20-7.58(7H, m), 8.02-8.15(2H, m).
IR(ATR): 3380, 2229, 1483, 1444, 1371, 1302, 1232, 761, 737, 710 cm⁻¹.

### [Reference Example 150]

### 3-Methyl-1-(3-oxocyclopentyl)-2-phenylbenzo[4,5]imidazo[1,2-a]pyridine-4-carbonitrile (I-150):

65 µl (0.74 mmol) of oxalyl chloride was dissolved in dichloromethane (1 ml), and a solution of 66 µl (0.93 mmol) of dimethylsulfoxide in dichloromethane (1 ml) was added thereto under nitrogen atmosphere at -78°C. At the temperature, this was stirred for 15 minutes, and a solution of 1-(3-hydroxycyclopentyl)-3-methyl-2-phenylbenzo[4,5]imidazo[1,2-a]pyridine-4-carbonitrile (I-149) (158 mg, including impurity) in dichloromethane (3 ml) was added thereto. This was further stirred for 1.5 hours, and 258 µl (1.85 mmol) of triethylamine was added thereto. Next, this was stirred at 0°C for 1 hour, and aqueous saturated ammonium chloride solution was added thereto. This solution was extracted with dichloromethane, and the organic layer was washed with brine and dried over anhydrous sodium sulfate. The solvent was evaporated, and the resulting residue was purified through preparative thin-layer chromatography (eluent: chloroform/acetone=9/1) to obtain 82.5 mg (yield in the two steps, 61 %) of the entitled compound as a yellow powder.
MS(EI)m/z:365(M⁺).
HRMS(EI)m/z:365.1548(Calcd for C₂₄H₁₉ON₃ 365.1529).
¹H-NMR(CDCl₃)δ: 2.10-2.65(6H, m), 2.20 (3H, s), 4.37(1H, br s), 7.28-7.33(3H, m), 7.50-7.65(5H, m), 8.03(1H, d, J=8.3Hz).
IR(ATR): 2222, 1741, 1481, 1446, 1302, 1230, 758, 735, 706cm⁻¹.

### [Example 121]

### 1-(3-Dimethylaminocyclopentyl)-3-methyl-2-phenylbenzo[4,5]imidazo[1,2-a]pyridine-4-carbonitrile (#121):

64 mg (0.175 mmol) of 3-methyl-1-(3-oxocyclopentyl)-2-phenylbenzo[4,5]imidazo[1,2-a]pyridine-4-carbonitrile (I-150) was dissolved in methanol (3 ml)/chloroform (3 ml) , and 52 µl (0.876 mmol) of acetic acid and 438 µl (0.876 mmol) of dimethylamine (2.0 M methanol solution) were added thereto at room temperature. At the temperature, this was stirred for 5 minutes, and 33 mg (0.525 mmol) of sodium cyanoborohydride was added thereto. At room temperature, this was stirred for 4 hours, and then aqueous saturated sodium hydrogencarbonate solution was added to this reaction mixture and extracted with chloroform. The organic layer was washed with brine, and dried over anhydrous sodium sulfate. The solvent was evaporated, and the resulting residue was purified through preparative thin-layer chromatography (eluent: chloroform/7N-ammonia-methanol solution = 96/4) to obtain 65 mg (94 %) of the entitled compound as a pale yellow powder.
MS (EI)m/z:394 (M⁺).
HRMS (EI)m/z:394.2131 (Calcd for C₂₆H₂₆N₄ 394.2157).
¹H-NMR(CDCl₃)δ: 1.88-2.30(6H, m), 2.12(6H, s), 2.21 (3H, s), 2.57-2.69(1H, m), 3.92-4.10(1H, m), 7.22-7.30(3H, m), 7.47-7.59(5H, m), 8.06(1H, dd, J=1.0, 8.5Hz).
IR(ATR): 2224, 1481, 1446, 1296, 1232, 762, 737, 706 cm⁻¹.

| Elemental analysis: C₂₆H₂₆N₄ | | | |
|---|---|---|---|
| Calcd. | C, 79.16%; | H, 6.64%; | N, 14.20% |
| Found | C, 78.71%; | H, 6.65%; | N, 14.15%. |

### [Example 122]

### 1-[(3-Dimethylamino)-1-azetidinyl]-3-methyl-2-phenylpyrido[1,2-a]benzimidazole-4-carbonitrile (#122):

200 mg (0.63 mmol) of 1-chloro-3-methyl-2-phenylpyrido[1,2-a]benzimidazole-4-carbonitrile (I-8) was suspended in dimethylsulfoxide (4 ml), and 0.37 ml (2.64 mmol) of triethylamine and 142 mg (0.82 mmol) of N,N-dimethyl-3-azetidinamine dihydrochloride were added thereto, and heated with stirring at 90°C for 18 hours. After restored to room temperature, water was added to the reaction mixture, and the resulting precipitate was collected by filtration and washed with n-hexane. The crude crystal was recrystallized from a mixed solvent of chloroform/ethyl acetate/n-hexane to obtain 131 mg (53 %) of the entitled compound as a yellow solid.
MS(ESI)m/z:382(M+1)⁺.
¹H-NMR(DMSO-d₆)δ: 1.91(6H, s), 2.22(3H, s), 2.98-3.01(1H, m), 3.35-3.39(2H, m), 3.72(2H, t, J=8.1Hz), 7.37(1H, dt, J=1.0, 7.3Hz), 7.46-7.51(2H, m), 7.53-7.55(4H, m), 7.82(1H, d, J=7.8Hz), 8.17(1H, d, J=8.3Hz).
IR(ATR): 2214, 1583, 1442 cm⁻¹.

| Elemental analysis: C₂₄H₂₃N₅·0.5H₂O | | | |
|---|---|---|---|
| Calcd. | C, 73.82%; | H, 6.19%; | N, 17.93% |
| Found | C, 73.76%; | H, 5.94%; | N, 17.79%. |

### [Example 123]

### 3-Methyl-2-phenyl-1-(1-piperazinyl)pyrido[1,2-a]benzimidazole-4-carbonitrile (#123):

200 mg (0.63 mmol) of 1-chloro-3-methyl-2-phenylpyrido[1,2-a]benzimidazole-4-carbonitrile (I-8) was suspended in dimethylsulfoxide (4 ml), and 0.19 ml (1.32 mmol) of triethylamine and 70 mg (0.82 mmol) of piperazine were added thereto, and heated with stirring at 80°C for 20 hours. After restored to room temperature, water was added to the reaction mixture, and the resulting precipitate was collected by filtration and washed with n-hexane. The crude crystal was recrystallized from a mixed solvent of chloroform/ethyl acetate/n-hexane to obtain 156 mg (64 %) of the entitled compound as a yellow solid.
MS(ESI)m/z:368(M+1)⁺.
¹H-NMR(DMSO-d₆)δ: 2.16(3H, s), 2.39-2.44(2H, m), 2.64(2H, d, J=12.0Hz), 2.96-3.06(4H, m), 7.38-7.43(3H, m), 7.52-7.58(4H, m), 7.88(1H, d, J=8.1Hz), 8.76(1H, d, J=8.5Hz).
IR(ATR): 2224, 1475, 1442, 1302 cm⁻¹.

| Elemental analysis: C₂₃H₂₁N₅ · 1H₂O | | | |
|---|---|---|---|
| Calcd. | C, 71.67%; | H, 6.01%; | N, 18.17% |
| Found | C, 71.42%; | H, 5.69%; | N, 17.81%. |

### [Example 124]

### 3-Methyl-1-[(3S)-3-methylpiperazinyl]-2-phenylpyrido[1,2-a]benzimidazole-4-carbonitrile (#124):

200 mg (0.63 mmol) of 1-chloro-3-methyl-2-phenylpyrido[1,2-a]benzimidazole-4-carbonitrile (I-8) was suspended in dimethylsulfoxide (4 ml), and 0.19 ml (1.32 mmol) of triethylamine and 82 mg (0.82 mmol) of (S) - (+) -2-methylpiperazine were added thereto, and heated with stirring at 90°C for 5 hours. After restored to room temperature, water was added to the reaction mixture, and the resulting precipitate was collected by filtration and washed with n-hexane. The thus-obtained crude crystal was recrystallized from a mixed solvent of chloroform/ethyl acetate/n-hexane to obtain 141 mg (58 %) of the entitled compound as a yellow solid.
MS(ESI)m/z:382(M+1)⁺.
¹H-NMR(DMSO-d₆)δ: 0.76(3H, d, J=6.1Hz), 1.89(1H, t, J=10.5Hz), 2.17(3H, s) , 2.30(1H, dt, J=2.7, 11.2Hz), 2.70(1H, d, J=12.4Hz), 2.98-3.11(4H, m), 7.37-7.42(3H, m), 7.52-7.57(4H, m), 7.88(1H, d, J=8.0Hz), 8.80(1H, d, J=8.5Hz).
IR(ATR): 2833, 2220, 1481, 1442 cm⁻¹.

| Elemental analysis: C₂₄H₂₃N₅·0.25H₂O | | | |
|---|---|---|---|
| Calcd. | C, 74.68%; | H, 6.14%; | N, 18.14% |
| Found | C, 74.43%; | H, 5.95%; | N, 18.00%. |

### [Example 125]

### 2-Butyl-2-[4-(dimethylamino)phenyl]-3-methylpyrido[1,2-a]benzimidazole-4-carbonitrile (#125):

Under nitrogen atmosphere, 150 mg (0.50 mmol) of 2-butyl-1-chloro-3-methylpyrido[1,2-a]-benzimidazole-4-carbonitrile (I-4) and 83 mg (0.50 mmol) of 4-(dimethylamino)phenylboric acid were dissolved in a mixture of 1,2-dimethoxymethane (3 ml) and water (0.8 ml), and 107 mg (1.01 mmol) sodium carbonate and tetrakis(triphenylphosphine)palladium (3 mg) were added thereto, and heated under reflux for 19 hours. After restoring to room temperature, the reaction mixture was concentrated, and diluted with ethyl acetate, and the insoluble material was removed through filtration. The filtrate was washed with water and brine, and the resulting organic phase was dried over anhydrous sodium sulfate, and the solvent was evaporated under reduced pressure. The resulting residue was applied to a silica gel column chromatography, and eluted with a mixed solvent of n-hexane/ethyl acetate (4/1, v/v). Thus obtained crude crystal was recrystallized and purified from a mixed solvent of chloroform/ethyl acetate/n-hexane to obtain 100 mg (51 %) of the entitled compound as a yellow solid. MS(ESI)m/z:383(M+1)⁺.
¹H-NMR(CDCl₃)δ: 0.82(3H, t, J=7.1Hz), 1.24-1.31(2H, m), 1.36-1.42(2H, m), 2.47(2H, m) , 2.75(3H, m), 3.13(6H, s), 6,17(1H, d, J=8.5Hz), 6.89-6.97(3H, m), 7.19(2H, d, J=8.8Hz), 7.39(1H, dd, J=7.1, 8.3Hz), 7.94(1H, d, J=8.3Hz).
IR(ATR): 2229, 1608, 1490, 1196, 741 cm⁻¹.

| Elemental analysis: C₂₅H₂₆N₄·0.25H₂O | | | |
|---|---|---|---|
| Calcd. | C, 77.59%; | H, 6.90%; | N, 14.48% |
| Found | C, 77.30%; | H, 6.75%; | N, 14.53%. |

### [Example 126]

### 2-Butyl-3-methyl-1-(3-pyridinyl)pyrido[1,2-a]benzimidazole-4-carbonitrile (#126):

Under nitrogen atmosphere, 150 mg (0.50 mmol) of 2-butyl-1-chloro-3-methylpyrido[1,2-a]benzimidazole-4-carbonitrile (I-4) and 62 mg (0.50 mmol) of pyridine-3-boronic acid were dissolved in a mixture of 1,2-dimethoxymethane (3 ml) and water (0.8 ml), and 107 mg (1.01 mmol) sodium carbonate and tetrakis(triphenylphosphine)palladium (3 mg) were added thereto, and heated under reflux in an oil bath at 85°C for 16 hours. Toluene (4 ml) was added thereto, and further heated under reflux in an oil bath at 110°C for 7 hours. After restoring to room temperature, the reaction mixture was concentrated, and diluted with ethyl acetate, and the insoluble material was removed through filtration. The filtrate was washed with water and brine, and the resulting organic phase was dried over anhydrous sodium sulfate, and the solvent was evaporated under reduced pressure. The resulting residue was applied to a silica gel column chromatography, and eluted with a mixed solvent of chloroform/methanol (50/1, v/v). Thus obtained, the crude crystal was recrystallized and purified from a mixed solvent of ethyl acetate/n-hexane to obtain 36 mg (21 %) of the entitled compound as a pale yellow solid.
MS(ESI)m/z:341(M+1)⁺.
¹H-NMR(CDCl₃)δ: 0.80(3H, t, J=7.3Hz), 1.22-1.2,9(2H, m), 1.38-1.63(2H, m) , 2.41(2H, m), 2.77(3H, s), 5.92(1H, dd, J=0.7, 8.6Hz), 6.95(1H, t, J=8.3Hz), 7.42(1H, dt, J=1.0, 8.3Hz), 7.66-7.69(1H, m) , 7.82-7.85(1H, m), 7.96(1H, dd, J=0.7, 8.3Hz) , 8.75(1H, s), 9.01(1H, dd, J=1.7, 4.9Hz).
IR(ATR): 2227, 1446, 1024, 735 cm⁻¹.

| Elemental analysis: C₂₂H₂₀N₄·0.25H₂O | | | |
|---|---|---|---|
| Calcd. | C, 76.61%; | H, 5.99%; | N, 16.24% |
| Found | C, 76.40%; | H, 5.72%; | N, 16.35%. |

### [Reference Example 151]

### 1,2-Dichloro-3-methylpyrido[1,2-a]benzimidazole-4-carbonitrile (I-151):

1.20 g (6.49 mmol) of (2-benzimidazolyl)acetonitrile, 898 µl (6.49 mmol) of ethyl 2-chloroacetoacetate and 442 mg (6.49 mmol) of sodium ethoxide were suspended in ethanol (22 ml), stirred at room temperature for 1 hour and then heated under reflux for 24 hours. The reaction mixture was left cooled, the solvent was evaporated under reduced pressure, and the resulting residue was applied to a silica gel column chromatography. This was eluted with a mixed solvent of n-hexane/ethyl acetate (2/1, v/v) to obtain 54 mg of a crude pyridone compound as a pale greenish gray solid. The resulting crude pyridone compound was dissolved in phosphoryl chloride (2 ml) and heated under reflux for 24 hours. The reaction mixture was restored to room temperature, the solvent was evaporated under reduced pressure, and the residue was diluted with chloroform, neutralized with aqueous saturated sodium hydrogencarbonate solution, and washed with brine. Thus obtained, the organic phase was dried over anhydrous sodiumsulfate, the solvent was evaporated under reduced pressure, and the resulting residue was applied to a silica gel column chromatography, and eluted with amixed solvent of n-hexane/ethyl acetate (3/1, v/v). This was recrystallized and purified from a mixed solvent of chloroform/n-hexane/ethyl acetate to obtain 29 mg (54 %) of the entitled compound as a yellow solid.
MS(EI)m/z:276(M⁺).
¹H-NMR(CDCl₃)δ: 2.83(3H, s), 7.47(1H, dt, J=1.2, 8.5Hz), 7.65 (1H, t, J=8.3Hz ), 8.07(1H, d, J=8.3Hz), 8.59(1H, d, J=8.8Hz).

### [Example 127]

### 2-Chloro-1-[(3S)-3-(dimethylamino)pyrrolidinyl]-3-methylpyrido[1,2-a]benzimidazole-4-carbonitrile (#127):

29 mg (0.11 mmol) of 1,2-dichloro-3-methylpyrido[1,2-a]benzimidazole-4-carbonitrile (I-151) was suspended in dimethylsulfoxide (180 ml) , and 31 µl (0.22 mmol) of triethylamine and 15 µl (0.12 mmol) of (3S) -methylaminopyrrolidine were added thereto and heated with stirring at 80°C for 12 hours. After restoring to room temperature, the solvent was evaporated under reduced pressure, and the resulting residue was dissolved in chloroform and washed with aqueous saturated sodium hydrogencarbonate solution and brine. The organicphase was dried over anhydrous sodium sulfate, the solvent was evaporated under reduced pressure, and the resulting residue was applied to a preparative silica gel column chromatography. This was eluted with a mixed solvent of chloroform/methanol (20/1, v/v), and recrystallized and purified from chloroform/n-hexane/ethyl acetate to obtain 13 mg (35 %) of the entitled compound as a yellow crystal.
MS(ESI)m/z:354(M+1)⁺.
¹H-NMR(DMSO-d₆)δ: 2.09(1H, m), 2.23(6H, s), 2.26-2.32 (1H, m), 2.67(3H, s), 3.04 (1H, br), 3.23-3.36 (2H, m), 3.51-3.60(2H, m), 7.45(1H, t, J=7.3Hz), 7.57(1H, t, J=8.1Hz), 7.87 (1H, d, J=8.1Hz), 8.23(1H, br).
IR(ATR): 2781, 2225, 1502, 1473, 1440, 1302, 1277 cm⁻¹.

| Elemental analysis: C₁₉H₂₀ClN₅·0.25H₂O | | | | |
|---|---|---|---|---|
| Calcd. | C, 63.68%; | H, 5.77%; | N, 19.54%; | Cl, 9.89% |
| Found | C, 63.88%; | H, 5.67%; | N, 19.38%; | Cl, 10.03%. |

### [Reference Example 152]

### Methyl 2-(1-benzothiophen-3-yl)acetate (I-152):

3.00 g (15.61 mmol) of benzo[b]-thiophene-3-acetic acid was dissolved in a mixed solvent of benzene (90 ml) and methanol (30 ml) , and 9.40 ml (18.73 mmol) of trimethylsilyldiazomethane (2 N n-hexane solution) was added thereto with cooling with ice, and stirred at room temperature for 1 hour. The reaction mixture was evaporated under reduced pressure, and the resulting residue was applied to a silica gel column chromatography. This was eluted with a mixed solvent of n-hexane/ethyl acetate (4/1, v/v) to obtain 3.40 g (quantitative) of the entitled compound as a brown oil.
MS (ESI)m/z:207 (M +1)⁺.
¹H-NMR(CDCl₃)δ: 3.71(3H, s), 3.87(2H, s), 7.35(1H, s), 7.34-7.42(1H, m), 7.77(1H, d, J=7.6Hz) , 7.86(1H, d, J=7.6Hz).

### [Reference Example 153]

### Methyl 2-(1-benzothiophen-3-yl)acetoacetate (I-153):

5.90 ml (3.78 mmol) of n-butyllithium (1.56 M n-hexane solution) was dissolved in tetrahydrofuran (40 ml), and 1.29 ml (9.21 mmol) of diisopropylamine was added dropwise thereto at -20°C. At the temperature, this was stirred for 15 minutes, and the reaction mixture was cooled to -40°C. Tetrahydrofuran solution (5 ml) of 2.00 g (9.70 mmol) of methyl 2- (1-behzothiophen-3-yl) acetate (I-152) was added thereto, and stirred for 1 hour at the temperature. Further, 0.44 ml (4.61 mmol) of acetic anhydride was added dropwise thereto, and with warming up to room temperature, this was stirred for 26.5 hours. Aqueous saturated ammonium chloride solution was added to the reaction mixture, extracted with ethyl acetate, and washed with brine. The resulting organic phase was dried over anhydrous sodium sulfate, and the solvent was evaporated under reduced pressure. The resulting residue was applied to a silica gel column chromatography, and eluted with a mixed solvent of n-hexane/ethyl acetate (5/1, v/v) to obtain 731 mg (64 %) of the entitled compound as a pale brown oil of keto-enol mixture. MS(ESI)m/z:249(M+1)⁺.
¹H-NMR(CDCl₃)δ: 1,83(3H, s), 3.64(3H, s), 7.22(1H, s), 7.34-7.41(1H, m), 7.51-7.53(1H, m), 7.77(1H, d, J=7.6Hz), 7.85-7.88(1H, m).

### [Reference Example 154]

### 2-(1-Benzothiophen-3-yl)-3-methyl-1-oxo-1,5-dihydropyrido[1,2-a]benzimidazole-4-carbonitrile (I-154):

A mixture of 463 mg (2.94 mmol) of (2-benzimidazolyl)acetonitrile, 731 ml (2.94 mmol) of methyl 2-(1-benzothiophen-3-yl)acetoacetate (I-153) and 454 mg (5.89 mmol) of ammonium acetate was heated at 140 to 150°C for 1 hour. After cooling, water was added thereto, and extracted with chloroform. The resulting organic phase was dried over anhydrous sodium sulfate, and the solvent was evaporated under reduced pressure. The resulting residue was applied to a silica gel column chromatography, and eluted with a mixed solvent of chloroform/methanol (20/1, v/v) to obtain 614.5 mg (59 %) of the entitled compound as a brown amorphous solid.
MS(ESI)m/z: 356(M+1)⁺.
¹H-NMR(CDCl₃)δ: 2.33(3H, s), 7.00-7.03(1H, m), 7.26-7.34(4H, m), 7.51(1H, d, J=7.8Hz), 7.54(1H, s), 7.71(1H, d, J=7.8Hz), 8.69-8.72(1H, m).

### [Reference Example 155]

### 2-(1-Benzothiophen-3-yl)-1-chloro-3-methylpyrido[1,2-a]benzimidazole-4-carbonitrile (I-155):

614mg (1.72 mmol) of 2-(1-benzothiophen-3-yl)-3-methyl-1-oxo-1,5-dihydropyrido[1,2-a]benzimidazole-4-carbonitrile (I-154) was heated under reflux in phosphoryl chloride (6.10 ml) for 1 hour. After cooling, the resulting reaction mixture was poured into ice-water (10 ml), neutralized with saturated sodium hydrogencarbonate solution, and extracted with chloroform, and the insoluble material was taken out through filtration. The resulting organic phase was dried over anhydrous sodium sulfate, and the solvent was evaporated under reduced pressure. The resulting residue and the insoluble material taken out previously were dissolved in a small amount of methanol, and recrystallized from n-hexane/ethyl acetate to obtain 462 mg (72 %) of the entitled compound as a yellow solid.
MS(ESI)m/z:374 (M+1)⁺.
¹H-NMR(CDCl₃)δ: 2.29 (3H, s), 7.38(1H, t, J=7.6Hz), 7.46 (2H, m), 7.61 (1H, d, J=8.1Hz), 7.66(1H, t, J=8. 3Hz) , 7.97(1H, s), 7.99(1H, d, J=7.6Hz), 8.12(1H, d, J=8.1Hz), 8.65(1H, d, J=8.3 Hz).

### [Example 128]

### 2-(1-Benzothiopen-3-yl)-1-[(3S)-3-(dimethylamino)pyrrolidinyl]-3-methylpyrido[1,2-a]benzimidazole-4-carbonitrile (#128):

300ml (0.80mmol) of 2-(1-benzothiophen-3-yl)-1-chloro-3-methylpyrido[1,2-a]benzimidazole-4-carbonitrile (I-155) was suspended in dimethylsulfoxide (6 ml), and 0.22 ml (1.60 mmol) of triethylamine and 0.12 ml (0.96 mmol) of (3S)-dimethylaminopyrrolidine were added thereto and heated with stirring at 80°C for 3 hours. After restoring to room temperature, the solvent was evaporated under reduced pressure, and the resulting residue was dissolved in chloroform and washed with aqueous saturated sodium hydrogencarbonate solution and brine. The organi c phase was dried over anhydrous sodium sulfate, the solvent was evaporated under reduced pressure, and the resulting residue was applied to a silica gel column chromatography. This was eluted with a mixed solvent of chloroform/acetone (2/1, v/v) to obtain 141 mg (39 %) of the entitled compound as a yellow-white solid.
MS(FAB)m/z:452(M+1)⁺.
¹H-NMR(DMSO-d₆)δ: 1.89(3H, s), 1.91(3H, s), 2.17(3H, d, J=2.2Hz), 3.00-3.18(7H, m), 7.39-7.46(3H, m), 7.54-7.58(2H, m) , 7.85(1H, d, J=8.3 Hz), 7.88(1H, d, J=8.1Hz), 8.08-8.12(2H, m).
IR(ATR): 2220, 1442, 1288 cm⁻¹.

| Elemental analysis: C₂₅H₂₅N₅·0.5H₂O | | | |
|---|---|---|---|
| Calcd. | C, 70.41%; | H, 5.69%; | N, 15.20% |
| Found | C, 70.63%; | H, 5.46%; | N, 15.21%. |

### [Reference Example 156]

### Methyl 2-(1-benzofuran-3-yl)acetate (I-156):

10.00 g (23.34 mmol) of phenol iodide, 4.25 ml (35.02 mmol) of 2,5-dihydroxy-2,5-dimethoxyfuran, 7.96ml (46.68mmol) of diisopropylethylamine and 4.99 g (23.34 mmol) of benzyltriethylammonium chloride were dissolved in dimethylformamide (100 ml) , and 100 mg (0.47 mmol) of palladium acetate was added thereto and stirred at 70°C for 6 hours. The reaction mixture was restored to room temperature, extracted with diethyl ether, and washed with aqueous saturated sodium hydrogencarbonate solution and brine. The resulting organic phase was dried over anhydrous sodium sulfate, and the solvent was evaporated under reduced pressure. The resulting residue was dissolved in dichloromethane (100ml) without being purified, and 8.8 ml (70.12 mmol) of boron trifluoride/diethyl ether complex was added thereto and stirred at room temperature for 19 hours. Aqueous saturated sodium hydrogencarbonate solution was added to the reaction mixture, extracted with dichloromethane and washed with brine. The resulting organic phase was dried over anhydrous sodium sulfate, and the solvent was evaporated under reduced pressure. The resulting residue was applied to a silica gel column chromatography, and eluted with a mixed solvent of n-hexane/ethyl acetate (8/1, v/v) to obtain 3.97 g (89 %) of the entitled compound as a brown oil.
MS(ESI)m/z:191 (M +1)⁺.
¹H-NMR(CDCl₃)δ: 3.72(2H, s), 3.73(3H, s), 7.24-7.33(2H, m), 7.48(1H, dd, J=0.7, 8.8Hz), 7.57(1H, d, J=8.8Hz), 7.63(d, 1H, J=0.7Hz).

### [Reference Example 157]

### Methyl 2-(1-benzofuran-3-yl)acetoacetate (I-157):

315 mg (7.87 mmol) of sodium hydride (60 % mineral oil suspension) was dissolved in dimethoxyethane (8 ml), and a catalytic amount of 15-crown-5 was added thereto. With cooling with ice, dimethoxyethane solution (2 ml) of 500 mg (2.63 mmol) of methyl 2- (1-benzofuran-3-yl) acetate (I-156) was added thereto, and stirred for 20 minutes at the temperature. 1.28 ml (13.15 mmol) of ethyl acetate was added dropwise thereto, stirred at room temperature for 30 minutes, and then heated under reflux for 30 minutes. Aqueous saturated ammonium chloride solution was added to the reaction mixture, extracted with ethyl acetate, and washed with brine. The resulting organic phase was dried over anhydrous sodium sulfate, and the solvent was evaporated under reduced pressure. The resulting residue was applied to a silica gel column chromatography, and eluted with a mixed solvent of n-hexane/ethyl acetate (8/1, v/v) to obtain 290 mg (44 %) of the entitled compound as a yellow oil of keto-enol mixture.
MS(ESI)m/z:247(M+1)⁺.
main product (enol)
¹H-NMR(CDCl₃)δ: 1.15(3H, t, J=7.1Hz), 1.95(3H, d, J=1.0Hz), 4.17(2H, q, J=7.1Hz), 7.22-7.56(5H, m), 13.38(1H, s).
by-product (keto)
¹H-NMR(CDCl₃)δ: 1.26(1H, br), 1.29(3H, t, J=7.3Hz), 2.24(3H, s), 4.26(2H, q, J=7.3Hz), 7.22-7.56(4H, m), 7.83(1H, s).

### [Reference Example 158]

### 2-(1-(Benzofuran-3-yl)-3-methyl-1-oxo-1,5-dihydropyrido[1,2-a]benzimidazole-4-carbonitrile (I-158):

A mixture of 186 mg (1.19 mmol) of (2-benzimidazolyl)acetonitrile, 275 mg (1.19 mmol) of methyl 2-(1-benzofuran-3-yl)acetoacetate (I-157) and 183 mg (2.37 mmol) of ammonium acetate was heated at 150°C for 16 hours. After cooling, water was added thereto, and extracted with chloroform. The resulting organic phase was dried over anhydrous sodium sulfate, the solvent was evaporated under reduced pressure, and the resulting residue was applied to a silica gel column chromatography. This was eluted with a mixed solvent of chloroform/methanol (30/1, v/v) to obtain 244 mg (61 %) of the entitled compound as a brown amorphous solid.
MS (ESI)m/z:340 (M +1)⁺.
¹H-NMR(CDCl₃)δ: 2.43(3H, s), 7.15-7.42(7H, m), 7.74(1H, s), 8.72(1H, d, J=7.8Hz)

### [Reference Example 159]

### 2-(1-Benzofuran-3-yl)-1-chloro-3-methylpyrido[1,2-a] benzimidazole-4-carbonitrile (I-159):

243 mg (0. 72 mmol) of 2- (1-benzofuran-3-yl) -3-methyl-1-oxo-1,5-dihydropyrido[1,2-a]benzimidazole-4-carbonitrile (I-158) was heated under reflux in phosphoryl chloride (2.50 ml) for 1 hour. After cooling, the resulting reaction mixture was poured into ice-water, neutralized with saturated sodium hydrogencarbonate solution, and extracted with chloroform. The organic phase was dried over anhydrous sodium sulfate, and the solvent was evaporated under reduced pressure. The resulting residue was recrystallized from n-hexane/ethylacetate/ethanol to obtain 188 mg (73 %) of the entitled compound as a brown solid.
MS (ESI)m/z:358 (M+1)⁺.
¹H-NMR(CDCl₃)δ: 2.44 (3H, s), 7.31(1H, t, J=7.3Hz), 7.43(1H, t, J=7.3Hz), 7.48(1H, t, J=7.8Hz), 7.56(1H, d, J=7.3Hz), 7.67(1H, t, J=8.0Hz), 7.75(1H, d, J=8.5Hz) 8.00(1H, d, J=8.8Hz), 8.27(1H, s), 8.66(1H, d, J=8.5Hz).

### [Example 129]

### 2-(1-Benzofuran-3-yl)-1-[(3S)-3-(dimethylamino)-pyrrolidinyl]-3-methylpyrido[1,2-a]benzimidazole-4-carbonitrile (#129):

187 mg (0.52 mmol) of 2-(1-benzofuran-3-yl)-1-chloro-3-methylpyrido[1,2-a]benzimidazole-4-carbonitrile (I-159) was suspended in dimethylsulfoxide (3.70ml), and 0.15ml (1.10mmol) of triethylamine and 86 µl (0.68 mmol) of (3S)-dimethylaminopyrrolidine were added thereto and heated with stirring at 90°C for 5 hours. After restoring to room temperature, the solvent was evaporated under reduced pressure, and the resulting residue was dissolved in chloroform and washed with aqueous saturated sodium hydrogencarbonate solution and brine. The organic phase was dried over anhydrous sodium sulfate, the solvent was evaporated under reduced pressure, and the resulting residue was recrystallized from n-hexane/ethyl acetate to obtain 170 mg (73 %) of the entitled compound as a yellow crystal.
MS(ESI)m/z:436(M+1)⁺.
¹H-NMR(CDCl₃)δ: 1.40-2.30(11H, br), 2.37(3H, s each) , 3.00-3.30(3H, br), 7.26-7.47(4H, m), 7.57(1H, t, J=7.3Hz), 7.64-7.68(2H, m), 8.03(1H, d, J=8.3Hz).
IR(ATR): 2220, 1298, 1090, 742 cm⁻¹.

| Elemental analysis: C₂₇H₂₅N₅O·0.75H₂O | | | |
|---|---|---|---|
| Calcd. | C, 72.22%; | H, 5.95%; | N, 15.60% |
| Found | C, 72.13%; | H, 5.67%; | N, 15.47%. |

### [Reference Example 160]

### Methyl 2-(1-benzofuran-2-yl)acetoacetate (I-160):

216 mg (5.36 mmol) of sodium hydride (60 % mineral oil suspension) and 15-crown-5 (one drop) were dissolved in dimethoxyethane (6.8 ml). With cooling with ice, dimethoxyethane solution (3.4ml) of 340mg (1.79 mmol) of methyl 2-(1-benzofuran-2-yl)acetate that had been produced with reference to Journal of Medicinal Chemistry, Vol. 32, p. 522 (1989) was added dropwise thereto. At the temperature, this was stirred for 20 minutes, and 0.72 ml (8.94 mmol) of methyl acetate was added thereto and heated under reflux for 17 hours. Aqueous saturated ammonium chloride solution was added to the reaction mixture, extracted with ethyl acetate, and washed with brine. The resulting organic phase was dried over anhydrous sodium sulfate, and the solvent was evaporated under reduced pressure. The resulting residue was applied to a silica gel column chromatography, and eluted with a mixed solvent of n-hexane/ethyl acetate (20/1, v/v) to obtain 218 mg (52 %) of the entitled compound as a pale yellow oil.
MS(ESI)m/z:233(M +1)⁺.
¹H-NMR(CDCl₃)δ: 2.06(3H, s) , 3.75(3H, d, J=0.7Hz), 6.60(1H, s), 7.20-7.29(2H, m), 7.46(1H, d, J=8.1Hz), 7.56(1H, d, J=7.6Hz), 13.38(1H, s).

### [Reference Example 161]

### 2-(1-(Benzofuran-2-yl)-1-chloro-3-methylpyrido[1,2-a]benzimidazole-4-carbonitrile (I-161):

A mixture of 217 mg (0.94 mmol) of methyl 2-(1-benzothiophen-3-yl)acetoacetate, 147 mg (0.94 mmol) of (2-benzimidazolyl)acetonitrile and 144 mg (1.87 mmol) of ammonium acetate was heated at 140 to 150°C for 5 hours. After cooling, water was added thereto, and extracted with chloroform. The resulting organic phase was dried over anhydrous sodium sulfate, the solvent was evaporated under reduced pressure, and the resulting residue was applied to a silica gel column chromatography. This was eluted with a mixed solvent of chloroform/methanol (40/1, v/v) to obtain 101 mg (0.30 mmol) of crude 2-(1-(benzofuran-2-yl)-3-methyl-1-oxo-1,5-dihydropyrido[1,2-a]benzimidazole-4-carbonitrile as a brown oil. The resul ting product was dissolved in phosphoryl chloride (1 ml) and heated under reflux for 1 hour. After cooling, the reaction mixture was poured into ice-water (10 ml) , neutralized wi th saturated sodiumhydrogencarbonate solution, and extracted with chloroform. The insoluble material was removed through filtration, and the resulting organic phase was dried over anhydrous sodium sulfate, and the solvent was evaporated under reduced pressure. The resulting residue was applied to a silica gel column chromatography and eluted with a mixed solvent of n-hexane/ethyl acetate (2/1, v/v) to obtain 45 mg (42 %) of the entitled compound as a yellow solid.
MS(ESI)m/z:358(M+1)⁺.
¹H-NMR(CDCl₃)δ: 2.61(3H, s), 6.98(1H, s), 7.36-7.48(4H, m), 7.58(1H, d, J=8.3Hz) , 7.66(1H, t, J=8.3Hz), 7.72(1H, d, J=8.1Hz), 8.09(1H, d, J=7.8Hz), 8.60(1H, d, J=8.8Hz).

### [Example 130]

### 2-(1-Henzofuran-2-yl)-1-[(3S)-3-(dimethylamino)-pyrrolidinyl]-3-methylpyrido[1,2-a]benzimidazole-4-carbonitrile (#130):

44 mg (0.12 mmol) of 2-(1-benzofuran-2-yl)-1-chloro-3-methylpyrido[1,2-a]benzimidazole-4-carbonitrile (I-161) was suspended in dimethylsulfoxide (2 ml), and 36 µl (0.26 mmol) of triethylamine and 20 µl (0.16 mmol) of (3S)-dimethylaminopyrrolidine were added thereto and heated with stirring at 90°C for 8 hours. The reaction mixture was restored to room temperature, and the solvent was evaporated under reduced pressure. The resulting residue was dissolved in ethyl acetate and washed with aqueous saturated sodium hydrogencarbonate solution and brine. The organic phase was dried over anhydrous sodium sulfate, the solvent was evaporated under reduced pressure, and the resulting residue was applied to a preparative silica gel column chromatography. This was eluted with a mixed solvent of chloroform/methanol (15/1, v/v), and recrystallized from ethyl acetate/n-hexane to obtain 15 mg (28 %) of the entitled compound as a yellow crystal.
MS(ESI)m/z:436(M+1)⁺.
¹H-NMR(CDCl₃)δ: 1.88(1H, br), 2.09(6H, s), 2.10(1H, br), 2.51 (3H, s), 2.60-3.60(5H, br), 6.86(1H, d, J=1.0Hz), 7.32-7.42(2H, m), 7.56(1H, dt, J=1.0, 8.3Hz) , 7.58 (1H, dd, J=1.0, 7.1Hz), 7.68(1H, ddd, J=0.7, 1.5, 8.3Hz), 8.03(1H, d, J=8.3Hz), 8.05(1H, br).
IR(ATR): 2225, 1500, 1442, 1255 cm⁻¹.

| Elemental analysis: C₂₇H₂₅N₅O·0.5H₂O | | | |
|---|---|---|---|
| Calcd. | C, 72.95%; | H, 5.90%; | N, 15.75% |
| Found | C, 73.32%; | H, 5.69%; | N, 15.49%. |

### [Reference Example 162]

### Ethyl α-(furan-2-yl)acetoacetate (I-162):

4.55 ml (2.2 M, 10.0 mmol) of dichloromethane solution of dichlorozinc/diethyl ether complex was added dropwise to dichloromethane/diethyl ether (1/1, v/v, 80 ml) solution of 1.22 ml (10.0 mmol) of 2,5-dimethoxy-2,5-dihydrofuran and 1.26 ml (10. 0 mmol) of ethyl acetoacetate. The system was replaced with nitrogen and sealed up, and heated at room temperature for 18 hours. Diethyl ether was added thereto, and the reaction mixture was washed with water. The organic layer was dried over anhydrous sodium sulfate, the solvent was evaporated under reduced pressure, and the resulting residue was applied to a silica gel column chromatography. This was eluted with a mixed solvent of hexane/ethyl acetate (97/3, v/v) to obtain 281 mg (14 %) of the entitled compound as an oil.
¹H-NMR(CDCl₃)δ: 1.24(3H, t, J=7.1Hz ), 1.97(3H, s), 4.21(2H, q, J=7.1Hz), 6.20(1H, d, J=3.2Hz), 6.40(1H, d, J=3.2Hz), 7.41(1H, s), 13.36(1H, s).

### [Reference Example 163]

### 2-(Furan-2-yl)-3-methyl-1-oxo-1H,5H-pyrido[1,2-a]benzimidazole-4-carbonitrile (I-163):

A mixture of 226 mg (1.43 mmol) of (2-benzimidazolyl)acetonitrile, 281 ml (1.43 mmol) of ethyl α-(furan-2-yl)acetoacetate (I-162) and 221 mg (2.87 mmol) of ammonium acetate was heated at 140 to 150°C for 3 hours. After cooling, chloroform was added thereto, and washed with water. The organic layer was dried over magnesium sulfate, and the solvent was evaporated. The resulting residue was applied to a flash silica gel column chromatography, and eluted with a mixed solvent of chloroform/ethyl acetate (97/3, v/v) to obtain a crude product of the entitled compound. This was washed with acetonitrile and was collected by filtration to obtain 102 mg (25 %) of the entitled compound as a pale green crystal.
MS(EI)m/z:290(M+1)⁺.
¹H-NMR(DMSO-d₆)δ: 2.42(3H, s), 6.59(1H, dd, J=3.4Hz, 1.7Hz), 6.65(1H, dd, J=3.4Hz, 0.7Hz), 7.38-7.42(1H, m), 7.52-7.58(2H, m), 7.74(1H, dd, J=1.7Hz, 0.7Hz), 7.31-7.37(1H, m), 8.58(1H, d, J=8.1Hz).

### [Reference Example 164]

### 1-Chloro-2-(furan-2-yl)-3-methylpyrido[1,2-a]benzimidazole-4-carbonitrile (I-164):

100 mg (0.346 mmol) of 2-(furan-2-yl)-3-methyl-1-oxo-1H,5H-pyrido[1,2-a]benzimidazole-4-carbonitrile (I-163) was heated under reflux in phosphoryl chloride (2 ml) for 90 minutes. After cooling, phosphoryl chloride was evaporated under reduced pressure, and ice-water and chloroform were added to the residue. While the resulting mixture was stirred, aqueous 1 N sodium hydroxide solution was added thereto until the pH of its aqueous layer could be at least 10. This was further stirred for 30 minutes, and the organic layer was extracted with chloroform. The combined chloroform layers were washed with brine, and dried over anhydrous sodium sulfate, and the solvent was evaporated under reduced pressure. The resulting residue was washed with a small amount of diisopropyl ether and a solid was collected by filtration to obtain 86.0 mg (81 %) of the entitled compound as a pale yellow-green crystal (this was used in the next reaction without further purification).
MS(EI)m/z:308(M+1)⁺.

### [Example 131]

### 1-[(3S)-Dimethylaminopyrrolidin-1-yl]-2-(furan-2-yl)-3-methylpyrido[1,2-a]benzimidazole-4-carbonitrile (#131):

To N,N-dimethylformamide (6 ml) suspension of 61.5 mg (0.20 mmol) of 1-chloro-2-(furan-2-yl)-3-methylpyrido[1,2-a]benzimidazole-4-carbonitrile (I-164) were added 50.8 µl (0.40 mmol) of (3S)-dimethylaminopyrrolidine and 83.6 µl (0.60 mmol) of triethylamine. The system was replaced with nitrogen and sealed up, and heated at 80°C for 3.5 hours. After cooling, the solvent was evaporated under reduced pressure, and the resulting residue was dissolved in dimethylsulfoxide, and purified through preparative reversed-phase HPLC (eluent solvent: water/acetonitrile) to obtain a crude product of the entitled compound. This was applied to a thin-layer silica gel column chromatography and developed with a mixed solvent of chloroform/methanol (95/5, v/v) to obtain 46.5 mg (60 %) of the entitled compound. This was washed with diisopropyl ether and was collectedby filtration to obtain 24.6 mg (32%) of the entitled compound as a yellow crystal.
MS(EI)m/z:386(M+1)⁺.
¹H-NMR(DMSO-d₆)δ: 1.79(1H, brs), 2.12(8H, brs), 2.34(3H, s), 2.76(1H, brs), 3.14(2H, m), 6.70-6.74(2H, m), 7.43(1H, t, J=7.7Hz), 7.57(1H, t, J=7.6Hz), 7.87(1H, d, J=8.3Hz), 7.99(1H, s), 8.31(1H, m).
IR(ATR): 2777, 1626, 1581, 1469, 758 cm⁻¹.

| Elemental analysis: C₂₃H₂₃N₅O | | | |
|---|---|---|---|
| Cacld. | C, 71.67%; | H, 6.01%; | N, 18.17% |
| Found | C, 71.45%; | H, 5.89%; | N, 17.64%. |

### [Reference Example 165]

### Ethyl α-(thiophen-2-yl)acetoacetate (I-165):

1. 22 ml (10.0 mmol) of ethyl 2-thiopheneacetoacetate was added to tetrahydrofuran suspension (10 ml) of 240 mg (60 wt.%, 6. 00 mmol) of sodium hydride washed with hexane, with replacing the system with nitrogen at 0°C. At the temperature, this was stirred for 2 hours, and then 944 µl (10.0 mmol) of acetic anhydride was added dropwise thereto at 0°C. The reaction mixture was heated under reflux at 80°C for 3 hours. After cooling, diethyl ether was added thereto, and 1 N hydrochloric acid was added thereto with stirring at 0°C. The organic layer was extracted with ethyl acetate, and washed with brine. The organic layer was dried over anhydrous sodium sulfate, the solvent was evaporated under reduced pressure, and the resulting residue was applied to a silica gel column chromatography. This was eluted with a mixed solvent of hexane/ethyl acetate (4/1, v/v) to obtain 441 mg (42 %) of the entitled compound as an oil. ¹H-NMR(CDCl₃)δ: 1.25(3H, t, J=7.1Hz), 2.07(3H, s) , 2.17(1H, s), 4.20(2H, q, J=7.1Hz), 6.97-7.02(2H, m), 7.35(1H, d, J=5.1Hz

### [Reference Example 166]

### 3-Methyl-1-oxo-2-(thiophen-2-yl)-1H,5H-pyrido[1,2-a]benzimidazole-4-carbonitrile (I-166):

A mixture of 327 mg (2.08 mmol) of (2-benzimidazolyl)acetonitrile, 441 ml (2.08 mmol) of ethyl α-(thiophen-2-yl)acetoacetate (I-165) and 320 mg (4.16 mmol) of ammonium acetate was heated at 140 to 150°C for 30 minutes. After cooling, chloroform was added thereto, and washed with water. The organic layer was dried over magnesium sulfate, and the solvent was evaporated. The resulting residue was applied to a silica gel column flash chromatography, and eluted with a mixed solvent of chloroform/ethyl acetate (9/1, v/v) to obtain a crude compound. This was washed with ethyl acetate and was collected by filtration to obtain 234 mg (37 %) of the entitled compound as a purple powder.
MS(EI)m/z:306(M+1)⁺.
¹H-NMR(DMSO-d₆)δ: 2.37(3H, s), 7.06(1H, d, J=3.4Hz), 7.12-7.15(1H, m), 7.39(1H, t, J=8.1Hz), 7.53-7.58(2H, m), 7.62(1H, d, J=5.1Hz), 8.57(1H, d, J=8.1Hz).

### [Reference Example 167]

### 1-Chloro-3-methyl-2-(thiophen-2-yl)pyrido[1,2 a]benzimidazole-4-carbonitrile (I-167):

234 mg (0.767 mmol) of 3-methyl-1-oxo-2-(thiophen-2-yl)-1H,5H-pyrido[1,2-a]benzimidazole-4-carbonitrile (I-166) was heated under reflux in phosphoryl chloride (4 ml) for 12 hours. After cooling, phosphoryl chloride was evaporated under reduced pressure, and ice-water and chloroform were added to the residue. While the resulting mixture was stirred, aqueous 1 N sodium hydroxide solution was added thereto until the pH of its aqueous layer could be at least 10. This was stirred for further 30 minutes, and the organic layer was extracted with chloroform. The combined chloroform layers were washed with brine and dried over anhydrous sodium sulfate, and the solvent was evaporated under reduced pressure. The resulting residue was washed with a small amount of diisopropyl ether and a precipitated solid was collected by filtration to obtain 246 mg (99 %) of theentitled compound as a yellow crystal (this was used in the next reaction without further purification).
MS (EI)m/z:324 (M+1)⁺.
¹H-NMR(CDCl₃)δ: 2.52 (3H, s), 7.06(1H, d, J=3.4Hz) , 7.22(1H, dd, J=3.4Hz, 5.1Hz), 7.43(1H, dd, J=7.3, 8.5Hz) , 7.57(1H, d, J=5.1Hz), 7.61-7.65(1H, m) , 8.07(1H, d, J=8.3Hz), 8.57(1H, d, J=8.5Hz).

### [Example 132]

### 1-[(3S)-Dimethylaminopyrrolidin-1-yl]-3-methyl-2-(thiophen-2-yl)-pyrido[1,2-a]benzimidazole-4-carbonitrile (#132):

To N,N-dimethylformamide (8ml) suspension of 246 mg (0.75 mmol) of 1-chloro-3-methyl-2-(thiophen-2-yl)pyrido[1,2-a]benzimidazole-4-carbonitrile were added 145 µl (1.14 mmol) of (3S)-dimethylaminopyrrolidine and 211.8 µl (1.52 mmol) of triethylamine. The system was replaced with nitrogen and sealed up, and heated at 80°C for 2 hours. After cooling, the crystal precipitated was washed with ethanol to obtain a crude product of the entitled compound. This was recrystallized from ethanol to obtain 170 mg (56 %) of the entitled compound as a yellow crystal. The filtrate was again recrystallized from ethanol to obtain 9.4 mg (3 %) of the entitled compound.
MS(EI)m/z:402(M+1)⁺.
¹H-NMR(DMSO-d₆)δ: 1.72(1H, brs), 2.08(8H, brs), 2.35(3H, s), 2.76(1H, brs), 3.15-3.59(4H, m), 7.22(1H, d, J=3.42Hz), 7.27(1H, dd, J=3.42Hz, 5.14Hz), 7.42(1H, t, J=7.70Hz), 7.57(1H, t, J=7.70Hz), 7.81(1H, d, J=5.14Hz), 8.08(1H, m).
IR(KBr): 2777, 1591, 1491, 1471, 1444 cm⁻¹.

| Elemental analysis: C₂₃H₂₃N₅S | | | | |
|---|---|---|---|---|
| Calcd. | C, 68.80%; | H, 5.77%; | N, 17.44%; | S, 7.99% |
| Found | C, 68.79%; | H, 5.75%; | N, 17.41%; | S, 8.12%. |

### [Example 133]

### 1-[(3S)-Methylaminopyrrolidin-1-yl]-3-methyl-2-phenylpyrido[1,2-a]benzimidazole-4-carbonitrile (#133):

To dimethylsulfoxide (5 ml) suspension of 318 mg (1.00 mmol) of 1-chloro-3-methyl-2-phenylpyrido[1,2-a]benzimidazole-4-carbonitrile (I-8) were added 128 µl (1.20 mmol) of (3S) -methylaminopyrrolidine and 278 µl (2.00 mmol) of triethylamine. The system was replaced wi th nitrogen and sealed up, and heated at 80°C for 3.5 hours. After cooling, the solvent was evaporated under reduced pressure, and the resulting residue was dissolved in chloroform, and washed with saturated sodium bicarbonate solution and brine. The organic layer was dried over anhydrous sodium sulfate, the solvent was evaporated under reduced pressure, and the resulting residue was applied to a silica gel column chromatography. This was eluted with a mixed solvent of chloroform/methanol (98/2, v/v to 95/5, v/v) to obtain a crude product of the entitled compound. The crystal was washed with ethanol/diethyl ether to obtain 140 mg (37 %) of the entitled compound as a yellow crystal.
MS(EI)m/z:381(M⁺).
¹H-NMR(CDCl₃)δ: 1.40-3.80(7H, m), 2.30(3H, s), 2.33(3H, brs), 7.20-7.40(3H, m), 7.41-7.65(4H, m), 8.00(1.5H, d, J=8.0Hz), 8.25-8.40(0.5H, m).
IR(ATR): 2222, 1622, 1587, 1468, 1441 cm⁻¹.

| Elemental analysis: C₂₄H₂₃N₅·0.25H₂O | | | |
|---|---|---|---|
| Calcd. | C, 74.68%; | H, 6.14%; | N, 18.14% |
| Found | C, 74.94%; | H, 6.05%; | N, 18.17%. |

### [Example 134]

### 1-[(3S)-N-Cyclopropylaminopyrrolidin-1-yl)-3-methyl-2-phenylpyrido[1,2-a]benzimidazole-4-carbonitrile (#134):

To dimethylsulfoxide (10 ml) suspension of 318 mg (1.00 mmol) of 1-chloro-3-methyl-2-phenylpyrido[1,2-a]benzimidazole-4-carbonitrile (I-8) were added 461 mg (1.30 mmol) of (3S)-N-cyclopropylaminopyrrolidine trifluoroacetate and 696 µl (5.00 mmol) of triethylamine. The system was replaced with nitrogen and sealed up, and heated at 80°C for 5 hours. After cooling, the solvent was evaporated under reduced pressure, and the resul ting residue was dissolved in chloroform, and washed with saturated sodium bicarbonate solution and brine. The organic layer was dried over anhydrous sodium sulfate, the solvent was evaporated under reduced pressure, and the resulting residue was applied to a silica gel column chromatography. This was eluted with a mixed solvent of chloroform/methanol (99/1, v/v) to obtain a crude product of the entitled compound. The crystal was washed with ethanol to obtain 190 mg (47 %) of the entitled compound as a yellow crystal.
MS(EI)m/z:407(M⁺).
¹H-NMR(CDCl₃)δ: 0.25-0.50(4H, m), 1.30-3.80(8H, m), 2.30(3H, s), 7.18-7.32(4H, m), 7.40-7.60(3H, m), 7.98-8.05(1.5H, m), 8.20-8.30(0.5H, m).
IR(ATR): 2222, 1624, 1591, 1469, 1441 cm⁻¹.

| Elemental analysis: C₂₆H₂₅N₅·0.25H₂O | | | |
|---|---|---|---|
| Calcd. | C, 75.79%; | H, 6.24%; | N, 17.00% |
| Found | C, 76.05%; | H, 6.18%; | N, 17.05%. |

### [Example 135]

### 1-(2,8-Diazabicyclo[4.3.0]non-8-yl)-3-methyl-2-phenylpyrido[1,2-a]benzimidazole-9-carbonitrile (#135):

To dimethylsulfoxide (10 ml) suspension of 318 mg (1.00 mmol) of 1-chloro-3-methyl-2-phenylpyrido[1,2-a]benzimidazole-4-carbonitrile (C1-1) were added 2-tertiary butoxycarbonyl-2,8-diazabicyclo[4.3.0]nonane (corresponding to 2.00 mmol) and 500 µl of triethylamine. The system was replaced with nitrogen and sealed up, and heated at 80°C for 20 hours. After cooling, the solvent was evaporated under reduced pressure, and the resulting residue was dissolved in chloroform, and washed with saturated sodium bicarbonate solution and brine. The organic layer was dried over anhydrous sodium sulfate, the solvent was evaporated under reduced pressure, and the resulting residue was applied to a silica gel column chromatography. This was eluted with a mixed solvent of chloroform/methanol (97/3, v/v) to obtain the main product. Concentrated hydrochloric acid was added thereto and stirred at room temperature for 10 minutes, and this was made alkaline with aqueous saturated sodium hydroxide solution and aqueous saturated sodium bicarbonate solution added thereto, and then extracted with chloroform. The organic layer was dried over anhydrous sodium sulfate, the solvent was evaporated under reduced pressure, and the resulting residue was applied to a silica gel column chromatography. This was eluted with a mixed solvent of chloroform/methanol (95/5, v/v) to obtain a crude product of the entitled compound. The crystal was washed with ethanol to obtain 290 mg (71 %) of the entitled compound as a yellow crystal.
MS(EI)m/z:407(M⁺).
¹H-NMR(CDCl₃)δ: 1.20-4.00(12H, m), 2.30(3H, s), 7.15-7.40(3H, m), 7.40-7.60(4H, m) , 7.95-8.03(1.5H, m) , 8.80-9.15(0.5H, m). IR(ATR): 2211, 1622, 1587, 1498, 1475, 1436 cm⁻¹.

| Elemental analysis: C₂₆H₂₅N₅ | | | |
|---|---|---|---|
| Calcd. | C, 76.63%; | H, 6.18%; | N, 17.19% |
| Found | C, 76.42%; | H, 6.18%; | N, 17.21%. |

### [Example 136]

### 1-[(3S,4S)-4-Hydroxymethyl-3-dimethylaminopyrrolidin-1-yl]-3-methyl-2-phenylpyrido[1,2-a]benzimidazole-4-carbonitrile (#136):

To dimethylsulfoxide (10 ml) suspension of 318 mg (1.00 mmol) of 1-chloro-3-methyl-2-phenylpyrido[1,2-a]benzimidazole-4-carbonitrile (I-8) were added (3S,4S)-3-tertiary butoxycarbonylamino-4-hydroxymethylpyrrolidine (corresponding to 1.5 mmol) and 500 µl of triethylamine. The system was replaced with nitrogen and sealed up, and heated at 80°C for 15 hours. After cooling, the solvent was evaporated under reduced pressure, and the resulting residue was dissolved in chloroform, and washed with saturated sodium bicarbonate solution and brine. The organic layer was dried over anhydrous sodium sulfate, the solvent was evaporated under reduced pressure, and the resulting residue was applied to a silica gel column chromatography. This was eluted with a mixed solvent of chloroform/methanol (98/2, v/v) to obtain the main product. Concentrated hydrochloric acid (9 ml) was added thereto and stirred at room temperature for 10 minutes, and, with cooling with ice, this was made alkaline with aqueous saturated sodium hydroxide solution and aqueous saturated sodium bicarbonate solution added thereto, and then extracted with chloroform. This was dissolved in a mixed solvent of acetonitrile (20 ml) and tetrahydrofuran (8 ml) and thereto, 0.5 ml of aqueous 37 % formaldehyde solution, 150 mg (2.39 mmol) of sodium cyanotrihydroborate and 500 µl of acetic acid were added and stirred at room temperature for 30 minutes. 0.5 ml of aqueous 37 % formaldehyde solution and 150 mg (2.39 mmol) of sodium cyanotrihydroborate were again added thereto, and further stirred for 6 hours at room temperature. Aqueous saturated sodium bicarbonate solution was added to the reaction mixture, and extracted with chloroform. The organic layer was washed with brine, and dried over anhydrous sodium sulfate, and the solvent was evaporated under reduced pressure. The resulting residue was applied to a silica gel column chromatography, and eluted with a mixed solvent of chloroform/methanol (97/3, v/v) to obtain a crude product of the entitled compound. The crystal was washed with ethanol/diethyl ether to obtain 140 mg (33 %) of the entitled compound as a pale yellow crystal. MS(EI)m/z:425(M⁺).
¹H-NMR(CDCl₃)δ: 2.00-4.50(14H, m), 2.26(3H, s) , 7.21-7.40(3H, m) , 7.50-7.65(4H, m), 7.86-8.30(1H, brs), 8.01(1H, d, J=8.4 Hz). IR(ATR): 2217, 1625, 1591, 1466, 1442 cm⁻¹.

| Elemental analysis: C₂₆H₂₇N₅O·0.25H₂O | | | |
|---|---|---|---|
| Calcd. | C, 72.62%; | H, 6.45%; | N, 16.29% |
| Found | C, 72.29%; | H, 6.37%; | N, 16.31%. |

### [Reference Example 168]

### 2-Fluoro-3-methyl-1-oxo-1H,5H-pyrido[1,2-a]benzimidazole-4-carbonitrile (I-168):

A mixture of 629 mg (4.00 mmol) of (2-benzimidazolyl)acetonitrile, 502 µl (4.00 mmol) of ethyl 2-fluoroacetoacetate and 617 mg (8.00 mmol) of ammonium acetate was heated at 120°C for 20 minutes. After cooling, water was added thereto, then the solid was crushed and decanted, and acetonitrile was added thereto and washed. The crystal was collected by filtration to obtain 613 mg (64 %) of the entitled compound as a colorless crystal.
MS(EI)m/z:241(M⁺).
¹H-NMR(DMSO-d₆)δ: 2.34(3H, d, J=2.7Hz), 7.32-7.39(1H, m), 7.50-7.55(2H, m), 8.52(1H, dd, J=0.7, 8.0Hz), 13.62(1H,brs). IR(ATR): 2212, 1670, 1595, 1533, 1464 cm⁻¹.

### [Reference Example 169]

### 1-Chloro-2-fluoro-3-methylpyrido[1,2-a]benzimidazole-4-carbonitrile (I-169):

591 mg (2.45 mmol) of 2-fluoro-3-methyl-1-oxo-1H,5H-pyrido[1,2-a]benzimidazole-4-carbonitrile (I-168) was heated under reflux in phosphoryl chloride (8 ml) for 5 hours. After cooling, phosphoryl chloride was evaporated under reduced pressure, and ice-water was added to the residue. This was extracted with a mixed solvent of chloroform/methanol (95/5, v/v). The organic layer was dried over anhydrous sodium sulfate, and the solvent was evaporated under reduced pressure. The resulting residue was washed with ethanol/diethyl ether and a precipitate was collected by filtration to obtain 521 mg (82 %) of the entitled compound as a yellow crystal.
MS(EI)m/z:259(M⁺).
¹H-NMR(DMSO-d₆)δ: 2.62(3H, d, J=2.2Hz), 7.47(1H, dt, J=1.0, 7.1Hz), 7.64(1H, dt, J=1.0, 7.3Hz), 7.95(1H, d, J=8.3Hz), 8.60(1H, d, J=8.6Hz).
IR(ATR): 2235, 1489, 1448 cm⁻¹.

### [Example 137]

### 2-Fluoro-1-[(3S)-dimethylaminopyrrolidin-1-yl]-3-methylpyrido[1,2-a]benzimidazole-4-carbonitrile (#137):

To N,N-dimethylformamide (5ml) suspensionof 236mg (0.91 mmol) of 1-chloro-2-fluoro-3-methylpyrido[1,2-a]benzimidazole-4-carbonitrile (I-169) were added 288 µl (2.27 mmol) of (3S)-dimethylaminopyrrolidine and 417 µl (3.00 mmol) of triethylamine. The system was replaced with nitrogen and sealed up, and heated at 60°C for 3 hours. After cooling, the solvent was evaporated under reduced pressure, and the residue was dissolved in chloroform, and washed with saturated sodium bicarbonate solution and brine. The organic layer was dried over anhydrous sodium sulfate. The solvent was evaporated under reduced pressure, and the resulting residue was separated and purified by preparative TLC (developed with chloroform/methanol (95/5, v/v) ) to obtain a crude product of the entitled compound. The crystal was washed with ethanol/diethyl ether to obtain 200 mg (65 %) of the entitled compound as a yellow crystal.
MS(EI)m/z:337(M⁺).
¹H-NMR(CDCl₃)δ: 2.05-2.17(1H, m), 2.30-2.40(1H, m), 2.34(6H, s), 2.64(3H, d, J=2.4Hz), 3.01-3.09(1H, m), 3.42-3.50(1H, m), 3.51-3.72(3H, m) , 7.35-7.39(1H, m), 7.55(1H, dt, J=1.2, 8.3Hz), 7.99(1H, d, J=8.3Hz), 8.14(1H, d, J=8.6Hz).
IR(ATR): 2225, 1633, 1599, 1508, 1479, 1442 cm⁻¹.

| Elemental analysis: C₁₉H₂₀FN₅ | | | |
|---|---|---|---|
| Calcd. | C, 67.46%; | H, 5.97%; | N, 20.76% |
| Found | C, 67.27%; | H, 5.94%; | N, 20.81%. |

### [Example 138]

### 3-Fluoro-1-[(3S)-dimethylaminopyrrolidin-1-yl]-2-phenylpyrido[1,2-a]benzimidazole-4-carbonitrile (#138):

To N,N-dimethylformamide (3ml) suspensionof 138mg (0.41 mmol) of 1,3-dichloro-3-phenylpyrido[1,2-a]benzimidazole-9-carbonitrile which was produced according to the method described in Journal of Heterocyclic Chemistry, Vol. 25, p. 1087 (1988) were added 155 µl (1.22 mmol) of (3S) -dimethylaminopyrrolidine and 200 µl of triethylamine. The system was replaced with nitrogen and sealed up, and heated at 60°C for 15 minutes. After cooling, the solvent was evaporated under reduced pressure, and the resulting residue was dissolved in chloroform, and washed with saturated sodium bicarbonate solution and brine. The organic layer was dried over anhydrous sodium sulfate. The solvent was evaporated under reduced pressure, and the resulting residue was purified by preparative TLC (developed with chloroform/methanol (97/3, v/v)) to obtain a crude product of the entitled compound. The crystal was washed with ethanol to obtain 50 mg (29 %) of the entitled compound as a yellow crystal.
MS(EI)m/z:415(M⁺).
¹H-NMR(CDCl₃)δ: 2.00-3.80(7H, m), 2.13(6H, s), 7.28-7.32(1H, m), 7.38-7.43(1H, m), 7.50-7.61(4H, m), 7.90-8.08(2H, m).
IR(ATR): 2235, 1622, 1583, 1471, 1441 cm⁻¹.

| Elemental analysis: C₂₄H₂₂ClN₅ | | | |
|---|---|---|---|
| Calcd. | C, 69.31%; | H, 5.33%; | N, 16.84% |
| Found | C, 69.08%; | H, 5.27%; | N, 16.93%. |

### [Example 139]

### Ethyl 3-chlaro-1-[(3S)-dimethylaminopyrrolidin-1-yl]-2-phenylpyrido[1,2-a]benzimidazole-4-carboxylate (#139):

To N,N-dimethylformamide (2 ml) suspension of 200 mg (0.52 mmol) of ethyl 1,3-dichloro-3-phenylpyrido[1,2-a]-benzimidazole-4-carboxylate which was produced according to the method described in Journal of Heterocyclic Chemistry, Vol. 25, p. 1087 (1988) were added 79 µl (0.62 mmol) of (3S)-dimethylaminopyrrolidine and 209 µl (1.50 mmol) of triethylamine. The system was replaced with nitrogen and sealed up, and heated at 60°C for 4 hours and then at 80°C for 8 hours (during the heating, 79 µl of (3S) -dimethylaminopyrrolidine was added to the system). After cooling, the solvent was evaporated under reduced pressure, and the resulting residue was dissolved in chloroform, and washed with saturated sodium bicarbonate solution and brine. The organic layer was dried over anhydrous sodium sulfate. The solvent was evaporated under reduced pressure, and the resulting residue was purified by preparative TLC (developed with chloroform/methanol (97/3, v/v)) to obtain a crude product of the entitled compound. This was recrystallized from isopropyl ether to obtain 70 mg (29 %) of the entitled compound as a pale yellow crystal.
MS(EI)m/z:462(M⁺).
¹H-NMR(CDCl₃)δ: 1.45(3H, t, J=7.3Hz) , 1.85-2.45(3H, m), 2.12(6H, s), 2.80-3.65(4H, m), 4.60(2H, q, J=7.1Hz), 7.28-7.35(3H, m), 7.48-7.56(4H, m), 7.90-8.18(2H, m).
IR(ATR): 1730, 1618, 1587, 1537, 1477, 1442 cm⁻¹.

| Elemental analysis: C₂₆H₂₇ClN₄O₂ | | | |
|---|---|---|---|
| Calcd. | C, 67.45%; | H, 5.88%; | N, 12.10% |
| Found | C, 67.20%; | H, 5.89%; | N, 12.03%. |

### [Example 140]

### 3-Chloro-1-[(3S)-dimethylaminopyrrolidin-1-yl]-2-phenylpyrido[1,2-a]benzimidazole-4-carboxylic acid (#140)

An aqueous solution (1 ml) of 20 mg (0.48 mmol) of lithium hydroxide (monohydrate) was added to tetrahydrofuran (2 ml) solution of 84 mg (0.18 mmol) of ethyl 3-chloro-1-[(3S)-dimethylaminopyrrolidin-1-yl]-2-phenylpyrido[1,2-a]benzimidazole-4-carboxylate (#139), and stirred at room temperature for 4 days (during this, ethanol (1 ml), water (1 ml) and aqueous 1 N sodium hydroxide solution (5 ml) were added). The reaction mixture was neutralized with concentrated hydrochloric acid added thereto, and then extracted with chloroform/methanol (95/5, v/v). The organic layer was dried over anhydrous sodium sulfate, the solvent was evaporated under reduced pressure, and the resulting residue was washed with ethanol/diethyl ether to obtain 50 mg (63 %) of the entitled compound as a colorless crystal.
MS(EI)m/z:434(M⁺).
¹H-NMR(DMSO-d₆)δ: 1.85-3.50(13H, m), 7.20-7.60(7H, m), 7.72-8.20 (2H, m).
IR(ATR): 1620, 1587, 1508, 1473, 1444 cm⁻¹.

| Elemental analysis: C₂₄H₂₃ClN₄O₂·0.5H₂O | | | | |
|---|---|---|---|---|
| Calcd. | C, 64.93%; | H, 5.45%; | N, 12.62%; | Cl, 7.99% |
| Found | C, 64.97%; | H, 5.38%; | N, 12.49%; | Cl, 8.06%. |

### [Example 141]

### 3-Chloro-4-hydroxymethyl-1-[(3S)-dimethylaminopyrrolidin-1-yl]-2-phenylpyrido[1,2-a]benzimidazole (#141):

200 mg of lithium aluminium hydride was added to tetrahydrofuran (15 ml) solution of 367 mg (0.79 mmol) of ethyl 3-chloro-1-[(3S)-dimethylaminopyrrolidin-1-yl]-2-phenylpyrido[1,2-a]benzimidazole-4-carboxylate (#139) with cooling with ice, and then stirred for 10 minutes at the temperature. Water (200 µl), aqueous 15 % sodium hydroxide solution (600 µl) and water (200 µl) were added to the reaction mixture, and then stirred at room temperature for 10 minutes. The insoluble material was removed through filtration, and the filtrate was evaporated under reduced pressure. The resulting residue was purified by preparative TLC (developed with chloroform/methanol (97/3, v/v)) to obtain 20 m (6 %) of the entitled compound as a pale yellow crystal.
HRMS(EI)m/z: 420.1729 (Calcd for C₂₄N₂₅ClN₄O 420.9345).
¹H-NMR(CDCl₃)δ: 1.86-2.02(2H, m), 2.12(6H, brs), 2.40-3.15(2H, m), 3.22-3.36(2H, m), 5.25(2H, s), 7.20-7.28(3H, m), 7.45-7.55(4H, m), 7.85-8.18(2H, m).

### [Reference Example 170]

### Ethyl 2-amino-3-nitrobenzoate (I-170):

With cooling with ice, 4.11 ml (80.2 mmol) of bromine and 16.1 g (76.4 mmol) of 2-carbamoyl-3-nitrobenzoic acid that had been produced according to the method described in Journal of Medicinal Chemistry, Vol. 43, p. 4084 (2000) were added to an aqueous solution prepared from 40 g (0.71 mol) of potassium hydroxide and water (180 ml), and heated at 60°C for 4 hours. After cooling at room temperature, the precipitate formed was washed with a small amount of water and taken out through filtration. The precipitate was dissolved in water and controlled to have pH of 4 with concentrated hydrochloric acid added thereto, and the crystal thus formed was taken out through filtration (2-amino compound, I-170'-1). The filtrate was controlled to have pH of 4 with concentrated hydrochloric acid added thereto, and the crystal thus formed was washed with water and taken out through filtration (2-amino compound, I-170'-2). The filtrate was extracted with ethyl acetate, the organic layer was washed with brine and then dried over anhydrous sodiumsulfate, and the solvent was evaporated under reduced pressure to obtain a residue (2-amino compound, I-170'-3). Thus obtained, the 2-amino compounds (I-170'-1, I-170'-2, I-170'-3) were combined, and heated under reflux in ethanol (400 ml) in the presence of 15 ml of concentrated sulfuric acid, for 10 days. After cooling, ethanol was evaporated under reduced pressure, and water was added thereto, and extracted with ethyl acetate. The organic layer was washed with aqueous 5 % potassium carbonate solution, and dried over anhydrous sodium sulfate, and the solvent was evaporated under reduced pressure to obtain 10.5 g (65 %) of the enti tled compound as a yellow crystal. The aqueous potassium carbonate solution was made acidic with concentrated hydrochloric acid added thereto, with cooling with ice, and then extracted with ethyl acetate. The organic layer was dried over anhydrous sodium sulfate, and the solvent was evaporated under reduced pressure to obtain 3.07 g (22 %) of 2-amino-3-nitrobenzoic acid (I-170').
MS(EI)m/z:210(M⁺).
¹H-NMR(CDCl₃)δ: 1.41(3H, t, J=7.1Hz), 4.37(2H, q, J=7.1Hz), 6.65(1H, dd, J=7.8, 8. 6Hz) , 8.25 (1H, dd, J=1.7, 7.6Hz) , 8.37(1H, dd, J=1.7, 8.3Hz).
IR(ATR): 1687, 1618, 1574, 1558, 1516, 1435 cm⁻¹.

### [Reference Example 171]

### Ethyl 2,3-diaminobenzoate (I-171):

10 % palladium-carbon catalyst (water content 50 %, 1 g) was added to ethanol (150 ml) solution of 1.39 g (6.61 mmol) of ethyl 2-amino-3-nitrobenzoate (I-170), and the resulting mixture was stirred under atmospheric pressure of hydrogen at room temperature for 4 hours. The catalyst was removed through filtration, and the filtrate was evaporated under reduced pressure to obtain 1.09 g (91 %) of a crude product of the entitled compound as a brown oil. This compound was directly used in the next reaction.
¹H-NMR(CDCl₃)δ: 1.38 (3H, t, J=7.1Hz), 3.32(1H, brs), 4.33(2H, q, J=7.1Hz), 5.56(1H, brs), 6.60(1H, dd, J=7.6, 8.3Hz), 6.84 (1H, dd, J=1.5, 7.6Hz), 7.49(1H, dd, J=1.5, 8.3Hz).

### [Reference Example 172]

### Ethyl 2-cyanomethyl-1H-benzimidazole-4-carboxylate (I-172):

To N,N-dimethylformamide (20 ml) solution of 1. 08 g (5.99 mmol) of ethyl 2,3-diarninobenzoate (I-171) were added 561 mg (6.60 mmol) of cyanoacetic acid, 1.38 g (7.20 mmol) of 1-ethyl-3-(3-dimethylaminopropyl)carbodiimide hydrochloride and 810 mg (6.00 mmol) of 1-hydroxybenzotriazole, and then the resulting mixture was stirred at room temperature for 4 hours. Aqueous saturated sodium bicarbonate solution (50 ml) was added to the reaction mixture, and the resulting mixture was extracted with chloroform. The organic layer was washed with brine, and dried over anhydrous sodium sulfate. The solvent was evaporated under reduced pressure, and the resulting residue was heated under reflux in acetic acid (30 ml). After cooling, acetic acid was evaporated under reduced pressure, and the resulting residue was dissolved in chloroform, washed with aqueous saturated sodium bicarbonate solution and brine, and dried over anhydrous sodium sulfate. The solvent was evaporated under reduced pressure, the resulting residue was washed with ethanol, and the crystal was taken out through filtration to obtain 768 mg of the entitled compound as a colorless crystal. The solvent was evaporated from the mother phase, and the resulting residue was recrystallized from ethanol/diethyl ether and taken out through fil tration to obtain 216 mg of the enti tled compound as a colorless crystal. Combined, the products weighed 984 mg (72 %).
MS(EI)m/z:229(M⁺).
¹H-NMR(CDCl₃)δ: 1.42-1.48(3H, m), 4.16(2H, s), 4.41-4.50(2H, m), 7.32-7.42(1H, m), 7.95(2H, d, J=7.8Hz), 10.65(1H, brs). IR(ATR): 2268, 1678, 1599, 1552, 1531 cm⁻¹.

### [Reference Example 173]

### Ethyl 4-cyano-3-methyl-1-oxo-2-phenyl-1H,5H-pyrido[1,2-a]benzimidazole-6-carboxylate (I-173):

A mixture of 501 mg (2.18 mmol) of ethyl 2-cyanomethyl-1H-benzimidazole-4-carboxylate (I-172), 451 mg (2. 18 mmol) of ethyl 2-phenylacetoacetate and 337 mg (4.37 mmol) of ammonium acetate was heated at 140 to 150°C for 30 minutes. After cooling, water was added thereto, then the solidwas crushed and decanted, and acetonitrile was added thereto and washed. The crystal was taken out through filtration to obtain 607 mg (75 %) of the entitled compound as a pale yellow crystal.
MS(EI)m/z:371(M⁺).
¹H-NMR(CDCl₃)δ: 1.50(3H, t, J=7.1Hz), 2.36(3H, s), 4.53(2H, q, J=7.1Hz), 7.27-7.30(2H, m), 7.36-7.49(4H, m), 8.08(1H, dd, J=1.2, 8.1Hz), 8.93(1H, dd, J=1.0, 8.1Hz).
IR(ATR): 2206, 1655, 1614, 1433, 1367 cm⁻¹.

### [Reference Example 174]

### Ethyl 4-cyano-1-chloro-3-methyl-2-phenylpyrido[1,2-a]benzimidazole-6-carboxylate (I-174):

585 mg (1.58 mmol) of ethyl 4-cyano-3-methyl-1-oxo-2-phenyl-1H,5H-pyrido[1,2-a]benzimidazole-6-carboxylate (I-173) was heated under reflux in phosphoryl chloride (6 ml) for 3 hours. After cooling, phosphoryl chloride was evaporated under reduced pressure, and ice-water was added to the residue, and the resulting mixture was extracted with chloroform. The organic layer was washed with brine, and dried over anhydrous sodium sulfate, and the solvent was evaporated under reduced pressure. The resulting residue was washed with ethanol/diisopropyl ether to obtain 588 mg (96 %) of the entitled compound as a yellow crystal.
MS(EI)m/z:389(M⁺).
¹H-NMR(CDCl₃)δ: 1.54(3H, t, J=7.1Hz), 2.43(3H, s), 4.58(2H, q, J=7.1Hz), 7.27-7.30(2H, m), 7.45(1H, t, J=8.6Hz), 8.30(1H, d, J=7.6Hz), 8.78(1H, dd, J=1.0, 8.6Hz).
IR(ATR): 2225, 1697, 1616, 1579, 1533, 1489, 1460, 1419 cm⁻¹.

### [Example 142]

### Ethyl 4-cyano-1-[(3S)-dimethylaminopyrrolidin-1-yl]-3-methyl-2-phenylpyrido[1,2-a]benzimidazole-6-carboxylate (#142):

To N,N-dimethylformamide (8ml) suspension of 571 mg (1.46 mmol) of ethyl 4-cyano-1-chloro-3-methyl-2-phenylpyrido[1,2-a]benzimidazole-6-carboxylate (I-174) were added 279 µl (2.20 mmol) of (3S) -dimethylaminopyrrolidine and 408 µl (2.93 mmol) of triethylamine. The system was replaced with nitrogen and sealed up, and the resulting mixture was heated at 80°C for 8 hours. After cooling, the solvent was evaporated under reduced pressure, and the residue was dissolved in chloroform, and washed with saturated sodium bicarbonate solution and brine. The organic layer was dried over anhydrous sodium sulfate. The solvent was evaporated under reduced pressure, and the resulting residue was applied to a silica gel column chromatography. This was eluted with a mixed solvent of chloroform/methanol (97/3, v/v) to obtain a crude product of the entitled compound. The crystal was washed with ethanol to obtain 335 mg (49 %) of the entitled compound as a pale yellow crystal. The mother phase was combined with the impurity-containing fraction in silica gel column chromatography purification, and purified by preparative TLC (developed with chloroform/methanol (97/3, v/v)) to obtain 266 mg (39 %) of the entitled compound as a pale yellow compound (overall yield, 601 mg, 88 %).
MS(EI)m/z:467(M⁺).
¹H-NMR(CDCl₃)δ: 1.54(3H, t, J=7.1Hz), 1.80-2.40(3H, m), 2.12(6H, s), 2.33(3H, s), 2.70-3.65(4H, m), 4.58(2H, q, J=7.1Hz), 7.20-7.33(2H, m), 7.37(1H, t, J=8.1Hz), 7.50-7.60(3H, m), 8.05-8.90(1H, m), 8.23(1H, dd, J=1.0, 7.6Hz).
IR(ATR): 2222, 1724, 1699, 1617, 1576, 1525, 1471, 1410 cm⁻¹.

| Elemental analysis: C₂₈H₂₉N₅O₂ | | | |
|---|---|---|---|
| Calcd. | C, 69.77%; | H, 7.43%; | N, 15.65% |
| Found | C, 69.48%; | H, 7.45%; | N, 15.38%. |

### [Example 143]

### 4-Cyano-1-[(3S)-dimethylaminopyrrolidin-1-yl]-3-methyl-2-phenylpyrido[1,2-a]benzimidazole-6-carboxylic acid (#143):

266 mg (0.57 mmol) of ethyl 4-cyano-1-[(3S)-dimethylaminopyrrolidin-1-yl]-3-methyl-2-phenylpyrido[1,2-a]benzimidazole-6-carboxylate (#142) was dissolved in a mixed solvent (10 ml) of tetrahydrofuran/ethanol (1/1, v/v), and aqueous 1 N sodium hydroxide solution (3 ml) was added thereto, and the resulting mixture was stirred at room temperature for 2.5 hours. The mixture was controlled to have pH of 7 with 1 N hydrochloric acid added thereto, and then water was added thereto, and the mixture was extractedwith chloroform. The organic layer was washed with brine, and dried over anhydrous sodium sulfate, and the solvent was evaporated under reduced pressure. The resulting residue was washed with ethanol and taken out through filtration to obtain 140mg (56%) of theentitled compound as a yellow crystal.
MS(EI)m/z:439(M⁺).
¹H-NMR(CDCl₃)δ: 1.80-3.70(7H, m), 2.16(6H, s), 2.35(3H, s), 7.34-7.43(3H, m), 7.51-7.60(3H, m), 8.10-8.50(2H, m).
IR(ATR): 2222, 1741, 1617, 1587, 1529, 1491, 1468, 1423 cm⁻¹.

| Elemental analysis: C₂₆H₂₅N₅O₂·0.5H₂O | | | |
|---|---|---|---|
| Cacld. | C, 69.63%; | H, 5.84%; | N, 15.61% |
| Found | C, 69.99%; | H, 5.67%; | N, 15.86%. |

### [Example 144]

### 6-Hydroxymethyl-1-[(3S)-dimethylaminopyrrolidin-1-yl]-3-methyl-2-phenylpyrido[1,2-a]benzimidazole-4-carbonitrile (#144):

To tetrahydrofuran (5 ml) suspension of 153 mg (0.35 mmol) of 4-cyano-1-[(3S)-dimethylaminopyrrolidin-1-yl]-3-methyl-2-phenylpyrido[1,2-a]benzimidazole-6-carboxylic acid (#143) were added 84 µl (0.60 mmol) of triethylamine and 57 µl (0.60 mmol) of ethyl chlorocarbonate, and the resulting mixture was stirred at room temperature for 2 hours. The reaction mixture was cooled with ice, 50 mg of sodium borohydride and water (1 ml) were added thereto, and the mixture was stirred at the temperature for 10 minutes. Aqueous saturated ammonium chloride solution was added thereto, and the mixture was extracted with chloroform. The organic layer was dried over anhydrous sodium sulfate, the solvent was evaporated under reduced pressure, and the resulting residue was applied to a silica gel column chromatography. This was eluted with the lower layer of chloroform/methanol/water (20/3/1, v/v/v), and then with a mixed solvent of chloroform/methanol (98/2, v/v → 97/3, v/v→95/5, v/v) to obtain a crude product of the enti tled compound. The crystal was washed with isopropyl ether to obtain 30 mg (20 %) of the entitled compound as a yellow crystal.
MS(EI)m/z:425(M⁺).
¹H-NMR(CDCl₃)δ: 2.30-3.80(7H, m), 2.31(3H, s), 2.43(6H, s), 4.21(1H, t, J=6.7Hz), 5.22(2H, d, J=5.6Hz), 7.20-7.38 (4H, m), 7.43(1H, d, J=7.1Hz), 7.50-7.80(3H, m).
IR(ATR): 2222, 1622, 1587, 1467, 1407 cm⁻¹.

| Elemental analysis: C₂₆H₂₇N₅O·H₂O | | | |
|---|---|---|---|
| Calcd. | C, 70.41%; | H, 6.59%; | N, 15.79% |
| Found | C, 70.31%; | H, 6.66%; | N, 15.39%. |

### [Reference Example 175]

### 1-[(7S)-(tertiary-butoxycarbonyl)amino-5-azaspiro[2,4]hept-5-yl]-3-methyl-2-phenylpyrido[1,2-a]benzimidazole-4-carbonitrile (I-175):

To dimethylsulfoxide (8ml) solution of 750 mg (2.36mmol) of 1-chloro-3-methyl-2-phenylpyrido[1,2-a]benzimidazole-4-carbonitrile (I-8) were added 551 mg (2.60 mmol) of (7S)-(tertiary-butoxycarbonyl)amino-5-azaspiro[2,4]heptane and 658 µl (4.72 mmol) of triethylamine, and the resultingmixture was heated at 90°C for 3 hours. After cooling, water was added to the reaction mixture, the mixture was extracted with ethyl acetate, and the organic layer was washed with brine, and dried over anhydrous sodium sulfate. The solvent was evaporated under reduced pressure, and the resulting residue was applied to a silica gel column chromatography. This was eluted with a mixed solvent of chloroform/acetone (10/1, v/v) to obtain 1.11 g (95 %) of the entitled compound as a yellow crystal.
MS(ESI)m/z:494 (M+1)⁺.
¹H-NMR(CDCl₃)δ: 0.41-1.04 (4H, m), 1.48(9H, s), 2.34(3H, s), 2.70-4.03(5H, m), 7.36-7.42(2H, m) , 7.53-7.67(5H, m), 8.06(1H, d, J=8.3Hz), 8.12-8.26(1H, m).

### [Example 145]

### 1-[(7S)-amino-5-azaspiro[2,4]hept-5-yl]-3-methyl-2 phenylpyrido[1,2-a]benzimidazole-4-carbonitrile (#145):

With cooling with ice, trifluoroacetic acid (5.0 ml) was added dropwise to dichloromethane (10 ml) solution of 1.11 g (2.25 mmol) of 1-[(7S)-(tertiary-butoxycarbonyl)amino-5-azaspiro[2,4]hept-5-yl]-3-methyl-2-phenylpyrido[1,2-a]benzimidazole-4-carbonitrile (I-175), and the resulting mixture was stirred at room temperature for 2.5 hours. The reaction mixture was concentrated, and the residue was diluted with chloroform and neutralized with aqueous saturated sodium hydrogencarbonate solution. The organic layer was separated, and the aqueous layer was extracted with chloroform. The organic layers were combined, washed with brine, anddriedover anhydrous sodium sulfate. The solvent was evaporated under reduced pressure, and the resul ting crude crystal was washed with diethyl ether/methanol to obtain 855 mg (97 %) of the entitled compound as a yellow crystal.
MS(ESI)m/z:394 (M+1)⁺.
¹H-NMR(DMSO-d₆)δ: 0.23(1H, m), 0.35(1H, m) , 0.47(1H, m), 0.77(1H, m), 2.24(3H, s), 2.77-3.31(5H, m), 7.37-7.42 (4H, m), 7.51-7.58(5H, m), 7.85(1H, d, J=8.1Hz), 8.47(1H, m).
IR(ATR) : 3054, 2992, 2919, 2816, 2217, 1623, 1589, 1486, 1463, 1442, 1297 cm⁻¹.

| Elemental analysis: C₂₅H₂₃N₅·H₂O | | | |
|---|---|---|---|
| Calcd. | C, 72.97%; | H, 6.12%; | N, 17.02% |
| Found | C, 73.26%; | H, 6.23%; | N, 16.56%. |

### [Example 146]

### 1-[(7S)-dimethylamino-5-azaspiro[2,4]hept-5-yl]-3-methyl-2-phenylpyrido[1,2-a]benzimidazole-4-carbonitrile (#146):

With cooling with ice, 360 mg (5.71 mmol) of sodium borocyanohydride was gradually added to methanol (10ml) solution of a mixture of 750 mg (1.91 mmol) of 1-[(7S)-amino-5-azaspiro[2,4]hept-5-yl]-3-methyl-2-phenylpyrido[1,2-a]benzimidazole-4-carbonitrile (#145) and 3.0 ml of formaldehyde solution (37 %), and 0.2 ml (3.49 mmol) of acetic acid was added dropwise thereto. After stirring at room temperature for 30 minutes, the reaction mixture was neutralized with aqueous 1 N sodium hydroxide solution added thereto, and extracted with chloroform. The organic layer was washed with brine, and dried over anhydrous sodium sulfate. The solvent was evaporated under reduced pres sure, and the resulting residue was applied to a column chromatography. This was eluted with a mixed solvent of chloroform/methanol (20/1, v/v) to obtain 614 mg (76 %) of the entitled compound as a yellow crystal. MS(ESI)m/z:422(M+1)⁺.
¹H-NMR(CDCl₃)δ: 0.44-1.03 (4H, m), 2.03 (3H, s), 2.30 (6H, s), 2.70-4.00(5H, m), 7.28-7.36(2H, m) , 7.48-7.57 (5H, m), 8.01(1H, d, J=7.6Hz), 8.26(1H, m).
IR(ATR): 2219, 1623, 1589, 1461, 1442 cm⁻¹.

| Elemental analysis: C₂₅H₂₃N₅·0.25H₂O | | | |
|---|---|---|---|
| Calcd. | C, 76.12%; | H, 6.51%; | N, 16.44% |
| Found | C, 75.80%; | H, 6.36%; | N, 16.33%. |

### [Reference Example 176]

### 3-Methylbenzene-1,2-diamine (I-176):

4.0 g of 10 % palladium-carbon catalyst (water content, 51.5 %) was added to ethanol (200 ml) solution of 8.50 g (55.9 mmol) of 2-methyl-6-nitroaniline, and the resulting mixture was stirred under atmospheric pressure hydrogen at room temperature for 4 hours. The catalyst was removed through filtration, and the solvent was evaporated from the filtrate to obtain 6.64 g (97 %) of a crude product of the entitled compound as a black crystal. This compound was directly used in the next reaction. ¹H-NMR(CDCl₃)δ: 2.22(3H, s), 3.35-3.40(4H, m), 6.62-6.68(3H, m)

### [Reference Example 177]

### 2-Cyanomethyl-4-methyl-1H-benzimidazole (I-177):

With cooling with ice, 6.26 g (32.6 mmol) of 1-(3-dimethylaminopropyl)-3-ethylcarbodiimide hydrochloride was added to dichloromethane solution of 3.32 g (27.2 mmol) of 3-methylbenzene-1,2-diamine (I-176), 2.78 g (27.2 mmol) of cyanoacetic acid and 5.69ml (40.8 mmol) of triethylamine. After restoring to room temperature, 0.37 g (2.74 mmol) of 1-hydroxybenzotriazole was added thereto, and the resulting mixture was stirred at the temperature for 21 hours. Aqueous saturated sodium hydrogencarbonate solution was added to the reaction mixture, and the mixture was extracted with chloroform. The organic layer was washed with brine, and dried over anhydrous sodium sulfate. The solvent was evaporated under reduced pressure, and the residue was solidified with ethyl acetate/isopropyl ether to obtain 1.84 g of an amide compound as a crude brown crystal. This compound was dissolved in acetic acid (30 ml) and heated under reflux for 4 hours. After cooling, acetic acid was evaporated under reduced pressure. The residue wasdiluted with chloroform, washed with aqueous saturated sodium hydrogencarbonate solution and brine, and dried over anhydrous sodium sulfate. The solvent was evaporated under reduced pressure, and the residue was applied to a column chromatography. This was eluted with ethyl acetate to obtain a crude product of the entitled compound. The crystal was washed with isopropyl ether to obtain 666 mg (14 %) of the entitled compound as a brown crystal.
MS(ESI)m/z:172(M+1)⁺.
¹H-NMR(CDCl₃)δ: 2.47 and 2.52(total 3H, each s), 4.34 and 4.35(total 2H, each s), 7.00(1H, m), 7.08(1H, m), 7.28 and 7.41 (total 1H, each d, J=7.8 Hz) , 12.51 and 12.59(total 1H, each brs).

### [Reference Example 178]

### 2-Butyl-3,6-dimethyl-1-oxo-1H,5H-pyrido[1,2-a]benzimidazole-4-carbonitrile (I-178):

A mixture of 666 mg (3.89 mmol) of 2-cyanomethyl-4-methyl-1H-benzimidazole (I-177), 763 µl (3.89 mmol) of ethyl 2-butylacetoacetate and 600 mg (7.78 mmol) of ammonium acetate was heated at 130 to 140°C for 1 hour. After cooling, water was added thereto, then the solid was crushed and decanted, and acetonitrile was added thereto and washed. The crystal was taken out through filtration to obtain 810 mg (71 %) of the entitled compound as a brown crystal.
MS(ESI)m/z:293(M⁺).
¹H-NMR(DMSO-d₆)δ: 0.93(3H, t, J=7.1Hz), 1.32-1.46(4H, m), 2.40(3H, s), 2.56(3H, s), 2.58-2.59(2H, m), 7.22(1H, t, J=7.8Hz), 7.28(1H, d, J=7.8Hz), 8.46(1H, d, J=7.8Hz), 13.19(1H, brs).

### [Reference Example 179]

### 2-Butyl-1-chloro-3,6-dimethylpyrido[1,2-a]benzimidazole-4-carbonitrile (I-179):

810 mg (2.76 mmol) of 2-butyl-3,6-dimethyl-1-oxo-1H,5H-pyrido[1,2-a]benzimidazole-4-carbonitrile (I-178) was heated under reflux in phosphoryl chloride (4 ml) for 2 hours. After cooling, phosphoryl chloride was evaporated under reduced pressure, and ice was added to the residue to decompose the excess phosphoryl chloride. The crystal thus formed was taken out through filtration, washed with water and dried to obtain 844 mg (98 %) of the entitled compound as a yellow crystal.
MS(ESI)m/z: 312(M+1)⁺.
¹H-NMR(CDCl₃)δ: 1.01(3H, t, J=7.1Hz), 1.48-1.60(4H, m), 2.73(3H, s), 2.80(3H, s), 2.83-2.87(2H, m), 7.29(1H, m), 7.39(1H, d, J=7.4Hz), 8.42(1H, d, J=8.8Hz).

### [Example 147]

### 2-Butyl-1-[(3S)-dimethylaminopyrrolidin-1-yl]-3,6-dimethylpyrido[1,2-a]benzimidazole-4-carbonitrile (#147):

To N,N-dimethylformamide (5 ml) suspension of 312 mg (1.00 mmol) of 2-butyl-1-chloro-3,6-dimethylpyrido[1,2-a]benzimidazole-4-carbonitrile (I-179) were added 140 µl (1.10 mmol) of (3S) -dimethylaminopyrrolidine and 278 µl (2.00 mmol) of triethylamine. The system was replaced with nitrogen and sealed up, and the mixture was heated at 120°C for 3 hours. After cooling, the solvent was evaporated under reduced pressure, and the resulting residue was dissolved in chloroform, and washed with saturated sodium hydrogencarbonate solution and brine. The organic layer was dried over anhydrous sodium sulfate. The solvent was evaporated under reduced pressure, and the resulting residue was applied to a silica gel column chromatography. This was eluted with a mixed solvent of chloroform/methanol (50/1, v/v) to obtain 263 mg (68 %) of the entitled compound as a yellow crystal.
MS(ESI)m/z:390(M+1)⁺.
¹H-NMR(CDCl₃)δ: 1.02(3H, brs), 1.47-1.64(4H, m), 2.15(1H, m), 2.34(6H, s), 2.39(1H, m), 2.56-2.65(2H, m), 2.67(3H, s), 2.78(3H, s), 2.92-3.75(5H, m), 7.22(1H, m), 7.31(1H, m), 7,81-7.91(1H, m).
IR(ATR): 2954, 2861, 2815, 2767, 2221, 1621, 1589, 1482, 1454, 1407 cm⁻¹.

| Elemental analysis: C₂₄H₃₁N₅ | | | |
|---|---|---|---|
| Calcd. | C, 74.00%; | H, 8.02%; | N, 17.98% |
| Found | C, 73.79%; | H, 7.99%; | N, 17.91%. |

### [Reference Example 180]

### Methyl 3-bromophenylacetate (I-180):

To dichloromethane (580 ml) solution of 25 g (116 mmol) of 3-bromophenylacetic acid were added 9.4 ml (232 mmol) of methanol and 0.15 g (1.2 mmol) of 4-(dimethylamino)pyridine. After cooling with ice, 26.8 g (140 mmol) of 1-(3-dimethylaminopropyl)-3-ethylcarbodiimide hydrochloride was added thereto, and the resulting mixture was stirred overnight with gradually warming up to room temperature. The reaction mixture was washed with hydrochloric acid, saturated sodium bicarbonate solution and brine, and then dried over anhydrous magnesium sulfate. After filtrating and concentrating under reduced pressure, 27.3 g (quantitative) of the entitled compound was obtained as a pale yellow oil.
¹H-NMR(CDCl₃)δ: 3.60(2H, s), 3.70(3H, s), 7.16-7.23(2H, m), 7.39-7.45(2H, m).

### [Reference Example 181]

### Monobenzyl isophthalate (I-181):

Water (20 ml) and 14.1 ml (101 mmol) of triethylamine were added to methanol (200 ml) suspension of 16.6 g (100 mmol) of isophthalic acid, and the resulting mixture was stirred overnight at room temperature. The reaction mixture was concentrated under reduced pressure, and the residue was dissolved in N,N-dimethylformamide (250ml). 13.1ml (110 mmol) of benzyl bromide was added thereto, and stirred at 100°C for 2 hours. After cooling, aqueous 5 % sodium bicarbonate solution was added to the reaction mixture, and the mixture was washed twice with ethyl acetate. The aqueous layer was controlled to have pH of 6.0 with 6 N hydrochloric acid added thereto, and then extracted twice with ethyl acetate. The organic layer was washed with brine, and dried over anhydrous magnesium sulfate. After filtrating and concentrating under reduced pressure, 7.0 g (27 %) of the entitled compound was obtained as a white crystal. MS(FAB)m/z:257(M+1)⁺.
¹H-NMR(CDCl₃)δ: 5.40(2H, s), 7.32-7.58(6H, m), 8.28-8.32(2H, m), 8.79(1H, s).

### [Reference Example 182]

### Methyl 3-cyanophenylacetate (I-182):

Under nitrogen atmosphere, 8.4 g (71.3 mmol) of zinc cyanide and 5.0 g (4.3 mmol) of tetrakis(triphenylphosphino)palladium were added to N,N-dimethylformamide (300 ml) solution of 26.1 g (114 mmol) of methyl 3-bromophenylacetate (I-180), and the resulting mixture was stirred at 90°C for 2 hours. Aqueous 2 N ammonia was added to the reaction mixture, and the mixture was extracted three times with ethyl acetate. The organic layer was washed once with aqueous 2 N ammonia and three times with brine, and then dried over anhydrous magnesium sulfate. After filtrating and concentrating under reduced pressure, the resulting residue was subjected to silica gel column chromatography. From the eluate with n-hexane/ethyl acetate (9/1, v/v), 15.8 g (79 %) of the entitled compound was obtained as a yellow oil.
¹H-NMR(CDCl₃)δ: 3.66(2H, s), 3.72(3H, s), 7.26-7.59(4H, m).

### [Reference Example 183]

### Benzyl 3-ethoxycarbonylmethylbenzoate (I-183):

Three drops of N,N-dimethylformamide was added to dichloromethane (80 ml) solution of 6.36 g (24.8 mmol) of monobenzyl isophthalate (I-181), and 11 ml (126 mmol) of oxalyl chloride was added thereto at room temperature and stirred for 1 hour. The reaction mixture was concentrated under reduced pressure to obtain benzyl 3-chlorocarbonylbenzoate as a brown oil.

Diethyl ether solution of about 2.10 g (49.8 mmol) of diazomethane (prepared from 15.0 g of N-methyl-N-nitrosoparatoluenesulfonamide) was cooled to -10°C, and benzene (20 ml) solution of 1.7 g (6.2 mmol) of benzyl 3-chlorocarbonylbenzoate was added dropwise thereto, taking 30 minutes. Then, this was stirred overnight while gradually warmed up to room temperature. The reaction mixture was concentrated under reduced pressure to obtain a yellow crystal of benzyl 3-diazomethylcarbonylbenzoate.

Ethanol (40 ml) solution of benzyl 3-diazomethylcarbonylbenzoate was heated at 55°C, and silver oxide (960 mg) was added thereto, divided into 160-mg portions at intervals of 15 minutes. Then, this was further stirred at the temperature for 2 hours. After cooling, chloroform (40 ml) was added to the reaction mixture, and filtered through Celite, and the filtrate was concentrated under reduced pressure. The resulting residue was applied to a silica gel column chromatography. From the eluate with n-hexane/ethyl acetate (15/1, v/v), 1.31 g (71 %) of the entitled compound was obtained as a colorless oil.
MS(FAB)m/z:399 (M+1)⁺.
¹H-NMR(CDCl₃)δ: 1.25 (3H, t, J=7.2Hz), 3.66(2H, s), 4.15(2H, q, J=7.2Hz), 5.36(2H, s), 7.34-7.51(7H, m), 7.99(2H, m).

### [Reference Example 184]

### Methyl (3,5-difluorophenyl)acetate (I-184):

10 ml of ethanol and 354 mg (2.90 mmol) of 4-(dimethylamino)pyridine were added to dichloromethane (100 ml) solution of 5.0 g (29.0 mmol) of (3,5-difluorophenyl)acetic acid. After cooling with ice, 6.68 g (34.9 mmol) of 1-(3-dimethylaminopropyl)-3-ethylcarbodiimide hydrochloride was added thereto, and stirred for 22 hours with graduallywarming up to room temperature. The reaction mixture was concentrated under reduced pressure, and ethyl acetate and 1 N hydrochloric acid were added to the residue. The organic layer separated was washed with aqueous saturated sodium bicarbonate solution and brine, and dried over anhydrous magnesium sulfate. After filtrating and concentrating under reduced pressure, 27.3 g (quantitative) of the entitled compound was obtained as a pale yellow oil.
MS(FAB)m/z:201(M⁺).
¹H-NMR(CDCl₃)δ: 1.27(3H, t, J=7.1Hz), 3.59(2H, s), 4.17(2H, q, J=7.1H), 6.67-6.87(3H, m).

### [Reference Example 185]

### Methyl 2-(3-cyanophenyl)-3-oxobutanoate (I-185):

Anhydrous tetrahydrofuran (100 ml) solution of 8.75 g (50 mmol) of methyl (3-cyanophenyl)acetate (I-182) was cooled to -30°C, and 25 ml (50 mmol) of heptane/tetrahydrofuran/ethylbenzene solution of 2 mol/liter lithium N,N-diisopropylamide was added dropwise thereto, taking 25 minutes. At the temperature, the resulting mixture was stirred for 1.5 hours, and anhydrous tetrahydrofuran (20 ml) solution of 1.2 ml (16 mmol) of acetyl chloride was added dropwise thereto, taking 30 minutes. At the temperature, the mixture was stirred for 2.5 hours. Aqueous saturated ammonium chloride solution was added to the reaction mixture, the mixture was extracted twice with ethyl acetate, and the organic layer was washed with brine and dried over anhydrous magnesium sulfate. After filtrating and concentrating under reduced pressure, the resulting residue was applied to a silica gel column chromatography. From the eluate with n-hexane/ethyl acetate (15/1, v/v), a mixture (8.0 g) of the entitled compound and the starting material (DFK-3) was obtained as a yellow oil.
MS(FAB)m/z:218(M+1)⁺.

### [Reference Example 186]

### Benzyl 2-(1-ethoxycarbonyl-2-oxopropyl)benzoate (I-186):

Anhydrous tetrahydrofuran (100ml) solution of 5.5 g (18.5 mmol) of benzyl 3-ethoxycarbonylmethylbenzoate (I-183) was cooled to -40°C, and 9.3 ml (18.6 mmol) of heptane/tetrahydrofuran/ethylbenzene solution of 2 mol/liter lithium N,N-diisopropylamide was added dropwise thereto, taking 15 minutes. At the temperature, the resulting mixture was stirred for 1.5 hours, and anhydrous tetrahydrofuran (20 ml) solution of 1.3 ml (18.5 mmol) of acetyl chloride was added dropwise thereto, taking 30 minutes. At the temperature, the mixture was stirred for 3.5 hours. Aqueous saturated ammonium chloride solution was added to the reaction mixture, the resulting mixture was extracted twice with ethyl acetate, and the organic layer was washed with brine and dried over anhydrous magnesium sulfate. After filtrating and concentrating under reduced pressure, the resulting residue was applied to a silica gel column chromatography. From the eluate with n-hexane/ethyl acetate (15/1, v/v) , a mixture (4.5 g) of the entitled compound and the starting material (I-183) was obtained as a yellow oil.
MS(FAB)m/z:341(M+1)⁺.

### [Reference Example 187]

### Ethyl 2-(3,5-difluorophenyl)-3-oxobutanoate (I-187):

Anhydrous tetrahydrofuran (40ml) solution of 2.00 g (10.0 mmol) of ethyl 2-(3,5-difluorophenyl)acetate (I-184) was cooled to -30°C, and 5.0 ml (10.0 mmol) of heptane/tetrahydrofuran/ethylbenzene solution of 2 mol/liter lithium N,N-dii sopropylamide was added dropwise thereto, taking 5 minutes. At the temperature, the resulting mixture was stirred for 30 minutes, and anhydrous tetrahydrofuran (10 ml) solution of 711 µl (10.0 mmol) of acetyl chloride was added dropwise thereto, taking 30 minutes. At the temperature, the mixture was stirred for 2.5 hours. Aqueous saturated ammonium chloride solution was added to the reaction mixture, the resulting mixture was extracted twice with ethyl acetate, and the organic layer was washed with brine and dried over anhydrous magnesium sulfate. After filtrating and concentrating under reduced pressure, the resulting residue was applied to a silica gel column chromatography. From the eluate with n-hexane/ethyl acetate (20/1, v/v) , 1.90 g (78 %) of the entitled compound was obtained as a yellow oil.
¹H-NMR(CDCl₃)δ: 1.15-1.32(3H, s), 1.58(0.62H, s), 1.88(1H, s), 2.23(0.2H, s), 3.59(1.2H, s), 4.12-4.28(2H, m), 6.64-6.99(3H, m)

### [Reference Example 188]

### Methyl 2-(3-methoxyphenyl)-3-oxobutanoate (I-188):

Anhydrous tetrahydrofuran (200 ml) suspension of 6.66 g (166 mmol) of 60 % oily sodium hydride was cooled to 0°C, and anhydrous tetrahydrofuran (200 ml) solution of 25.0 g (139 mmol) of methyl (3-methoxyphenyl) acetate was added dropwise thereto, taking 40 minutes. Then, the resulting mixture was stirred for 2 hours at room temperature. 16.5 ml (208 ml) of methyl acetate was added thereto at room temperature, and the resulting mixture was stirred overnight, and further stirred in an oil bath at 70 (C for 1 hour. After cooling, the reaction mixture was poured into aqueous saturated ammonium chloride solution, and made acidic with 1 N hydrochloric acid added thereto. The product was extracted with ethyl acetate, the organic layer was washed with brine, and dried over anhydrous magnesium sulfate. After filtrating and concentrating under reduced pressure, the resulting residue was applied to a silica gel column chromatography. From the eluate with n-hexane/ethyl acetate (10/1, v/v), 21.3 g (69 %) of the entitled compound was obtained as a yellow oil.
MS(FAB)m/z:223(M+1)+.
1H-NMR(CDCl3)δ: 1.86 and 2.18(3H, s each), 3.68, 3.57 and 3.80 (6.5H, seach), 6.70-6.93 (3H, m) , 7.21-7.32 (1H, m), 13. 0 (0.5 H, s).

### [Reference Example 189]

### 2-(3-Cyanophenyl)-3-methyl-1-oxobenzo[4,5]imidazo[1,2-a]pyridine-4-carbonitrile (I-189):

A mixture of 1.2 g (7.5 mmol) of 2-benzimidazolylacetonitrile, 4.0 g (mixture with I-182) of methyl 2-(3-cyanophenyl)-3-oxobutanoate (I-185) and 1.2 g (16 mmol) of ammonium acetate was heated with stirring in an oil bath at 140 to 150 (C for 2.5 hours. After cooling, the reaction mixture was applied to a silica gel column chromatography. From the eluate with chloroform/methanol (100/1, v/v), 0.78 g (30 %) of the entitled compound was obtained as a yellow-brown crystal.
MS(FAB)m/z:325(M+1)+.
1H-NMR(DMSO-d6)δ: 2.22(3H, s), 7.36(1H, m), 7.53(2H, m), 7.64(2H, m), 7.81(2H, m), 8.51(1H, m).

### [Reference Example 190]

### Benzyl 3-(4-cyano-3-methyl-1-oxobenzo[4,5]imidazo[1,2-a]pyridin-2-yl)benzoate (I-190):

A mixture of 2.0 g (13 mmol) of 2-benzimidazolylacetonitrile, 4.5 g (mixture with I-183) of 3-(1-ethoxycarbonyl-2-oxopropyl)benzoic acid (I-186) and 1.3 g (17 mmol) of ammonium acetate was heated with stirring in an oil bath at 140 to 150°C for 2.5 hours. After cooling, the reaction mixture was applied to a silica gel column chromatography. From the eluate with chloroform/methanol (100/1, v/v), 1.87 g (32 %) of the entitled compound was obtained as a pale brown crystal.
MS(FAB)m/z: 434(M+1)⁺.
¹H-NMR(DMSO-d₆)δ: 2.20(3H, s), 5.35(2H, s), 7.30-7.63(10H, m), 7.63(1H, s), 7.95(1H, m) , 8.52(1H, d, J=7.8Hz), 13.65(1H, brs).

### [Reference Example 191]

### 2-(3,5-Difluorophenyl)-3-methyl-1-oxobenzo[4,5]imidazo[1,2-a]pyridine-4-carbonitrile (I-191):

A mixture of 531 mg (3.38 mmol) of 2-benzimidazolylacetonitrile, 900 mg (3.72 mmol) of ethyl 2-(3,5-difluorophenyl)-3-oxobutanoate (I-187) and 547 mg (7.10 mmol) of ammonium acetate was heated with stirring in an oil bath at 140 to 150°C for 1.5 hours. After cooling, water and acetonitrile were added thereto, and the crystal formed was taken out through filtration, and dissolved in methanol (about 100 ml). This was processed with activated charcoal and filtered, and the filtrate was concentrated into about 6 ml under reduced pressure. The crystal thus formed was taken out through filtration, and dried under reduced pressure to obtain 228 mg (20 %) of the entitled compound as a dark brown crystal.
MS(FAB)m/z:336(M+1)⁺.
¹H-NMR(DMSO-d₆)δ: 2.25 (3H, s), 6.98-7.08 (2H, m), 7.17-7.28(1H, m), 7.34-7.40(1H, m), 7.50-7. 60 (2H, m) , 8.53(1H, d, J=8.1Hz), 13.7(0.7H, brs).

### [Reference Example 192]

### 2-(3-Methoxyphenyl)-3-methyl-1-oxobenzo[4,5]imidazo[1,2-a]pyridine-4-carbonitrile (I-192):

A mixture of 1.00 g (6.36 mmol) of 2-benzimidazolylacetonitrile, 2.12 g (9.54 mmol) of methyl 2-(3-methoxyphenyl)-3-oxobutanoate (I-188) and 547 mg (7.10 mmol) of ammonium acetate was heated with stirring in an oil bath at 140 to 150°C for 2.5 hours. After cooling, water was added thereto, and the crystal formed was taken out through filtration, and washed with acetonitrile. The crystal taken out through filtration was dried under reduced pressure to obtain 1.60 g (76 %) of the entitled compound as a grayish purple solid. MS(FAB)m/z:330(M+1)⁺.
¹H-NMR(DMSO-d₆)δ: 2.21 (3H, s), 3.76 (3H, s), 6.79-6.88(2H, m), 6.89-6.95(1H, m), 7.30-7.40(2H, m), 7.48-7.58(2H, m) , 8.53(1H, d, J=8.1Hz), 13.6(0.6H, brs).

### [Reference Example 193]

### 1-Chloro-2-(3-cyanophenyl)-3-methylbenzo[4,5]imidazo[1,2-a]pyridine-4-carbonitrile (I-193):

Phosphoryl chloride (10.0 ml) suspension of 0.65 g (2.0 mmol) of 2-(3-cyanophenyl)-3-methyl-1-oxobenzo[4,5]imidazo[1,2-a]pyridine-4-carbonitrile (I-189) was stirred under reflux for 3 hours. After cooling, the reaction mixture was concentrated under reduced pressure, ice-water was carefully added to the residue, and the mixture was extracted with chloroform. The organic layer was washed with brine, and dried over anhydrous magnesium sulfate. After filtrating and concentrating under reduced pressure, 983 mg (quantitative) of the entitled compound was obtained as a yellow-brown crystal.
MS(FAB)m/z:343(M+1)⁺.
¹H-NMR(CD₃OD)δ: 2.55(3H, s), 7.72-8.05 (7H, m) , 8.93(1H, d, J=4.4Hz).

### [Reference Example 194]

### Benzyl 3-(1-chloro-4-cyano-3-methylbenzo[4,5]imidazo[1,2-a]pyridin-2-yl)benzoate (I-194):

Phosphoryl chloride (20.0 ml) suspension of 1.8 g (4.2 mmol) of benzyl 3-(4-cyano-3-methyl-1-oxobenzo[4,5]imidazo[1,2-a]pyridin-2-yl)benzoate (I-190) was stirred under reflux for 3 hours. After cooling, the reaction mixture was concentrated under reduced pressure, ice-water was carefully added to the residue, and the mixture was extracted with chloroform. The organic layer was washed with brine, and dried over anhydrous magnesium sulfate. After filtrating and concentrating under reduced pressure, 1.94 g (quantitative) of the entitled compound was obtained as a yellow crystal.
¹H-NMR(CDCl₃)δ: 2.40(3H, s), 5.40(2H, s), 7.26-7.70(9H, m), 8.00(1H, m), 8.06(1H, d, J=8.1Hz), 8.24(1H, m), 8.54(1H, d, J=8.4Hz).

### [Reference Example 195]

### 1-Chloro-2-(3,5-difluorophenyl)-3-methylbenzo[4,5]imidazo[1,2-a]pyridin-4-carbonitrile (I-195):

Phosphoryl chloride (5.0 ml) suspension of 200 mg (0.596 mmol) of 2-(3,5-difluorophenyl)-3-methyl-1-oxobenzo[4,5]imidazo[1,2-a]pyridine-4-carbonitrile (I-191) was stirred under reflux for 3 hours. After cooling, the reaction mixture was concentrated under reduced pressure, ice-water was carefully added to the residue, and the mixture was extracted with chloroform. The organic layer was washed with brine, and dried over anhydrous magnesium sulfate. After filtrating and concentrating under reduced pressure, 219 mg (quantitative) of the entitled compound was obtained as a yellow crystal.
¹H-NMR(DMSO-d₆)δ: 2.35(3H, s), 7.21-7.30(2H, m), 7.42-7.51(2H, m), 7.63-7.74(1H, m) , 7.98(1H, d, J=7.3Hz), 8.64(1H, d, J=8.4Hz).

### [Reference Example 196]

### 1-Chloro-2-(3-methoxyphenyl)-3-methylbenzo[4,5]-imidazo[1,2-a]pyridin-4-carbonitrile (I-196):

Phosphoryl chloride (37 ml) suspension of 1.70 g (5.16 mmol) of 2-(3-methoxyphenyl)-3-methyl-1-oxobenzo[4,5]imidazo[1,2-a]pyridine-4-carbonitrile (I-192) was stirred under reflux for 1.5 hours. After cooling, the reaction mixture was concentrated under reduced pressure, ice-water was carefully added to the residue, and the mixture was extracted with chloroform. The organic layer was washed with brine, and dried over anhydrous magnesium sulfate. After filtrating and concentrating under reduced pressure, 1.69 g (94 %) of the entitled compound was obtained as a yellow-brown solid.
¹H-NMR(CDCl₃)δ: 2.43(3H, s), 3.87(3H, s), 6.81(1H, t, J=2.1Hz), 6.85(1H, d, J=7.5Hz), 7.05(1H, dd, J=2.4, 8.1Hz), 7.37-7.43(1H, m), 7.47(1H, t, J=8.0Hz), 7.58-7.64 (1H, m), 8.06(1H, d, J=7.8Hz), 8.56(1H, d, J=8.4Hz).

### [Example 148]

### 2-(3-Cyanophenyl)-1-[(3S)-dimethylaminopyrrolidin-1-yl]-3-methylbenzo[4,5]imidazo[1,2-a]pyridine-4-carbonitrile (#148):

To N,N-dimethylformamide (10.0 ml) solution of 300 mg (0.88 mmol) of 1-chloro-2-(3-cyanophenyl)-3-methylbenzo[4,5]imidazo[1,2-a]pyridine-4-carbonitrile (I-193) were added 0.28 ml (2.0 mmol) of triethylamine and 0.13 ml (1.0 mmol) of (3S)-dimethylaminopyrrolidine, and the resulting mixture was stirred in an oil bath at 70 to 80°C for 4.5 hours. After cooling, the reaction mixture was concentrated under reduced pressure, and the residue was dissolved in chloroform. The organic layer was washed with aqueous saturated sodium bicarbonate solution and brine, and dried over anhydrous magnesium sulfate. After filtrating and concentrating under reduced pressure, the resulting residue was applied to a silica gel column chromatography. From the eluate with chloroform/methanol (50/1, v/v) , 204 mg (49 %) of the entitled compound was obtained as a yellow-brown crystal.
MS(FAB)m/z:421(M+1)⁺.
¹H-NMR(CDCl₃)δ: 1.79-3.49 (7H, br) , 2.15 (6H, d, J=4.8Hz), 2.22 (3H, s), 7.25(1H, m), 7.57 (3H, m) , 7.72(1H, m), 7.83(1H, m), 7.99(2H, m).
IR(KBr): 2866, 2227, 1623, 1588, 1497, 1469, 1300 cm⁻¹.

| Elemental analysis: C₂₆H₂₄N₆·H₂O | | | |
|---|---|---|---|
| Calcd. | C, 71.21%; | H, 5.98%; | N, 19.16% |
| Found | C, 71.00%; | H, 5.54%; | N, 18.84%. |

### [Example 149]

### Benzyl 3-[4-cyano-1-[(3S)-dimethylaminopyrrolidin-1-yl]-3-methylbenzo[4,5]imidazo[1,2-a]pyridin-2-yl]benzoate (#149):

To N,N-dimethylformamide (10.0ml) solution of 1.9 g (4.2 mmol) of benzyl 3-(1-chloro-4-cyano-3-methylbenzo[4,5]imidazo[1,2-a]pyridin-2-yl)benzoate (I-194) were added 1.26 ml (9.0 mmol) of triethylamine and 0.57 ml (4.5 mmol) of (3S)-dimethylaminopyrrolidine, and the resulting mixture was stirred in an oil bath at 70 to 80°C for 5.5 hours. After cooling, the reaction mixture was concentrated under reduced pressure, and the residue was dissolved in chloroform. The organic layer was washed with aqueous saturated sodium bicarbonate solution and brine, and dried over anhydrous magnesium sulfate. After filtrating and concentrating under reduced pressure, the resulting residue was applied to a silica gel column chromatography. From the eluate with chloroform/methanol (50/1, v/v), 1. 69 g (77 %) of the entitled compound was obtained as a yellow-brown crystal.
MS(FAB)m/z:530(M+1)⁺.
¹H-NMR(CDCl₃)δ: 1.77-3.49(7H, br), 2.01(6H, s), 2.29(3H, s), 5.40(2H, m), 7.32-7.57(8H, m) , 7.65 (1H, m), 8.00 (3H, m), 8.22 (1H, d, J=7.8Hz).
IR(KBr): 2867, 2221, 1712, 1623, 1588, 1472, 1269 cm⁻¹.

| Elemental analysis: C₃₃H₃₁N₅O₂·0.25H₂O | | | |
|---|---|---|---|
| Calcd. | C, 74.21%; | H, 5.94%; | N, 13.11% |
| Found | C, 74.14%; | H, 5.88%; | N, 13.06%. |

### [Example 150]

### 2-(3,5-Difluorophenyl)-1-[(3S)-dimethylaminopyrrolidin-1-yl]-3-methylbenzo[4,5]imidazo[1,2-a]pyridine-4-carbonitrile (#150):

To N,N-dimethylformamide (20 ml) solution of 200 mg (0.565 mmol) of 1-chloro-2-(3,5-difluorophenyl)-3-methylbenzo[4,5]imidazo[1,2-a]pyridin-4-carbonitrile (I-195) were added 150 µl (1.13 mmol) of triethylamine and 86 µl (0.678 mmol) of (3S)-dimethylaminopyrrolidine, and the resulting mixture was stirred in an oil bath at 80 to 90°C for 9.5 hours. After cooling, the reaction mixture was concentrated under reduced pressure, and the residue was dissolved in chloroform. The organic layer was washed with aqueous saturated sodium bicarbonate solution and brine, and dried over anhydrous magnesium sulfate. After filtrating and concentrating under reduced pressure, the resulting residue was applied to a silica gel column chromatography. From the eluate with chloroform/methanol (100/1 to 98/2, v/v), 170 mg (70 %) of the entitled compound was obtained as a yellow crystal.
MS(FAB)m/z:432(M⁺).
¹H-NMR(CDCl₃)δ: 1.54-1.65(m, overlapped with H₂O peak), 2.08-2.20(7H, m), 2 .34 (3H, s) , 2.60-3.70(5H, brm), 6.80-6.88(2H, m), 6.95-7.04(1H, m), 7.33-7.39(1H, m), 7.53-7.60(1H, m), 7.95-8.07(2H, m).
IR(KBr) : 3040, 2982, 2955, 2868, 2823, 2778, 2222, 1621, 1592, 1499, 1464, 1443, 1410, 1352 cm⁻¹.

| Elemental analysis: C₂₅H₂₃F₂N₅·0.25H₂O | | | |
|---|---|---|---|
| Calcd. | C, 68.87%; | H, 5.43%; | N, 16.06% |
| Found | C, 68.82%; | H, 5.27%; | N, 16.00%. |

### [Example 151]

### 1-[(3S)-Dimethylaminopyrrolidin-1-yl]-2-(3-methoxyphenyl)-3-methylbenzo[4,5]imidazo[1,2-a]pyridine-4-carbonitrile (#151):

To N,N-dimethylformamide (32 ml) solution of 1.59 g (4.57 mmol) of 1-chloro-2-(3-methoxyphenyl)-3-methylbenzo[4,5]imidazo[1,2-a]pyridin-4-carbonitrile (I-196) were added 1.27 ml (9.14 mmol) of triethylamine and 638 µl (5.03 mmol) of (3S)-dimethylaminopyrrolidine, and the resulting mixture was stirred in an oil bath at 80 to 90°C for 1.5 hours. After cooling, the reaction mixture was concentrated under reduced pressure, and the residue was dissolved in chloroform. The organic layer was washed with aqueous saturated sodium bicarbonate solution and brine, and dried over anhydrous magnesium sulfate. After filtrating and concentrating under reduced pressure, the resulting residue was applied to a silica gel column chromatography. From the eluate with chloroform/methanol (10/1 to 100/1, v/v), 1.84g (94 %) of the entitled compound was obtained as a yellow crystal.
MS(FAB)m/z:426(M+1)⁺.
¹H-NMR(CDCl₃)δ: 1.5-2.2(3H, br), 2.14 and 2.15(6H, s each) , 3.33(3H, s), 2.8-3.6(4H, br), 3.97(3H, s), 6.79 (1H, brs), 6.84(1H, d, J=7.5Hz), 7.02(1H, dd, J=2.7, 8.4Hz), 7.33(1H, t, J=7.8Hz), 7.45(1H, t, J=7.8Hz), 7.50-7.56(1H, m), 7.8-8.2(1H, brs), 8.01(1H, d, J=8.1Hz).
IR(KBr): 2948, 2866, 2770, 2221, 1589, 1474, 1442, 1298 cm⁻¹.

| Elemental analysis: C₂₆H₂₇N₅O·H₂O | | | |
|---|---|---|---|
| Calcd. | C, 70.41%; | H, 6.59%; | N, 15.79% |
| Found | C, 70.45%; | H, 6.11%; | N, 15.68%. |

### [Example 152]

### 3-[4-Cyano-1-[(3S)-dimethylaminopyrrolidin-1-yl]-3-methylbenzo[4,5]imidazo[1,2-a]pyridin-2-yl]benzoic acid (#152):

1.4 g (2.6 mmol) of benzyl 3-[4-cyano-1-[(3S)-dimethylaminopyrrolidin-1-yl]-3-methylbenzo[4,5]imidazo[1,2-a]pyridin-2-yl]benzoate (#149) was dissolved in amixed solvent of methanol /tetrahydrofuran/chloroform (1/1/2, v/v/v) (60 ml), and 5 % palladium-carbon catalyst (0.84 g) was added thereto and the resulting mixture was stirred in hydrogen of 6 atmospheres (initial pressure) for 1 hour. At the end of the reaction, a crystal was partly precipitated. Therefore, methanol and water were added to dissolve it. Then, this was filtered, and the filtrate was concentrated under reduced pressure. The residue was dissolved in aqueous sodium hydroxide solution, and the insoluble material was removed through filtration through a membrane filter. The filtrate was made to have pH of 7 with 1 N hydrochloric acid added thereto, and then concentrated under reduced pressure. The resulting residue was purified through an ODS column (30 % methanol/aqueous 0.1 % acetic acid solution to 80 % methanol/aqueous 0.1 % acetic acid solution), and the resulting mixture was concentrated under reduced pressure to obtain 305 mg (26 %) of an acetic acid salt of the entitled compound as a yellow crystal.
HRMS(FAB)m/z:440.2083(Calcd for C₂₆H₂₆N₅O₂ 440.2087).
¹H-NMR(DMSO-d₆)δ: 1.85(3H, s), 1.94(s), 2.19(6H, s), 1.68-3.86(br), 7.34(1H, t, J=7.5Hz), 7.54(1H, t, J=7.5Hz), 7.69(2H, m), 7.97(4H, m).
IR(KBr): 2874, 2223, 1706, 1624, 1590, 1474, 1299 cm⁻¹.

| Elemental analysis: C₂₆H₂₅N₅O₂·CH₃CO₂H | | | |
|---|---|---|---|
| Calcd. | C, 67.32%; | H, 5.85%; | N, 14.02% |
| Found | C, 67.63%; | H, 5.86%; | N, 14.59% |

### [Example 153]

### 3-[4-Cyano-1-[(3S)-dimethylaminopyrrolidin-1-yl]-3-methylbenzo[4,5]imidazo[1,2-a]pyridin-2-yl]-N,N-dimethylbenzamide (#153):

To N,N-dimethylformamide (5 ml) solution of 44 mg (0.1 mmol) of 3-[4-cyano-1-[(3S)-dimethylaminopyrrolidin-1-yl]-3-methylbenzo[4,5]imidazo[1,2-a]pyridin-2-yl]benzoic acid (#152) were added 84 µl (0.6 mmol) of triethylamine, 27 mg (0.2 mmol) of 1-hydroxybenzotriazole hydrate, 41 mg (0.5 mmol) of dimethylamine hydrochloride and 58 mg (0.3 mmol) of 1-(3-dimethylaminopropyl)-3-ethylcarbodiimide hydrochloride, and the resulting mixture was stirred overnight at room temperature. The reaction mixture was concentrated under reduced pressure, and the residue was dissolved in chloroform. The organic layer was washed with aqueous saturated sodium bicarbonate solution and brine, and dried over anhydrous magnesium sulfate. After filtrating and concentrating under reduced pressure, the resulting residue was applied to a silica gel column chromatography. From the eluate with chloroform/methanol (20/1, v/v), 40 mg (85 %) of the entitled compound was obtained as a yellow crystal.
MS(FAB)m/z:467(M⁺).
¹H-NMR(CDCl₃)δ: 1.60-3.38(br), 2.13 and 2.14 (6H, s, each), 2.28, 2.29and2.30(3H, s, each), 3.07 and 3.15(6H, brs, each), 7.32(3H, m), 7.57(3H, m), 7.99(2H, d, J=8.1Hz).
IR(KBr): 2948, 2360, 2222, 1636, 1498, 1472, 1300, 1186 cm⁻¹.

| Elemental analysis: C₂₈H₃₀N₆O·1.5H₂O | | | |
|---|---|---|---|
| Calcd. | C, 68.13%; | H, 6.74%; | N, 17.03% |
| Found | C, 68.49%; | H, 6.31%; | N, 16.66%. |

### [Example 154]

### 2-(3-Hydroxyphenyl)-1-[(3S)-dimethylaminopyrrolidin-1-yl] 3-methylbenzo[4,5]imidazo[1,2-a]pyridine-4-carbonitrile (#154):

To chloroform/acetonitrile/toluene (3/1/1, v/v/v) mixed solution (150 ml) of 1.53 g (3.60 mmol) of 1-[(3S)-dimethylaminopyrrolidin-1-yl]-2-(3-methoxyphenyl)-3-methylbenzo[4,5]imidazo[1,2-a]pyridine-4-carbonitrile (#151) was added 5.12 ml (36.0 mmol) of trimethylsilyl iodide, and the resulting mixture was stirred overnight at room temperature. On the next day, since the starting material was precipi tated, the reaction mixture was heated at 60°C and stirred for 6.5 hours (even though it was heated or a solvent was added thereto, it did not dissolve). The reaction mixture was poured into methanol, well shaken, and concentrated under reduced pressure. Aqueous saturated sodium bicarbonate solution and sodium thiosulfate were added thereto, and the mixture was extracted with chloroform. The organic layer was washed with brine, and dried over anhydrous magnesium sulfate. After filtrating and concentrating under reduced pressure, the resulting residue was applied to a silica gel column chromatography. A fraction obtained from the eluate with chloroform/methanol (50/1 to 100/3 to 10/1, v/v) was concentrated under reduced pressure, and further applied to a silica gel column chromatography. From the eluate with chloroform/methanol (100/1 to 100/3 to 10/1, v/v) , 147 mg (10 %) of an orange solid was obtained. The sample thus obtained would be a zwitterion, it was processed with 4 N hydrochloric acid/1,4-dioxane, the mixture was concentrated, and the resulting residue was suspended in diethyl ether, taken out through filtration and dried to obtain the entitled compound as an orange solid.
HRMS(FAB)m/z:412.2176(Calcd for C₂₅H₂₆ON₅ 412.2137).
¹H-NMR(CD₃OD)δ: 1.80-3.95(7H, br), 2.50(3H, s), 2.75(3H, brs), 2.87(3H, brs), 6.93 (2H, brs), 7.03-7.07(1H, m), 7.47-7.52(1H, m), 7.75-7.96(3H, m), 8.10-8.45(1H, brs).

### [Reference Example 197]

### 2-(3H-imidazo[4,5-b]pyridin-2-yl)acetonitrile (I-197):

A mixture of 10.0 g (89.9 mmol) of 2,3-diaminopyridine and 15.4 ml (135 mmol) of ethyl cyanoacetate was stirred under heating in an oil bath at 180 to 190°C for 25 minutes. After cooling to room temperature, diethyl ether, ethyl acetate and methanol were added to the reaction mixture, and the crystal formed was taken out through filtration. The crystal thus taken out through filtration was dissolved in N,N-dimethylformamide (about 400 ml) at 60 to 70°C, and the processed with activated charcoal. After filtration, the filtrate was concentrated under reduced pressure to obtain a pale brown solid. The solid was dissolved in warmed methanol (about 15000 ml), processed with activated charcoal and filtered, and the filtrate was concentrated to about 200 ml under reduced pressure. The crystal formed was taken out through filtration, and dried under reduced pressure to obtain 8.42 g (59 %) of the entitled compound as a colorless crystal.
MS(FAB)m/z:159(M+1)⁺.
¹H-NMR(CDCl₃)δ: 4.42(2H, s), 7.18-7.26(1H, m), 7.85-8.06(1H, m), 8.24-8.42(1H, m), 12.78-12.94(0.3H, brs), 13.10-13.30(0.5H, brs).

### [Reference Example 198]

### 7-n-Butyl-8-methyl-6-oxodipyrido[1,2-a;2',3'-d]imidazole-9-carbonitrile (I-198):

A mixture of 2.45 g (13.2 mmol) of 2-(3H-imidazo[4,5-b]pyridin-2-yl)acetonitrile (I-197), 2.45 g (13.2 mmol) of ethyl 2-acetylhexanoate and 2.02 g (26.2 mmol) of ammonium acetate was stirred under heating in an oil bath at 140 to 150°C for 1 hour. After the reaction mixture was cooled to room temperature, water and acetonitrile were added thereto, and the crystal formed was taken out through filtration. This crystal was dissolved in warmed mixed solvent of N,N-dimethylformamide/methanol (1/4, v/v) (about 500 ml), and then cooled to room temperature. The crystal formed was taken out through filtration, washed with methanol and diethyl ether, and dried under reduced pressure to obtain 2.33 g (66 %) of the entitled compound as a green solid.
MS(FAB)m/z:281(M+1)⁺.
¹H-NMR(DMSO-d₆)δ: 0.90(3H, t, J=7.1Hz), 1.26-1.48(4H, m), 2.39(3H, s), 2.50-2.62(m), 2.82-2.95(2H, m), 7.29-7.35(1H, m) , 8.36-8.41(1H, m), 8.78-8.83(1H, m).

### [Reference Example 199]

### 8-Methyl-7-(2-methylthiazol-4-yl)-6-oxodipyrido[1,2-a;2',3'-d]imidazole-9-carbonitrile (I-199):

A mixture of 500 mg (3.16 mmol) of 2-(3H-imidazo[4,5-b]pyridin-2-yl)acetonitrile (I-197), 790 mg (3.48 mmol) of ethyl 2-(2-methylthiazol-4-yl)-3-oxobutyrate (I-127) and 487 mg (6.32 mmol) of ammonium acetate was stirred under heating in an oil bath at 140 to 150°C for 1 hour. After the reaction mixture was cooled to room temperature, water and methanol were added thereto, and the crystal formed was taken out through filtration. This crystal was washed with methanol and diethyl ether, and dried under reduced pressure to obtain 688 mg (68 %) of the entitled compound as a dark brown solid.
MS(FAB)m/z:322(M+1)⁺.
¹H-NMR(DMSO-d₆)δ: 2.31 (3H, s), 2.69 (3H, s), 7.33-7.38(1H, m), 7.50(1H, s), 8.42(1H, d, J=5.5Hz), 8.81(1H, d, J=7.9Hz).

### [Reference Examples 200 to 201]

### Mixture of 8-methyl-6-oxo-7-phenyldipyrido[1,2-a;2',3'-d]imidazole-9-carbonitrile (I-200), and 7-methyl-9-oxo-8-phenyldipyrido[1,2-a;2',3'-d]imidazole-6-carbonitrile (I-201):

A mixture of 3.20 g (20.2 mmol) of 2-(3H-imidazo[4,5-b]pyridin-2-yl)acetonitrile(I-197), 4.58 g (22.2mmol) of ethyl 2-phenylacetoacetate, and 3.11 g (40.3 mmol) of ammonium acetate was stirred under heating in an oil bath at 140 to 150°C for 1 hour. After the reaction mixture was cooled to room temperature, water and acetonitrile were added thereto, and the crystal formed was taken out through filtration. This crystal was dried under reduced pressure to obtain 5.52 g (91 %) of the entitled compound (mixture of I-200 and I-201) as a yellow solid. Neither separated nor purified, the product was used in the next reaction.
MS(FAB)m/z:301(M+1)⁺.

### [Reference Example 202]

### 7-n-Butyl-6-chloro-8-methyldipyrido[1,2-a;2',3'-d]imidazole -9-carbonitrile (I-202):

Phosphoryl chloride (7 ml, 75.0 mmol) suspension of 500 mg (1.78 mmol) of 7-n-butyl-8-methyl-6-oxodipyrido[1,2-a;2',3'-d]imidazole-9-carbonitrile (I-198) was stirred under reflux for 3.5 hours. After cooling, the reaction mixture was concentrated under reduced pressure, ice-water was carefully added to the residue, and aqueous saturated sodium carbonate solution and chloroform were added thereto, and the mixture was stirred. The organic layer was collected, washed with brine and dried over anhydrous magnesium sulfate. After filtration, the filtrate was concentrated under reduced pressure, and the resulting residue was dissolved in methanol. This was processed with activated charcoal, filtered, and washed with hot methanol, and the combined filtrates were concentrated under reduced pressure. The resul ting residue was dried to obtain 158 mg (30 %) of the entitled compound as a brown solid.
MS(FAB)m/z:299(M+1)⁺.
¹H-NMR(CDCl₃)δ: 1.03 (3H, t, J=7.1Hz), 1.45-1.66(4H, m), 2.74(3H, s), 7.35-7.43(1H, m), 8.83-8.92(2H, m)

### [Reference Example 203]

### 6-Chloro-8-methyl-7-(2-methylthiazol-4-yl)dipyrido[1,2-a;2',3'-d]imidazole-9-carbonitrile (I-203):

Phosphoryl chloride (30 ml) suspension of 300 mg (0.99 mmol) of 8-methyl-7-(2-methylthiazol-4-yl)-6-oxodipyrido[1,2-a;2',3'-d]imidazole-9-carbonitrile (I-199) was stirred under reflux for 3.5 hours. After cooling, the reaction mixture was concentrated under reduced pressure, ice-water was carefully added to the residue, and aqueous saturated sodium carbonate solution was added thereto, and the insoluble material was removed through filtration. This was washed with water, and dried under reduced pressure to obtain a crude product of the entitled compound. This compound was directly used in the next reaction.
MS(FAB)m/z:340(M+1)⁺.

### [Reference Examples 204 to 205]

### 6-Chloro-8-methyl-7-phenyldipyrido[1,2-a;2',3'-d]imidazole-9-carbonitrile (I-209), and 9-Chloro-7-methyl-8-phenyldipyrido[1,2-a;2',3'-d]imidazole-6-carbonitrile (I-205):

Phosphoryl chloride (77 ml) suspension of 5.50 g (18.3 mmol) of the mixture of 8-methyl-6-oxo-7-phenyldipyrido[1,2-a;2',3'-d]imidazole-9-carbonitrile (I-200) and 7-methyl-9-oxo-8-phenyldipyrido[1,2-a;2',3'-d]imidazole-6-carbonitrile (I-201) was stirred under reflux for 5.5 hours. After cooling, the reaction mixture was concentrated under reduced pressure, ice-water was carefully added to the residue, and aqueous 10 % sodium bicarbonate solution and methanol were added thereto, and the mixture was stirred. The crystal formed was taken out through filtration, and applied to a silica gel column chromatography. From the eluate with chloroform/methanol (100/1 to 50/1, v/v), 532 mg (9.1 %) of the entitled compound (I-205) was obtained as a yellow solid; and 3.28 g (56 %) of the entitled compound (I-204) was as a yellow solid.
I-204:
MS(FAB)m/z:319(M+1)⁺.
¹H-NMR (CDCl₃) δ: 2.45 (3H, s), 7.22-7.38 (3H, overlapped with CHCl₃ peak), 7.50-7.63(3H, m), 8.80(1H, dd, J=1.5, 8.7Hz), 8.88(1H, dd, J=1.5, 4.5Hz).
I-205:
HRMS(FAB)m/z:319.0747 (Calcd for C₁₈H₁₂N₄Cl 319.0750).
¹H-NMR(CDCl₃)δ: 2.44 (3H, s) , 7.22-7.32 (2H, overlapped with CHCl₃ peak), 7.50-7.63(4H, m), 8.36(1H, dd, J=1.5, 8.4Hz), 8.60(1H, dd, J=1.5, 4.5Hz).

### [Reference Example 206]

### 2,3-Diamino-4-methylpyridine (I-206):

7.5 g of 5 % palladium-carbon catalyst (water content 50 %) was added to acetic acid (300 ml) solution of 15.0 g (97.9 mmol) of 2-amino-4-methyl-3-nitropyridine, and the resulting mixture was stirred under atmospheric pressure of hydrogen at room temperature for 3 hours. After filtrating and concentrating under reduced pressure, the residue was dissolved in aqueous 10 % sodium carbonate solution and concentrated under reduced pressure. The residue was dissolved in mixed solution of chloroform/methanol (3/1, v/v) , and the insoluble material was removed through filtration. This was applied to a silica gel column chromatography. From the eluate with chloroform/methanol (20/1, v/v) , a pink-brown solidwas obtained. From the ¹H-NMR, since the presence of acetic acid therein was confirmed, this solid was again dissolved in aqueous 10 % sodium carbonate solution and concentrated under reduced pressure. The residue was dissolved in chloroform, dried over anhydrous magnesium sulfate and filtered, and the filtrate was concentrated under reduced pressure and the resulting residue was dried to obtain 8.13 g (67 %) of the entitled compound as a yellow-brown solid.
MS(FAB)m/z:124(M+1)⁺.
¹H-NMR(CD₃OD)δ: 2.14(3H, s) , 6.46(2H, t, J=5.4Hz) , 7.27(2H, t, J=5.4Hz).

### [Reference Example 207]

### [7-Methyl-2-(3H-imidazo[4,5-b]pyridine)-2-yl]acetonitrile (1-207):

A mixture of 7.83 g (63.6 mmol) of 2,3-diamino-4-methylpyridine (I-206) and 10.1 ml (95.4 mmol) of ethyl cyanoacetate was stirred under heating in an oil bath at 180 to 190°C for 25 minutes. After dissolved, the reaction mixture became dark tar, and when left cooled, it solidified. The reaction mixture was dissolved in mixed solution of chloroform/methanol (7/3, v/v), the insoluble material was removed through filtration, and the filtrate was concentrated under reducedpressure. The residue was applied to a short silica gel chromatography, and the eluate with chloroform/methanol (4/1, v/v) was concentrated under reduced pressure. The resulting residue was suspended and washed in diethyl ether, and suspended and washed in acetoni trile, and then this was dried under reduced pressure to obtain 7.16 g (66 %) of the entitled compound as a yellow-brown solid.
MS(FAB)m/z:173(M+1)⁺.
¹H-NMR(DMSO-d₆)δ: 2.52(3H, s, overlapped with DMSO peak), 4.40(2H, s), 7.05(1H, d, J=4.8Hz), 8.09-8.25(1H, m), 12.8-13.2(1H, m).

### [Reference Example 208]

### 4,7-Dimethyl-9-oxo-8-phenyldipyrido[1,2-a;3',2'-d]imidazole -6-carbonitrile (I-208):

A mixture of 3.0 g (16.7 mmol) of [7-methyl-2-(3H-imidazo[4,5-b]pyridin)-2-yl]acetonitrile (I-207), 3.80 g (18.4 mmol) of ethyl 2-phenylacetoacetate and 2.58 g (33.5 mmol) of ammonium acetate was heated in an oil bath at 140 to 150(C for 1 hour. The reaction mixture solidified in about 20 minutes. After cooling, the reaction mixture was suspended and washed twice with acetonitrile, taken out through filtration, and dried under reduced pressure to obtain 4.21 g (80 %) of the entitled compound as an ocher solid.
MS(FAB)m/z:315(M+1)+.
1H-NMR(DMSO-d6)δ: 2.13(3H, s) , 2.56 (3H, s), 7.12(1H, d, J=4.5Hz), 7.20-7.28(3H, m), 7.32-7.38(2H, m), 7.97(1H, d, J=4.5Hz).

### [Reference Example 209]

### 9-Chloro-4,7-dimethyl-8-phenyldipyrido[1,2-a;3',2'-d]imidazole-6-carbonitrile (I-209):

Phosphoryl chloride (100 ml) suspension of 5.06 g (16.1 mmol) of 4,7-dimethyl-9-oxo-8-phenyldipyrido[1,2-a;3',2'-d]imidazole-6-carbonitrile (I-208) was stirred under reflux for 3 hours. After cooling, the reaction mixture was concentrated under reduced pressure, ice-water was carefully added to the residue, and the mixture was poured into aqueous saturated sodium bicarbonate solution. The mixture was extracted with chloroform, and the organic layer collected was washed with brine and dried over anhydrous magnesium sulfate. After filtration, the filtrate was concentrated under reduced pressure, and the resulting residue was applied to a short silica gel chromatography. From the eluate with chloroform/methanol (20/1, v/v), 4.48 g (84 %) of the entitled compound was obtained as a yellow-orange solid.
1H-NMR(CDCl3)δ: 2.42(3H, s), 2.85(3H, s), 7.26-7.30(2H, m), 7.39(1H, d, J=4.8Hz), 7.49-7.60(3H, m), 8.46(1H, d, J=4.8Hz).

### [Reference Example 210]

### 2-Amino-6-methyl-3-nitropyridine (I-210):

25.0 g (231 mmol) of 2-amino-6-picoline was cooled to -15(C, and dissolved very carefully in concentrated sulfuric acid (100 ml). This solution was cooled to 0(C, and 22.0 ml (60 %, d = 1.42, 347 mmol) of nitric acid was added dropwise thereto. After the addition, the ice-water bath was removed, and after the heat generation was stopped, the reaction mixture was stirred at room temperature for 2 hours. Thereactionmixture was poured into ice (400 g), and the mixture was controlled to have pH of from 4 to 5 with aqueous 4 N sodium hydroxide solution added thereto. The precipitate formed was taken out through filtration, and washed with hot water. This was dried, and applied to a silica gel column chromatography. From the eluate with chloroform/methanol (50/1, v/v), 7.60 g (22 %) of the entitled compound was obtained as a yellow solid.
¹H-NMR(DMSO-d₆)δ: 2.37(3H, s), 6.61(1H, d, J=8.7Hz), 7.86(2H, brs), 8.24(1H, d, J=8.7Hz).

### [Reference Example 211]

### 2,3-Diamino-6-methylpyridine (I-211):

7.50 g of 5 % palladium-carbon catalyst (water content 50 %) was added to methanol (300 ml) solution of 14.9 g (97.0 mmol) of 2-amino-6-methyl-3-nitropyridine (I-210), and the resulting mixture was stirred under atmospheric pressure of hydrogen at room temperature for 2 hours. The reaction mixture was filtered, and the filtrate was concentrated under reduced pressure, and the resulting residue was dried to obtain 11.8 g (quantitative) of the entitled compound as an ocher solid. ¹H-NMR(DMSO-d₆)δ: 2.11(3H, s), 4.38(2H, brs), 5.26(2H, brs), 6.18(1H, d, J=7.5Hz), 6.59(1H, d, J=7.5 Hz).

### [Reference Example 212]

### (5-Methyl-1H-imidazo[4,5-b]pyridin-2-yl)acetonitrile (I-212):

A mixture of 11.8 g (96.0 mmol) of 2,3-diamino-6-methylpyridine (I-211) and 15.3 ml (144 mmol) of ethyl cyanoacetate was stirred under heating in an oil bath at 180 to 190°C for 8 minutes. After dissolved, the reaction mixture became a black-brown solid. The reaction mixture was kept cooled, suspended and washed with methanol added thereto, filtered, and dried to obtain 13.4 g (81 %) of the entitled compound as a gray-brown solid.
¹H-NMR(DMSO-d₆)δ: 2.53(3H, s, overlapped with DMSO peak), 4.34(2H, s), 7.05(1H, d, J=8.1Hz), 7.81(1H, d, J=8.1Hz), 12.9(0.8H, brs).

### [Reference Example 213]

### 6-Hydroxy-2,8-dimethyl-7-phenyldipyrido[1,2-a;2',3' d]imidazole-9-carbonitrile triethylamine salt (I-213):

A mixture of 13.8 g (80.4 mmol) of (5-methyl-1H-imidazo[4,5-b]pyridin-2-yl)acetonitrile (I-212), 18.2 g (88.4 mmol) of ethyl 2-phenylacetoacetate and 12.7 g (165 mol) of ammonium acetate was stirred under heating in an oil bath at 140 to 150°C for 50 minutes. The reaction mixture solidified in about 15 minutes. After cooling, water was added to the reaction mixture, and the mixture was suspended and washed. After filtrating and drying under reduced pressure, the resulting residue was applied to a silica gel column chromatography. The solid obtained from the eluate with chloroform/methanol/triethylamine (30/1/1, v/v/v) was washed with diethyl ether and dried under reduced pressure to obtain 17.5 g (52 %) of the entitled compound as a yellow solid.
¹H-NMR(DMSO-d₆)δ: 1.11(9H, t, J=7.2 Hz), 2.13(3H, s), 2.49(3H, s, overlapped with DMSO peak), 3.34 (6H, q, J=7.2Hz), 6.83(1H, d, J=7.8Hz), 7.18-7.25(3H, m), 7.30-7.35(2H, m), 8.40(1H, d, J=7.8Hz).

### [Reference Example 214]

### 6-Chloro-2,8-dimethyl-7-phenyldipyrido[1,2-a;2',3'-d]imidazole-9-carbonitrile (I-214):

Phosphoryl chloride (195 ml) suspension of 10.0 g (24.1 mmol) of 6-hydroxy-2,8-dimethyl-7-phenyldipyrido[1,2-a;2',3'-d]imidazole-9-carbonitrile triethylamine salt (I-213) was stirred under reflux for 6 hours. After cooling, the reaction mixture was concentrated under reduced pressure, ice-water was carefully added to the residue, and the mixture was suspended and washed with aqueous 10 % sodium carbonate solution. After taken out through filtration, this was washed with water and dried under reduced pressure to obtain 7.97 g (quantitative) of the entitled compound as a yellow-green solid.
¹H-NMR(CDCl₃)δ: 2.43 (3H, s), 2.79(3H, s), 7.22(1H, d, J=8.7Hz) , 7.26-7.32(2H, m), 7.50-7.61(3H, m), 8.65(1H, d, J=8.7Hz).

### [Example 155]

### 7-n-Hutyl-6-[(3S)-dimethylaminopyrrolidin-1-yl]-8-methyldipyrido[1,2-a;2',3'-d]imidazole-9-carbonitrile (#155):

To N,N-dimethylformamide (5 ml) solution of 158 mg (0.529 mmol) of 7-n-butyl-6-chloro-8-methyldipyrido[1,2-a;2',3'-d]imidazole-9-carbonitrile (I-202) and 72.5 mg (0.635 mmol) of (3S)-dimethylaminopyrrolidine was added 140 µl (1.06 mmol) of triethylamine, and the resulting mixture was stirred in an oil bath at 80 to 90°C for 6.5 hours. After cooling, the reaction mixture was concentrated under reduced pressure, and the resulting residue was applied to a silica gel column chromatography. From the eluate with chloroform/methanol/triethylamine (100/1/1, v/v/v), 114 mg (57 %) of the entitled compound was obtained as a pale yellow crystal.
MS(FAB)m/z:377(M+1)⁺.
¹H-NMR(CDCl₃)δ: 1.04(3H, t, J=6.8Hz), 1.47-1.66(4H, m), 2.11-2.50(8H, m), 2.59-2.73(5H, m), 2.96-3.08(1H, m), 3.14-3.75(4H, m), 7.23-7.30(1H, m), 8.14-8.53(1H, m), 8.76-8.79(1H, m).
IR(KBr) : 2955, 2867, 2818, 2764, 2225, 1622, 1590, 1566, 1526, 1499, 1466 cm⁻¹.

| Elemental analysis: C₂₂H₂₈N₆ | | | |
|---|---|---|---|
| Calcd. | C, 70.18%; | H, 7.50%; | N, 22.32% |
| Found | C, 69.98%; | H, 7.52%; | N, 22.24%. |

### [Example 156]

### 6-[(3S)-Dimethylaminopyrrolidin-1-yl]-8-methyl-7-(2-methylthiazol-4-yl)dipyrido[1,2-a;2',3'-d]imidazole-9-carbonitrile (#156):

To N,N-dimethylformamide (15 ml) solution of 74.6 mg (0.933 mmol) of 6-chloro-8-methyl-7-(2-methylthiazol-4-yl)dipyrido[1,2-a;2',3'-d]imidazole-9-carbonitrile (I-203) and 118 µl (0.933mol) of (3S)-dimethylaminopyrrolidine was added 284 µl (1.87 mmol) of triethylamine, and the resulting mixture was stirred in an oil bath at 80 to 90°C for 6.5 hours. After cooling, the reaction mixture was concentrated under reduced pressure, and aqueous 10 % sodium carbonate solution was carefully added thereto with cooling with ice. The solid thus formed was taken out through filtration and dried under reduced pressure. The resulting residue was applied to a silica gel column chromatography. From the eluate with chloroform/methanol/triethylamine (100/1/0.5 to 100/1/1 to 100/2/1, v/v/v), 131 mg (34 %) of the entitled compound was obtained as a yellow crystal.
MS(FAB)m/z:918 (M+1)⁺.
¹H-NMR(CDCl₃)δ: 1.50-1.70(1H, overlapped with H₂O peak), 1.70-1.90(1H, m), 2.05-2.23(7H, m), 2.37(3H, s), 2.40-2.75(1H, m), 2.83(3H, s), 2.86-3.44 (3H, m) , 7.22-7.32 (2H, m, overlapped with CHCl₃ peak), 8.31(1H, brm), 8.76(1H, dd, J=1.5, 4.8Hz). IR(KBr): 3404, 3081, 2979, 2950, 2868, 2820, 2773, 2224, 1618, 1588, 1567, 1492, 1464, 1404, 1368 cm⁻¹.

| Elemental analysis: C₂₂H₂₃N₇S·0.75 H₂O | | | |
|---|---|---|---|
| Calcd. | C, 61.30%; | H, 5.73%; | N, 22.75% |
| Found | C, 61.32%; | H, 5.63%; | N, 22.85%. |

### [Example 157]

### 6-[(3S)-Dimethylaminopyrrolidin-1-yl]-8-methyl-7-phenyldipyrido[1,2-a;2',3'-d]imidazole-9-carbonitrile (#157):

To N,N-dimethylformamide (30 ml) solution of 470 mg (1.47 mmol) of 6-chloro-8-methyl-7-phenyldipyrido[1,2-a;2',3'-d]imidazole-9-carbonitrile (I-204) and 202 mg (1.77 mmol) of (3S)-dimethylaminopyrrolidine was added 390 µl (2.94 mmol) of triethylamine, and the resulting mixture was stirred in an oil bath at 80 to 90°C for 6.5 hours. After cooling, the solid formed was taken out through filtration, warmed methanol was added thereto and the mixture was stirred. Then, this was kept static at room temperature. The crystal was taken out through filtration, and recrystallized and purified from chloroform/diethyl ether, and dried to obtain 223 mg (27 %) of the entitled compound as a yellow crystal.
HRMS (FAB)m/z:397.2177 (Calcd for C₂₄H₂₅N₆ 397.2141).
¹H-NMR(CDCl₃)δ: 1.96-2.25(9H, m), 2.25-2.35 (4H, m), 2.55-3.70(3H, brm), 7.20-7.35(3H, m), 7.48-7.62(3H, m), 8.10-8.50(1H, brm), 8.74-8.78(1H, m).
IR(KBr): 2975, 2949, 2853, 2823, 2776, 2227, 1621, 1583, 1567, 1488, 1471, 1407, 1368 cm⁻¹.

| Elemental analysis: C₂₄H₂₄N₆·0.2.5H₂O | | | |
|---|---|---|---|
| Calcd. | C, 71.89%; | H, 6.16%; | N, 20.96% |
| Found | C, 72.28%; | H, 6.01%; | N, 21.07%. |

### [Example 158]

### 8-Methyl-6-[(3S)-methylaminopyrrolidin-1-yl]-7-phenyldipyrido[1,2-a;2',3'-d]imidazole-9-carbonitrile (#158):

To N,N-dimethylformamide (150ml) solution of 2.50 g (7.84 mmol) of 6-chloro-8-methyl-7-phenyldipyrido[1,2-a;2',3'-d]imidazole-9-carbonitrile (I-204) and 943 mg (9.41 mmol) of (3S)-methylaminopyrrolidine was added 2.08 ml (15.7 mmol) of triethylamine, and the resulting mixture was stirred in an oil bath at 80 to 90°C for 3.5 hours. After it was cooled and concentrated, aqueous 10 % sodium carbonate solution was added thereto with cooling with ice, and the mixture was extracted with chloroform. The organic layer collected was washed with brine and dried over anhydrous magnesium sulfate. After filtration,the filtrate was concentrated under reduced pressure, and the resulting residue was applied to a silica gel column chromatography. From the eluate with chloroform/methanol (50/1 to 30/1 to 20/1, v/v) and then with chloroform/methanol/triethylamine (20/1/1, v/v/v), a pale brown solid was obtained. This solid was stirred in methanol as slurry, and then the mixture was kept static at room temperature. The crystal formed was taken out through filtration, and dried under reduced pressure to obtain 2.03 g (54 %) of the entitled compound as a yellow crystal.
MS (FAB)m/z:383 (M+1)⁺.
¹H-NMR(CDCl₃)δ: 1.40-2.00 (overlapped with H₂O), 2.31(3H, s), 2.36(3H,s), 2.60-3.60(3H, brm) , 7.19-7.35(5H, m), 7.46-7.58(2H, m), 8.75(1H, dd, J=1.5, 4.8Hz).
IR(KBr): 3036, 2956, 2863, 2788, 2222, 1620, 1589, 1568, 1528, 1470, 1442, 1407 cm⁻¹.

| Elemental analysis: C₂₃H₂₂N₆·0.25H₂O | | | |
|---|---|---|---|
| Calcd. | C, 71.39%; | H, 5.86%; | N, 21.72% |
| Found | C, 71.33%; | H, 5.64%; | N, 21.51%. |

### [Example 159]

### 6-(3-Hydroxypyrrolidin-1-yl)-8-methyl-7-phenyldipyrido[1,2-a;2',3'-d]imidazole-9-carbonitrile (#159):

To N,N-dimethylformamide (30 ml) solution of 500 mg (1.57 mmol) of 6-chloro-8-methyl-7-phenyldipyrido[1,2-a;2',3'-d]imidazole-9-carbonitrile (I-204) and 164 mg (1.88 mmol) of 3-hydroxypyrrolidine was added 417 µl (3.14 mmol) of triethylamine, and the resulting mixture was stirred at 80 to 90°C for 5.5 hours. After it was cooled and concentrated, ethyl acetate and brine were added to the residue, and the mixture was stirred. The solid formed was taken out through filtration, and the solid was stirred in warmed methanol as slurry. The solid formed was taken out through filtration, and dried under reduced pressure to obtain 181 mg (31 %) of the entitled compound as a yellow solid. Further, the filtrates in the above were combined, concentrated under reduced pressure and filtered. The resulting filtrate was concentrated under reduced pressure, and the residuewas applied toa silica gel column chromatography. From the eluate with chloroform/methanol (100/1 to 98/1 to 20/1, v/v), 281 mg (39 %) of the entitled compound (second crystal) was obtained as a yellow solid.
MS(FAB)m/z:370(M+1)⁺.
¹H-NMR(DMSO-d₆)δ: 1.60-1.80(2H, brm), 2.26 (3H, s) , 2.45-2.80 (m, overlapped with DMSO peak), 4.14-4.23(1H, m), 5.18(1H, m), 7.32-7.58(6H, s), 8.58-8.70(2H, m).
IR(KBr): 2930, 2869, 2222, 1619, 1589, 1568, 1529, 1472, 1442, 1409 cm⁻¹.

| Elemental analysis: C₂₂H₁₉N₅O·HCl·0.5H₂O | | | | |
|---|---|---|---|---|
| Calcd. | C, 63.69%; | H, 5.10%; | N, 16.88%; | Cl, 8.54% |
| Found | C, 63.39%; | H, 4.76%; | N, 16.69%; | Cl, 9.46%. |

### [Example 160]

### 8-Methyl-7-phenyl-6-(piperazin-1-yl)dipyrido[1,2-a;2',3'-d]imidazole-9-carbonitrile (#160):

To N,N-dimethylformamide (35 ml) solution of 350 mg (1.10 mmol) of 6-chloro-8-methyl-7-phenyldipyrido[1,2-a;2',3'-d]imidazole-9-carbonitrile (I-204) and 473 mg (5.49 mol) of anhydrous piperazine was added 306 µl (2.20 mmol) of triethylamine, and the resulting mixture was stirred in an oil bath at 80 to 90°C for 50 minutes. After it was cooled and concentrated, the residue was dissolved in chloroform, and the organic layer was washed with aqueous saturated sodium bicarbonate solution, and dried over anhydrous magnesium sulfate. After filtration, the filtrate was concentrated under reduced pressure, and the resulting residue was applied to a silica gel column chromatography. The solid obtained from the eluate with from chloroform/methanol (50/1, v/v) to chloroform/methanol/triethylamine (100/2/1, v/v/v) was stirred with methanol as slurry. This was filtered and dried under reduced pressure to obtain 285 mg (70 %) of the entitled compound as a yellow solid.
MS (FAB) m/z : 369 (M+1)⁺.
¹H-NMR(CD₃OD/CDCl₃)δ: 2.25(3H, s), 2.55-2.63(2H, m), 2.85-2.90(2H, m), 3.10-3.20(4H, m), 7.26-7.36(3H, m), 7.55-7.65(3H, m), 8.72(1H, dd, J=1.5, 4.5Hz), 8.98(1H, dd, J=1.5, 8.1Hz).
IR(KBr): 2936, 2226, 1620, 1478, 1367, 780 cm⁻¹.

| Elemental analysis: C₂₂H₂₀N₆O·0.75H₂O | | | |
|---|---|---|---|
| Calcd. | C, 69.18%; | H, 5.67%; | N, 22.00% |
| Found | C, 69.44%; | H, 5.39%; | N, 21.95%. |

### [Example 161]

### 8-Methyl-7-phenyl-6-[(3S)-methylpiperazin-1-yl]dipyrido[1,2-a;2',3'-d]imidazole-9-carbonitrile (#161):

To N,N-dimethylformamide (35 ml) solution of 350 mg (1.10 mmol) of 6-chloro-8-methyl-7-phenyldipyrido[1,2-a;2',3'-d]imidazole-9-carbonitrile (I-204) and 132 mg (1.32 mol) of (2S)-methylpiperazine was added 306 µl (2.20 mmol) of triethylamine, and the resulting mixture was stirred in an oil bath at 80 to 90°C for 9.5 hours. Further, 132 mg (1.32 mmol) of (2S) -methylpiperazine was added thereto, and the mixture was stirred in an oil bath at 80 to 90°C for 2.5 hours. After it was cooled and concentrated, the residue was dissolved in chloroform, and the organic layer was washed with aqueous saturated sodium bicarbonate solution, and dried over anhydrous magnesium sulfate. After filtration, the filtrate was concentrated under reduced pressure, and the resulting residue was applied to a silica gel column chromatography. The solid obtained from the eluate with from chloroform/methanol (50/1 to 20/1, v/v) to chloroform/methanol/triethylamine (100/5/3, v/v/v) was stirred with methanol as slurry. This was filtered and dried under reduced pressure to obtain 255 mg (61 %) of the entitled compound as a yellow solid.
HRMS(FAB)m/z:383.1989(Calcd for C₂₃H₂₃N₆ 383.1984).
¹H-NMR(CD₃OD/CDCl₃)δ: 0.88 and 0.96(3H, d each, J=6.0Hz), 2.11(1H, t, J=10.2Hz ), 2.24(3H, s), 2.50(1H, dt, J=3.0, 11.7Hz), 2.90-3.00(1H, m), 3.10-3.28(4H, m), 7.26-7.40(3H, m), 7.55-7.63(3H, m), 8.72(1H, dd, J=1.5, 4.8Hz), 8.98(1H, dd, J=1.5, 8.1Hz).
IR(KBr): 2947, 2855, 2229, 1475, 1365, 775 cm⁻¹.

| Elemental analysis: C₂₃H₂₂N₆ | | | |
|---|---|---|---|
| Calcd. | C, 72.23%; | H, 5.80%; | N, 21.97% |
| Found | C, 71.95%; | H, 5.74%; | N, 21.61%. |

### [Example 162]

### 8-Methyl-6-[3-(N-methylamino)azetidin-1-yl]-7-phenyldipyrido[1,2-a;2',3'-d]imidazole-9-carbonitrile (#162):

To N,N-dimethylformamide (20 ml) solution of 600 mg (1.88 mmol) of 6-chloro-8-methyl-7-phenyldipyrido[1,2-a;2',3'-d]imidazole-9-carbonitrile (I-204) and 461 mg (2.07 mol) of 3-(N-tert-butoxycarbonyl-N-methylamino)azetidine was added 749 µl (5.64 mmol) of triethylamine, and the resulting mixture was stirred at 80 to 90°C for 6.5 hours. After it was cooled and concentrated, aqueous 10 % sodium carbonate solution was added to the residue and the mixture was stirred. The insoluble material was taken out through filtration, washed with water and dried under reduced pressure. The resulting solid was applied to a silica gel column chromatography. From the eluate with from chloroform/methanol (100/1 to 50/1, v/v), 543mg (62%) of 8-methyl-6-[3-(N-tert-butoxycarbonyl-N-methylamino)azetidin-1-yl]-7-phenyldipyrido[1,2-a;2',3'-d]imidazole-9-carbonitrile was obtained as a yellow solid.

Thus obtained, 510 mg (1.09 mmol) of 8-methyl-6-[3-(N-tert-butoxycarbonyl-N-methylamino)azetidin-1-yl]-7-phenyldipyrido[1,2-a;2',3'-d]imidazole-9-carbonitrile was added to 1.4-dioxane solution (75 ml) of 4 N hydrochloric acid with cooling with ice, and the mixture was stirred at the temperature for 2 hours. The reaction mixture was concentrated under reduced pressure, aqueous 10 % sodium carbonate solution was added to the residue and the mixture was stirred. The insoluble.material was taken out through filtration, and dried under reduced pressure, and the resulting solid was applied to a silica gel column chromatography. The solid obtained from the eluate with chloroform/methanol/triethylamine (100/1/0 to 100/1/0.5 to 100/2/1, v/v/v) was further applied to a silica gel column chromatography; and from the eluate with chloroform/methanol (50/1 to 30/1 to 20/1 to 10/1 to 5/1, v/v), 174 mg (58 %) of the entitled compound was obtained as a yellow solid.
HRMS(FAB)m/z:369.1820(Calcd for C₂₂H₂₁N₆ 369.1828).
¹H-NMR(CDCl₃)δ: 2.30(3H, s), 2.31(3H, s), 3.38-3.56(3H, m), 3.88-3.95(2H, m), 7.25-7.33(1H, m), 7.36-7.43(2H, m), 7.53-7.58(3H, m), 8.40(1H, dd, J=1.5, 8.4Hz), 8.69(1H, dd, J=1.8, 4.8Hz).
IR(KBr): 3581, 3329, 2942, 2882, 2359, 2220, 1616, 1588, 1563, 1530, 1484, 1461, 1405, 1371 cm⁻¹.

| Elemental analysis: C₂₂H₂₀N₆·0.5H₂O | | | |
|---|---|---|---|
| Calcd. | C, 70.01%; | H, 5.61%; | N, 22.27% |
| Found | C, 70.14%; | H, 5.52%; | N, 22.47%. |

### [Example 163]

### 8-Methyl-6-[3-(N,N-dimethylamino)azetidin-1-yl]-7-phenyldipyrido[1,2-a;2',3'-d]imidazole-9-carbonitrile (#163):

130 mg (0.353 mmol) of 8-methyl-6-[3-(N-methylamino)azetidin-1-yl]-7-phenyldipyrido[1,2-a;2',3'-d]imidazole-9-carbonitrile (#162), and 147 µl (1.76 mmol) of aqueous 36 % formaldehyde solution were dissolved in mixed solution (20 ml) of anhydrous tetrahydrofuran/dichloromethane (1/1, v/v), and 384 mg (1.76 mmol) of sodium triacetoxyborohydride was added thereto, and the resulting mixture was stirred at room temperature for 140 minutes. The reaction mixture was concentrated under reduced pressure, aqueous 10 % sodium carbonate solution was added to the residue, and the mixture was extracted from chloroform. The organic layer collected was washed with brine and dried over anhydrous magnesium sulfate. After filtration, the filtrate was concentrated under reduced pressure and the resulting residue was dried to obtain 116 mg (86 %) of the entitled compound as a yellow solid.
HRMS(FAB)m/z:383.1973(Calcd for C₂₃H₂₃N₆ 383.1984).
¹H-NMR(CDCl₃)δ: 2.03(6H, s), 2.32(3H, s), 2.92-3.03(1H, m), 3.45(2H, dd, J=6.3, 8.7Hz), 3.73(2H, dd, J=6.9, 8.7Hz), 7.23-7.28 (1H, m) , 7.34-7.39(2H, m), 7.49-7.60(3H,m), 8.36(1H, dd, J=1.5, 8.1Hz), 8.75(1H, dd, J=1.5, 4.8 Hz).
IR(KBr): 2971, 2944, 2820, 2770, 2219, 1618, 1589, 1564, 1529, 1485, 1460, 1406, 1373 cm⁻¹.

| Elemental analysis: C₂₃H₂₂N₆·0.25H₂O | | | |
|---|---|---|---|
| Calcd. | C, 71.39%; | H, 5.86%; | N, 21.72% |
| Found | C, 71.50%; | H, 5.79%; | N, 21.73%. |

### [Example 164]

### 9-[(3S)-Dimethylaminopyrrolidin-1-yl]-7-methyl-8-phenyldipyrido[1,2-a;3',2'-d]imidazole-6-carbonitrile (#164):

To N,N-dimethylformamide (15ml) solutionof250mg (0.748 mmol) of 9-chloro-7-methyl-8-phenyldipyrido[1,2-a;3',2'-d]imidazole-6-carbonitrile (I-205) and 119 µl (0.941 mmol) of (3S)-dimethylaminopyrrolidine was added 390 µl (2.94 mmol) of triethylamine, and the resulting mixture was stirred in an oil bath at 80 to 90°C for 6.5 hours. After cooling and concentration, aqueous 10 % sodium carbonate solution was added to the residue and the mixture was stirred. The insoluble material was taken out through filtration, washed with water and dried under reduced pressure. The resulting solid was applied to a silica gel column chromatography. From the eluate with chloroform/methanol/triethylamine (100/1/0.5 to 100/2/0.5, v/v/v) , 234 mg (75 %) of the entitled compound was obtained as a yellow solid.
HRMS(FAB)m/z:397.2154(Calcd for C₂₄H₂₅N₆ 397.2141).
¹H-NMR(CDCl₃)δ: 1.72-1.82(1H, m), 2.04-2.13(7H, m), 2.36(3H, s), 2.78-2.96(2H, m), 3.07-3.16(1H, m), 3.34-3.45(2H, m), 7.20-7.26(2H, m), 7.43-7.55(4H, m) , 8.26(1H, dd, J=1.5, 8.1Hz), 8.49(1H, dd, J=1.5, 4.8Hz).
IR(KBr): 3051, 2974, 2958, 2864, 2819, 2767, 2219, 1623, 1578, 1527, 1489, 1470, 1441, 1416, 1392 cm⁻¹.

| Elemental analysis: C₂₄H₂₄N₆ | | | |
|---|---|---|---|
| Calcd. | C, 72.70%; | H, 6.10%; | N, 21.10% |
| Found | C, 72.60%; | H, 6.03%; | N, 20.95%. |

### [Example 165]

### 7-Methyl-8-phenyl-9-(piperazin-1-yl)dipyrido[1,2-a;3',2'-d]imidazole-6-carbonitrile (#165):

To N,N-dimethylformamide (5 ml) solution of 80 mg (0.251 mmol) of 9-chloro-7-methyl-8-phenyldipyrido[1,2-a;3',2'-d]imidazole-6-carbonitrile (I-205) and 25.9 mg (0.301 mmol) of anhydrous piperazine was added 66.7 µl (0.502 mmol) of triethylamine, and the resulting mixture was stirred in an oil bath at 80 to 90°C for 5.5 hours. After cooling and concentration, aqueous 10 % sodium carbonate solution was added to the residue and the mixture was stirred. The insoluble material was taken out through filtration, washed with water and dried under reduced pressure. The resulting solid was applied to a silica gel column chromatography. From the eluate with from chloroform/methanol (50/1, v/v) to chloroform/methanol/triethylamine (30/1/0 to 30/1/1, v/v/v), 51 mg (55 %) of the entitled compound was obtained as a yellow solid.
HRMS (FAB)m/z:369.1835 (Calcd for C₂₂H₂₁N₆ 369.1828).
¹H-NMR(CDCl₃)δ: 2.35(3H, s), 2.40-2.90(4H, brm), 3.00-3.50 (4H, brm), 7.19-7.28(2H, m) , 7.44-7.56 (4H, m), 8.28(1H, dd, J=1.5, 8.1Hz), 8.51(1H, dd, J=1.5, 4.5Hz).
IR(KBr): 3058, 2930, 2849, 2806, 2222, 1620, 1577, 1526, 1486, 1441, 1394, 1370, 1303 cm⁻¹.

| Elemental analysis: C₂₂H₂₀N₆·0.25H₂O | | | |
|---|---|---|---|
| Calcd. | C, 70.85%; | H, 5.54%; | N, 22.53% |
| Found | C, 70.91%; | H, 5.40%; | N, 22.67%. |

### [Example 166]

### 9-[(3S)-Dimethylaminopyrroldin-1-yl]-4,7-dimethyl-8-phenyldipyrido[1,2-a;3',2'-d]imidazole-6-carbonitrile (#166):

To N,N-dimethylformamide (20 ml) solution of 1.00 g (3.00 mmol) of 9-chloro-9,7-dimethyl-8-phenyldipyrido[1,2-a;3',2'-d]imidazole-6-carbonitrile (I-209) and 838 µl (6.01 mmol) of (3S)-dimethylaminopyrrolidine was added 419 µl (3.31 mmol) of triethylamine, and the resulting mixture was stirred in an oil bath at 80 to 90°C for 2.5 hours. After cooling and concentration, the residue was dissolved in chloroform, and the organic layer was washed with aqueous saturated sodium bicarbonate solution and brine. This was dried over anhydrous magnesium sulfate and filtered, and the filtrate was concentrated under reduced pressure. The resulting residue was applied to a silica gel column chromatography. From the eluate with chloroform/methanol (100/1 to 20/1, v/v) , 1.15 g (93 %) of the entitled compound was obtained as a yellow solid.
MS(FAB)m/z:411(M+1)⁺.
¹H-NMR(CDCl₃)δ: 1.67-1.79(1H, m), 2.04-2.15(1H, m), 2.15(6H, s), 2.35(3H, s), 2.76-2.96(2H, m), 2.82(3H, s), 3.09(1H, dt, J=3.6, 8.7Hz), 3.34-3 46(2H, m), 7.22-7.29(3H, m), 7.92-7.54(3H, m), 8.35(1H, d, J=4.8Hz).
IR(KBr): 2940, 2855, 2770, 2219, 1618, 1593, 1507, 1357 cm⁻¹.

| Elemental analysis: C₂₅H₂₆N₆ | | | |
|---|---|---|---|
| Calcd. | C, 73.14%; | H, 6.38%; | N, 20.47% |
| Found | C, 73.01%; | H, 6.37%; | N, 20.35%. |

### [Example 167]

### 6-[(3S)-Dimethylaminopyrroldin-1-yl]-2,8-dimethyl-7-phenyldipyrido[1,2-a;2',3'-d]imidazole-9-carbonitrile (#167):

To N,N-dimethylformamide (50 ml) solution of 1.00 g (3. 00 mmol) of 6-chloro-2,8-dirnethyl-7-phenyldipyrido[1,2-a;2',3'-d]imidazole-9-carbonitrile (I-214) and 458 µl (3.61 mmol) of (3S) -dimethylaminopyrrolidine was added 838 µl (6.01 mmol) of triethylamine, and the resulting mixture was stirred in an oil bath at 80 to 90°C for 6.5 hours. After it was cooled and concentrated, aqueous 10 % sodium carbonate solution was added to the residue and the mixture was stirred, and the insoluble material was taken out through filtration, washed with water and dried under reduced pressure. The resulting solid was applied to a silica gel column chromatography. The solid obtained from the eluatewith chloroform/methanol (100/1 to 50/1, v/v) and with chloroform/methanol/triethylamine (100/2/1, v/v/v) was stirred in methanol as slurry, taken out through filtration and dried under reduced pressure to obtain 1.03 g (84 %) of the entitled compound as a yellow-green solid.
¹H-NMR(CDCl₃)δ: 1.60-3.60(7H, br), 2.13(6H, s), 2.28(3H, s), 2.74(3H, s), 7.12(1H, d, J=8.4Hz), 7.24-7.35(2H, m), 7.46-7.57(3H, m), 8.06-8.30(1H, brm).

| Elemental analysis: C₂₅H₂₆N₆·0.25H₂O | | | |
|---|---|---|---|
| Calcd. | C, 72.35%; | H, 6.44%; | N, 20.25% |
| Found | C, 72.28%; | H, 6.32%; | N, 20.06%. |

### [Reference Example 215]

### Methyl 2-benzoyloxyacetate (I-215):

To dichloromethane (100 ml) solution of 4.5 g (50.0 mmol) of methyl glycolate was added 7.7 ml (55.0 mmol) of triethylamine, the mixture was cooled with ice, 6.00 ml (52.0 mmol) of benzoyl chloride was added thereto, and the resuling mixture was stirred at room temperature for 3 hours. Aqueous saturated sodium bicarbonate solution was added to the reaction mixture, and the mixture was extracted with chloroform. The organic layer was washed with 1 N hydrochloric acid and brine, and dried over anhydrous magnesium sulfate. This was filtered and concentrated under reduced pressure to obtain 11.3 g (quantitative) of the entitled compound as a pale yellow oil.
¹H-NMR(CDCl₃)δ: 3.80(3H, s), 4.87(2H, s), 7.46(1H, m), 7.59(1H, m), 8.10(2H, m).

### [Reference Example 216]

### 2-Benzoyloxyacetic acid (I-216):

To methanol (125 ml) solution of 9.70 g (50.0 mmol) of methyl 2-benzoyloxyacetate (I-215) was added water (25 ml) solution of 2. 00 g (47.5 mmol) of lithium hydroxide monohydrate, and the resulting mixture was stirred overnight at room temperature. The reaction mixture was concentrated under reduced pressure, water was added to the residue to dissolve it, and the mixture was then washed twice with diethyl ether. The aqueous layer was made acidic with 6 N hydrochloric acid added thereto, and then extracted three times with diethyl ether. The organic layer was washed with brine and then dried over anhydrous magnesium sulfate. After filtrating and concentrating under reduced pressure, 11.3 g of the entitled compound was obtained as a white crystal.
MS(FAB)m/z:181(M+1)⁺.
¹H-NMR(CDCl₃)δ: 4.92(2H, s), 7.46(2H, m), 7.61(1H, m), 8.10(2H, m), 8.36(1H, brs).

### [Reference Example 217]

### Ethyl 4-benzoyloxy-3-oxo-2-phenylbutyrate (I-217):

To tetrahydrofuran (45 ml) solution of 2.34 g (13.0 mmol) of 2-benzoyloxyacetic acid (I-216) wereadded2.10ml (15.0 mmol) of triethylamine and 1.70 ml (14.0 mmol) of pivaloyl chloride at -15°C, and the resulting mixture was stirred at the temperature for 1 hour to prepare a mixed acid anhydride. Under nitrogen atmosphere, tetrahydrofuran (45 ml) solution of 2.30 g (14.0 mmol) of ethyl phenylacetate was cooled to -40°C, and 7.0 ml (14.0 mmol) of 2 M lithium diisopropylamide was added thereto and the mixture was stirred at the temperature for 2 hours. Then, the mixed acid anhydride solution prepared previously was added dropwise thereto, and the mixture was stirred overnight with gradually warming up to room temperature. Aqueous saturated ammonium chloride solution was added to the reaction mixture, and the mixture was extracted twice with ethyl acetate. The organic layer was washed with aqueous saturated sodium bicarbonate solution and brine, and dried over anhydrous magnesium sulfate. After filtrating and concentrating under reduced pressure, the resulting residue was applied to a silica gel column chromatography. From the eluate with n-hexane/ethyl acetate (10/1, v/v), 1.98 g (47 %) of the entitled compound was obtained as a yellow oil.
MS (FAB)m/z:327 (M+1)⁺.
¹H-NMR(CDCl₃)δ: 1.09-1.27(m), 3.93(m), 4.22(m), 4.77(s), 4.88(s), 5.00(s), 5.60(s), 7.24-7.62 (m), 7.95-8.07(m), 13.05(s).

### [Reference Example 218]

### 3-Benzoyloxymethyl-1-hydroxy-2-phenylpyrido[1,2-a]benzimidazole-4-carbonitrile (I-218):

A mixture of 500 mg (3.20 mmol) of (2-benzimidazolyl)acetonitrile, 980 mg (3.00 mmol) of ethyl 4-benzoyloxy-3-oxo-2-phenylbutyrate (I-217) and 500 mg (6.50 mmol) of ammonium acetate was stirred under heating in an oil bath at 140 to 150°C for 1 hour. The reaction mixture was cooled to room temperature, and water and acetonitrile were added thereto. The crystal precipitated was taken out through filtration and dried under reduced pressure to obtain 322 mg (26 %) of the entitled compound as a black-brown crystal.
MS(FAB)m/z:420(M+1)⁺.
¹H-NMR(DMSO-d₆)δ: 5.17(s), 7.32-7.69(m), 7.91(m), 8.56(d, J=8.1Hz), 13.82 (brs) .

### [Reference Example 219]

### 3-Benzoyloxymethyl-1-chloro-2-phenylpyrido[1,2-a]benzimidazole-4-carbonitrile (I-219):

Phosphoryl chloride (10 ml) suspension of 590 mg (1.40 mmol) of 3-benzoyloxymethyl-1-hydroxy-2-phenylpyrido[1,2-a]benzimidazole-4-carbonitrile (I-218) was stirred under reflux for 2.5 hours. After cooling, the reaction mixture was concentrated under reduced pressure, ice-water was carefully added to the residue, and the mixture was extracted with chloroform. The organic layer was washed with aqueous saturated sodium bicarbonate solution and brine, and dried over anhydrous magnesium sulfate. After it was filtered and concentrated under reduced pressure, the resulting residue was applied to a silica gel column chromatography. From the eluate with chloroform/methanol (50/1, v/v), 324 mg (51 %) of the entitled compound was obtained as a black-brown crystal.
¹H-NMR(CDCl₃)δ: 5.32(2H, s), 7.28-7.70(10H, m), 7.95(2H, d, J=8.4Hz), 8.13(1H, d, J=8.1Hz), 8.62(1H, d, J=8.7Hz).

### [Example 168]

### 3-Benzoyloxymethyl-1-[(3S)-dimethylaminopyrrolidin-1-yl]-2-phenylpyrido[1,2-a]benzimidazole-4-carbonitrile (#168):

To N,N-dimethylformamide (10 ml) solution of 300 mg (0.69 mmol) of 3-benzoyloxymethyl-1-chloro-2-phenylpyrido[1,2-a]benzimidazole-4-carbonitrile (I-219) and 0.10 ml (0.76 mmol) of (3S) -dimethylaminopyrrolidine was added 0.14 ml (1.00 mmol) of triethylamine, and the resulting mixture was stirred in an oil bath at 70 to 80°C for 2 hours. After it was cooled and concentrated, the residue was dissolved in chloroform, washed with aqueous saturated sodium bicarbonate solution and brine, and dried over anhydrous magnesium sulfate. After it was filtered and concentrated under reduced pressure, the resulting residue was applied to a silica gel column chromatography. From the eluate with chloroform/methanol (50/1, v/v), 240 mg (68 %) of the entitled compound was obtained as a yellow-brown crystal. MS(FAH)m/z:516(M+1)⁺.
¹H-NMR(CDCl₃)δ: 1.75(2H, brs), 2.11(8H, s), 2.90-3.36(3H, br), 5.25(2H, s), 7.33-7.62(10H, m), 7. 96 (2H, d, J=8.4Hz), 8.07(2H, d, J=8.1Hz), 8.09(1H, brs).

| Elemental analysis: C₃₂H₂₉N₅O₂·0.25H₂O | | | |
|---|---|---|---|
| Calcd. | C, 73.90%; | H, 5.72%; | N, 13.46% |
| Found | C, 74.02%; | H, 5.61%; | N, 13.46%. |

### [Example 169]

### 3-Hydroxymethyl-1-[(3S)-dimethylaminopyrrolidin-1-yl]-2-phenylpyrido[1,2-a]benzimidazole-4-carbonitrile (#169):

To methanol (15 ml) solution of 200 mg (0.39 mmol) of 3-benzoyloxymethyl-1-[(3S)-dimethylaminopyrrolidin-1-yl]-2-phenylpyrido[1,2-a]benzimidazole-4-carbonitrile (#168) was added 0.29ml (1.17 mmol) of aqueous 4 N sodiumhydroxide solution, and the resulting mixture was stirred at room temperature for 3 hours. The reaction mixture was controlled to have pH of 7 with 1 N hydrochloric acid added thereto, and then concentrated under reduced pressure. The resulting residue was applied to an ODS silica gel column chromatography with 0.1 % acetic acid to 10 % methanol/0.1 % acetic acid, and then concentrated under reduced pressure. This was further applied to a silica gel column chromatography, and from the eluate with chloroform/methanol (10/1, v/v), 37 mg (23 %) of the entitled compound was obtained as a yellow crystal.
MS(FAB)m/z:412(M+1)⁺.
¹H-NMR(CDCl₃)δ: 2.05-3.69(7H, br), 2.17(6H, s), 4.87-5.08(2H, m), 7.32-7.58(8H, m), 8.01(1H, d, J=8.4Hz), 8.02(1H, brs).

### [Test Example 1]

The antifungal activity of the compound of the invention was determined according to the US NCCLS Standard Method (M27-A, 1997) against yeasts (*Candida, Cryptococcus*); and according to the US NCCLS Standard Method (M38-P, 1998) against molds (*Aspergillus*). The result is expressed in values of MIC (minimal inhibitory concentration for growth, µg/ml) in Table 7.

**Table 7**

| Example | *Saccharomyces cerevisiae* | *Candida glabrata* | *Candida krusei* |
|---|---|---|---|
| 1 | 16 | 2 | 8 |
| 6 | <0.063 | <0.063 | 0.5 |
| 11 | 2 | 16 | 32 |
| 12 | >32 | 128 | >128 |
| 60 | 64 | 16 | >128 |
| 66 | 16 | 32 | 16 |
| 82 | 32 | 8 | >128 |
| 87 | 2 | 1 | 8 |
| 95 | 16 | 32 | 64 |

### INDUSTRIAL APPLICABILITY

The invention provides a compound capable of specifically or selectively expressing an antifungal activity in a broad spectrum based on the novel mechanism thereof of 1,6-β-glucan synthesis inhibition, and provides an antifungal agent containing any of such compound, its salts or solvates thereof.

## Claims

1. A compound represented by the following formula (I), its salt or solvates thereof: 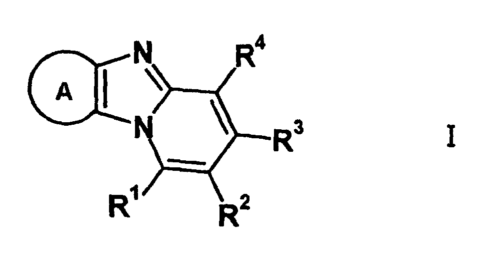 [wherein the moiety A means a benzene ring or a heteroaryl ring (the heteroaryl ring is a 5-membered ring or 6-membered ring and contains from 1 to 3 hetero atoms of one or more types selected from the group consisting of a nitrogen atom, an oxygen atom and a sulfur atom), and the benzene ring and heteroaryl ring each may have one or more substituents of one or more types selected from the group consisting of an amino group, a hydroxyl group, a mercapto group, a nitro group, a cyano group, a formyl group, a carboxyl group, a sulfo group, a halogen atom, a heteroaryl group (the heteroary group is a 5-membered or 6-membered ring containing from 1 to 3 hetero atoms of one or more types selected from the group consisting of a nitrogen atom, an oxygen atom and a sulfur atom), a group of the following formula: 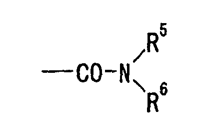 (wherein R⁵ and R⁶ each independently represents a hydrogen atom, an alkyl group having from 1 to 6 carbon atoms or an aryl group having from 6 to 10 carbon atoms),
an alkyl group having from 1 to 6 carbon atoms, an alkenyl group having from 2 to 6 carbon atoms, an alkynyl group having from 2 to 6 carbon atoms, an alkoxy group having from 1 to 6 carbon atoms, a hydroxyalkyl group having from 1 to 6 carbon atoms, an alkylamino group having from 1 to 6 carbon atoms, an alkylthio group having from 1 to 6 carbon atoms, an acyl group having from 2 to 5 carbon atoms, an alkoxycarbonyl group having from 2 to 5 carbon atoms, a cycloalkyl group having from 3 to 6 carbon atoms, an aryl group having from 6 to 10 carbon atoms and an aralkyl group having from 7 to 16 carbon atoms, and the substituents containing a carbon chain of them may bond to each other to form a cyclic structure and fuse to the benzene ring or the heteroaryl ring;
R¹ represents a hydrogen atom, a halogen atom, an amino group, a hydroxyl group, a mercapto group, a nitro group, a cyano group, a formyl group, a carboxyl group, a group of the following formula: 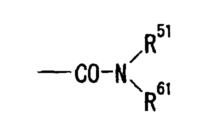 (wherein R⁵¹ and R⁶¹ each independently represents a hydrogen atom, an alkyl group having from 1 to 6 carbon atoms or an aryl group having from 6 to 10 carbon atoms),
an alkyl group having from 1 to 10 carbon atoms, an alkenyl group having from 2 to 10 carbon atoms, an alkynyl group having from 2 to 10 carbon atoms, an alkylamino group having from 1 to 10 carbon atoms, an alkoxy group having from 1 to 10 carbon atoms, an alkylthio group having from 1 to 10 carbon atoms, an acyl group having from 2 to 6 carbon atoms, an alkoxycarbonyl group having from 2 to 7 carbon atoms, a cycloalkyl group having from 3 to 10 carbon atoms, a cycloalkylamino group having from 3 to 10 carbon atoms, a cycloalkyloxy group having from 3 to 10 carbon atoms, a cycloalkylthio group having from 3 to 10 carbon atoms, a cycloalkenyl group having from 4 to 10 carbon atoms, a cycloalkenylamino group having from 4 to 10 carbon atoms, a cycloalkenyloxy group having from 4 to 10 carbon atoms, a cycloalkenylthio group having from 4 to 10 carbon atoms, an aryl group having from 6 to 10 carbon atoms, an arylamino group having from 6 to 10 carbon atoms, an aryloxy group having from 6 to 10 carbon atoms, an arylthio group having from 6 to 10 carbon atoms, a monocyclic-, bicyclic- or spirocyclic-heterocyclic group having from 3 to 10 carbon atoms (containing from 1 to 4 hetero atoms of one or more types selected from the group consisting of a nitrogen atom, an oxygen atom and a sulfur atom), a heteroarylamino group having from 5 to 10 carbon atoms, a heteroaryloxy group having from 5 to 10 carbon atoms, a heteroarylthio group having from 5 to 10 carbon atoms, in which the amino group, the hydroxyl group and the mercapto group may be protected with a protective group;
when R¹ is an amino group, an alkyl group, an alkenyl group, an alkynyl group, an alkylamino group, an alkoxy group, an alkylthio group, an acyl group, an alkoxycarbonyl group, a cycloalkyl group, a cycloalkyl amino group, a cycloalkyloxy group, a cycloalkylthio group, a cycloalkenyl group, a cycloalkenylamino group, a cycloalkenyloxy group, a cycloalkenylthio group, an aryl group, an arylamino group, an aryloxy group, an arylthio group, a heteroarylamino group, a heteroaryloxy group or a heteroarylthio group, they may have one or more substituents of one or more types selected from the group consisting of an amino group, an aminoalkyl group having from 1 to 6 carbon atoms (the amino group and the amino group moiety of the aminoalkyl group having from 1 to 6 carbon atoms may be protected with a protective group, and may have one or two alkyl groups each having from 1 to 6 carbon atoms, and when they have two alkyl groups, the groups may be the same or different), a hydroxyl group, a mercapto group, a halogen atom, an alkoxy group having from 1 to 6 carbon atoms, an alkylthio group having from 1 to 6 carbon atoms, an acyl group having from 2 to 7 carbon atoms, an alkoxycarbonyl group having from 2 to 7 carbon atoms, an aryl group having from 6 to 10 carbon atoms, a monocyclic- or bicyclic-heterocyclic group having from 3 to 10 carbon atoms (containing from 1 to 4 hetero atoms of one or more types selected from the group consisting of a nitrogen atom, an oxygen atom and a sulfur atom), an alkylcarbonylamino group having from 2 to 7 carbon atoms and a cycloalkyl group having from 3 to 10 carbon atoms;
when R¹ is a heterocyclic group, it may have one or more substituents of one or more types selected from the group consisting of a halogen atom, an amino group, a hydroxyl group, an oxo group, a group of the following formula: 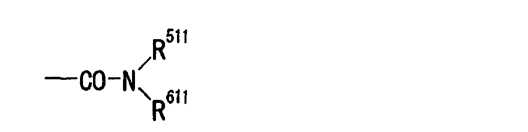 (wherein R⁵¹¹ and R⁶¹¹ each independently represents a hydrogen atom, an alkyl group having from 1 to 6 carbon atoms or an aryl group having from 6 to 10 carbon atoms),
an alkyl group having from 1 to 6 carbon atoms, an alkylamino group having from 1 to 6 carbon atoms, a cycloalkylamino group having from 3 to 6 carbon atoms, an aryl group having from 6 to 10 carbon atoms, a monocyclic- or bicyclic-heterocyclic group having from 3 to 10 carbon atoms (containing from 1 to 4 hetero atoms of one or more types selected from the group consisting of a nitrogen atom, an oxygen atom and a sulfur atom), an alkoxy group having from 1 to 6 carbon atoms, an alkylthio group having from 1 to 6 carbon atoms, a halogenoalkyl group having from 1 to 6 carbon atoms and an aminoalkyl group having from 1 to 6 carbon atoms; and the alkyl group and the alkyl moiety of the alkylamino group, the cycloalkylamino group, the alkoxy group, the alkylthio group, the halogenoalkyl group and the aminoalkyl group may have one or more substituents of one or more types selected from the group consisting of a halogen atom, a hydroxyl group, a carboxyl group, an alkyl group having from 1 to 6 carbon atoms, an alkoxy group having from 1 to 6 carbon atoms, an alkoxycarbonyl group having from 2 to 7 carbon atoms, an alkoxycarbonylamino group having from 2 to 7 carbon atoms, an aryl group having from 6 to 10 carbon atoms, and a monocyclic- or bicyclic-heterocyclic group having from 3 to 10 carbon atoms (containing from 1 to 4 hetero atoms of one or more types selected from the group consisting of a nitrogen atom, an oxygen atom and a sulfur atom); the amino group and the amino group moiety of the aminoalkyl group and the alkylamino group may be protected withaprotectivegroup, andmayhave, as thesubstituentthereof, one or two alkyl groups each having from 1 to 6 carbon atoms (optionally having one or more substituents of one or more types selected from the group consisting of a hydroxyl group, a halogen atom, an alkoxy group having from 1 to 6 carbon atoms and an alkyl thio group) , and an amino acid, a dipeptide or a polypeptide comprising from 3 to 5 amino acids may be bonded thereto; R² represents a hydrogen atom, a halogen atom, an amino group, a hydroxyl group, a nitro group, a cyano group, a carboxyl group, a group of the following formula: 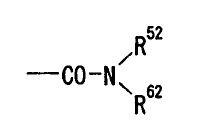 (wherein R⁵² and R⁶² each independently represents a hydrogen atom, an alkyl group having from 1 to 6 carbon atoms or an aryl group having from 6 to 10 carbon atoms),
an alkyl group having from 1 to 20 carbon atoms, an alkenyl group having from 2 to 20 carbon atoms, an alkynyl group having from 2 to 20 carbon atoms, an alkylamino group having from 1 to 20 carbon atoms, an alkoxy group having from 1 to 20 carbon atoms, an acyl group having from 2 to 18 carbon atoms, an alkoxycarbonyl group having from 2 to 18 carbon atoms, a cycloalkyl group having from 3 to 10 carbon atoms, a cycloalkenyl group having from 5 to 10 carbon atoms, a cycloalkylamino group having from 3 to 10 carbon atoms, a cycloalkylalkyl group having from 4 to 16 carbon atoms, a cycloalkyloxy group having from 3 to 10 carbon atoms, an aryl group having from 6 to 10 carbon atoms, an arylamino group having from 6 to 10 carbon atoms, an aralkyl group having from 7 to 16 carbon atoms, an aryloxy group having from 6 to 10 carbon atoms, a monocyclic- or bicyclic-heterocyclic group having from 5 to 10 carbon atoms (containing from 1 to 4 hetero atoms of one or more types selected from the group consisting of a nitrogen atom, an oxygen and a sulfur atom), a heteroarylamino group having from 5 to 10 carbon atoms, a heteroarylalkyl group having from 6 to 16 carbon atoms or a heteroaryloxy group having from 5 to 10 carbon atoms, and the amino group and the hydroxyl group may be protected with a protective group;
when R² is an alkyl group, an alkenyl group, an alkynyl group, an alkylamino group, an alkoxy group, an acyl group, an alkoxycarbonyl group, a cycloalkyl group, a cycloalkylamino group or a cycloalkyloxy group, these groups may have one or more substituents of one or more types selected from the group consisting of a halogen atom, an amino group, an imino group, a nitro group, a hydroxyl group, a mercapto group, a carboxyl group, a cyano group, a sulfo group, a dialkylphosphoryl group, a group of the following formula: 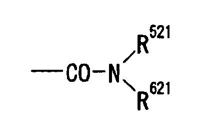 (wherein R⁵²¹ and R⁶²¹ each independently represents a hydrogen atom, an alkyl group having from 1 to 6 carbon atoms or an aryl group having from 6 to 10 carbon atoms),
an alkoxy group having from 1 to 10 carbon atoms, an alkylthio group having from 1 to 10 carbon atoms, an acyl group having from 2 to 8 carbon atoms, an alkoxycarbonyl group having from 2 to 8 carbon atoms, a cycloalkyl group having from 3 to 10 carbon atoms, an aryl group having from 6 to 10 carbon atoms and an arylthio group having from 6 to 10 carbon atoms; the amino group may have one or two substituents selected from the group consisting of a formyl group, an alkyl group having from 1 to 10 carbon atoms, an aminoalkyl group having from 1 to 8 carbon atoms, a hydroxyalkyl group having from 1 to 8 carbon atoms, a mercaptoalkyl group having from 1 to 8 carbon atoms, an acyl group having from 2 to 8 carbon atoms, an alkoxycarbonyl group having from 2 to 8 carbon atoms, a cycloalkyl group having from 3 to 10 carbon atoms, an aryl group having from 6 to 10 carbon atoms, an aralkyl group having from 7 to 16 carbon atoms, a heteroaryl group (the heteroaryl group is a 5-membered or 6-membered ring and contains from 1 to 4 hetero atoms of one or more types selected from the group consisting of a nitrogen atom, an oxygen atom and a sulfur atom), an alkylsulfonyl group having from 1 to 10 carbon atoms and an arylsulfonyl group having from 6 to 10 carbon atoms; when the amino group has two substituents, they may bond to each other to form a cycl i c structure; the hydroxyl group and the mercapto group may have a substituent selected from the group consisting of an alkyl group having from 1 to 10 carbon atoms, an aminoalkyl group having from 1 to 8 carbon atoms, a hydroxyalkyl group having from 1 to 8 carbon atoms, a mercaptoalkyl group having from 1 to 8 carbon atoms, an acyl group having from 2 to 8 carbon atoms, a cycloalkyl group having from 3 to 10 carbon atoms, an aryl group having from 6 to 10 carbon atoms, an aralkyl group having from 7 to 16 carbon atoms, and a heteroaryl group (the heteroaryl group is a 5-membered or 6-membered ring and contains from 1 to 4 hetero atoms of one or more types selected from the group consisting of a nitrogen atom, an oxygen atom and a sulfur atom); the cycloalkyl group may have one or more substituents of one or more types selected from the group consisting of a halogen atom, an amino group, an imino group, a nitro group, ahydroxyl group, a mercapto group, a carboxyl group, a cyano group, a sulfo group, a group of the following formula: 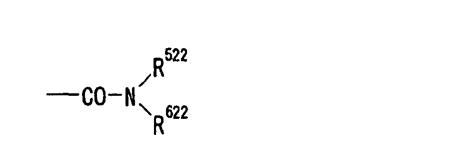 (wherein R⁵²² and R⁶²² each independently represents a hydrogen atom, an alkyl group having from 1 to 6 carbon atoms or an aryl group having from 6 to 10 carbon atoms),
an alkoxy group having from 1 to 10 carbon atoms, an alkylthio group having from 1 to 10 carbon atoms, an acyl group having from 2 to 8 carbon atoms and an alkoxycarbonyl group having from 1 to 8 carbon atoms; the amino group may have one or two substituents selected from the group consi sting of a formyl group, an alkyl group having from 1 to 10 carbon atoms, an aminoalkyl group having from 1 to 8 carbon atoms, a hydroxyalkyl group having from 1 to 8 carbon atoms, a mercaptoalkyl group having from 1 to 8 carbon atoms, an acyl group having from 2 to 8 carbon atoms, an alkoxycarbonyl group having from 2 to 8 carbon atoms, a cycloalkyl group having from 3 to 10 carbon atoms, an aryl group having from 6 to 10 carbon atoms, an aralkyl group having from 7 to 16 carbon atoms, a heteroaryl group (the heteroaryl group is a 5-membered or 6-membered ring and contains from 1 to 4 hetero atoms of one or more types selected from the group consisting of a nitrogen atom, an oxygen atom and a sulfur atom), an alkylsulfonyl group having from 1 to 10 carbon atoms and an arylsulfonyl group having from 6 to 10 carbon atoms; when the amino group has two substituents, they may bond to each other to form a cyclic structure;
when R² is an aryl group, an arylamino group, an aralkyl group, an aryloxy group, a heteroaryl group, a heteroarylamino group, a heteroarylalkyl group or a heteroaryloxy group, they may have one or more substituents of one or more types selected from the group consisting of a halogen atom, an amino group, an imino group, a nitro group, a hydroxyl group, a mercapto group, a carboxyl group, a cyano group, a sulfo group, a group of the following formula: 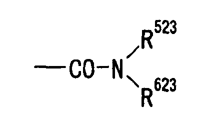 (wherein R⁵²³ and R⁶²³ each independently represents a hydrogen atom, an alkyl group having from 1 to 6 carbon atoms or an aryl group having from 6 to 10 carbon atoms),
an alkoxy group having from 1 to 10 carbon atoms, an alkylthio group having from 1 to 10 carbon atoms, an acyl group having from 2 to 8 carbon atoms, an alkoxycarbonyl group having from 2 to 8 carbon atoms, an aralkyloxy group having from 7 to 16 carbon atoms, an aralkyloxycarbonyl group having from 8 to 17 carbon atoms, an aryl group, and a monocyclic- or bicyclic-heterocyclic group having from 5 to 10 carbon atoms (containing from 1 to 4 hetero atoms of one or more types selected from the group consisting of a nitrogen atom, an oxygen atom andasulfuratom); the amino group may have one or two substi tuents selected from the group consisting of a formyl group, an alkyl group having from 1 to 10 carbon atoms, an aminoalkyl group having from 1 to 8 carbon atoms, a hydroxyalkyl group having from 1 to 8 carbon atoms, a mercaptoalkyl group having from 1 to 8 carbon atoms, an acyl group having from 2 to 8 carbon atoms, an alkoxycarbonyl group having from 2 to 8 carbon atoms, a cycloalkyl group having from 3 to 10 carbon atoms, an aryl group having from 6 to 10 carbon atoms, an aralkyl group having from 7 to 16 carbon atoms, a heteroaryl group (the heteroaryl group is a 5-membered or 6-membered ring and contains from 1 to 4 hetero atoms of one or more types selected from the group consisting of a nitrogen atom, an oxygen atom and a sulfur atom), an alkylsulfonyl group having from 1 to 10 carbon atoms and an arylsulfonyl group having from 6 to 10 carbon atoms; and when the amino group has two substituents, they may bond to each other to form a cyclic structure;
when R² is a heterocyclic group, i tmay have one or two substituents selected from the group consisting of a halogen atom, an amino group, a hydroxyl group, mercapto group, a carboxyl group, a group of the following formula: 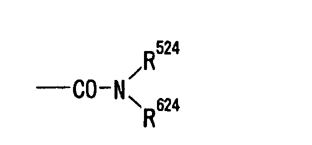 (wherein R⁵²⁴ and R⁶²⁴ each independently represents a hydrogen atom, an alkyl group having from 1 to 6 carbon atoms or an aryl group having from 6 to 10 carbon atoms),
an alkyl group having from 1 to 10 carbon atoms, an alkenyl group having from 2 to 10 carbon atoms, an alkynyl group having from 2 to 10 carbon atoms, an alkoxy group having from 1 to 10 carbon atoms, an alkylthio group having from 1 to 10 carbon atoms, a halogenoalkyl group having from 1 to 10 carbon atoms, an acyl group having from 2 to 10 carbon atoms, an alkoxycarbonyl group having from 2 to 10 carbon atoms and an aryl group having from 6 to 10 carbon atoms; the amino group may have one or two substituents selected from the group consisting of a formyl group, an alkyl group having from 1 to 10 carbon atoms, an aminoalkyl group having from 1 to 8 carbon atoms, a hydroxyalkyl group having from 1 to 8 carbon atoms, a mercaptoalkyl group having from 1 to 8 carbon atoms, an acyl group having from 2 to 8 carbon atoms, an alkoxycarbonyl group having from 2 to 8 carbon atoms, a cycloalkyl group having from 3 to 10 carbon atoms, an aryl group having from 6 to 10 carbon atoms, an aralkyl group having from 7 to 16 carbon atoms, a monocyclic- or bicyclic-heterocyclic group having from 5 to 10 carbon atoms (containing from 1 to 4 hetero atoms of one or more types selected from the group consisting of a nitrogen atom, an oxygen atom and a sulfur atom), a heteroaryl group (the heteroaryl group is a 5-membered or 6-membered ring and contains from 1 to 4 hetero atoms of one or more types selected from the group consisting of a nitrogen atom, an oxygen atom and a sulfur atom), an alkylsulfonyl group having from 1 to 10 carbon atoms and an arylsulfonyl group having from 6 to 10 carbon atoms; and when the amino group has two substituents, they may bond to each other to form a cyclic structure;
R¹ and R² may cooperate to form a 5-membered or 6-membered heterocyclic group (containing from 1 to 3 hetero atoms of one or more types selected from the group consisting of a nitrogen atom, an oxygen atom and a sulfur atom);
R³ represents a hydrogen atom, a halogen atom, an amino group, a hydroxyl group, a mercapto group, a nitro group, a cyano group, a formyl group, a carboxyl group, a groupof the following formula: 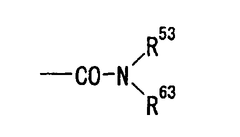 (wherein R⁵³ and R⁶³ each independently represents a hydrogen atom, an alkyl group having from 1 to 6 carbon atoms or an aryl group having from 6 to 10 carbon atoms),
an alkyl group having from 1 to 6 carbon atoms, an alkenyl group having from 2 to 6 carbon atoms, an alkynyl group having from 2 to 6 carbon atoms, an alkoxy group having from 1 to 6 carbon atoms, an alkylthio group having from 1 to 6 carbon atoms, an acyl group having from 2 to 5 carbon atoms, an alkoxycarbonyl group having from 2 to 5 carbon atoms, a cycloalkyl group having from 3 to 7 carbon atoms, a cycloalkenyl group having from 4 to 7 carbon atoms, an aryl group having from 6 to 10 carbon atoms, an aralkyl group having from 7 to 16 carbon atoms and a heteroaryl group (the heteroaryl group is a 5-membered or 6-membered ring and contains from 1 to 4 hetero atoms of one or more types selected from the group consisting of a nitrogen atom, an oxygen atom and a sulfur atom); and the amino group, the hydroxyl group and the mercapto group may be protected with a protective group; when R³ is an alkyl group, an alkenyl group, an alkynyl group, an alkoxy group, an alkylthio group, an acyl group, an alkoxycarbonyl group, a cycloalkyl group, a cycloalkenyl group, an aryl group, an aralkyl group or a heteroaryl group, they may have one or more substituents of one or more types selected from the group consisting of an amino group, a hydroxyl group, a mercapto group, a halogen atom, an alkoxy group having from 1 to 6 carbon atoms, an alkylthio group having from 1 to 6 carbon atoms, an acyl group having from 2 to 5 carbon atoms and an alkoxycarbonyl group having from 2 to 5 carbon atoms; the amino group may have one or two substituents selected from the group consisting of a formyl group, an alkyl group having from 1 to 6 carbon atoms, a cycloalkyl group having from 3 to 6 carbon atoms, an aryl group having from 6 to 10 carbon atoms, a heteroaryl group (the heteroaryl group is a 5-membered or 6-membered ring and contains from 1 to 3 hetero atoms of one or more types selected from the group consisting of a nitrogen atom, an oxygen atom and a sulfur atom) , an acyl group having from 2 to 5 carbon atoms and an alkoxycarbonyl group having from 2 to 5 carbon atoms; and when the amino group has two substituents, they may bond to each other to form a cyclic structure;
R⁴ represents a hydrogen atom, a halogen atom, an amino group, a hydroxyl group, a nitro group, a cyano group, a carboxyl group, a sulfo group, a carbamoyl group, a group of the following formula: 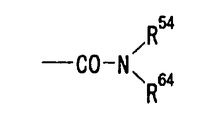 (wherein R⁵⁴ and R⁶⁴ each independently represents a hydrogen atom, an alkyl group having from 1 to 6 carbon atoms or an aryl group having from 6 to 10 carbon atoms),
an alkyl group having from 1 to 6 carbon atoms, an alkenyl group having from 2 to 6 carbon atoms, an alkynyl group having from 2 to 6 carbon atoms, an alkoxy group having from 1 to 6 carbon atoms, an acyl group having from 2 to 5 carbon atoms, an alkoxycarbonyl group having from 2 to 5 carbon atoms, an alkylcarbonyloxy group having from 1 to 6 carbon atoms, an alkyloxysulfonyl group having from 1 to 6 carbon atoms, a cycloalkyl group having from 3 to 6 carbon toms, an aryl group having from 6 to 10 carbon atoms or a heteroaryl group (the heteroaryl group is a 5-membered or 6-membered ring and contains from 1 to 3 hetero atoms of one or more types selected from the group consisting of a nitrogen atom, an oxygen atom and a sulfur atom) ; and the amino group and the hydroxyl group may be protected with a protective group;
when R⁴ is an alkyl group, an alkenyl group, an alkynyl group, an alkoxy group, an acyl group, an alkoxycarbonyl group, a cycloalkyl group, an aryl group or a heterocyclic group, they may have one or more substituents of one or more types selected from the group consisting of an amino group, a hydroxyl group, a mercapto group, a halogen atom, an alkoxy group having from 1 to 6 carbon atoms, an alkylthio group having from 1 to 6 carbon atoms, an acyl group having from 2 to 5 carbon atoms and an alkoxycarbonyl group having from 2 to 5 carbon atoms; the amino group may have one or two substituents selected from the group consisting of a formyl group, an alkyl group having from 1 to 6 carbon atoms, a cycloalkyl group having from 1 to 6 carbon atoms, an aryl group having from 6 to 10 carbon atoms , a heteroaryl group (the heteroaryl group is a 5-membered or 6-membered ring and contains from 1 to 3 hetero atoms of one or more types selected from the group consisting of a nitrogen atom, an oxygen atom and a sulfur atom), an acyl group having from 2 to 5 carbon atoms, an alkoxycarbonyl group having from 2 to 5 carbon atoms, an alkylsulfonyl group having from 1 to 6 carbon atoms and an arylsulfonyl group having from 6 to 10 carbon atoms; and when the amino group has two substituents, they may bond to each other to form a cyclic structure].

2. The compound, its salt or solvates thereof as claimed in claim 1, wherein A is a benzene ring having one or more substituents of one or more types selected from the group consisting of a carboxyl group, a halogen atom, an alkyl group having from 1 to 6 carbon atoms, an alkoxy group having from 1 to 6 carbon atoms, a hydroxyalkyl group having from 1 to 6 carbon atoms and an alkoxycarbonyl group having from 2 to 5 carbon atoms.

3. The compound, its salt or solvates thereof as claimed in claim 1, wherein A is a pyridine ring having one or more substituents of one or more types selected from the group consisting of a carboxyl group, a halogen atom, an alkyl group having from 1 to 6 carbon atoms, an alkoxy group having from 1 to 6 carbon atoms, a hydroxyalkyl group having from 1 to 6 carbon atoms and an alkoxycarbonyl group having from 2 to 5 carbon atoms.

4. The compound, its salt or solvates thereof as claimed in any one of Claims 1 to 3, wherein R¹ is a halogen atom, a substituted or unsubstituted amino group, a hydroxyl group, a substituted or unsubstituted alkylamino group having from 1 to 10 carbon atoms, a substituted or unsubstituted alkylthio group having from 1 to 10 carbon atoms, a substituted or unsubstituted cycloalkyl group having from 3 to 10 carbon atoms, a substituted or unsubstituted cycloalkenyl group having from 4 to 10 carbon atoms, or a substi tuted or unsubstituted monocyclic-, bicyclic- or spirocyclic-heterocyclic group having from 3 to 10 carbon atoms (containing from 1 to 4 hetero atoms of one or more types selected from the group consisting of a nitrogen atom, an oxygen atom and a sulfur atom).

5. The compound, its salt or solvates thereof as claimed in any one of claims 1 to 4, wherein R² is a hydrogen atom, a halogen atom, a group of the following formula: 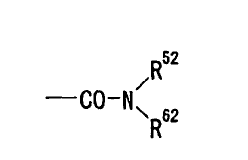 (wherein R⁵² and R⁶² each independently represents a hydrogen atom, an alkyl group having from 1 to 6 carbon atoms or an aryl group having from 6 to 10 carbon atoms),
a substituted or unsubstituted alkyl group having from 1 to 20 carbonatoms, a substituted or unsubstituted alkenyl group having from 2 to 20 carbon atoms, a substituted or unsubstituted cycloalkyl group having from 3 to 10 carbon atoms, a substituted or unsubstituted cycloalkenyl group having from 5 to 10 carbon atoms, a substituted or unsubstituted aryl group having from 6 to 10 carbon atoms, a substituted or unsubstituted aralkyl group having from 7 to 16 carbon atoms, or a substituted or unsubstituted monocyclic- or bicyclic-heterocyclic group having from 5 to 10 carbon atoms (containing from 1 to 4 hetero atoms of one or more types selected from the group consisting of a nitrogen atom, an oxygen atom and a sulfur atom).

6. The compound, its salt or solvates thereof as claimed in any one of claims 1 to 5, wherein R³ is a hydrogen atom, a halogen atom, a substituted or unsubstituted alkyl group having from 1 to 6 carbon atoms or a substituted or unsubstituted cycloalkyl group having from 3 to 7 carbon atoms.

7. The compound, its salt or solvates thereof as claimed in any one of claims 1 to 6, wherein R⁴ is a cyano group, a carboxyl group, a carbamoyl group, a substituted or unsubstituted alkyl group having from 1 to 6 carbon atoms or a substituted or unsubstituted alkoxycarbonyl group having from 2 to 5 carbon atoms.

8. Apharmaceutical composition containing the compound, its salt or solvates thereof as described in any one claims 1 to 7.

9. An agent for treating an infectous disease, containing the compound, its salt or solvates thereof as described in any one of claims 1 to 7.

10. An antifungal agent containing the compound, its salt or solvates thereof as described in any one of claims 1 to 7.
